# EUROPEAN PATENT APPLICATION

(11) **EP 3 034 610 A1**
(43) Date of publication of application: **22.06.2016**
(21) Application number: 14836017.5
(22) Date of filing: 27.05.2014
(51) Int. Cl.: C12P 5/00, C12N 1/21, C12N 9/00, C12N 15/09, C12P 7/02

(54) **NOVEL TERPENOID COMPOUND AND METHOD FOR PRODUCING SAME**

(30) Priority: 13.08.2013 JP 2013168230
(71) Applicant: The Kitasato Institute, Tokyo 108-8641 (JP); Nagase & Co., Ltd., Osaka 550-8668 (JP)
(72) Inventor: IKEDA, Haruo, Sagamihara-shi Kanagawa 252-0373 (JP); SOTA, Masahiro, Kobe-shi Hyogo 651-2241 (JP)
(74) Representative: dompatent von Kreisler Selting Werner - Partnerschaft von Patent- und Rechtsanwälten mbB
(86) International application number: PCT/JP2014/064029
(87) International publication number: WO 2015/022798

(57) **Abstract**

The present invention provides a novel terpenoid synthetase gene; a method of preparing a terpenoid compound, comprising introducing the novel terpenoid synthetase gene into a host cell to give a transformant, and culturing the transformant; and a novel terpenoid compound.

## Description

### TECHNICAL FIELD

The present invention relates to a novel terpenoid compound and a method of producing the same.

### BACKGROUND ART

Terpenoid metabolites including monoterpenes, sesquiterpenes and diterpenes are present in plants, liverworts, and fungi and are also present in prokaryotes with only a relatively minor fraction. A lot of terpenoid metabolites such as menthol, camphor, artemisinin, and taxol serve as a medicine, flavor, a raw material therefor, and the like and are industrially important. It is generally difficult to produce terpenoid metabolites on a large scale in view of the cost and workload to be expended. In recent years, methods of biotechnologically-preparing terpenoids, which use genetically-modified microorganisms, have been studied.

In biosynthesis of a terpenoid,
isopentenyl diphosphate (IPP) and dimethylallyl diphosphate (DMAPP) are synthesized via "mevalonate pathway" wherein mevalonate is used as a biosynthesis intermediate, or "non-mevalonate pathway" wherein mevalonate is not used;
geranyl diphosphate (GPP), which has 10 carbon atoms, is synthesized by the condensation of DMAPP with one IPP;
farnesyl diphosphate (FPP), which has 15 carbon atoms, is synthesized by the condensation of DMAPP with two IPPs; and
geranylgeranyl diphosphate (GGPP), which has 20 carbon atoms, is synthesized by the condensation of DMAPP with three IPPs; and
monoterpenoid, sesquiterpenoid, and diterpenoid are synthesized, via a catalytic reaction using a terpenoid synthetase, from GPP, FPP, and GGPP, respectively.

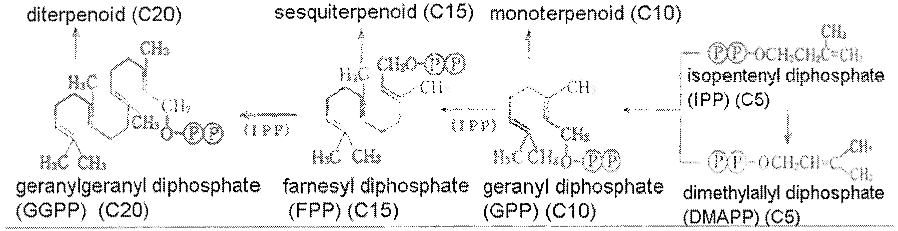

Study results regarding terpenoid synthetases derived from plants, fungi, and bacterium (Non-patent document 1) show that the terpenoid synthetases derived from these organisms typically display two highly conserved metal-binding motifs: an acidic amino acid-rich motif, -DDxx[DE]- or -DDxxx[DE]-; and an triad motif located downstream of the acidic amino acid-rich motif, -[ND]Dxx[ST]xx[KR][DE]-.

It has been reported that these two motifs are involved in the activation of the precursor (GPP or FPP) (Non-patent document 2, Non-patent document 3). The activation is important in order to trigger the cyclization cascade in a biosynthesis of terpenoids.

As for the two highly conserved metal-binding motifs, presumptive sequences of terpenoid synthetases were obtained by bioinformatics based on a search method using hidden Markov models and Pfam search of bacterial databases (Non-patent document 4), and then 2-methylisoborneol (monoterpene) synthase was discovered (Non-patent document 5, Non-patent document 6).

A method of evaluating a candidate terpenoid synthetase gene wherein the candidate gene is transferred into actinomycetes has been studied (Non-patent document 7).

For example, *Streptomyces avermitilis,* which is one of actinomycetes and whose genome has been wholly sequenced, is widely used in the industrial production of an anthelmintic agent avermectin. A series of SUKA which is a genetically-modified *S. avermitilis* has been developed. The mutant SUKA retains the ability of the parent S. *avermitilis* to synthesize components which are necessary to survive and also retains the ability to export biosynthetic end products. The mutant lacks a majority of endogenous biosynthetic gene clusters, which corresponds to 1.5 Mb of the left subtelomeric region, and thereby the mutant is suitable for evaluating a function of a biosynthesis-related gene (Non-patent document 5, Non-patent document 8).

An efficient method of preparing a terpenoid compound wherein a genetically modified *E.coli* is used has been studied (Non-patent document 9, Non-patent document 10, Non-patent document 11).

It has been known that when a gene of a terpenoid precursor synthetase which synthesizes GPP, FPP, or GGPP is coexpressed with a gene of the corresponding terpenoid synthetase, the productivity of the terpenoid compound is improved (Non-patent document 11).

### CITATION LIST

### [NON-PATENT DOCUMENT]

Non-patent document 1: Felicetti, B. and Cane, D. E. (2004). Aristolochene synthase: Mechanistic analysis of active site residues by site-directed mutagenesis. J. Am. Chem. Soc., 126, 7212-7221.
Non-patent document 2: Christianson, D.W. (2006). Structural biology and chemistry of the terpenoid cyclases. Chem. Rev., 106, 3412-3442
Non-patent document 3: Christianson, D.W. (2008). Unearthing the roots of the terpenome, Curr. Opin. Chem. Biol., 12, 141-150.
Non-patent document 4: Finn, R.D., Mistry, J., Tate, J., Coggill, P., Heger, A., Pollington, J.E., Gavin, O.L., Gunasekaran, P., Ceric, G., Forslund, K., Holm, L., Sonnhammer, E.L., Eddy, S.R. and Bateman, A. (2010). The Pfam protein families database. Nucleic Acids Res., 38, D211-222.
Non-patent document 5: Komatsu, M., Tsuda, M., Omura, S., Oikawa, H. and Ikeda, H. (2008). Identification and functional analysis of genes controlling biosynthesis of 2-methylisoborneol. Proc. Natl. Acad. Sci. USA., 105, 7422-7427.
Non-patent document 6: Giglio, S., Chou, W.K., Ikeda, H., Cane, D.E. and Monis, P.T. (2011). Biosynthesis of 2-methylisoborneol in cyanobacteria. Environ. Sci. Technol., 45, 992-998.
Non-patent document 7: Yamada et al. Methods in Enzymology Vol. 515, pp123-162, 2012.
Non-patent document 8: Komatsu, M., Uchiyama, T., Omura, S., Cane, D.E. and Ikeda, H. (2010). Genomeminimized Streptomyces host for the heterologous expression of secondary metabolism. Proc. Natl. Acad. Sci. USA., 107, 2646-2651.
Non-patent document 9: Harada, H., Yu, F., Okamoto, S., Kuzuyama, T., Utsumi, R., and Misawa, N. (2009) Efficient synthesis of functional isoprenoids from acetoacetate through metabolic pathway-engineered Escherichia coli. Appl. Microbiol. Biotechnol., 81, 915-925
Non-patent document 10: Hu, Y., Chou, W.K.W., Hopson, R. and Cane, D.E. (2011). Genome mining in Streptomyces clavuligerus: Expression and biochemical characterization of two new cryptic sesquiterpene synthases. Chem. Biol., 18, 32-37.
Non-patent document 11: Martin, V.J., Pitera, D.J., Withers, S.T., Newman, J.D. and Keasling, J.D. (2003). Engineering a mevalonate pathway in Escherichia coli for production of terpenoids, Nat. Biotechnol., 21, 796-802.
Non-patent document 12: Chou, WKW et al. J. Am. Chem. Soc. 132, 8850-8851, 2010.
Non-patent document 13: Ikeda, H.; Takada, Y.; Pang, C. H.; Tanaka, H.; Omura, S. J. Bacteriol. 1993, 175, 2077-2082.
Non-patent document 14: Alexeyev, MF et al. Gene 160, 63-67, 1995.

### SUMMARY OF THE INVENTION

### (PROBLEMS TO BE SOLVED BY INVENTION)

An object of the present invention is to provide a novel terpenoid synthetase gene, a production method of a terpenoid compound by using the gene, and a novel terpenoid compound.

### (MEANS TO SOLVE PROBLEMS)

The present inventors have studied to find out a solution of said problems, and then they have successfully identified the novel terpenoid synthetase genes.

Specifically, the present invention provides the following genes:
Monoterpenoid synthetase gene: sclav_p0982 (SEQ ID NO:1);
Sesquiterpenoid synthetase gene: sclav_p0985 (SEQ ID NO:3), sclav_p1407 (SEQ ID NO:5), sgr_6065 (SEQ ID NO:7), sven_0552 (SEQ ID NO:9), sk_3051 (SEQ ID NO:11), slt_1718 (SEQ ID NO:13), mmar_3220 (SEQ ID NO:15), mmar_3220A (SEQ ID NO:58), haur_2988 (SEQ ID NO:27), haur_2988A (SEQ ID NO:61), rcas_0622 (SEQ ID NO:29), rcas_0622A (SEQ ID NO:62), roseRS_3509 (SEQ ID NO:31), roseRS_3509A (SEQ ID NO:63), sce6369 (SEQ ID NO:33), sce6369A (SEQ ID NO:64), sce8552 (SEQ ID NO:35), sce8552A (SEQ ID NO:65), and slt_1880 (SEQ ID NO:92);
Diterpenoid synthetase gene: sclav_p0765 (SEQ ID NO:17), sclav_p1169 (SEQ ID NO:19), slt_1078 (SEQ ID NO:21), rxyl_0493 (SEQ ID NO:23), rxyl_0493A (SEQ ID NO:59), haur_2987 (SEQ ID NO:25), and haur_2987A (SEQ ID NO:60).

The terpenoid synthetase genes of the present invention may have different sequences from those of the above genes as long as they encode the same amino acids as the above genes.

In addition, the terpenoid synthetase genes of the present invention may be the gene consisting of a base sequence having 80% or more, 90% or more, 95% or more, or 97% or more identity to the above genes.

The present invention provides the following amino acid sequences which are encoded by the above terpenoid synthetase genes:
Monoterpenoid synthetase protein :SEQ ID NO:2;
Sesquiterpenoid synthetase protein :SEQ ID NO:4, SEQ ID NO:6, SEQ ID NO:8, SEQ ID NO:10, SEQ ID NO:12, SEQ ID NO:14, SEQ ID NO:16, SEQ ID NO:28, SEQ ID NO:30, SEQ ID NO:32, SEQ ID NO:34, SEQ ID NO:36, and SEQ ID NO:93;
Diterpenoid synthetase protein :SEQ ID NO:18, SEQ ID NO:20, SEQ ID NO:22, SEQ ID NO:24, and SEQ ID NO:26.

In addition, the present invention provides a method of preparing a terpenoid compound, comprising:
transferring the above terpenoid synthetase gene into a host cell to give a transformant, and
culturing the transformant to give a terpenoid compound of interest.

In the method of the present invention, when a terpenoid precursor synthetase gene and a terpenoid synthetase gene are introduced into a single host cell, the resulting transformant can be used to prepare the terpenoid compound more efficiently. Alternatively, in the method of the present invention, a terpenoid synthetase obtained from a transformant which is made by introducing a terpenoid synthetase gene into a host cell, the transformant per se, a culture supernatant of the transformant, or a preparation from the culture supernatant may be applied to a terpenoid precursor (GPP, FPP, or GGPP).

The host used in the method of the present invention is not particularly limited as long as the host cell is able to express a gene of interest. For example, bacterium (actinomycetes, *E. coli, etc.*)*,* yeasts, fungi, basidiomycetes, insect cells, plant cells, and animal cells may be used.

The present invention also provides the following novel terpenoids which can be prepared by the method of the present invention:

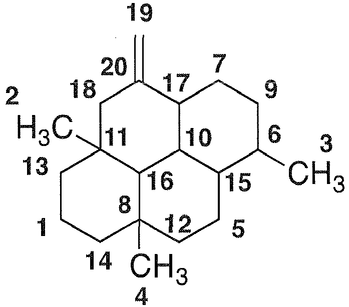

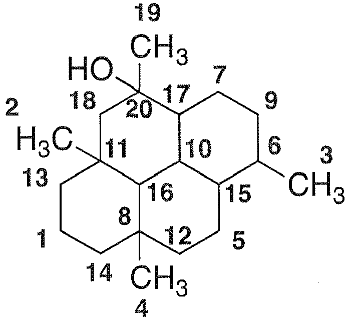

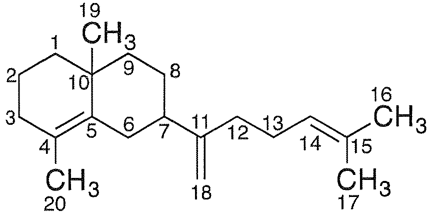

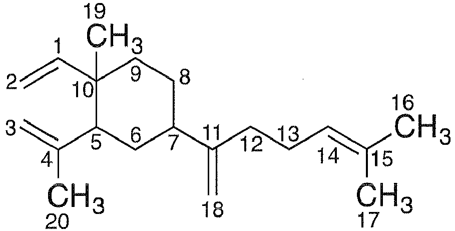

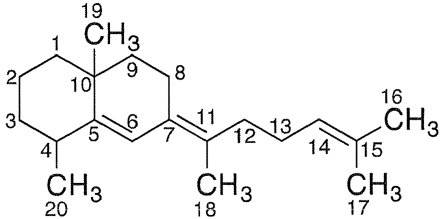

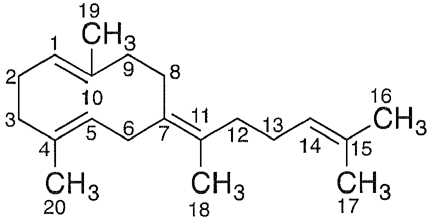

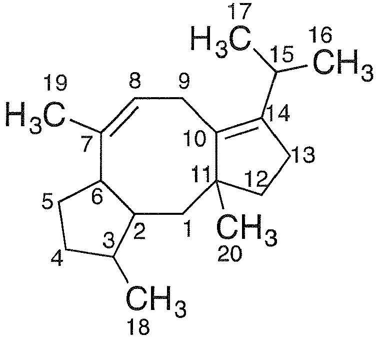

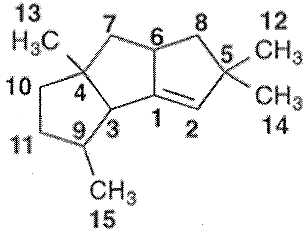

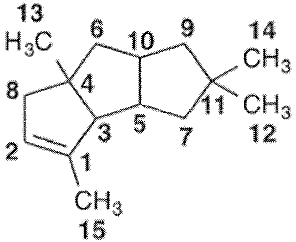

### (EFFECT OF THE INVENTION)

The genes of the present invention are useful in the biosynthesis of various terpenoid compounds.

Various terpenoid compounds may be prepared efficiently and in high productivity by the present invention.

Further, the novel terpenoid compounds of the present invention may be used as a drug (for example, a drug for treating ovarian cancer, leukemia, tuberculosis, Hansen's disease etc.), a raw material for drug, an antimicrobial agent or a raw material therefor, or flavor or a raw material therefor.

### BRIEF DESCRIPTION OF THE DRAWINGS

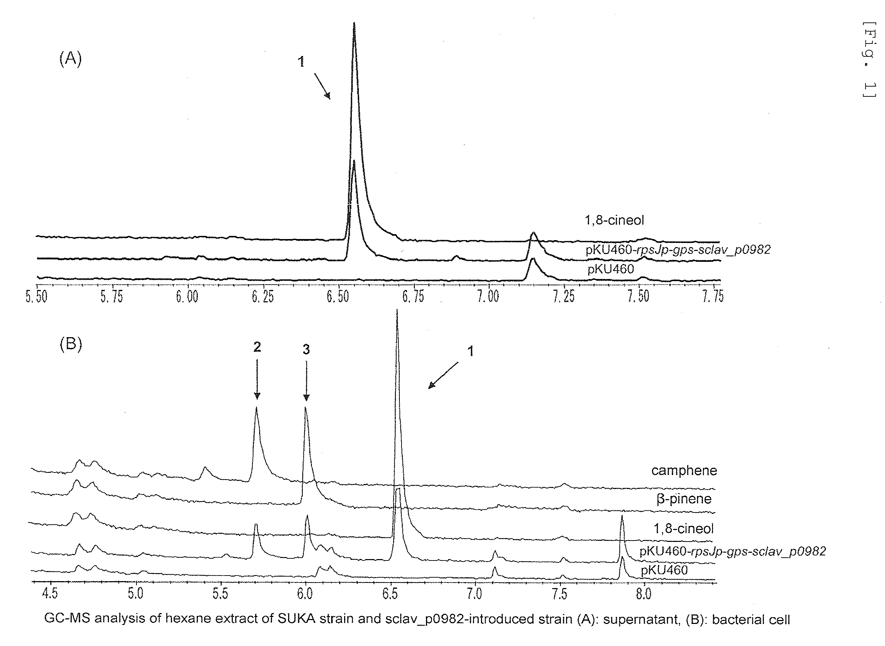

[Fig. 1] GC-MS analysis of hexane extract of SUKA strain and sclav_p0982-introduced strain (A): supernatant, (B): bacterial cell

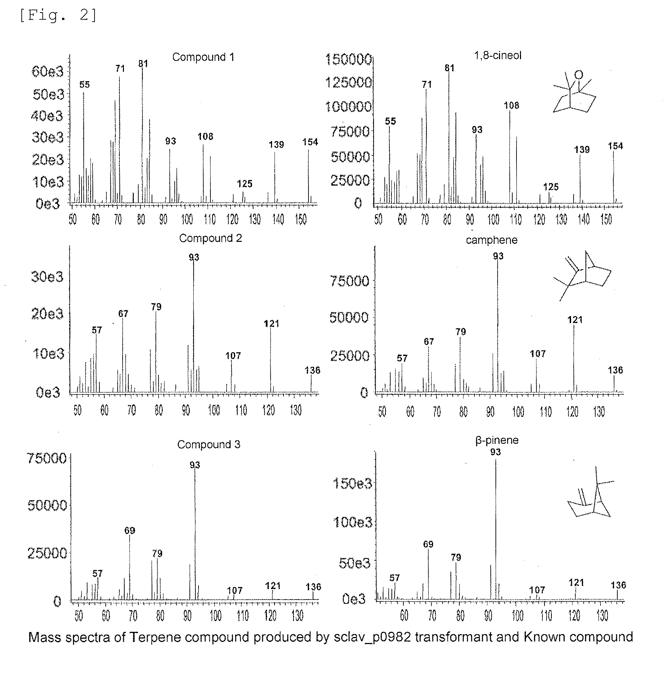

[Fig. 2] Mass spectra of Terpene compound produced by sclav_p0982 transformant and Known compound

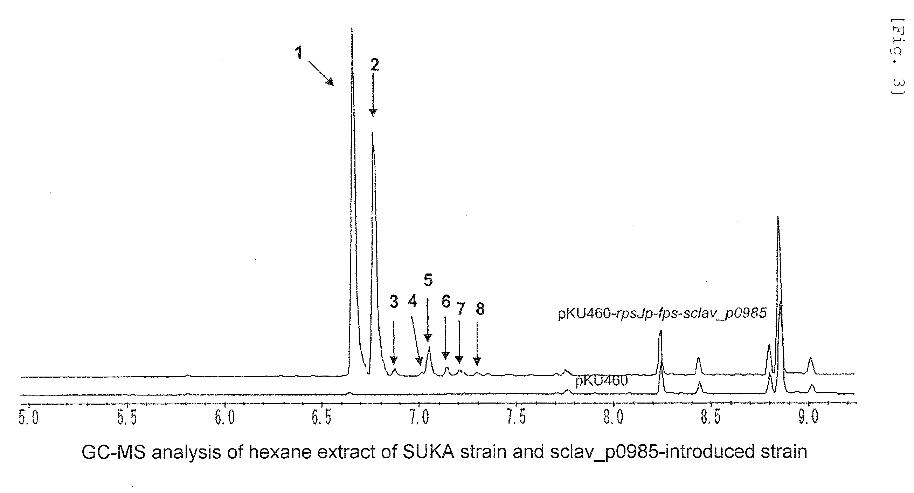

[Fig. 3] GC-MS analysis of hexane extract of SUKA strain and sclav_p0985-introduced strain

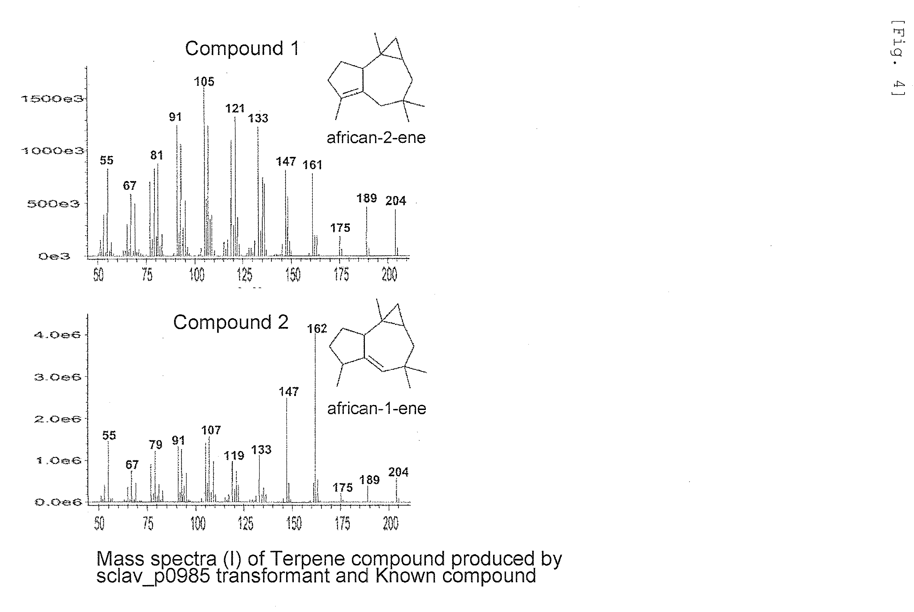

[Fig. 4] Mass spectra (I) of terpene compound produced by sclav_p0985 transformant and Known compound

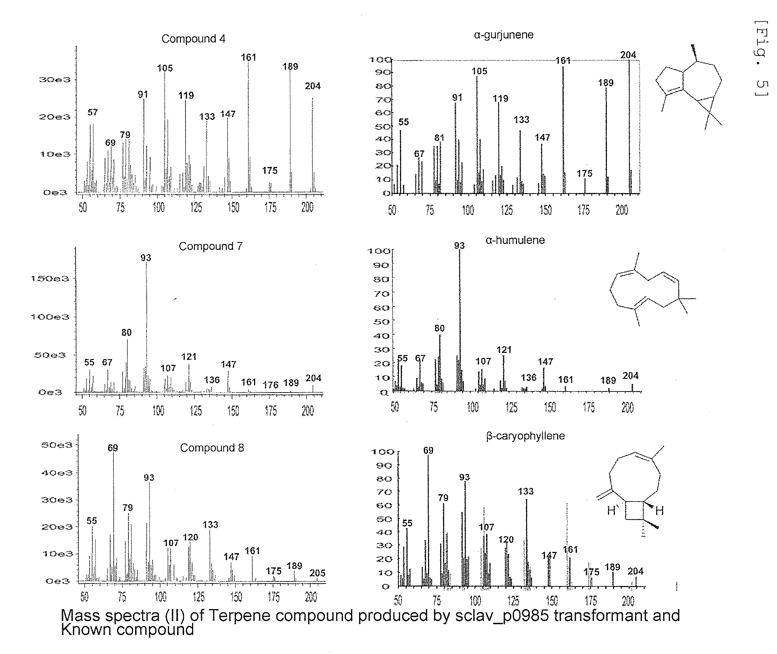

[Fig. 5] Mass spectra (II) of Terpene compound produced by sclav_p0985 transformant and Known compound

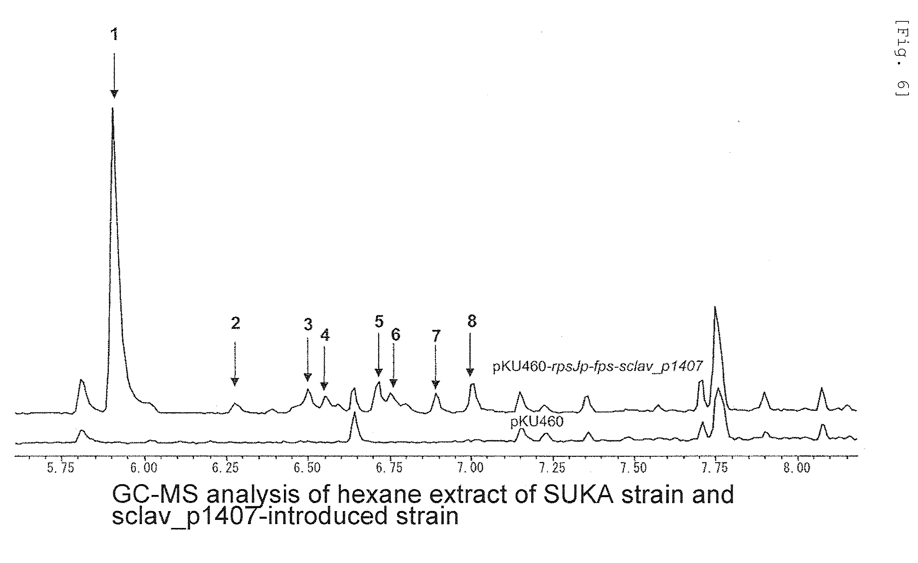

[Fig. 6] GC-MS analysis of hexane extract of SUKA strain and sclav_p1407-introduced strain

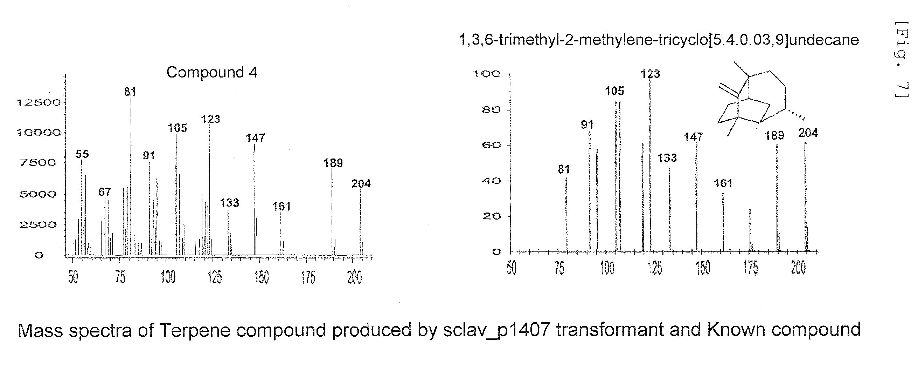

[Fig. 7] Mass spectra of Terpene compound produced by sclav_p1407 transformant and Known compound

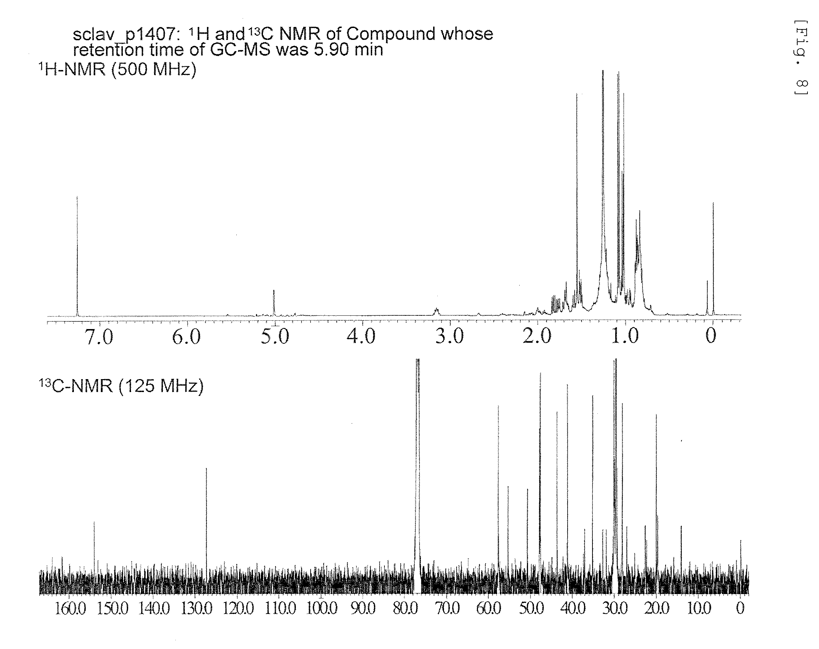

[Fig. 8] sclav_p1407: ¹H and ¹³C NMR of Compound whose retention time of GC-MS was 5.90 min

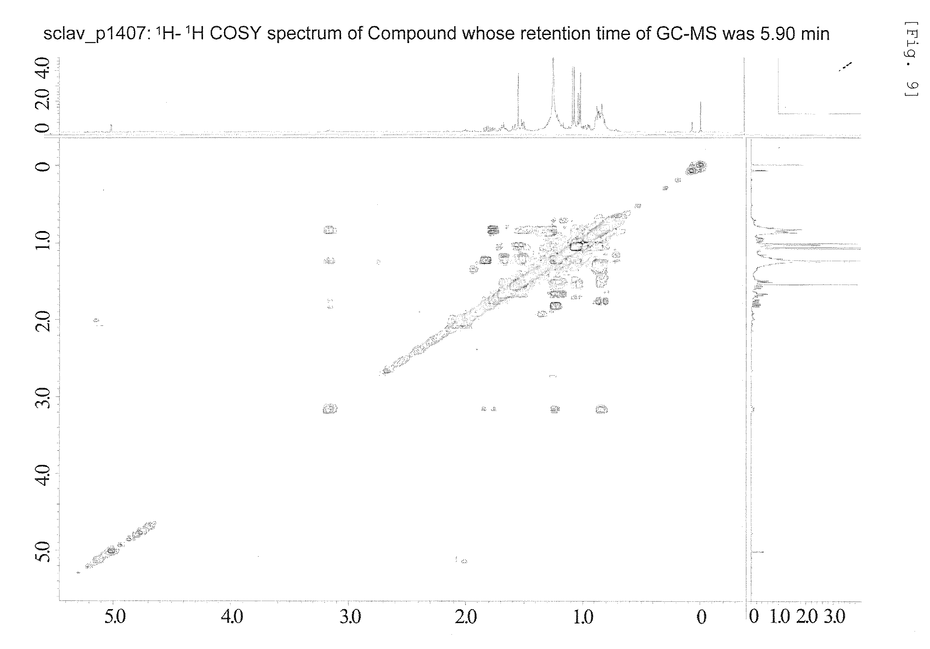

[Fig. 9] sclav_p1407: ¹H-¹H COSY spectrum of Compound whose retention time of GC-MS was 5.90 min

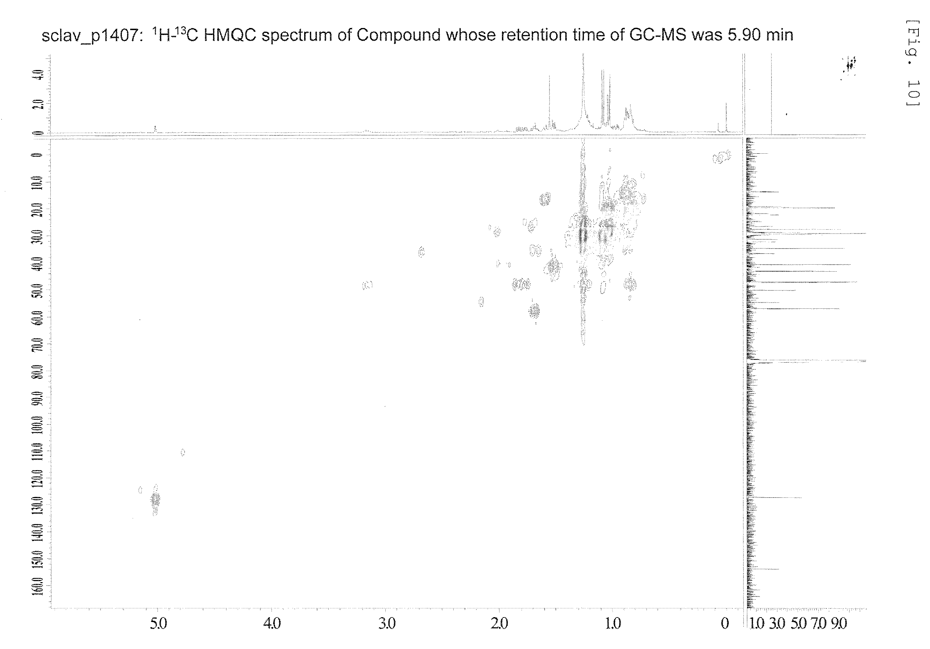

[Fig. 10] sclav_p1407: ¹H-¹³C HMQC spectrum of Compound whose retention time of GC-MS was 5.90 min

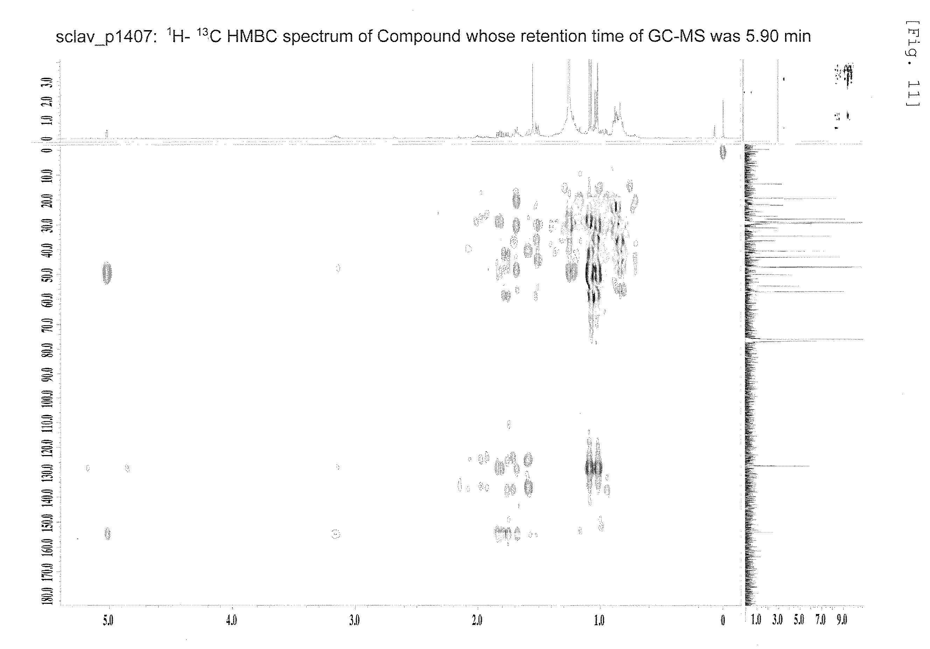

[Fig. 11] sclav_p1407: ¹H-¹³C HMBC spectrum of Compound whose retention time of GC-MS was 5.90 min

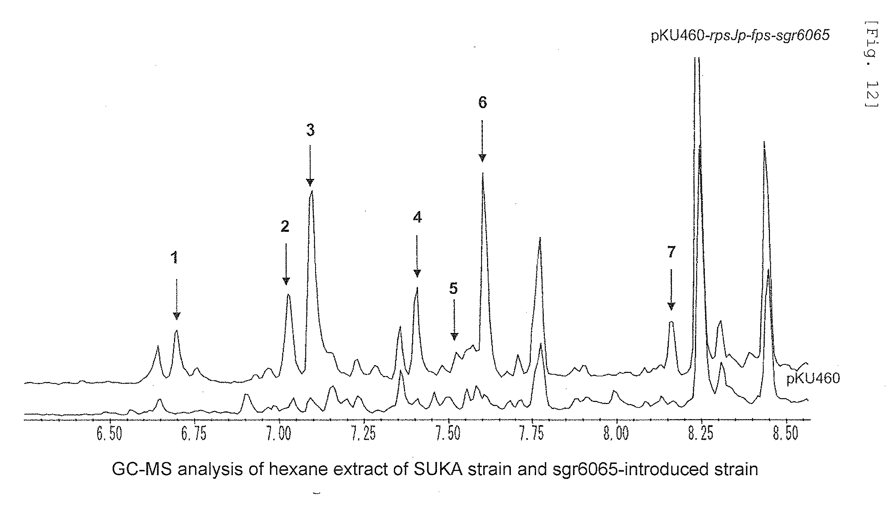

[Fig. 12] GC-MS analysis of hexane extract of SUKA strain and sgr6065-introduced strain

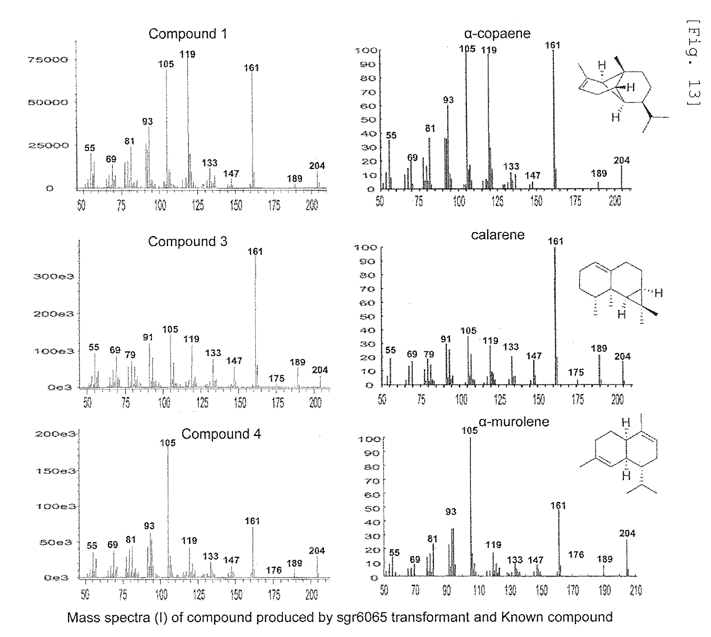

[Fig. 13] Mass spectra (I) of compound produced by sgr6065 transformant and Known compound

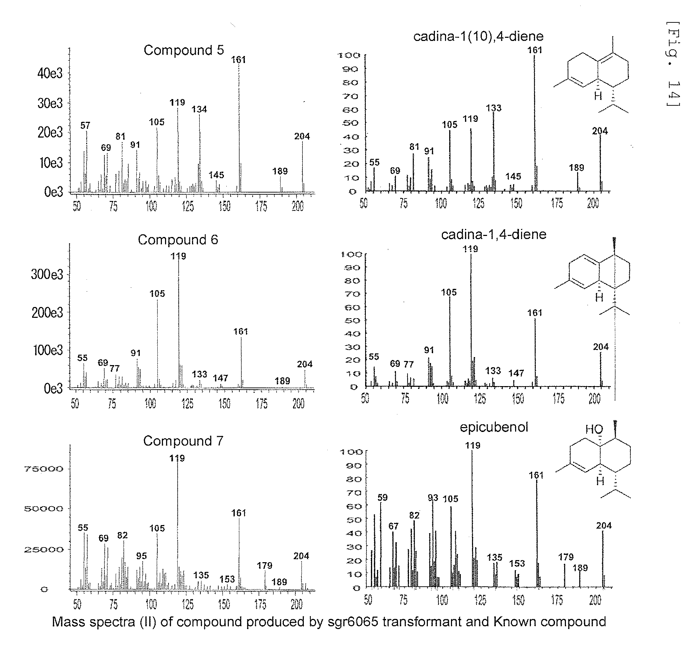

[Fig. 14] Mass spectra (II) of compound produced by sgr6065 transformant and Known compound

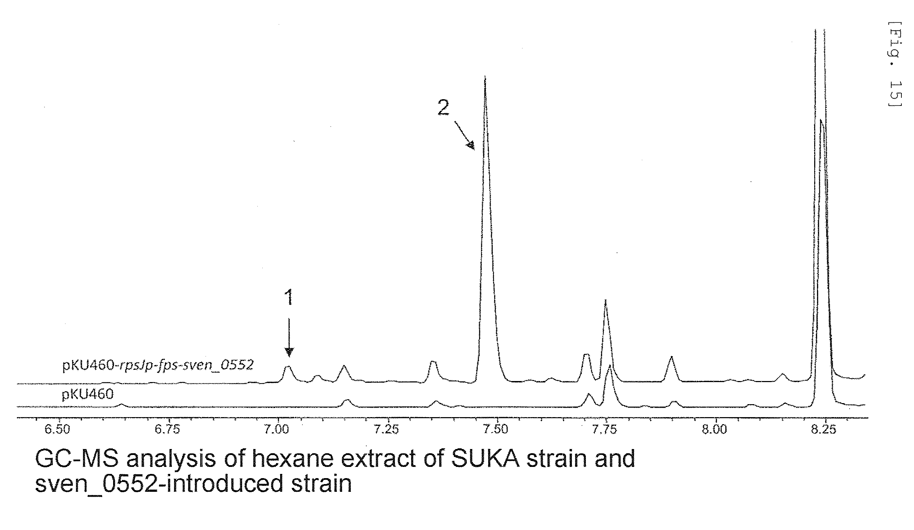

[Fig. 15] GC-MS analysis of hexane extract of SUKA strain and sven_0552-introduced strain

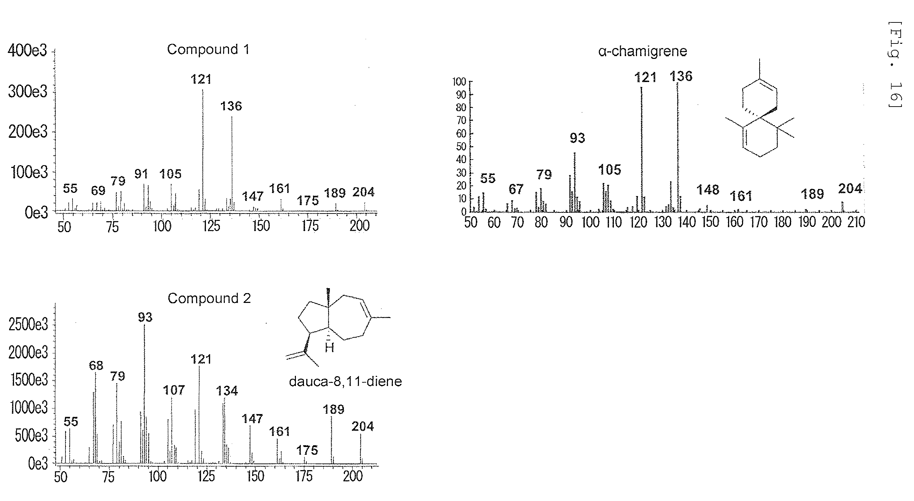

[Fig. 16] Mass spectra of compound produced by sven_0552 transformant and Known compound

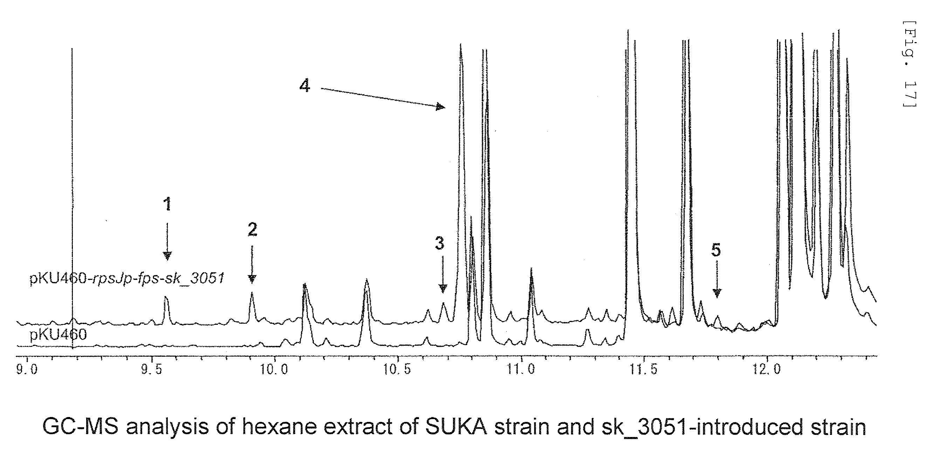

[Fig. 17] GC-MS analysis of hexane extract of SUKA strain and sk_3051-introduced strain

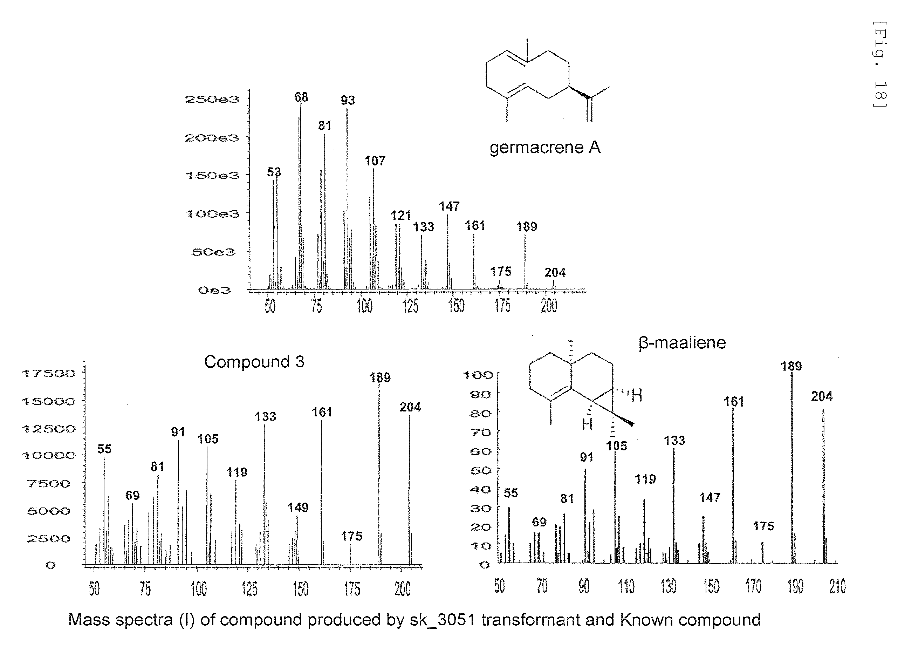

[Fig. 18] Mass spectra (I) of compound produced by sk_3051 transformant and Known compound

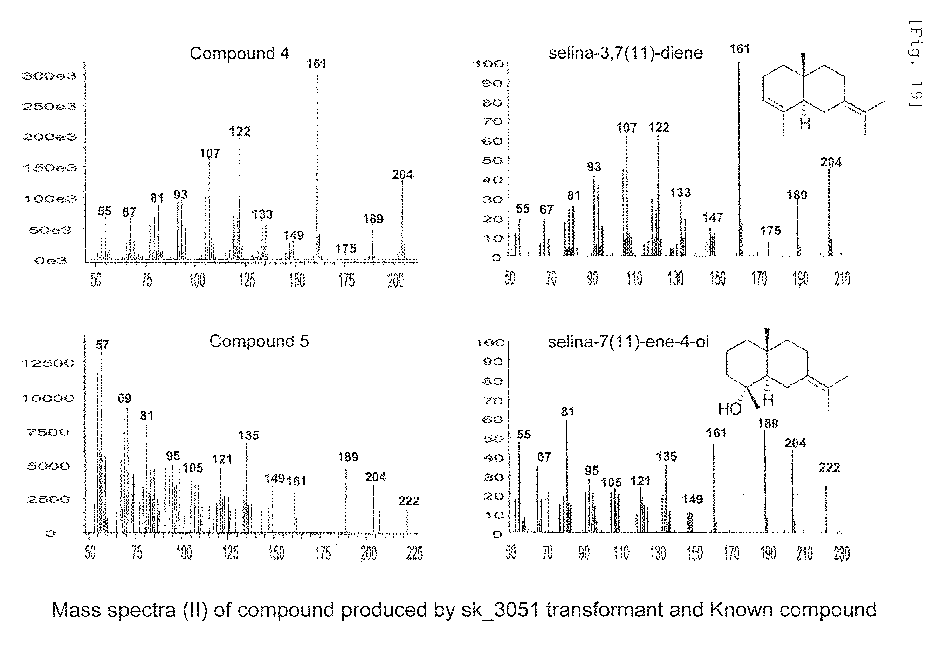

[Fig. 19] Mass spectra (II) of compound produced by sk_3051 transformant and Known compound

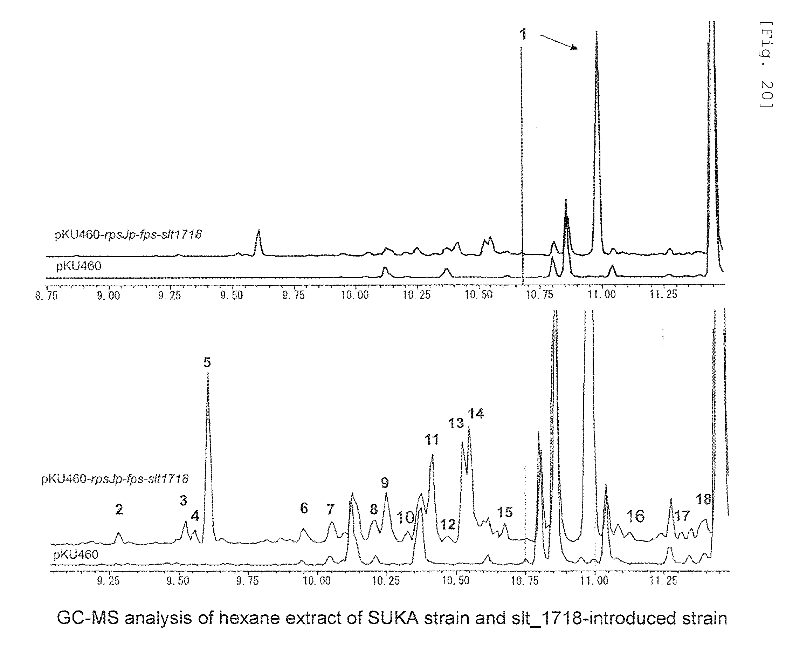

[Fig. 20] GC-MS analysis of hexane extract of SUKA strain and slt_1718-introduced strain

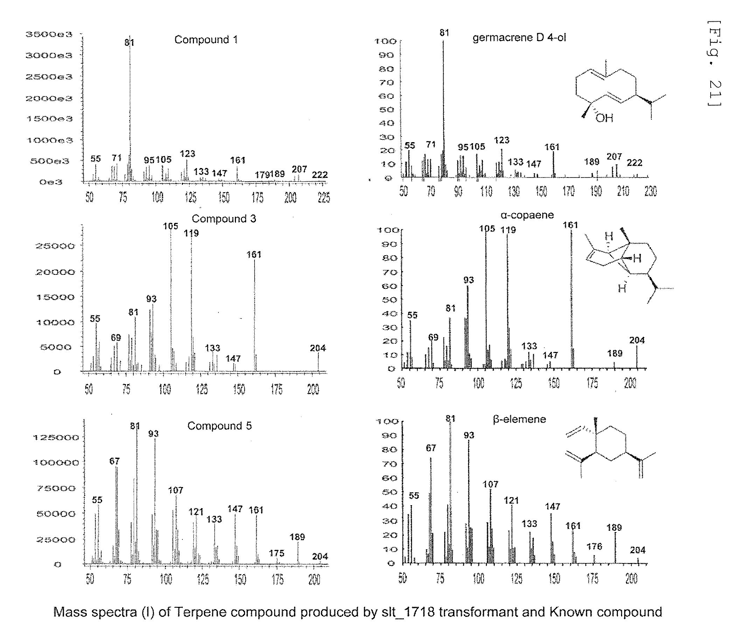

[Fig. 21] Mass spectra (I) of terpene compound produced by slt_1718 transformant and Known compound

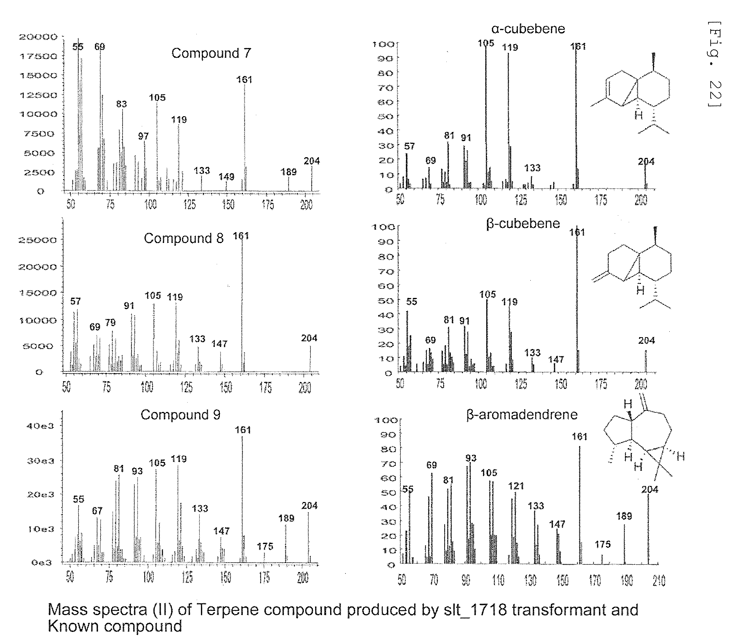

[Fig. 22] Mass spectra (II) of terpene compound produced by slt_1718 transformant and Known compound

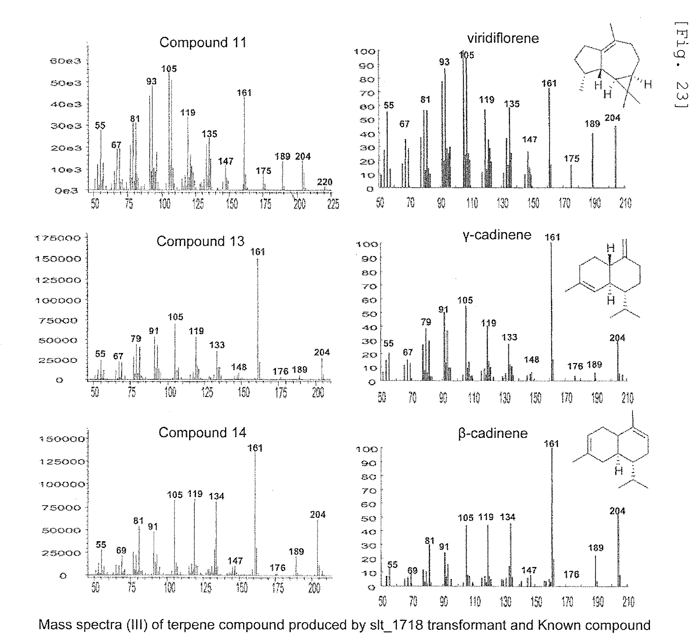

[Fig. 23] Mass spectra (III) of terpene compound produced by slt_1718 transformant and Known compound

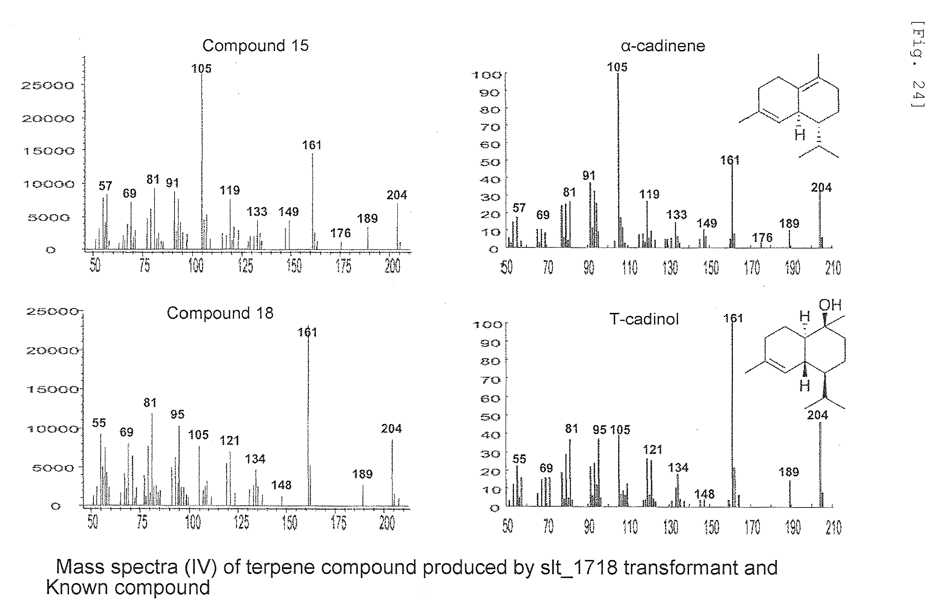

[Fig. 24] Mass spectra (IV) of terpene compound produced by slt_1718 transformant and Known compound

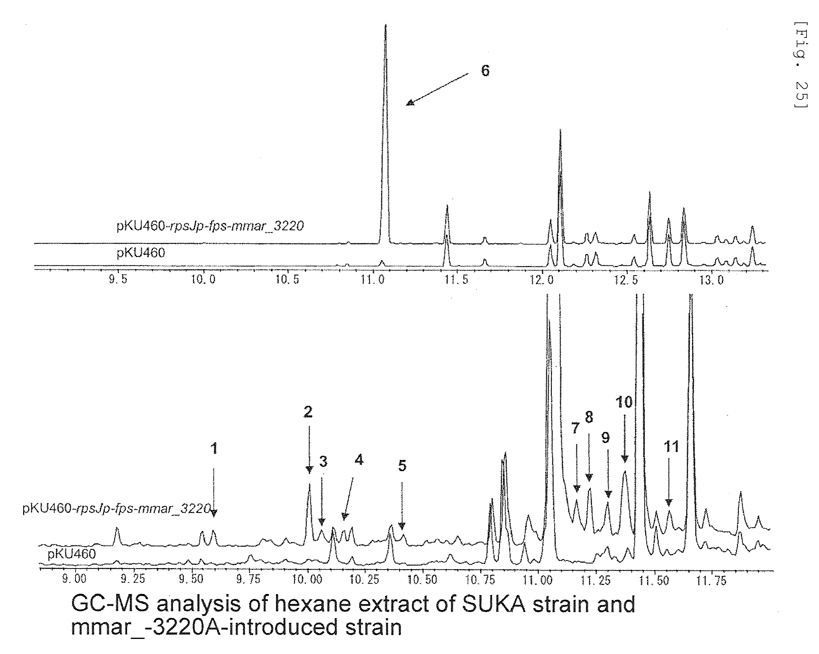

[Fig. 25] GC-MS analysis of hexane extract of SUKA strain and mmar_-3220A-introduced strain

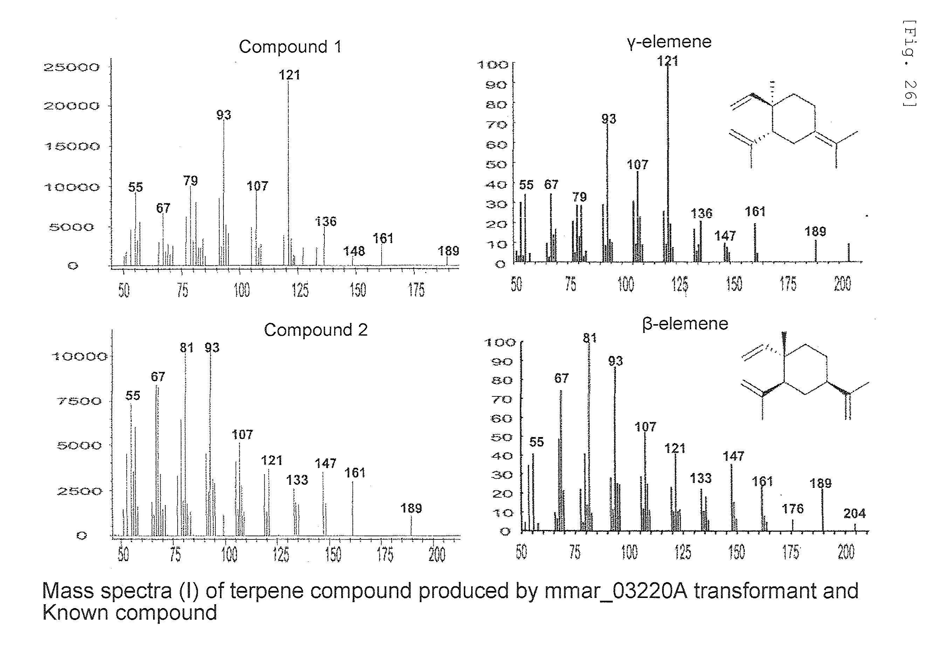

[Fig. 26] Mass spectra (I) of terpene compound produced by mmar_03220A transformant and Known compound

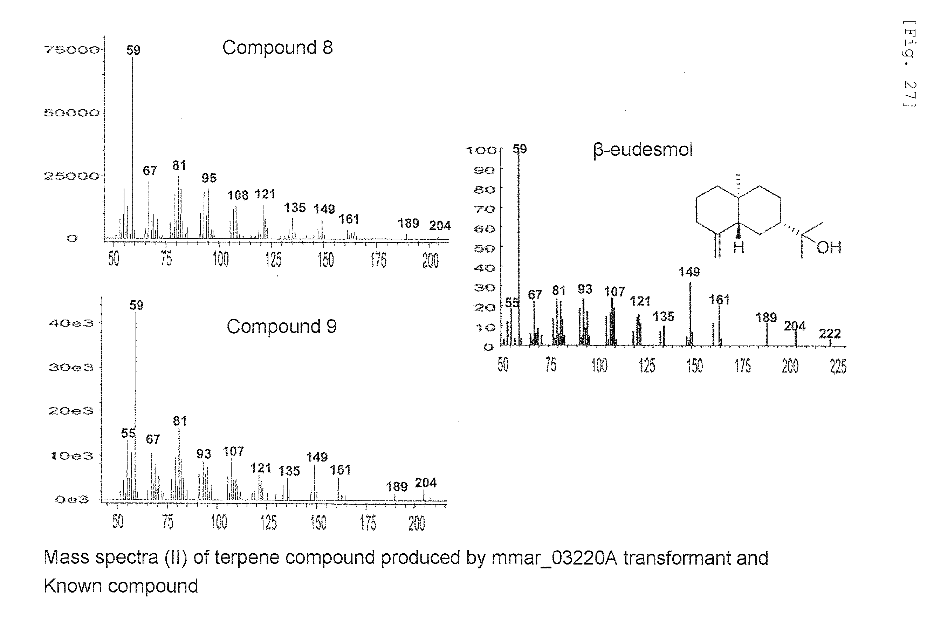

[Fig. 27] Mass spectra (II) of terpene compound produced by mmar_03220A transformant and Known compound

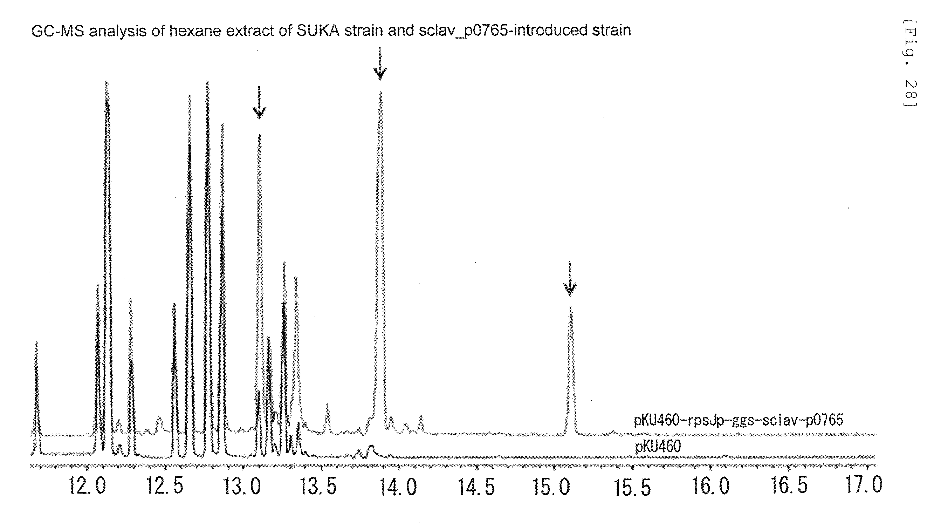

[Fig. 28] GC-MS analysis of hexane extract of SUKA strain and sclav_p0765-introduced strain

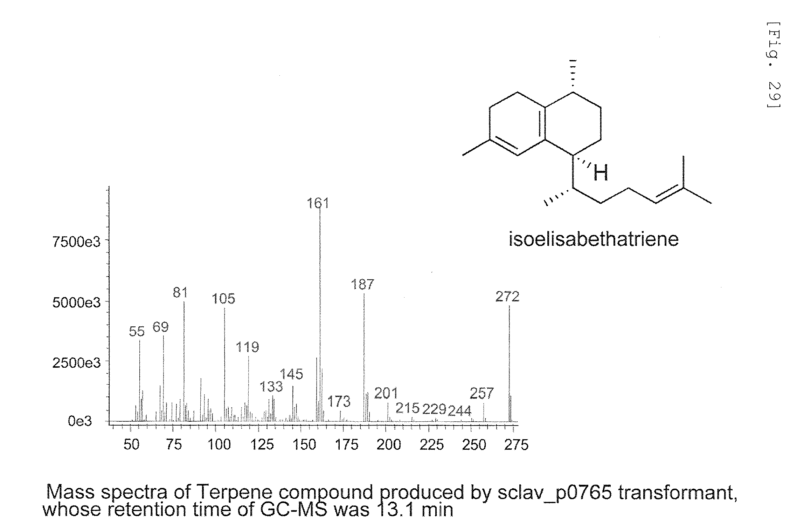

[Fig. 29] Mass spectra of Terpene compound produced by sclav_p0765 transformant

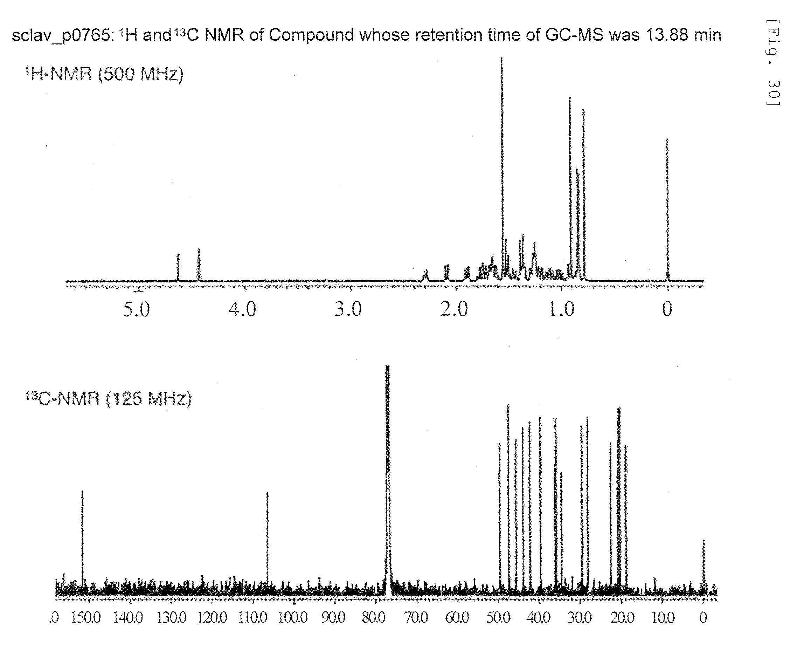

[Fig. 30] sclav_p0765: ¹H and ¹³C NMR of Compound whose retention time of GC-MS was 13.88 min

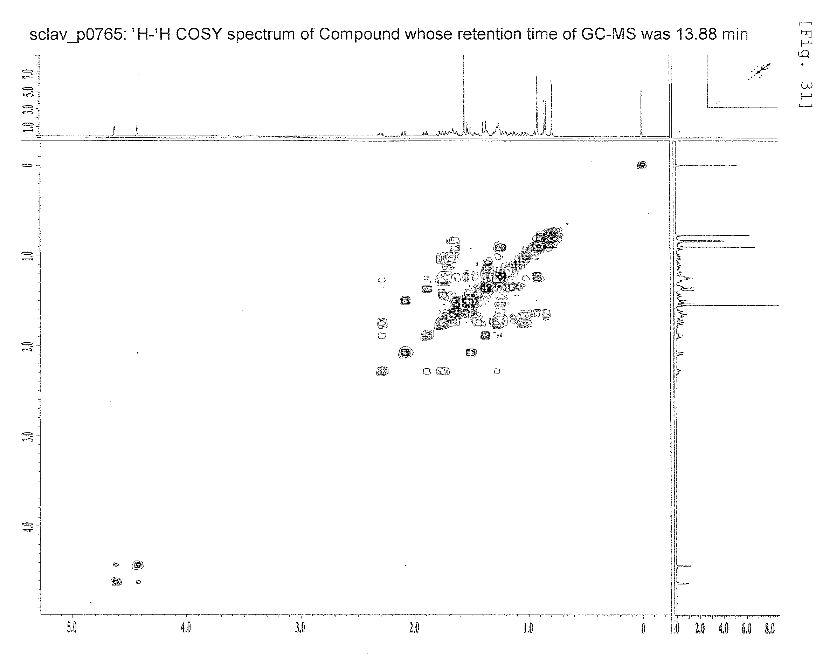

[Fig. 31] sclav_p0765: ¹H-¹H COSY spectrum of Compound whose retention time of GC-MS was 13.88 min

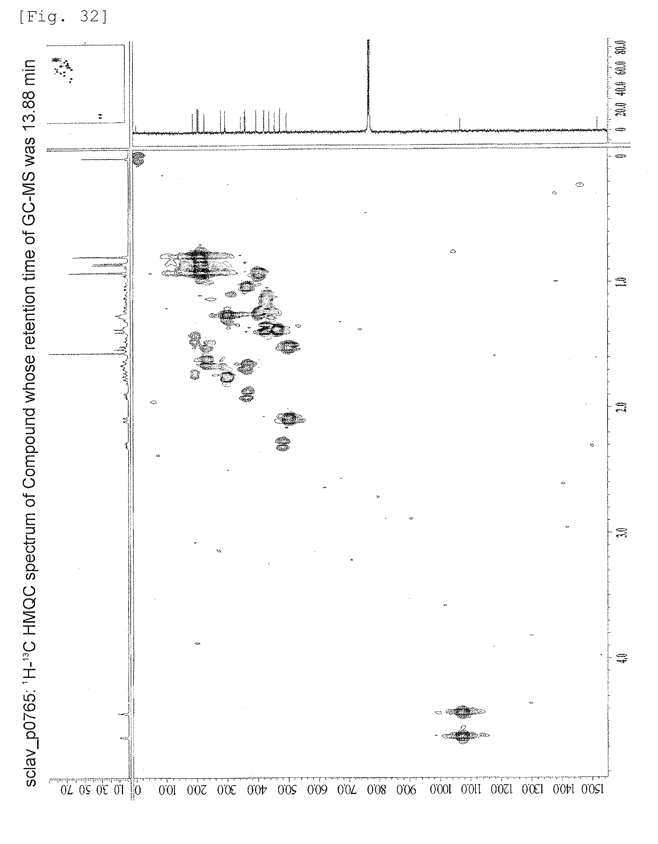

[Fig. 32] sclav_p0765: ¹H-¹³C HMQC spectrum of Compound whose retention time of GC-MS was 13.88 min

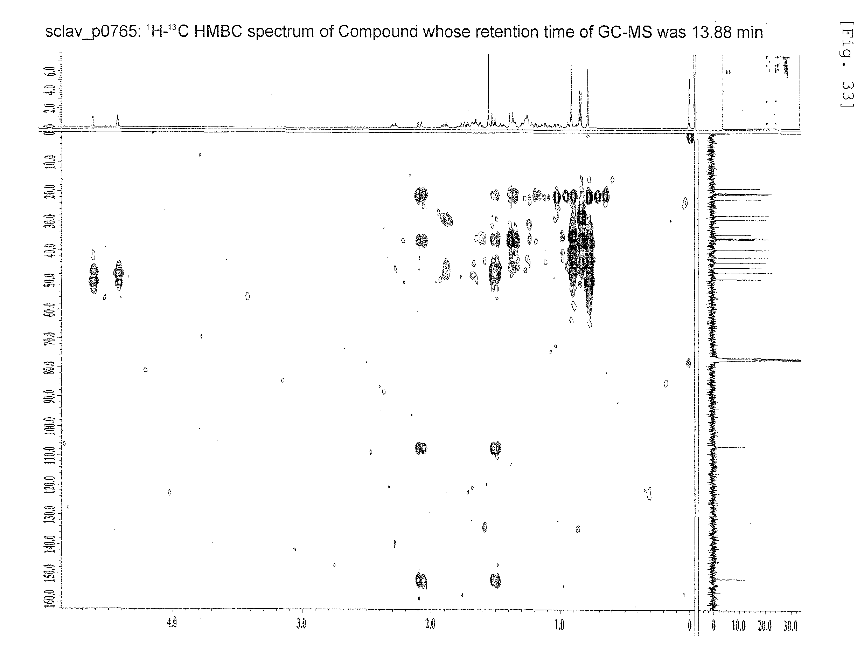

[Fig. 33] sclav_p0765: ¹H-¹³C HMBC spectrum of Compound whose retention time of GC-MS was 13.88 min

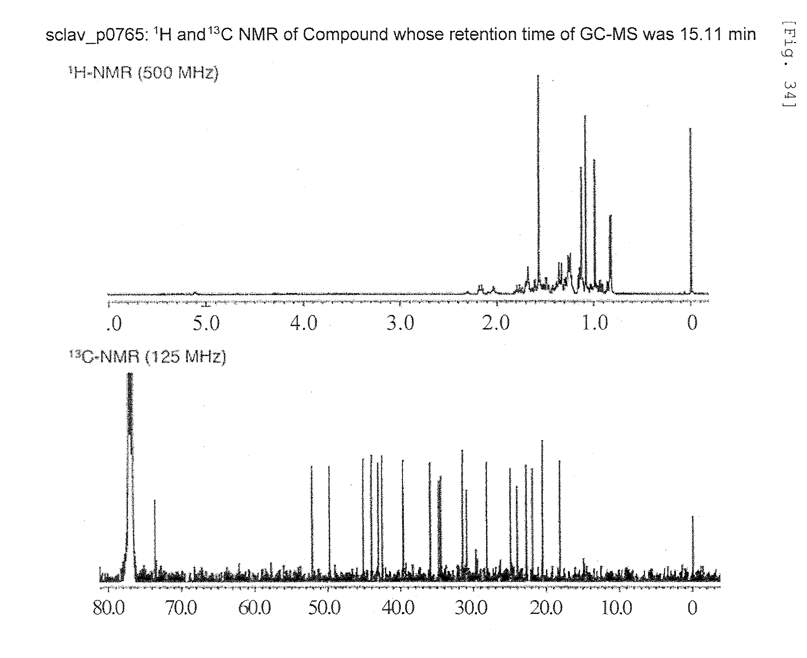

[Fig. 34] sclav_p0765: ¹H and ¹³C NMR of Compound whose retention time of GC-MS was 15.11 min

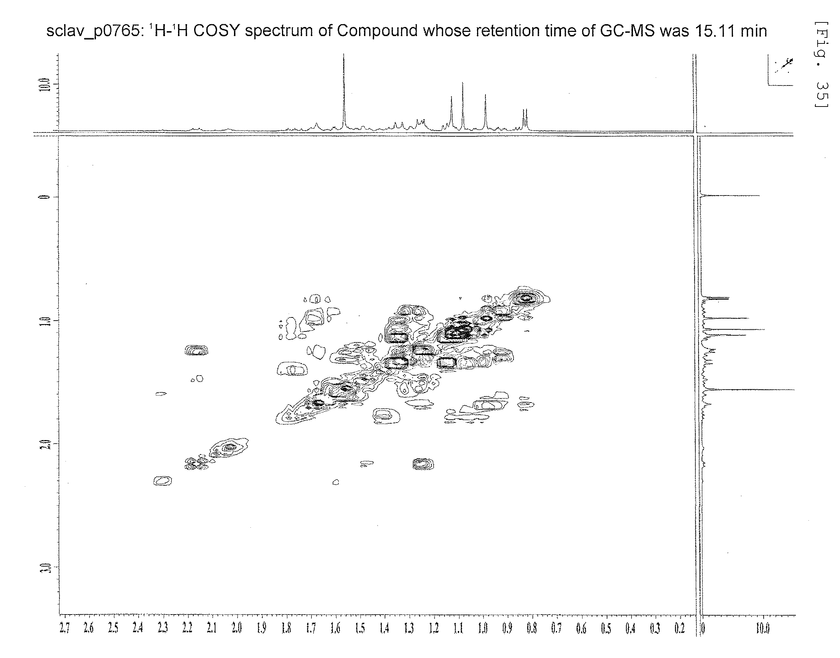

[Fig. 35] sclav_p0765: ¹H-¹H COSY spectrum of Compound whose retention time of GC-MS was 15.11 min

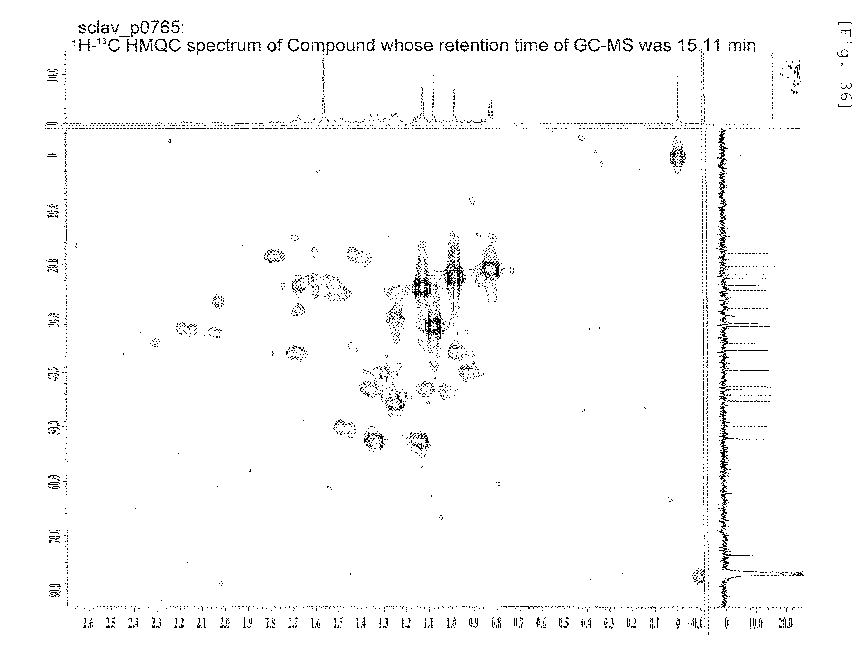

[Fig. 36] sclav_p0765: ¹H-¹³C HMQC spectrum of Compound whose retention time of GC-MS was 15.11 min

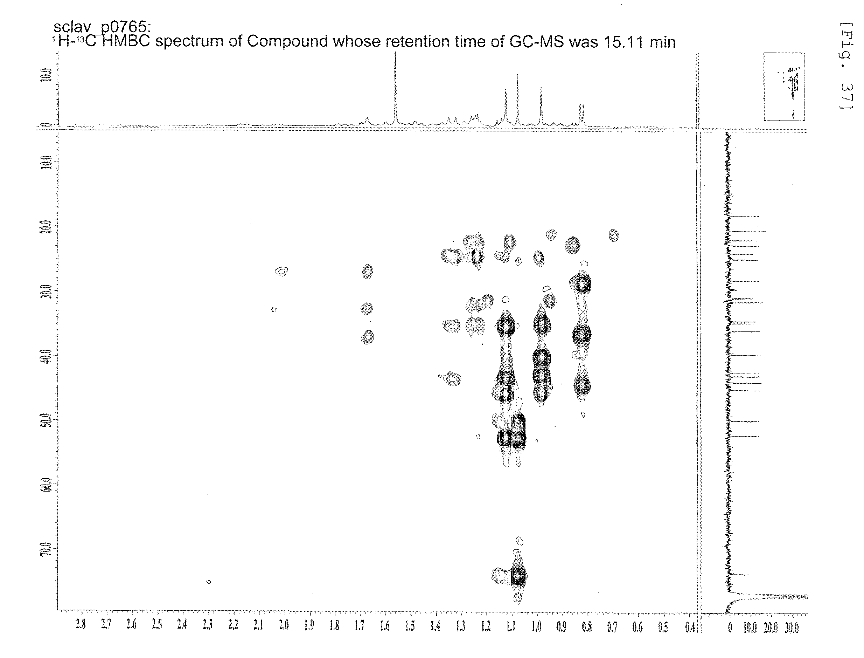

[Fig. 37] sclav_p0765: ¹H-¹³C HMBC spectrum of Compound whose retention time of GC-MS was 15.11 min

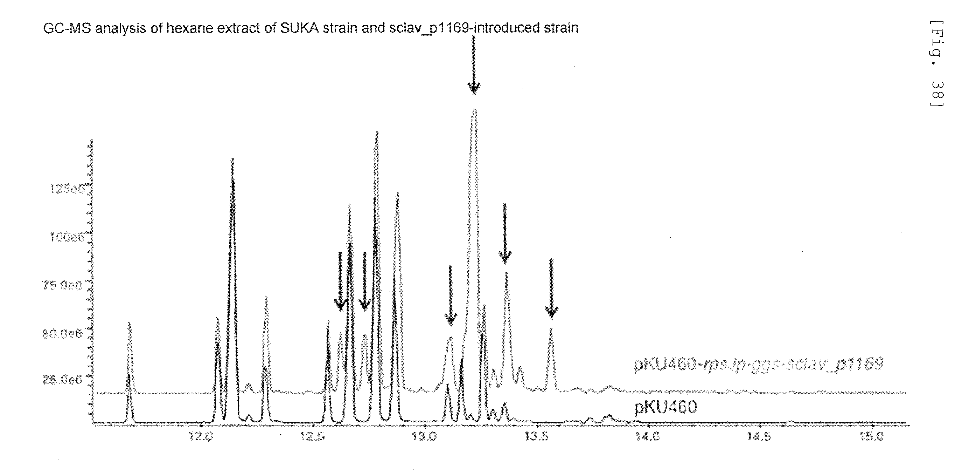

[Fig. 38] GC-MS analysis of hexane extract of SUKA strain and sclav_p1169-introduced strain

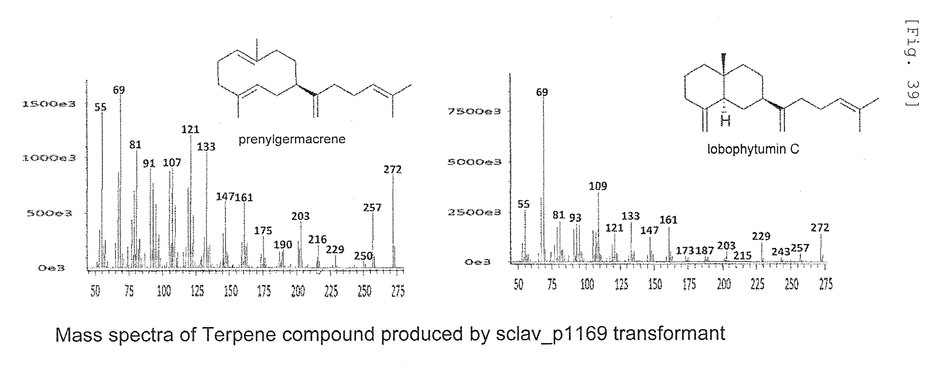

[Fig. 39] Mass spectra of Terpene compound produced by sclav_p1169 transformant

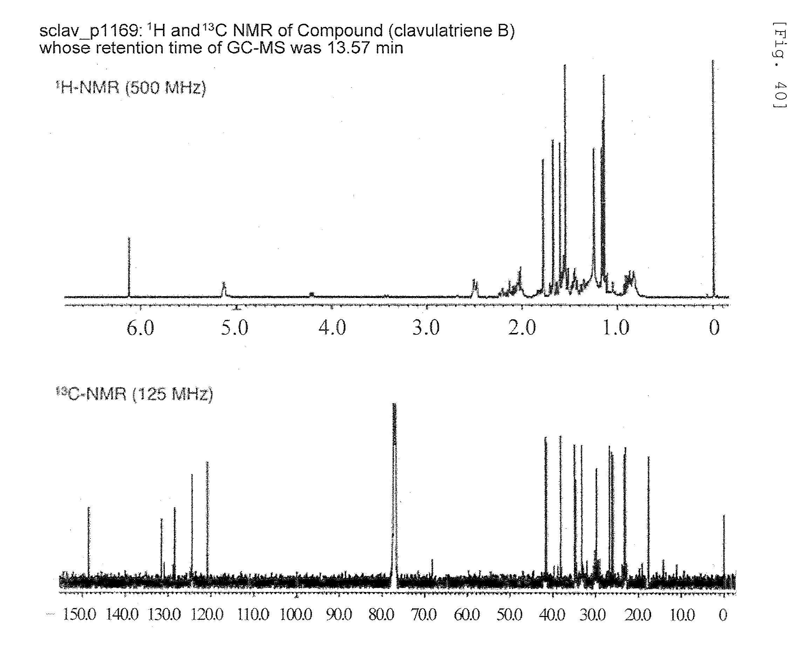

[Fig. 40] sclav_p1169: ¹H and ¹³C NMR of Compound (clavulatriene B) whose retention time of GC-MS was 13.57 min

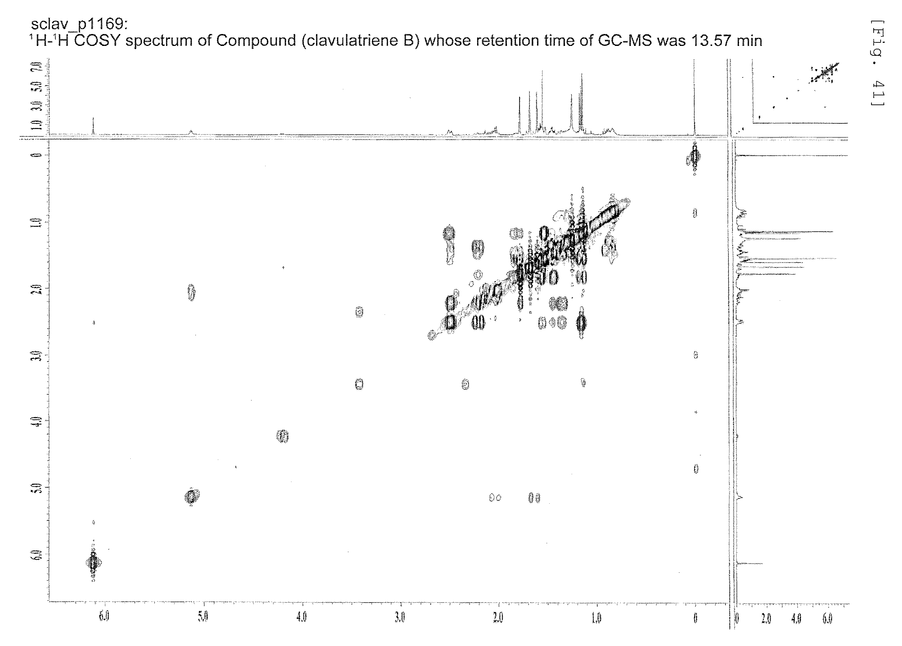

[Fig. 41] sclav_p1169: ¹H-¹H COSY spectrum of Compound (clavulatriene B) whose retention time of GC-MS was 13.57 min

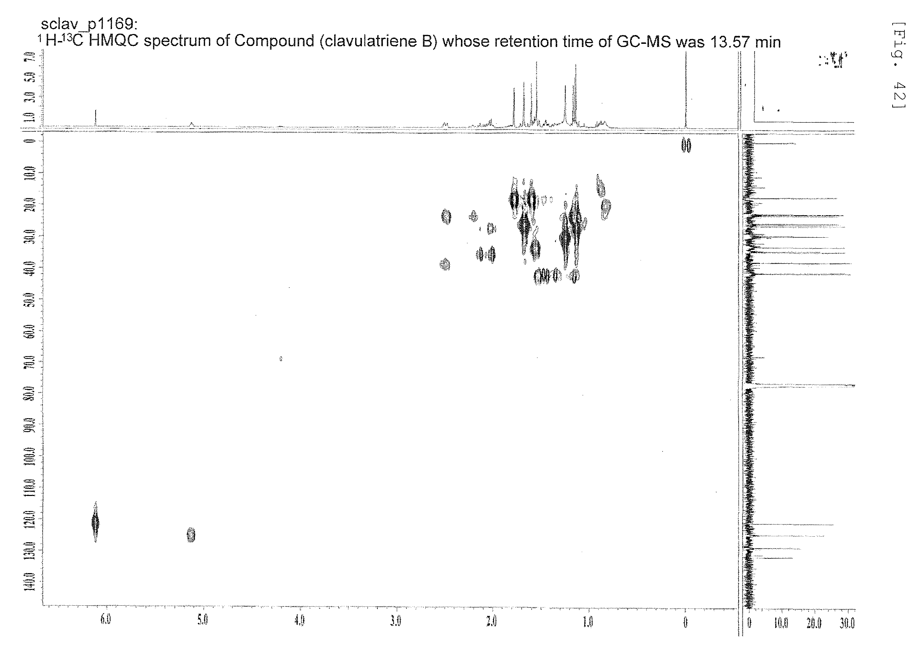

[Fig. 42] sclav_p1169: ¹H-¹³C HMQC spectrum of Compound (clavulatriene B) whose retention time of GC-MS was 13.57 min

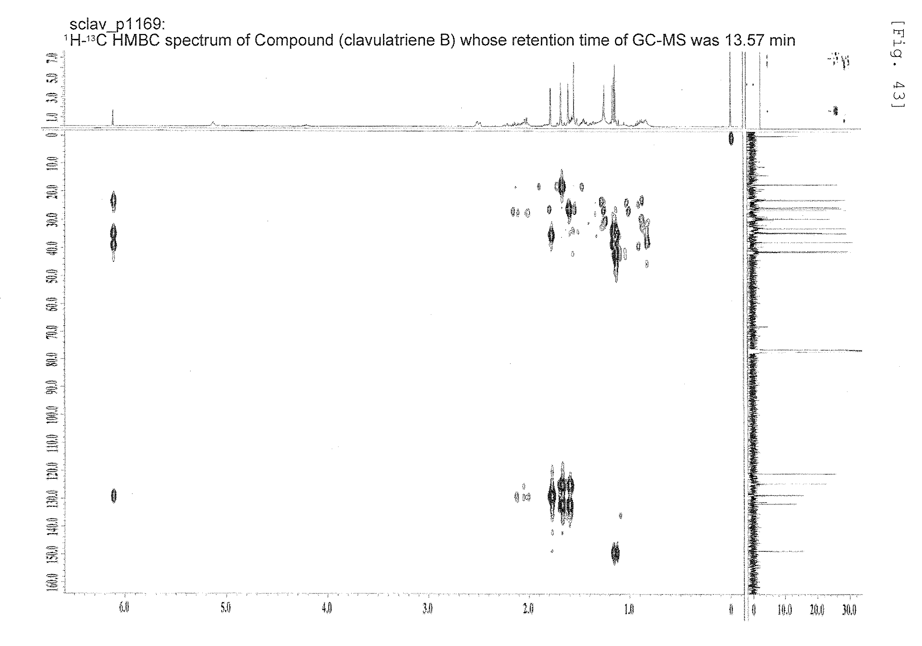

[Fig. 43] sclav_p1169: ¹H-¹³C HMBC spectrum of Compound (clavulatriene B) whose retention time of GC-MS was 13.57 min

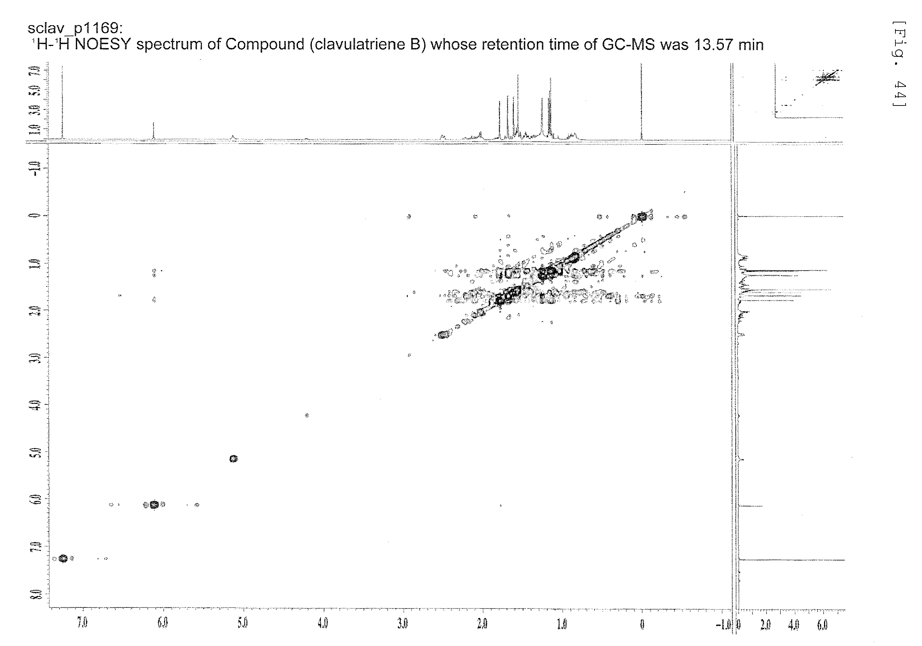

[Fig. 44] sclav_p1169: ¹H-¹H NOESY spectrum of Compound (clavulatriene B) whose retention time of GC-MS was 13.57 min

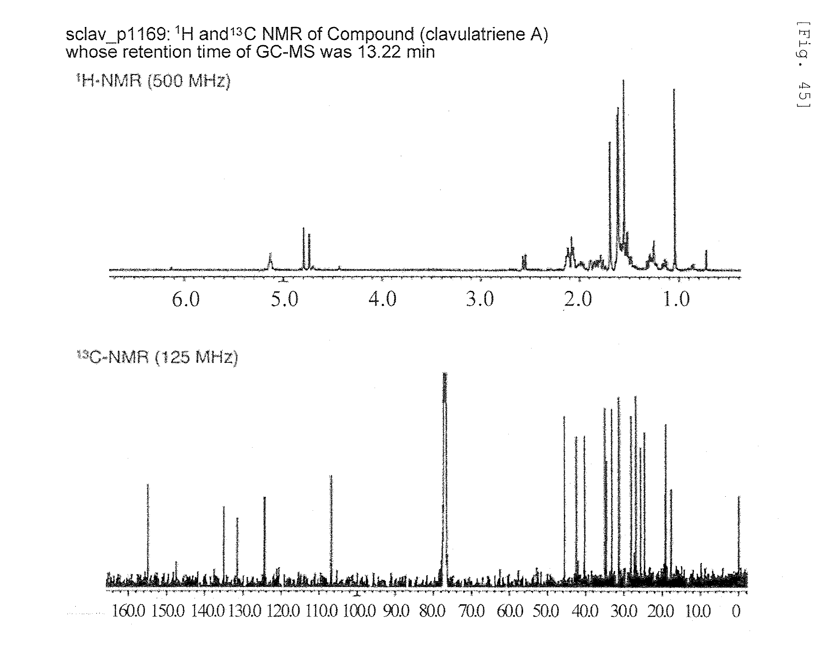

[Fig. 45] sclav_p1169: ¹H and ¹³C NMR of Compound (clavulatriene A) whose retention time of GC-MS was 13.22 min

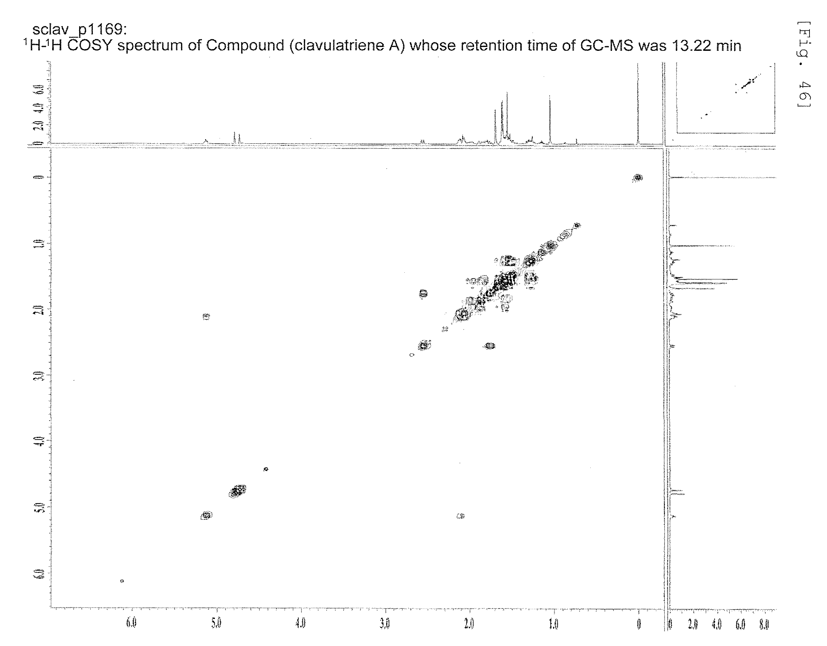

[Fig. 46] sclav_p1169: ¹H-¹H COSY spectrum of Compound (clavulatriene A) whose retention time of GC-MS was 13.22 min

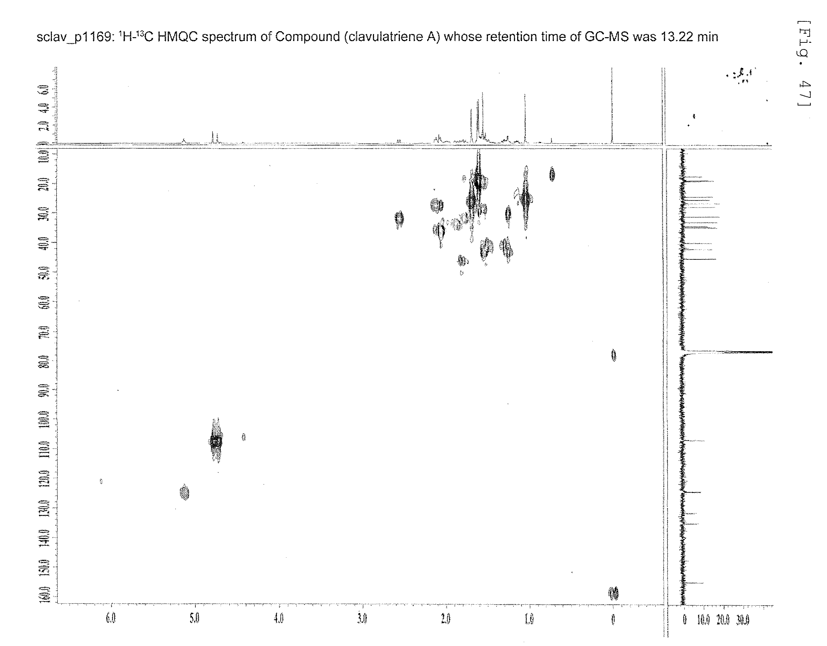

[Fig. 47] sclav_p1169: ¹H-¹³C HMQC spectrum of Compound (clavulatriene A) whose retention time of GC-MS was 13.22 min

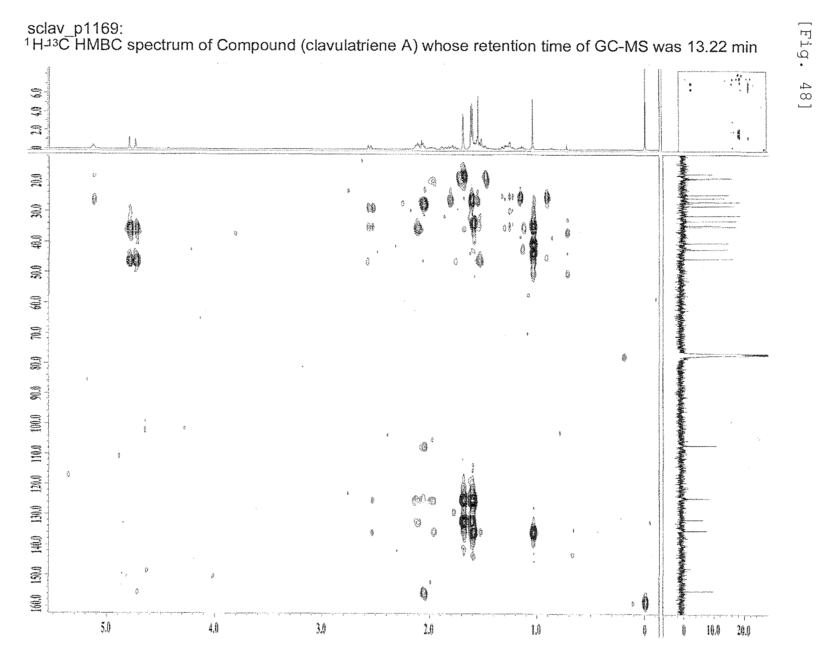

[Fig. 48] sclav_p1169: ¹H-¹³C HMBC spectrum of Compound (clavulatriene A) whose retention time of GC-MS was 13.22 min

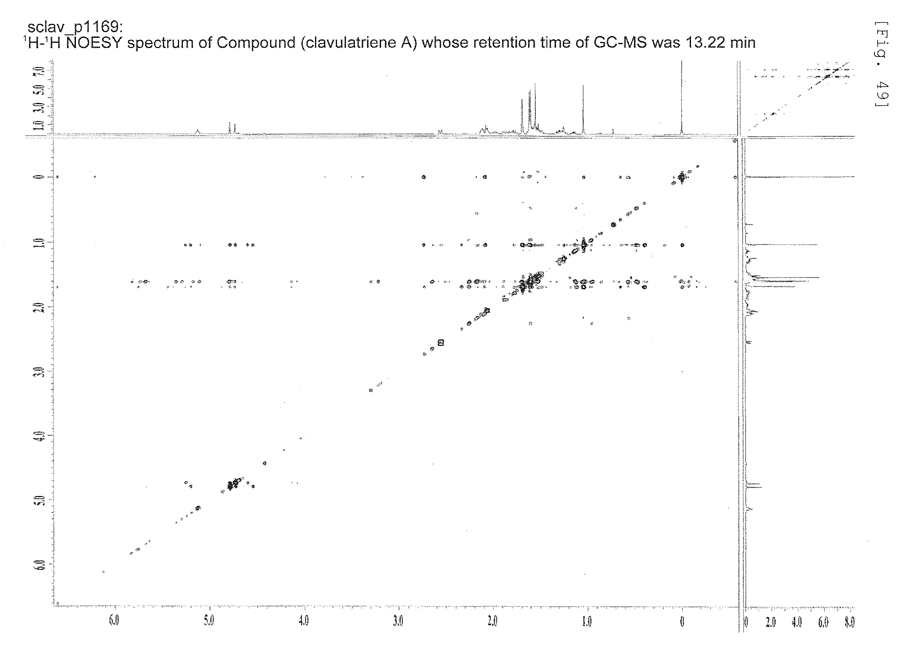

[Fig. 49] sclav_p1169: ¹H-¹H NOESY spectrum of Compound (clavulatriene A) whose retention time of GC-MS was 13.22 min

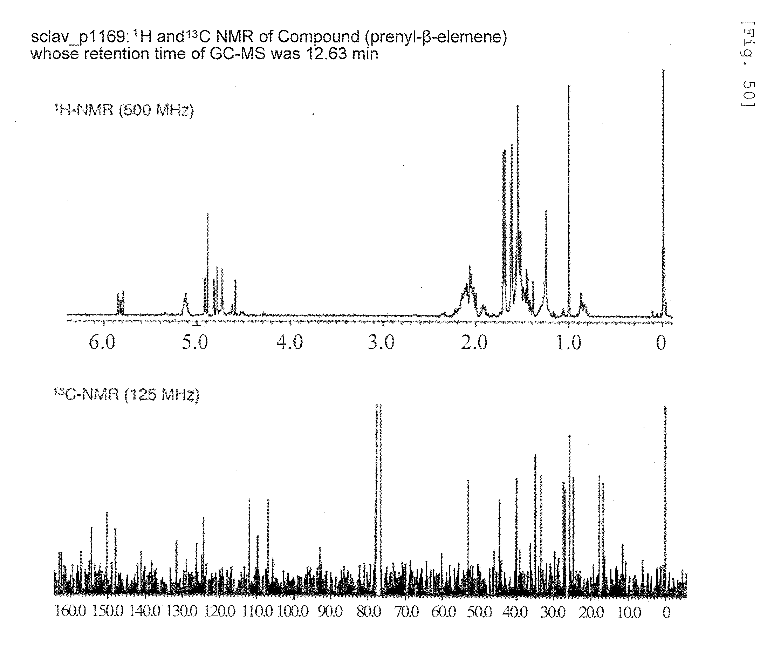

[Fig. 50] sclav_p1169: ¹H and ¹³C NMR of Compound (prenyl-β-elemene) whose retention time of GC-MS was 12.63 min

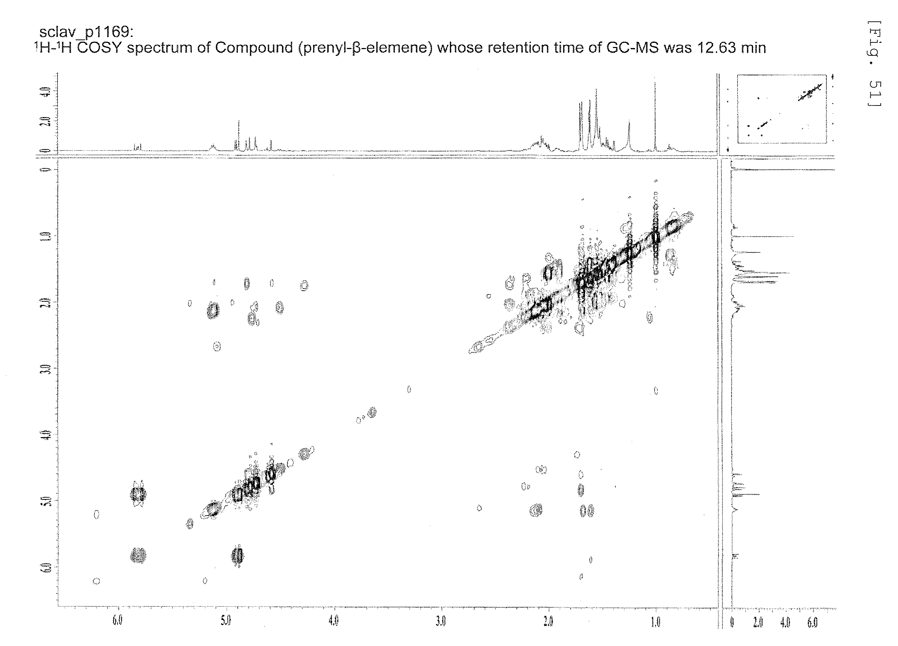

[Fig. 51] sclav_p1169: ¹H-¹H COSY spectrum of Compound (prenyl-β-elemene) whose retention time of GC-MS was 12.63 min

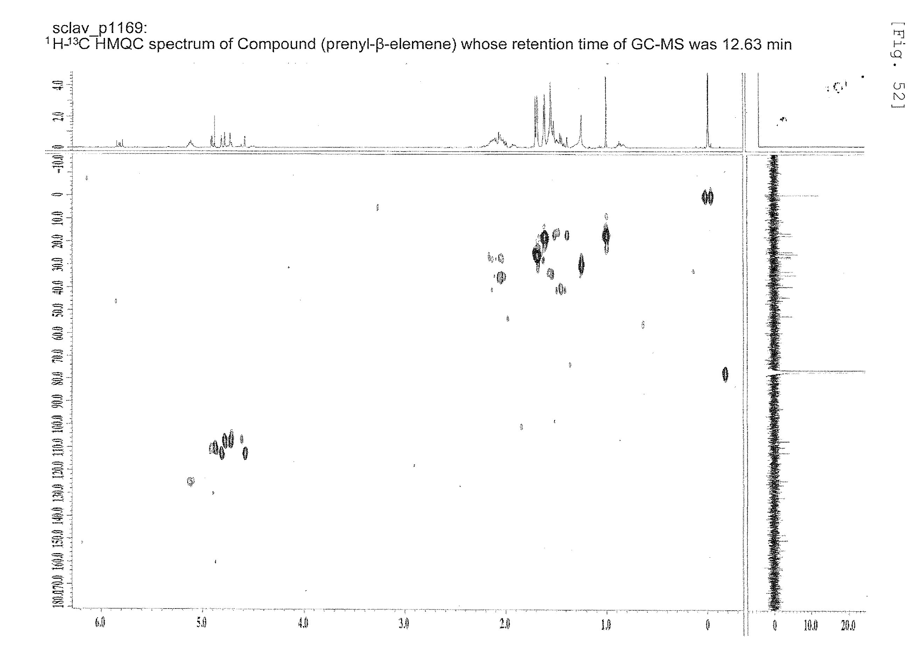

[Fig. 52] sclav_p1169: ¹H-¹³C HMQC spectrum of Compound (prenyl-β-elemene) whose retention time of GC-MS was 12.63 min

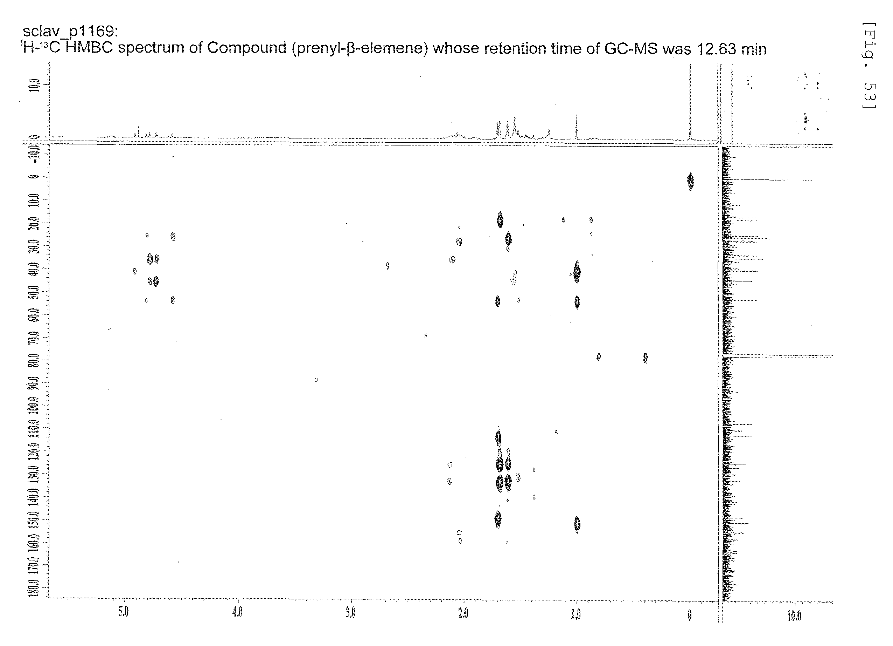

[Fig. 53] sclav_p1169: ¹H-¹³C HMBC spectrum of Compound (prenyl-β-elemene) whose retention time of GC-MS was 12.63 min

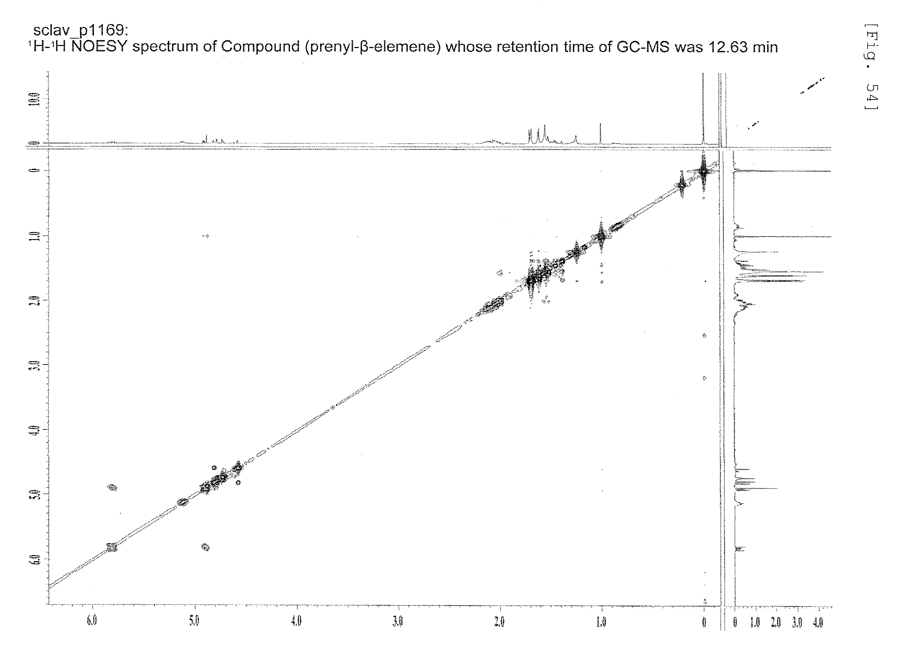

[Fig. 54] sclav_p1169: ¹H-¹H NOESY spectrum of Compound (prenyl-β-elemene) whose retention time of GC-MS was 12.63 min

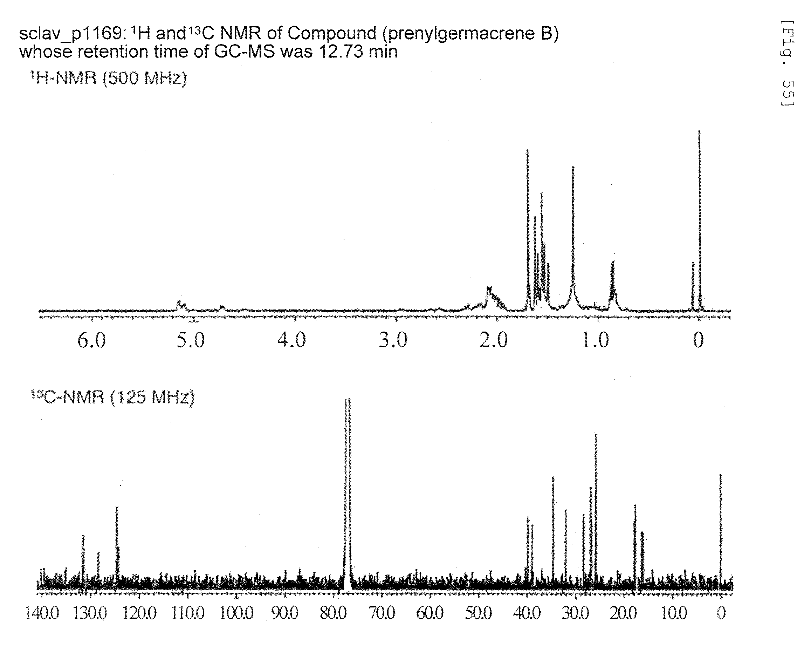

[Fig. 55] sclav_p1169: ¹H and ¹³C NMR of Compound (prenylgermacrene B) whose retention time of GC-MS was 12.73 min

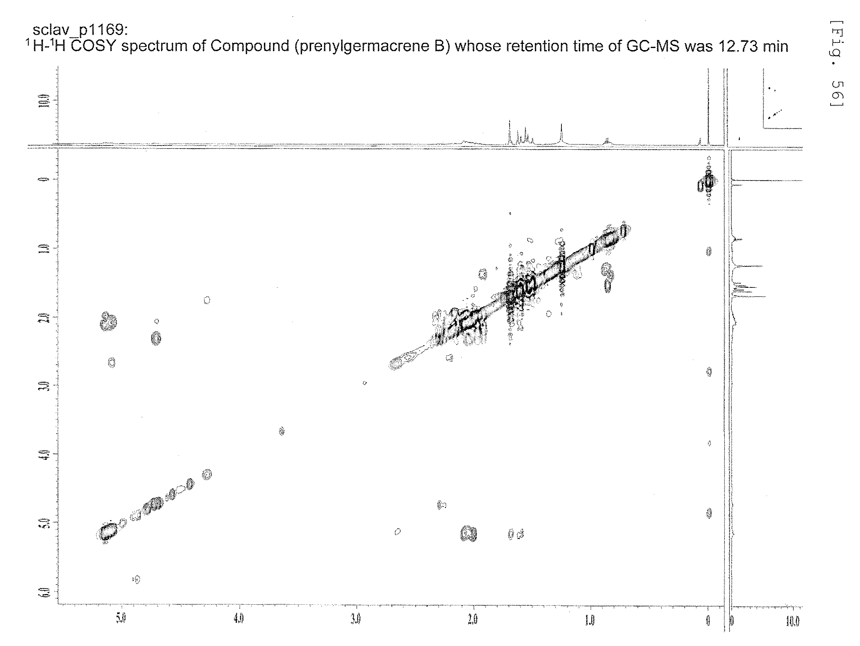

[Fig. 56] sclav_p1169: ¹H-¹H COSY spectrum of Compound (prenylgermacrene B) whose retention time of GC-MS was 12.73 min

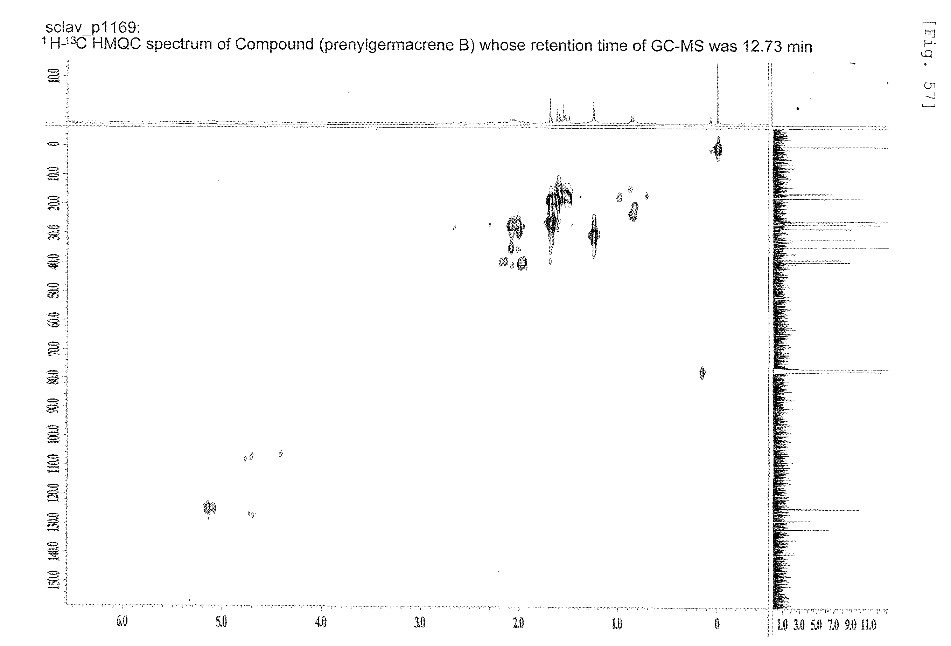

[Fig. 57] sclav_p1169: ¹H-¹³C HMQC spectrum of Compound (prenylgermacrene B) whose retention time of GC-MS was 12.73 min

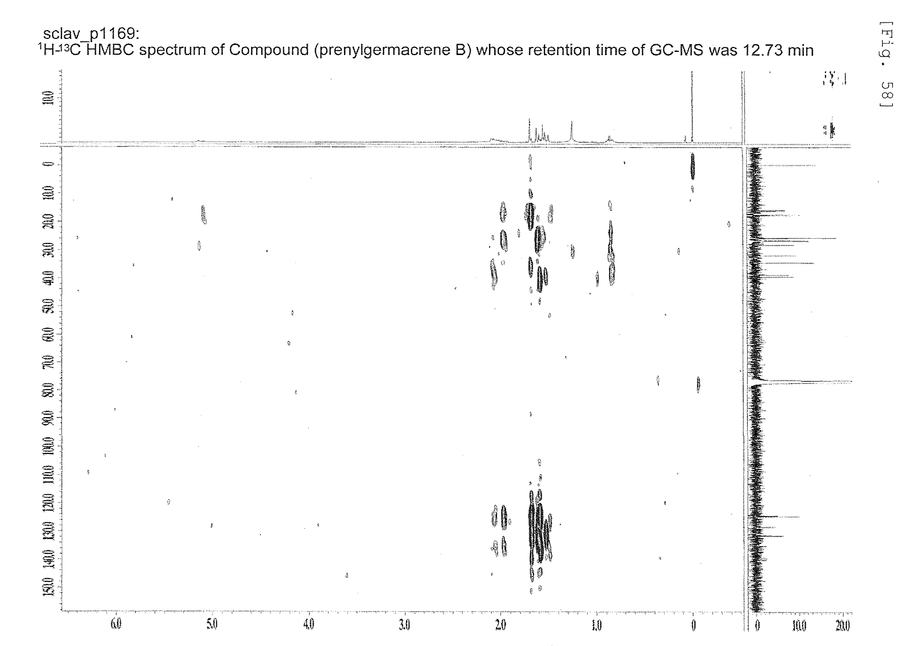

[Fig. 58] sclav_p1169: ¹H-¹³C HMBC spectrum of Compound (prenylgermacrene B) whose retention time of GC-MS was 12.73 min

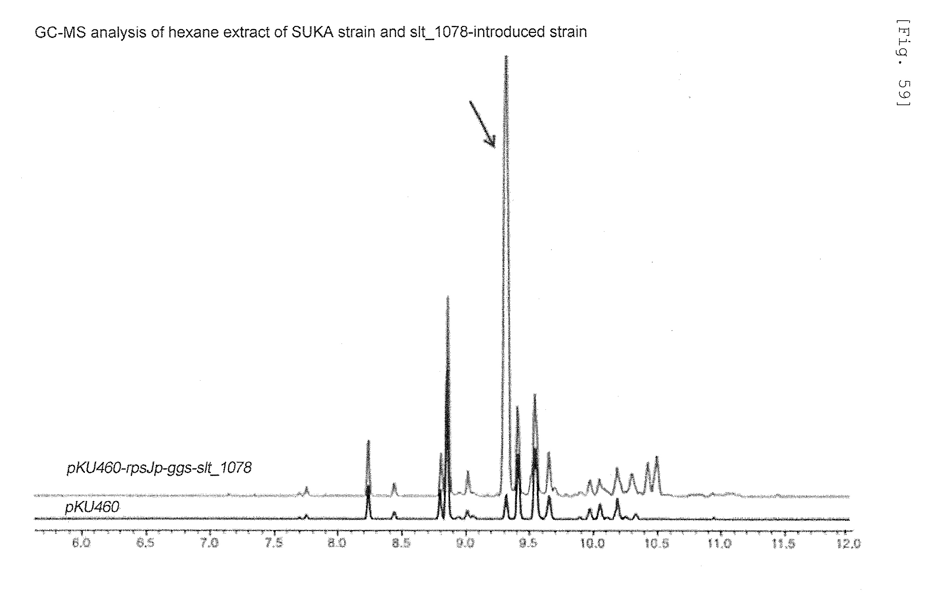

[Fig. 59] GC-MS analysis of hexane extract of SUKA strain and slt_1078-introduced strain

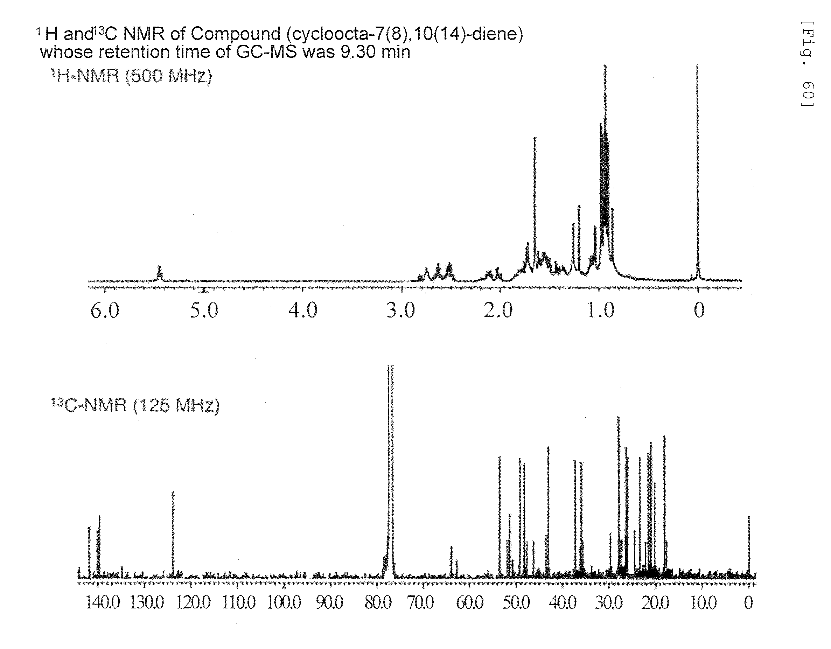

[Fig. 60] ¹H and ¹³C NMR of Compound (cycloocta-7(8),10(14)-diene) whose retention time of GC-MS was 9.30 min

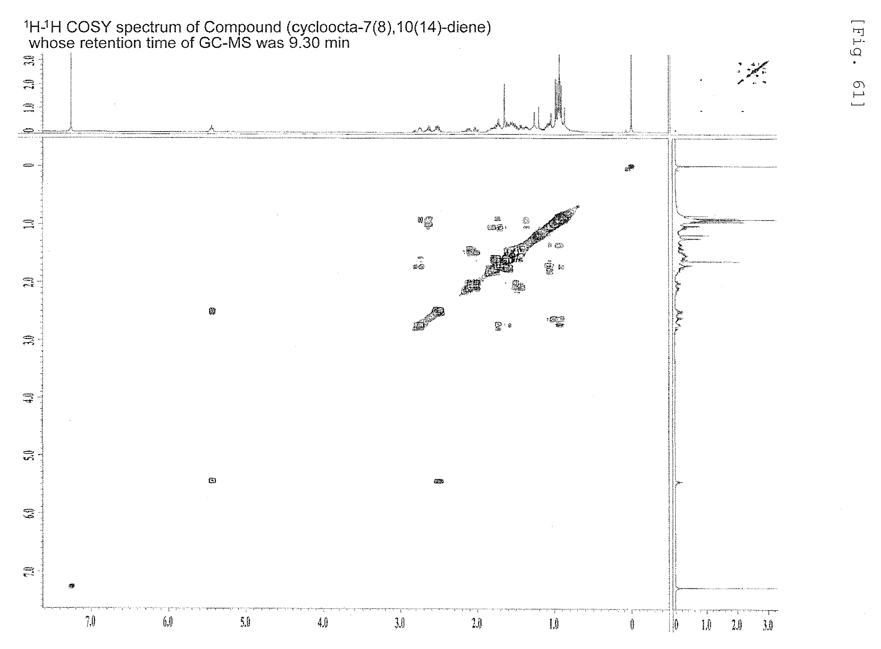

[Fig. 61] ¹H-¹H COSY spectrum of Compound (cycloocta-7(8),10(14)-diene) whose retention time of GC-MS was 9.30 min

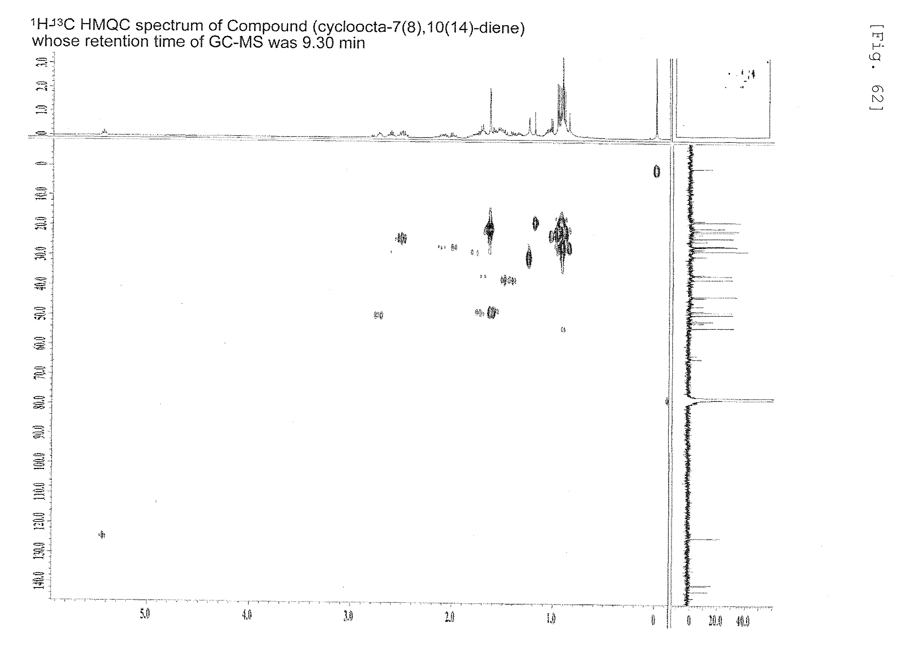

[Fig. 62] ¹H-¹³C HMQC spectrum of Compound (cycloocta-7(8),10(14)-diene) whose retention time of GC-MS was 9.30 min

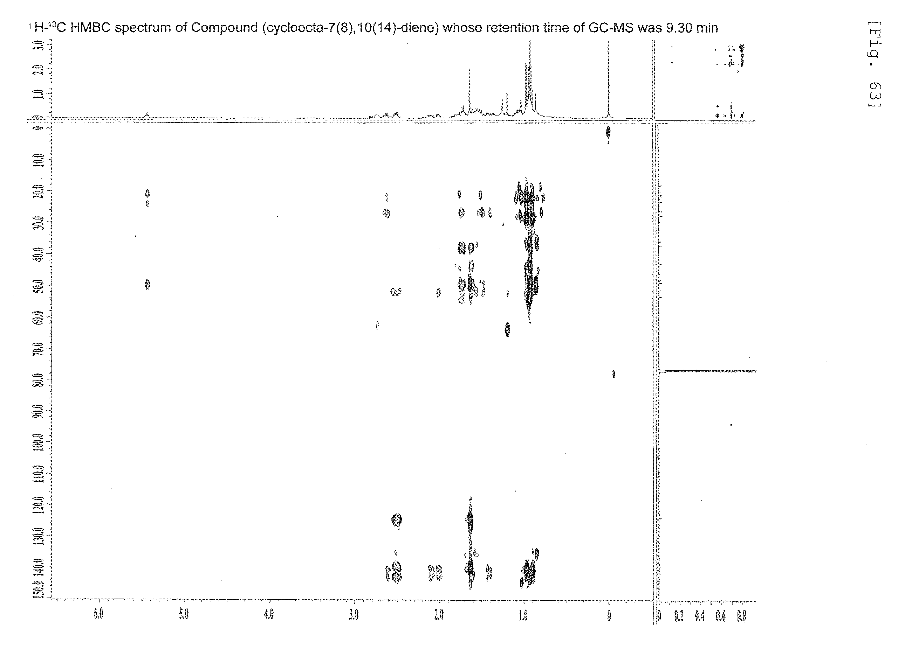

[Fig. 63] ¹H-¹³C HMBC spectrum of Compound (cycloocta-7(8),10(14)-diene) whose retention time of GC-MS was 9.30 min

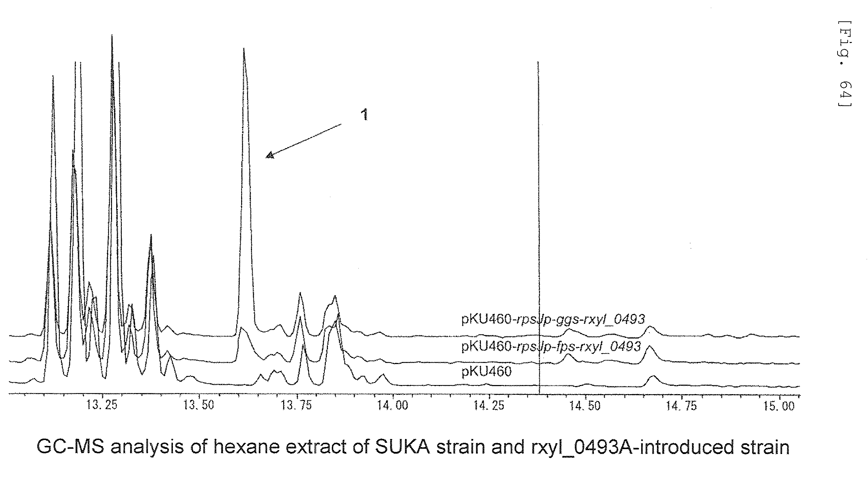

[Fig. 64] GC-MS analysis of hexane extract of SUKA strain and rxyl_0493A-introduced strain

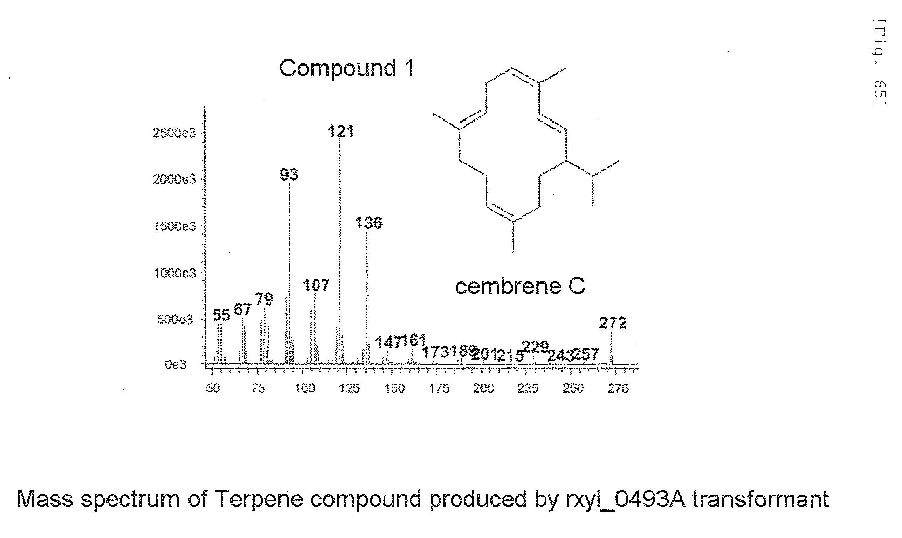

[Fig. 65] Mass spectra of Terpene compound produced by rxyl_0493A transformant

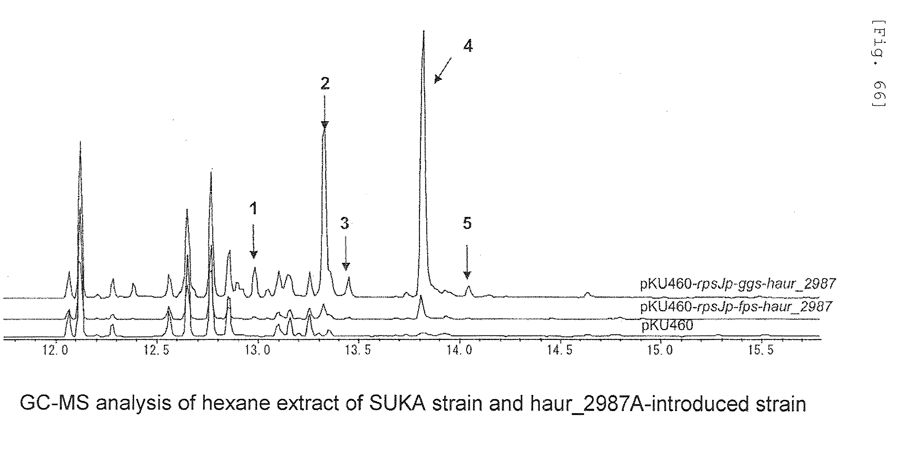

[Fig. 66] GC-MS analysis of hexane extract of SUKA strain and haur_2987A-introduced strain

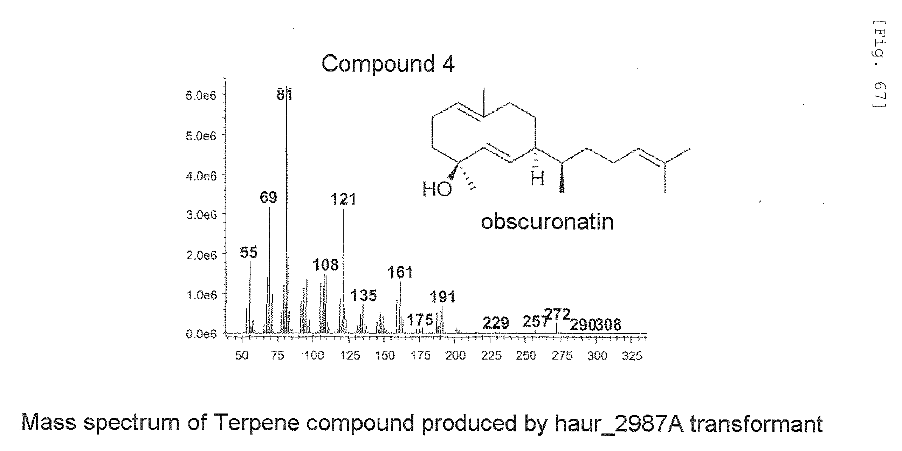

[Fig. 67] Mass spectra of Terpene compound produced by haur_2987A transformant

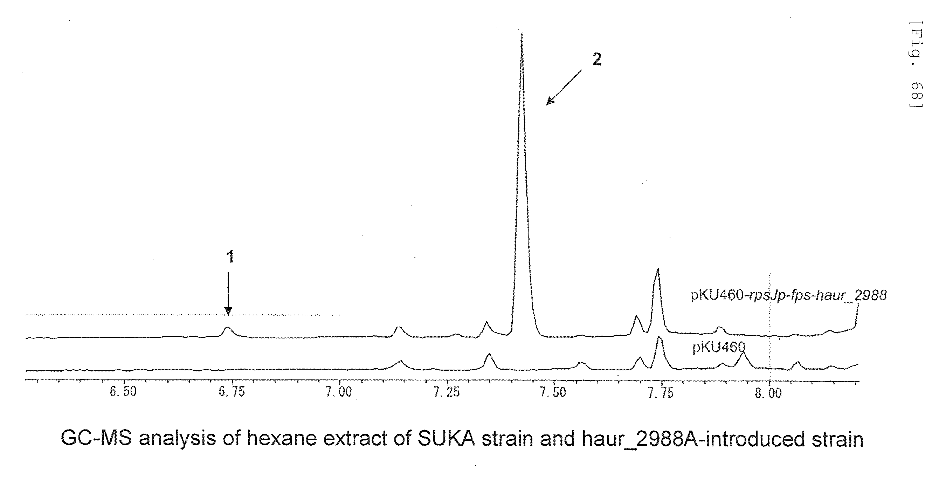

[Fig. 68] GC-MS analysis of hexane extract of SUKA strain and haur_2988A-introduced strain

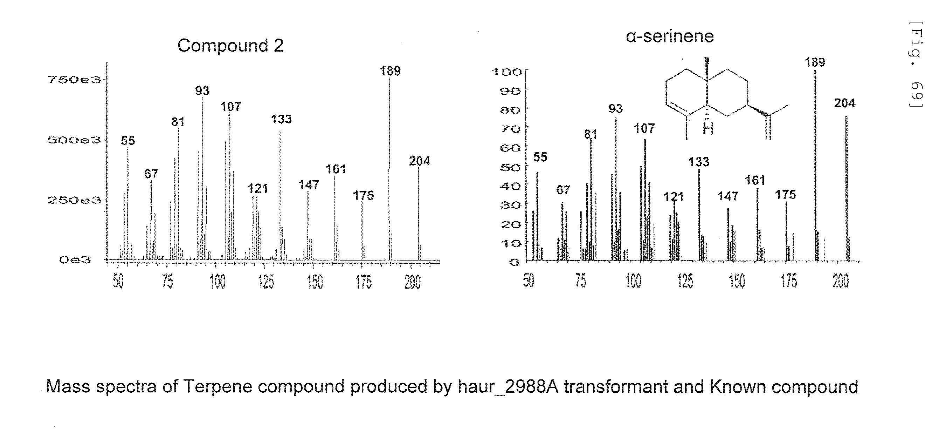

[Fig. 69] Mass spectra of Terpene compound produced by haur_2988A transformant and Known compound

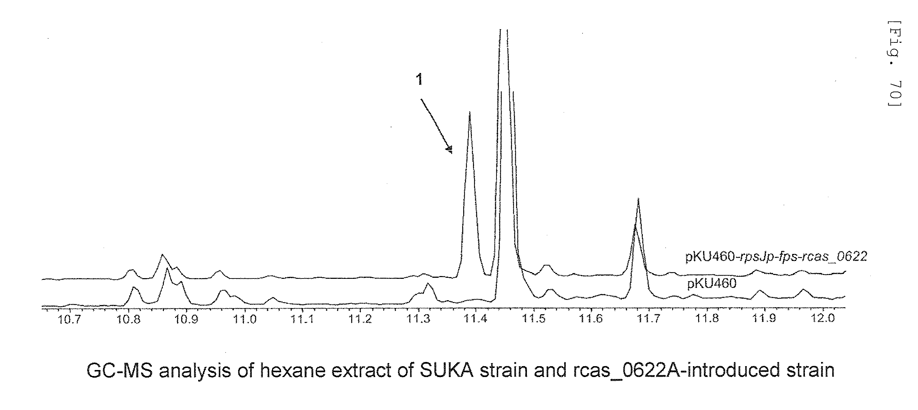

[Fig. 70] GC-MS analysis of hexane extract of SUKA strain and rcas_0622A-introduced strain

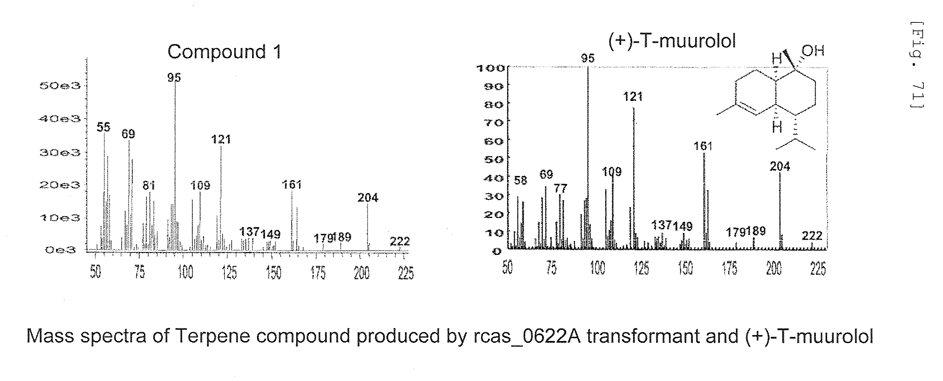

[Fig. 71] Mass spectra of Terpene compound produced by rcas_0622A transformant and (+)-T-muurolol

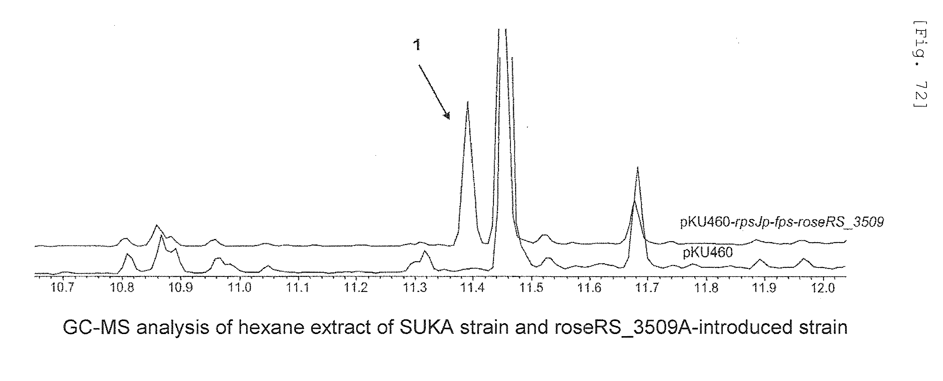

[Fig. 72] GC-MS analysis of hexane extract of SUKA strain and roseRS_3509A-introduced strain

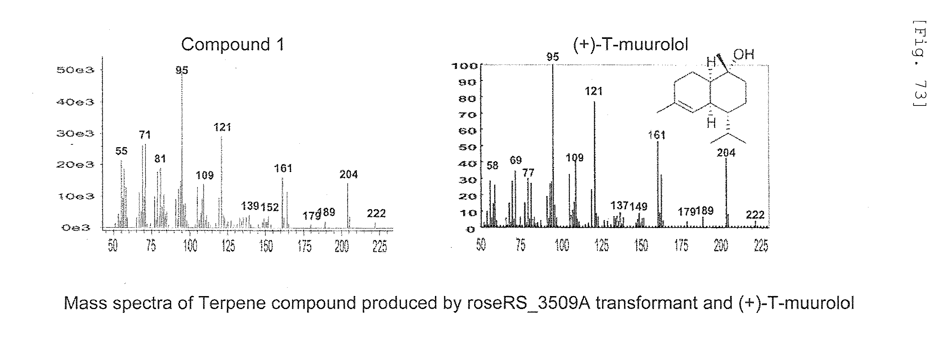

[Fig. 73] Mass spectra of Terpene compound produced by roseRS_3509A transformant and (+)-T-muurolol

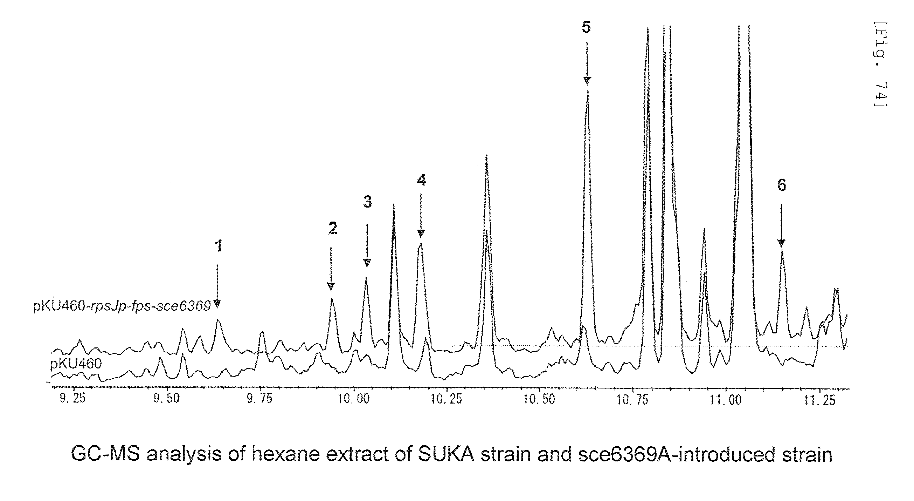

[Fig. 74] GC-MS analysis of hexane extract of SUKA strain and sce6369A-introduced strain

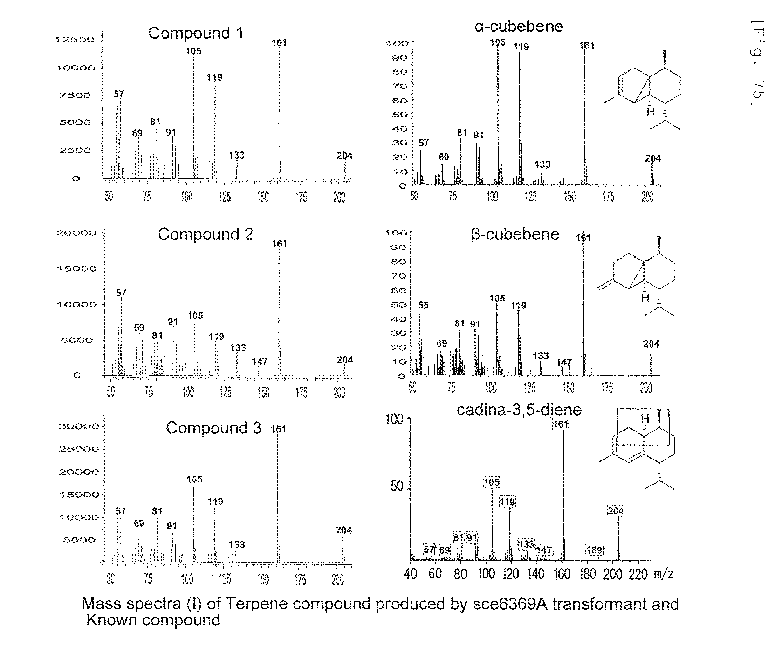

[Fig. 75] Mass spectra (I) of terpene compound produced by sce6369A transformant and Known compound

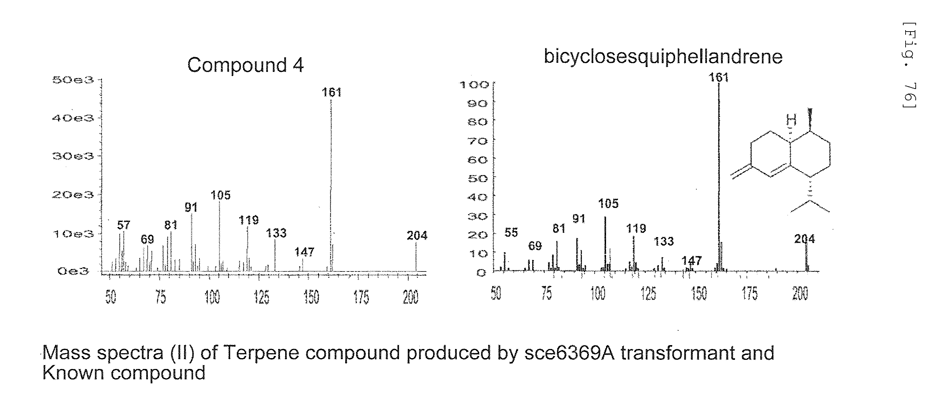

[Fig. 76] Mass spectra (II) of terpene compound produced by sce6369A transformant and Known compound

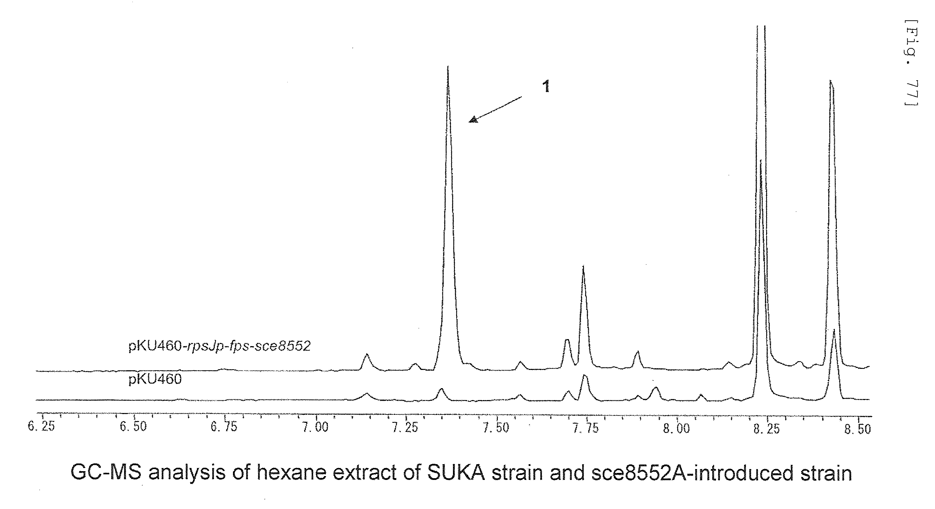

[Fig. 77] GC-MS analysis of hexane extract of SUKA strain and sce8552A-introduced strain

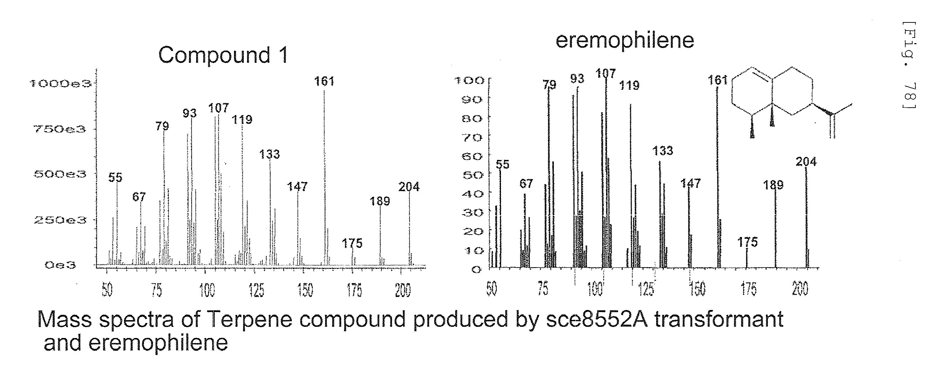

[Fig. 78] Mass spectra of Terpene compound produced by sce8552A transformant and eremophilene

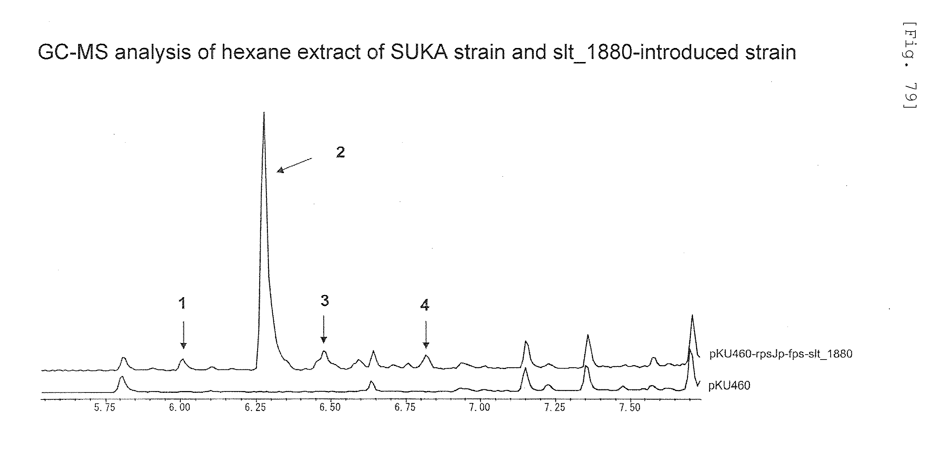

[Fig. 79] GC-MS analysis of hexane extract of SUKA strain and slt_1880-introduced strain

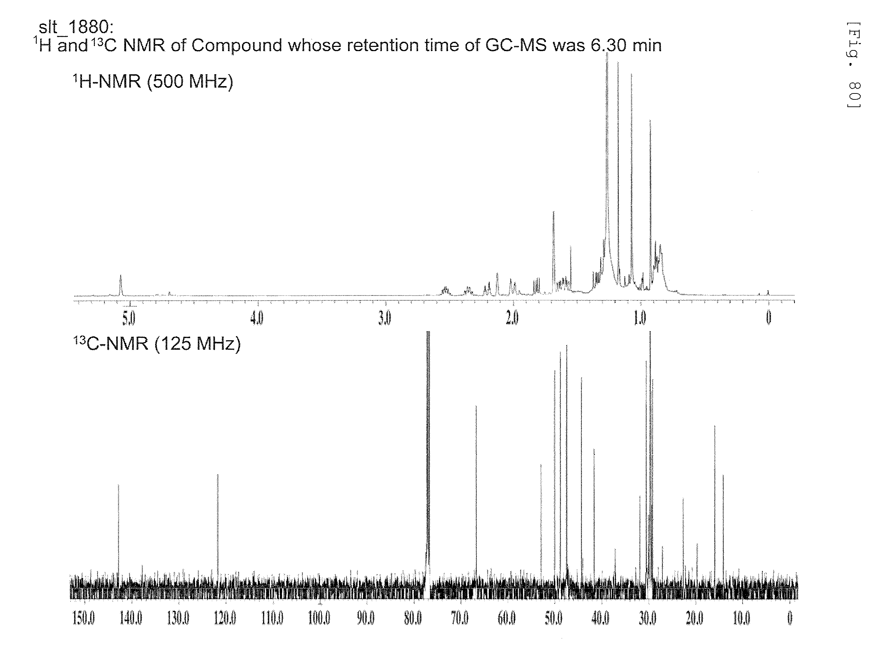

[Fig. 80] slt_1880: ¹H and ¹³C NMR of Compound whose retention time of GC-MS was 6.30 min

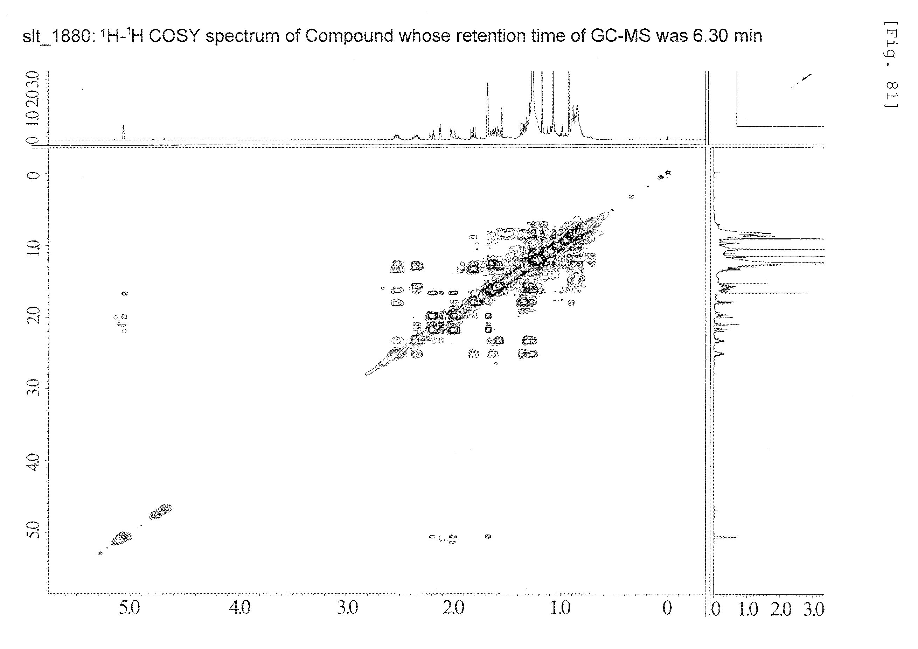

[Fig. 81] slt_1880: ¹H-¹H COSY spectrum of Compound whose retention time of GC-MS was 6.30 min

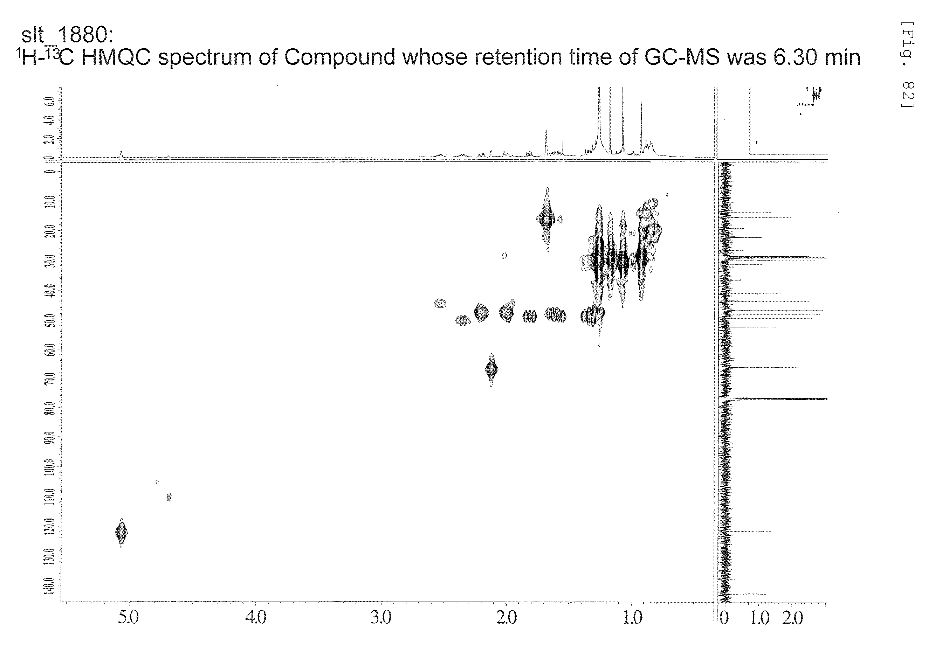

[Fig. 82] slt_1880: ¹H-¹³C HMQC spectrum of Compound whose retention time of GC-MS was 6.30 min

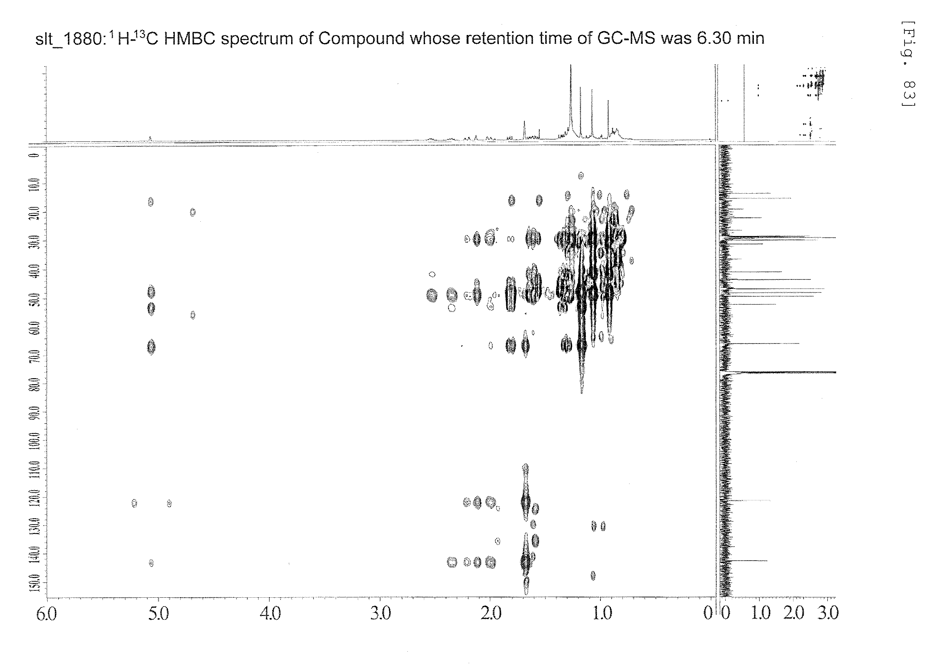

[Fig. 83] slt_1880: ¹H-¹³C HMBC spectrum of Compound whose retention time of GC-MS was 6.30 min

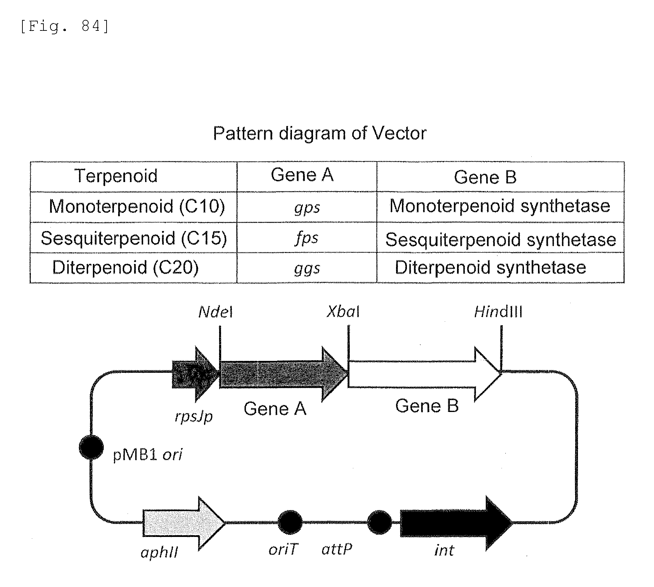

[Fig. 84] Pattern diagram of Vector

In the present invention, the term "terpenoid synthetase" refers to an enzyme capable of synthesizing a terpenoid compound from a corresponding terpenoid precursor, and is used as a generic term that includes monoterpenoid synthetase, sesquiterpenoid synthetase, and diterpenoid synthetase.

Monoterpenoid synthetase, sesquiterpenoid synthetase, and diterpenoid synthetase are able to synthesize a monoterpenoid compound, a sesquiterpenoid compound, and a diterpenoid compound respectively from the corresponding terpenoid precursor: geranyl diphosphate (GPP), farnesyl diphosphate (FPP), or geranylgeranyl diphosphate (GGPP).

In the present invention, the term "terpenoid synthetase gene" refers to a gene encoding terpenoid synthetase, and is used as a generic term that includes a monoterpenoid synthetase gene, a sesquiterpenoid synthetase gene, and a diterpenoid synthetase gene.

Monoterpenoid synthetase gene, sesquiterpenoid synthetase gene, and diterpenoid synthetase gene refer to genes encoding monoterpenoid synthetase, sesquiterpenoid synthetase, or diterpenoid synthetase, respectively.

### Host cell

The host used in the method of the present invention is not particularly limited as long as the host cell is able to express a gene of interest. Preferred examples of the host cell include actinomycetes (for example, Streptomyces) and a mutant thereof, and *E. coli* and a mutant thereof. It is preferable that the host cell has an ability to synthesize isopentenyl diphosphate (IPP) and dimethylallyl diphosphate (DMAPP) which are starting materials for the terpenoid precursors. Many of actinomycetes are originally able to synthesize terpenoid compounds and have capabilities necessary to synthesize a terpenoid compound. Therefore actinomycetes are a preferred host cell in the present invention. Mutants of *E. coli* which are suitable for synthesizing terpenoids have been developed by using gene-recombination technology (Non-patent document 11). Such mutants of *E.coli* are also a preferred host in the present invention.

Further preferred examples of the host cell include *Streptomyces avermitilis* and a mutant thereof. In the present invention, preferred examples of the mutant of *Streptomyces avermitilis* include a series of SUKA (for example SUKA 1 to 17 (Non-patent document 8)), which is prepared by removing the majority of endogenous biosynthetic gene clusters from *S. avermitilis.* Especially preferred examples include but not limited to SUKA 17. The wild strain of *Streptomyces avermitilis* produces terpenoid compounds such as avermectin, therefore, it may necessary to remove undesired terpenoid compounds in order to obtain a desired terpenoid compound. SUKA 17 does not produce any terpenoid compound due to lack of any terpenoid synthetase gene. Using SUKA 17 allows a terpenoid compound of interest to be given without any step of removing undesired terpenoid compounds.

### Transformation

In the method of the present invention, a gene of interest may be introduced into a host cell by using a genetic recombination technology known to a person skilled in the art. Examples of the gene introduction include a method wherein a DNA fragment is directly introduced, the conjugative transfer, and a method wherein a vector such as transposon, phage vector or viral vector is used.

In the method of the present invention, a gene of interest being integrated into a chromosome, a plasmid, a plastid or a mitochondrial DNA may be kept in a host cell to transform the host cell. Preferably, a gene of interest is integrated into a chromosome of the host cell.

In the method of the present invention, preferably, a vector is used to introduce a gene of interest into the host cell.

In the method of the present invention, a vector to be used for transforming a host cell is not particularly limited, and may be a self-replicating vector or an integrating vector which induces homologous recombination.

In the method of the present invention, when a gene of interest is introduced into a chromosome of a host cell, the gene introduction method is not particularly limited but, for example, an integrating vector may be used.

It is desirable that vectors have a so-called regulatory sequence such as operator, promoter, terminator, and enhancer, which controls the expression of a gene of interest, and a selection marker.

The vector may appropriately be selected depending on a host to be transformed. For example, when actinomycetes are used as a host, a vector which may work in actinomycetes is selected. And, when *E. coli* is used as a host, a vector which may work in *E.coli* is selected.

In the method of the present invention, a vector to be used to transform a host cell can be produced by a method known to a person skilled in the art. For example, a terpenoid precursor synthetase gene and/or a terpenoid synthetase gene are amplified by using appropriate primers and restriction enzyme sites are added to the both ends, and then the resulting fragment is inserted into an original vector cleaved by the restriction enzymes to give a vector to be used to transform a host cell. An expression cassette wherein a promoter to express the gene(s) of interest in a vector is inserted upstream of the gene(s) of interest may be used.

The vector may be introduced into a host cell by a technique well known to a person skilled in the art, for example, a calcium chloride method, protoplast transformation, conjugative transfer, electroporation.

When a host cell is a bacterium belonging to the genus Streptomyces, for example, a chromosomal integration vector pKU460 (Non-patent document 5, Non-patent document 12) can be used.

The chromosomal integration vector pKU460 may be introduced into a host cell by a known method. Preferred examples include PEG mediated protoplast transformation (Non-patent document 13) and conjugative transfer.

### Promoter

In the production method of a terpenoid compound of the present invention, preferably, a gene of interest is introduced into an adjacent downstream of a promoter capable of controlling a transcription of the gene.

The promoter in the present invention is not particularly limited as long as the promoter is capable of controlling a transcription of the gene(s) of interest (terpenoid precursor synthetase gene and/or terpenoid synthetase gene) in a host cell.

In the case where a host cell is a bacterium belonging to the genus Streptomyces, a preferred promoter is rpsJ (SEQ ID NO:37), which is a constitutive strong expression promoter derived from *S. avermitilis* (Non-patent document 5).

**[Table 1]**

| **Table 1 (SEQ ID NO:37)** |
|---|
| 1 CACGGACATG TTCTGTTCAC CCGGAAGGGG GCTGCCGGGA TGAGCACATG 51 TGCTCACGTT CCCGGCCGGC CGGTGGCACC GGGGTCGGAC GCGTGTGCCC 101 CGGGCGGATC GGTGTGCGAC CCTTGCTGAG TGCCCGGGGT TTGACCTGCG 151 TCACCCTCGG GGTACGATCC TCGGCCCGAT TGGCCAGGCC CGTGCCCCGT 201 ATGGCAGACT GTCGGAGTTG CTCGGTCGAG TGCCGATGCT GTGCGCCTCC 251 CGCCGGGAGG ACTGGAAGCG AGTCCCACAG TACTCGTCGC CCGATCTGTC 301 GAAAGGCAGC GCTGGGGCGG ACGTACGGGA ATCTTCCGGG AAGTTTCAGC 351 GGGGTGCCGA CCAGGCGCCC GGTGGGTGTT GTGCCCCCGG TCCTGCGGTT 401 TTTTTCGGGC CGTGTCCCTC GGTAGGGAAA TCCATATCGG ATATCTCTGT 451 CAGCAAGAGC GCGACACACC CGACCGCGTG GGTCGGAGGT CAGGACGCGA 501 ACCCCCGGGC TCCAGAGCGT TTTATCGAGA CGAAGGACTA CTGAGTAC |

In the method of the present invention, in order to introduce a gene of interest into a host cell, an expression cassette comprising a promoter, a terpenoid precursor synthetase gene and/or a terpenoid synthetase gene may be used.

In the method of the present invention, an expression cassette means a nucleic-acid construct comprising a promoter and a gene of interest, which may comprise a terminator sequence, an intron, a spacer sequence, an enhancer sequence and the like.

In the production method of a terpenoid compound of the present invention, preferred examples of the expression cassette include:
For a synthesis of monoterpenoid: rpsJ-sclav_p0982, rpsJ-gps-sclav_p0982;
For a synthesis of sesquiterpenoid:
   rpsJ-sclav_p0985, rpsJ-sclav_p1407, rpsJ-sgr_6065, rpsJ-sven_0552, rpsJ-sk_3051, rpsJ-slt_1718, rpsJ-mmar_3220, rpsJ-mmar_3220A, rpsJ-haur_2988, rpsJ-haur_2988A, rpsJ-rcas_0622, rpsJ-rcas_0622A, rpsJ-roseRS_3509, rpsJ-roseRS_3509A, rpsJ-sce6369, rpsJ-sce6369A, rpsJ-sce8552, rpsJ-sce8552A, rpsJ-slt_1880,
   rpsJ-fps-sclav_p0985, rpsJ-fps-sclav_p1407, rpsJ-fps-sgr_6065, rpsJ-fps-sven_0552, rpsJ-fps-sk_3051, rpsJ-fps-slt_1718, rpsJ-fps-mmar_3220, rpsJ-fps-mmar_3220A, rpsJ-fps-haur_2988, rpsJ-fps-haur_2988A, rpsJ-fps-rcas_0622, rpsJ-fps-rcas_0622A, rpsJ-fps-roseRS_3509, rpsJ-fps-roseRS_3509A, rpsJ-fps-sce6369, rpsJ-fps-sce6369A, rpsJ-fps-sce8552, rpsJ-fps-sce8552A, rpsJ-fps-slt_1880;
For a synthesis of diterpenoid:
   rpsJ-sclav_p0765, rpsJ-sclav_p1169, rpsJ-slt_1078, rpsJ-rxyl_0493, rpsJ-rxyl_0493A, rpsJ-haur_2987, rpsJ-haur_2987A,
   rpsJ-ggs-sclav_p0765, rpsJ-ggs-sclav_p1169, rpsJ-ggs-slt_1078, rpsJ-ggs-rxyl_0493, rpsJ-ggs-rxyl_0493A, rpsJ-ggs-haur_2987, rpsJ-ggs-haur_2987A.

In the above expression cassettes, gps (SEQ ID NO:38) is a geranyl diphosphate synthetase gene, fps (SEQ ID NO: 40) is a farnesyl diphosphate synthetase gene, ggs (SEQ ID NO:42) is a geranylgeranyl diphosphate synthetase gene.

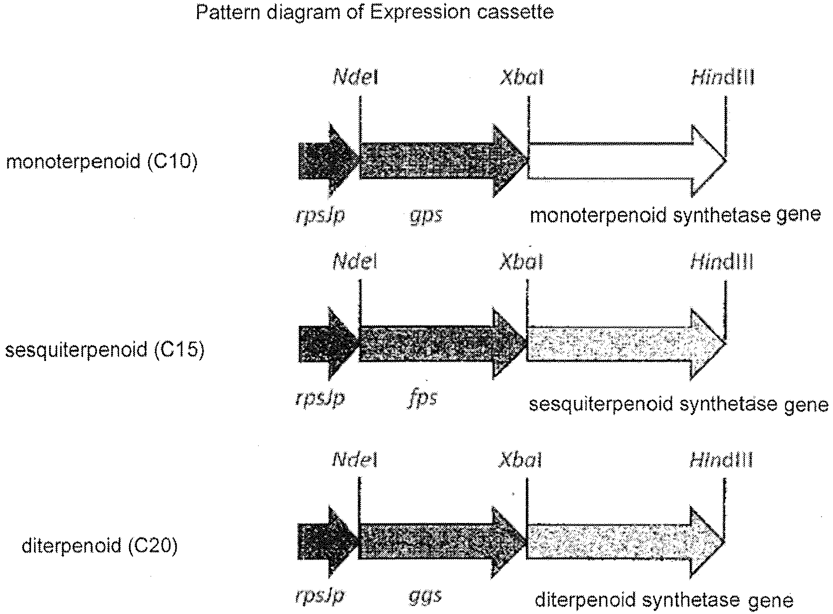
Pattern diagram of Expression cassette

NdeI XbaI HindIII
monoterpenoid (C10)
rpsJp gps monoterpenoid synthetase gene

NdeI XbaI HindIII
sesquiterpenoid (C15)
rpsJp fps sesquiterpenoid synthetase gene

NdeI XbaI HindIII
diterpenoid (C20)
rpsJp ggs diterpenoid synthetase gene

### Cultivation

A culture condition for a transformant may optionally be determined depending on the type of host cell and the type of terpenoid to be produced. Further, the culture condition may vary depending on an object of the cultivation (the cultivation is performed to synthesize a terpenoid, or the cultivation is performed to produce a terpenoid synthetase). A person skilled in the art can optionally determine the culture condition on the basis of a well-known technique.

A medium for synthesizing a terpenoid is not particularly limited as long as the terpenoid can be synthesized by culturing a transformant in the medium. In the case where the transformant has a low ability to synthesize isopentenyl diphosphate (IPP) and/or dimethylallyl diphosphate (DMAPP), it is preferred to add a precursor which may be converted to IPP and/or DMAPP into the medium. Said precursor is a MEP/DOXP pathway intermediate in the formation of 1-hydroxy-2-methyl-2-butenyl 4-diphosphate from glyceraldehyde 3-phosphate.

In the case where terpenoid precursor synthetase gene is not used, it is preferred to add a compound (such as farnesol) which may be converted to the corresponding terpenoid precursor into the medium.

In the case where a host cell is a bacterium belonging to the genus Streptomyces, preferred examples of the medium include an inorganic salt synthetic medium such as a minimal medium (in the medium 1 L, L-asparagine 0.5 g; K₂HPO₄ 0.5 g; MgSO₄ · 7H₂O 0.2 g; FeSO₄ · 7H₂O 0.01 g; and glucose 10 g are contained) and a nutrient-rich medium such as Tryptone soya broth (manufactured by Difco). Further, preferred examples of the medium include a medium wherein glucose 60 g, calcium carbonate 5 g, sodium chloride 2 g, dipotassium hydrogen phosphate 0.5 g, 0.5% iron sulfate heptahydrate 10 mL, 0.5% zinc sulfate heptahydrate 10 mL, 0.5% manganese sulfate tetrahydrate 10 mL, 1% magnesium sulfate heptahydrate 10 mL, ammonium sulfate 2 g, and yeast extract 2 g are contained in the medium 1 L, and another medium wherein glucose 5 g, defatted soy flour 15 g, and yeast extract (manufactured by Difco) 5 g are contained in the medium 1 L.

A medium for producing a terpenoid synthetase is not particularly limited as long as the terpenoid synthetase is produced by culturing transformant in the medium. A person skilled in the art can optionally select the medium on the basis of a well-known art. The obtained bacterial cells; the obtained culture supernatant; the preparation from the culture which is prepared by crush, lysis or the like of the bacterial cells; or the terpenoid synthetase purified from the bacterial cells by a known technique may be applied to the terpenoid precursor to give the terpenoid compound.

A transformant can be cultivated by a conventional culture method such as a shaking culture and a continuous culture. The culture condition can be optionally determined depending on the culture method and the like. The culture condition is not particularly limited as long as the transformant grows and a terpenoid synthetase is produced under the culture condition. Usually, the initial pH of the medium is between 4 and 10, preferably between 6 and 8; and the culturing may be carried out at a temperature suitable for a growth of a host cell. The culture time is not particularly limited as long as a terpenoid compound is synthesized, or a terpenoid synthetase is produced. The culturing is usually carried out for 1 day to 20 days, preferably 3 days to 14 days.

### Terpenoid precursor synthetase gene

In the present invention, a terpenoid precursor synthetase refers to an enzyme which synthesizes geranyl diphosphate (GPP), farnesyl diphosphate (FPP), or geranylgeranyl diphosphate (GGPP) from dimethylallyl diphosphate (DMAPP) and isopentenyl diphosphate (IPP), and is used as a generic term that includes geranyl diphosphate synthetase, farnesyl diphosphate synthetase, and geranylgeranyl diphosphate synthetase.

In the present invention, an enzyme which synthesizes geranyl diphosphate (GPP), which has 10 carbon atoms, from one dimethylallyl diphosphate (DMAPP) and one isopentenyl diphosphate (IPP) is referred to as a geranyl diphosphate synthetase, and a gene thereof is referred to as a geranyl diphosphate synthetase gene.

In the present invention, an enzyme which synthesizes farnesyl diphosphate (FPP), which has 15 carbon atoms, from one dimethylallyl diphosphate (DMAPP) and two isopentenyl diphosphates (IPP) is referred to as a farnesyl diphosphate synthetase, and a gene thereof is referred to as a farnesyl diphosphate synthetase gene.

In the present invention, an enzyme which synthesizes geranylgeranyl diphosphate (GGPP), which has 20 carbon atoms, from on dimethylallyl diphosphate (DMAPP) and three isopentenyl diphosphates (IPP) is referred to as a geranylgeranyl diphosphate synthetase, and a gene thereof is referred to as a geranylgeranyl diphosphate synthetase gene.

In the method of the present invention, a transformant wherein a terpenoid precursor synthetase gene and a terpenoid synthetase gene are both introduced into the same host cell is preferably used. As for a transcription, the terpenoid precursor synthetase gene and the terpenoid synthetase gene may be each controlled by different promoters, or may be each controlled by the same promoter.

rpsJ (SEQ ID NO:37) can be used as a promoter for a terpenoid precursor synthetase gene alone and can also be used as a promoter for a terpenoid synthetase gene alone. rpsJ can be used as a common promoter which controls both terpenoid precursor synthetase gene and terpenoid synthetase gene. In the case where both terpenoid precursor synthetase gene and terpenoid synthetase gene are linked to rpsJ, the order of the terpenoid precursor synthetase gene and the terpenoid synthetase gene is not limited.

In the method of the present invention, a geranyl diphosphate synthetase gene may be used in combination with a monoterpenoid synthetase gene.

A preferred geranyl diphosphate synthetase gene is the gene encoding the protein of the amino acid sequence described in Table 2 (SEQ ID NO: 39), more preferably gps (SEQ ID NO:38) described in Table 2.

A geranyl diphosphate synthetase gene of the present invention includes the gene encoding a protein consisting of an amino acid sequence having 80% or more, 90% or more, 95% or more, or 97% or more identity to the amino acid sequence (SEQ ID NO:39) described in Table 2. For example, the gene consisting of a base sequence having 80% or more, 90% or more, 95% or more, or 97% or more identity to gps (SEQ ID NO:38) is included.

**[Table 2]**

| Table 2 |
|---|
| gps (geranyl diphosphate synthetase gene: for synthesis of monoterpenoid (C10)) derived from Streptomyces sp. |
| Base sequence (SEQ ID NO:38) |
| ATGACCACCGACACCGGCCAGGACGCCGTCGGCCTCGCCCGCCGCACCGGCGCCGATC TGCTGCACCGCGTGGAGGACCGGCTGCGCTCCCTCCTCGCGGTCGAGCGCGACGCCTG GGCCGCGGTGCACGAACAGGCGGTCGTCCCCGTCGACGCGCTGTCCGAGCTGATCGCG TCGGGCGGCAAGCGCATCCGGCCGGCCTTCTGCATCACCGGCTATCTCGCGGCCGGCG GCGATCCCGCCGAGCCCGGGATCGTGGCGGCCGGCGCGGCTCTGGAGATGCTCCACCT GTCGGCCCTCGTCCACGACGACGTCCTCGACGACTCCAGCAGCCGGCGCGGAGTGCCG ACCGTGCACACACAGGCCATGGCGCTGCACGAGAGCAGCGGATGGCAGGGCGAGCCGC GCAGGTACGGCGAGGGCGTGGCGATCCTCGTCGGCGACCTCGCGCTCGTCTACTCCGA GGAACTGATGGCCGAGGCGCCGCGGCGGGTGCTCCCGGAGTGGAACAAGCTGCGCTCC GAAGTGATGATCGGCCAGTACATGGACGTCCACGCGGCCGCCGAGTTCTCGGTCGACC CCCGAAGCTCCCGACTGATCGCCAGGATCAAGTCCGGCCGCTACACCATCCACCGGCC CCTCGTGGTGGGCGCGAAACGCGGGCGGGCCGAGGCGACCTCGCCCCCGCCCTGGA GGAGTACGGCGAGGCGGTCGGCGAGGCGTTCCAGCTCCGCGACGACCTGCTGGACGCT TCGGCGACTCCCGCGGAGACCGGCAAGCCCACCGGCCTCGACTTCACCCAGCACAAGA TGACGCTGCTGCTGGGCTGGGCGATGCAGCGCGACGAGCACATCCACACCCTCGTCAC GGAGCCGGGCCACACCCCCGACGAGGTGCGCCGCCGCCTCCTGGACACGGGCGTTCCC GCCGACGTCGAACGGCACATCGCGGGCCTCGTCGAGCGGGGCTGCAAGGCCATCGCCG ACGCGCCCGTCGACCAGGTGTGGCGCGGCGAACTGGCGGCCATGGCCGGACGGGTCGC CTACCGCACCGCCTGA |

| Amino acid sequence (SEQ ID NO:39) |
|---|
| MTTDTGQDAVGLARRTGADLLHRVEDRLRSLLAVERDAWAAVHEQAVVPVDALSELIASGGK RIRPAFCITGYLAAGGDPAEPGIVAAGAALEMLHLSALVHDDVLDDSSSRRGVPTVHTQAMAL HESSGWQGEPRRYGEGVAILVGDLALVYSEELMAEAPRRVLPEWNKLRSEVMIGQYMDVHAA AEFSVDPRSSRLIARIKSGRYTIHRPLVVGAKRGRGRGDLAPALEEYGEAVGEAFQLRDDLLDA SATPAETGKPTGLDFTQHKMTLLLGWAMQRDEHIHTLVTEPGHTPDEVRRRLLDTGVPADVE RHIAGLVERGCKAIADAPVDQVWRGELAAMAGRVAYRTA |

In the method of the present invention, a farnesyl diphosphate synthetase gene may be used in combination with a sesquiterpenoid synthetase gene.

A preferred farnesyl diphosphate synthetase gene is the gene encoding the protein of the amino acid sequence described in Table 3 (SEQ ID NO: 41), more preferably fps (SEQ ID NO:40) described in Table 3.

A farnesyl diphosphate synthetase gene of the present invention includes the gene encoding a protein consisting of an amino acid sequence having 80% or more, 90% or more, 95% or more, or 97% or more identity to the amino acid sequence (SEQ ID NO:41) described in Table 3. For example, the gene consisting of a base sequence having 80% or more, 90% or more, 95% or more, or 97% or more identity to fps (SEQ ID NO:40) is included.

**[Table 3]**

| Table 3 |
|---|
| fps (farnesyl diphosphate synthetase gene: for synthesis of sesquiterpenoid (C15)) derived from S. avermitilis MA-4680 |
| Base sequence (SEQ ID NO:40) |
| ATGACCGTGACCCCGGAAAGTCTGCACCGCAGCCGCGACGCGATCACACCGGCGTTGC GCACCGCGCTCCACCTGCTCGACGAGGAGACCCGCCATCAGGCGGCGTACCACCTCGG CTGGATCGAGGCCGACGGCACCCCGGCGGACGGCGGCGGAGGCAAGGCCGTACGGCC CGCGCTCGCGCTGCTGTCGGCGCAGGCGGCCGGCGCACCGGCGGAAACCGGCCTGCC CGGTGCGGTGGCCGTCGAACTGGTGCACAACTTCTCGTTGCTGCACGACGACCTGATG GACGGCGACGAGGAGCGTCGGCATCGACGTACGGTCTGGACGCTGTGGGGCGCCTCGG GCGCGATCCTCACCGGTGACGCGCTGCTGGCGCTCGCGCAGGAGGTGCTGCTCGACTG CGGGCTGCCCACGGGGGCGGCCGCGGCGCGGCTGCTCGCCCGCACCACCCGCGACCT CATCCGCGGGCAGGTGCAGGACGTGGCGTTCGAGCGGCGGGCCCATGTGCCGATCGAG GAGTGCGTCGACATGGCGGCCGGCAAGACCGGCGCGCTGCTGTCGGCCAGCGCCGCGA TCGGTGCCGTACTGGCCGGCGCGCCGGTGACCACCGTCGACGCCCTGGCGCTCTACGG GCAGCAGGTGGGCCTCGCCTTCCAGCTGACCGACGACGTCCTCGGCATCTGGGGCGAC CCGGCTGTCACCGGCAAGTCGGTCCACTCCGACCTGCGGGCCCGCAAGAAGTCGCTGC CGGTCACGTACGCGCTCAACCAGGGCGGACCGTGAGTGTCGAGCTGGCCACCTGGCT CTCCGCACCAGGCGAGCCCGACGAGGCAGAGCTGCGCCGGGCGGCCGACCTGGTGGC GGCGGCCGGCGGCCGCGACTGGGCCACCGCCGAGGCGGCCCGGCGCATGGCGCTCGG GGAGGCCGCGCTGCGCGGCGTCGACCTGGACGCCGCGGCGCGCGACGAGCTCATCGC CCTCGGACACTTCGTCGTCGACCGGGAGGTGTGA |

| Amino acid sequence (SEQ ID NO:41) |
|---|
| MTVTPESLHRSRDAITPALRTALHLLDEETRHQAAYHLGWIEADGTPADGGGGKAVRPALALL SAQAAGAPAETGLPGAVAVELVHNFSLLHDDLMDGDEERRHRRTVWTLWGASGAILTGDALL ALAQEVLLDCGLPTGAAAARLLARTTRDLIRGQVQDVAFERRAHVPIEECVDMAAGKTGALL SASAAIGAVLAGAPVTTVDALALYGQQVGLAFQLTDDVLGIWGDPAVTGKSVHSDLRARKKS LPVTYALNQGGTVSVELATWLSAPGEPDEAELRRAADLVAAAGGRDWATAEAARRMALGEA ALRGVDLDAAARDELIALGHFVVDREV |

In the method of the present invention, a geranylgeranyl diphosphate synthetase gene may be used in combination with a diterpenoid synthetase gene.

A preferred geranylgeranyl diphosphate synthetase gene is the gene encoding the protein of the amino acid sequence described in Table 4 (SEQ ID NO:43), more preferably ggs (SEQ ID NO:42) described in Table 4.

A geranylgeranyl diphosphate synthetase gene of the present invention includes the gene encoding a protein consisting of an amino acid sequence having 80% or more, 90% or more, 95% or more, or 97% or more identity to the amino acid sequence (SEQ ID NO: 43) described in Table 4. For example, the gene consisting of a base sequence having 80% or more, 90% or more, 95% or more, or 97% or more identity to ggs (SEQ ID NO:42) is included.

**[Table 4]**

| **Table 4** |
|---|
| **ggs (geranylgerany diphosphate synthetase gene:for synthesis of diterpenoid (C20)) derived from Streptomyces sp.** |
| **Base sequence (SEQ ID NO:42)** |
| ATGGCCGGTACCGCATGGACACCGATATCCCGACCCTTATATCCGTTCGTGACGATGT AGACGCGGTGCTCGAGGACTTCGTCGACGTGCAGGAGCGGCAGCGCACGGCCCGGAA ATGGCACCGCTGTTCACCACGGCCCGCCGGCTCCTCTTCTCCAACGGCAAGCGACTGC GCCCCTTGCTGTGCGTGGCGGATGGCAGGCCGCAGGCGCACCGGAGACCCCCACGC GGTCCTTCGCGTCGCCGCCGCCCTGGAGCTGTTCCAGACCTTCGCCCTGATCCACGAC GACGTCATGGACGACAGTGACACCCGCCGCGGACGGCCCTCGGCCCACCGCGCGCTGG CCGCCGAGTACATGAGCGGCGGTGGGCGGCTCAGCAAGGCGGAGGCGCACGGCCGAG GTGCCGCCATCCTCCTCGGTGACCTCCTGCTCGTGTGGTCGGACGAAATGCTGGCGGG CGCGGGGCTCGAAGCCCGGGCGCGGGCCAGGGTCCTGCCACTGGTGGACAGCATGCGC GCCGAGCTGGTCTACGGGCAGTACCTGGATCTGCTGTCGACCGACCGGATGTCCGGGG ACGTCGAGGCCGCGCTGCGGGTCGCCCGGTACAAGACGGGGAAGTACACCGTGGAACG GCCCCTGCACCTCGGCGTTTCCCTGGCCGGCGGCGATCCGCGGCTGCTGCAGACCTGC ACCGATTTCGCCCTCCCGCTGGGAGAAGCGTTTCAGCTGCGCGATGACCTGCTCAACGT CTTCGGTGACCCGGTGCGGACCGGCAAGCCTGTCGGAGACGACATCCGTGAGGGCAAA GCCACCGTGCTCCTGGCTCTCGCGATCCAGGACGCCTCCGAGGCCGACCTGAAGCTGC TCGGCACCCTGGTCGGCAGCCCGGAACTGGGGAGCGAGGACATCGACCGGTTCCGCAC CGTCATGGAGACCACCGGTGCCCGGCAGCGGGTCGAAGACATGATCATCGCCAGGCGC GACACCGCCCTCGCCACGCTGGAAGAAGCAGACCTGCCCCAGGACGTCGCCGAGACGT TGAGGCAGATCGTATGGATGGCCACGGAAAGGCAGTCATGA |

| **Amino acid sequence (SEQ ID NO:43)** |
|---|
| MAGTAWDTDIPTLISVRDDVDAVLEDFVDVQERAAHGPEMAPLFTTARRLLFSNGKRLRPLLC VAGWQAAGAPGDPHAVLRVAAALELFQTFALIHDDVMDDSDTRRGRPSAHRALAAEYMSGG GRLSKAEAHGRGAAILLGDLLLVWSDEMLAGAGLEARARARVLPLVDSMRAELVYGQYLDLL STDRMSGDVEAALRVARYKTGKYTVERPLHLGVSLAGGDPRLLQTCTDFALPLGEAFQLRDD LLNVFGDPVRTGKPVGDDIREGKATVLLALAIQDASEADLKLLGTLVGSPELGSEDIDRFRTVM ETTGARQRVEDMIIARRDTALATLEEADLPQDVAETLRQIVWMATERQS |

### Terpenoid synthetase gene and Produced terpenoid compound

Preferred monoterpenoid synthetase genes, sesquiterpenoid synthetase genes, and diterpenoid synthetase genes, which may be used in the production method of a terpenoid compound of the present invention, are described in Tables 5 to 22 and Table A.

Amino acid sequences encoded by these terpenoid synthetase genes are described in these tables.

The genes encoding the proteins consisting of these amino acid sequences can also be used in the production method of a terpenoid compound of the present invention, and are included in the scope of the present invention.

In the present invention, codons of a monoterpenoid synthetase gene, a sesquiterpenoid synthetase gene, or a diterpenoid synthetase gene may be optimized so as to be suitable for expression in a host cell. The genes which are obtained by optimization of codon are included in the scope of the present invention.

A terpenoid synthetase gene of the present invention includes the gene consisting of a base sequence having 80% or more, 90% or more, 95% or more, or 97% or more identity to each base sequence of the monoterpenoid synthetase gene, the sesquiterpenoid synthetase genes, or the diterpenoid synthetase genes described in Tables 5 to 22 and Table A. Amino acid sequences encoded by such genes are also included in the present invention.

Further, the terpenoid compounds which are produced by each terpenoid synthetase gene described in Tables 5 to 22 and Table A are also described below.

### 1-1. Monoterpenoid synthetase gene

### 1-1-1. sclav_p0982 (derived from Streptomyces clavuligerus ATCC 27064)

**[Table 5]**

| **Table 5** |
|---|
| **Base sequence (SEQ ID NO:1)** |
| ATGCCCGCCGGCCACGAAGAGTTCGACATACCCTTCCCGTCCCGAGTGAACCCCTTCCACGCCCGGGCCGAGGAC CGCCATGTGGCCTGGATGCGCGCCATGGGTCTGATCACCGGCGACGCCGCCGAAGCGACGTACCGCCGCTGGAGT CCCGCCAAGGTCGGAGCCCGCTGGTTCTATCTCGCACAGGGTGAGGACCTGGACCTGGGCTGCGACATCTTCGGC TGGTTCTTCGCCTATGACGACCACTTCGACGGACCCACCGGCACCGATCCCCGGCAGACCGCCGCGTTCGTGAAC CGCACCGTGGCCATGCTCGACCCCCGCGCCGACCCGACCGGCGAGCACCCCCTCAACATCGCCTTTCACGACTTA TGGCAACGTGAGAGCGCCCCCATGTCCCCCCTCTGGCAGCGGCGTGCCGTCGACCACTGGACGCAGTACCTCACC GCCCACATCACCGAGGCGACCAACCGCACCCGCCACACCAGTCCCACCATCGCCGACTACCTGGAGCTGCGCCAC CGCACCGGCTTCATGCCCCCGCTGCTCGACCTGATCGAACGGGTCTGGCGCGCCGAAATCCCCGCCCCCGTCTAC ACCACCCCCGAAGTGCAGACCCTTCTCCATACGACCAACCAGAACATCAACATCGTCAATGACGTCCTGTCCCTG GAGAAGGAAGAAGCCCACGGCGACCCGCACAACCTCGTCCTGGTCATCCAGCACGAGCGGCAGAGTACCCGACAG CAGGCCCTCGCCACCGCCCGGCGCATGATCGACGAGTGGACCGATACCTTCATCCGCACCGAACCCCGGCTCCCC GCCCTCTGCGGCCGCCTCGGCATTCCTCTCGCCGACCGCACATCCCTCTACACCGCCGTCGAAGGCATGCGCGCC GCCATCCGCGGCAACTACGACTGGTGCGCCGAGACCAACCGCTACGCCGTTCACCGCCCCACCGGCACGGGGCGG GCCACCACCCCTTGGTGA |

| **Amino acid sequence (SEQ ID NO:2)** |
|---|
| MPAGHEEFDIPFPSRVNPFHARAEDRHVAWMRAMGLITGDAAEATYRRWSPAKVGARWF YLAQGEDLDLGCDIFGWFFAYDDHFDGPTGTDPRQTAAFVNRTVAMLDPRADPTGEHPL NIAFHDLWQRESAPMSPLWQRRAVDHWTQYLTAHITEATNRTRHTSPTIADYLELRHRTG FMPPLLDLIERVWRAEIPAPVYTTPEVQTLLHTTNQNINIVNDVLSLEKEEAHGDPHNLVLV IQHERQSTRQQALATARRMIDEWTDTFIRTEPRLPALCGRLGIPLADRTSLYTAVEGMRAAI RGNYDWCAETNRYAVHRPTGTGRATTPW |

### Produced terpenoid compound

### (1) camphene

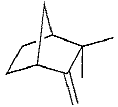

### (2) β-pinene

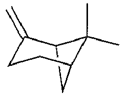

### (3) 1,8-cineol

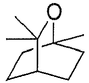

### 1-2. Sesquiterpenoid synthetase gene (I)

### 1-2-1. sclav_p0985 (derived from Streptomyces clavuligerus ATCC 27064)

**[Table 6]**

| **Table 6** |
|---|
| **Base sequence (SEQ ID NO:3)** |
| ATGCCGTTCCCCGCCCGCCCGCTGAACGCGGAATGGAGCAGGCCAGGCAGCGGA TGTGGGCATGGATCGACCACTTCGGCCTCGGCCCTTCCGAGGCGTCCCACCAGCGC CTGTCACAGCTGCGGTTAGAGGTGATCACGGCCCGCTACTACCCCTTCGTGGAGCC GTCCGCCCTGCCCCTGCTCGCGCTGCACATGGCATGGGCCTGGTGTGTCGACGAGC AGTTCGACGACGGGCCCGCCGGTCGTGATCCGCGCTGGTGTCTGACGGCCCTCCGC GGTCTGCGGGACGCCGTACTGGGCGGGAGCCACATACCGGGCAACCCCCTCGAAG CCGCCGCCGCCGACTTCCACCGGAGACTGGGAGAGGTCCATTCGCCCCGGTGGGTC CGGGACTACACGGACGTCACCACCCGCTGGCTGTGGAGTTACTACGCCGAGAGCCT GGACCGGGCCACCGACCGGCACCAACCGCTGCCGGACTACCGGCGCCATCGGCAG GTCACCAGCGGCGAGCACATGTTCTTCATGCTCGCCGAGCACGGAGCGGGCAGGGA GCTTCCCGACAGTGTGAGCAGGCTCCCGGCCTACATCGCGTTGCAGGACGCGGCGG CGGAGCACATGGGACTGCTCAACGACATCGTCTCCCTGCCCAAGGAGGAACCCATC GGCGACGTGCACAACGCGGTCACTCTTGTGTCGCGCCACTCCCGTGTGCCCGCCGC GGAGGCGGCCGAGGCCGTCAACGACATGCTGACCGACTGCGTCCACCGGATGCTGG CCGCGGAGGACGAACTCCCGGGACAGCTCGACGCCGTGGGCGTCACGGAGCGGAC CCGGGCGGACGCGCTGGCCATGGTCGAGGCGATCAAGGCCCATACCCGGGGAAAC TTCGACTGGCACTTCGAGGTGAGCCGCTACGCGGGGCCAGGACAGCTCGACGAAGC CGGAAGCCCCGCGTATGTGGCCGATGTGCTGGGCGGCGCACGCACCGGTGCCGAC TGA |

| **Amino acid sequence (SEQ ID NO:4)** |
|---|
| MPFPARPLNAGMEQARQRMWAWIDHFGLGPSEASHQRLSQLRLEVITARYYPFVEPSALP LLALHMAWAWCVDEQFDDGPAGRDPRWCLTALRGLRDAVLGGSHIPGNPLEAAAADFH RRLGEVHSPRWVRDYTDVTTRWLWSYYAESLDRATDRHQPLPDYRRHRQVTSGEHMFFM LAEHGAGRELPDSVSRLPAYIALQDAAAEHMGLLNDIVSLPKEEPIGDVHNAVTLVSRHSR VPAAEAAEAVNDMLTDCVHRMLAAEDELPGQLDAVGVTERTRADALAMVEAIKAHTRGN FDWHFEVSRYAGPGQLDEAGSPAYVADVLGGARTGAD |

### Produced terpenoid compound

### (1) african-2-ene

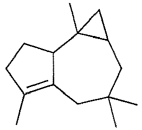

### (2) african-1-ene

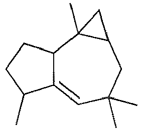

### (3) α-gurjunene

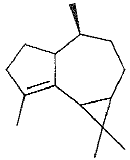

### (4) α-humulene

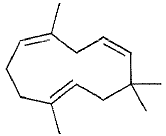

### (5) β-caryophyllene

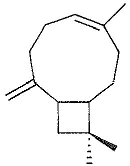

### 1-2-2. sclav_p1407 (derived from Streptomyces clavuligerus ATCC 27064)

**[Table 7]**

| **Table 7** |
|---|
| **Base sequence (SEQ ID NO:5)** |
| ATGGGTGGGGCGTACAGGACCGTCCACCGTACGCCGGGAGGGGCTGGGATGCCGC AGGATGTTGTGTTCGATCTTCCGTGGCCGCTGCGGAAGCACCCCGGTGTGGCCGGG GCCCGGGAGCACTGCCTCGGCTGGCTGGCCGCCCAGGGGCTGGCGGACCGGCGGG GGCTGACCGCCGAGACCTTTGTGACCTGGCAGCTCGACGAGTTGGCGGGCTACTTC TTCCCGCGTGCCACCCAGGAGGGCCTTGAACTCGCCACGGACCTGATGGTCTGGTA CTTCGCCCCGTTCGACGACCAGTTCGACGGCGCGCTCGGCCGCGACCCCCGCCGC ACGGCCGGTGTCTGCGCCGGGCTGGCCGAGGTCCTGTACGGGGTGCCGGAGCCCG GCCCGGTCGCCTCGTCCCCGGTGGGCCGGGCGCTGGGGGACCTGTGGCGGCGGAG TTGCACGGGCATGAGCCCGTTCTGGCGCACCCGTGCCCGTCACAACTGGACGGGCT ATCTCGCCGCGCACACCGCGGAATCGGTTCCCCGGTACCACGGGCGGACCGTGGAC GCCGCTTACTGCGTGCGCCAGCGGGATACGCGACCTCGTCACACGTGATCATGGA TCTGATCGAGCGGACCGGGGGCTTCGAGGTTCCGGCCATGGTCTGGCACCACCCCG TCCTCGTCGAGCTGCGGACGCTGACCTCCGAGATGATCGGAATCTCCAACGACCTC TGCTCCGCGGAGAAGGAGGAGGCGGACGGCGATCTGAGCAACAATCTGCTGCTGGT CCTGGAGAACCACGAGGGGCTGGACCGCCCGGAGGCCATCGAGCGGGCGCGCGCC CTGACGGCGGAACGGGTCGCCCGCTTCCTCGACGTCGAACGCGCCGTCACGGACGT GGACTGCCTGCTCGACGGCGCGGGCCGCGAGGCGGTGCGCCGGTTCGTGGAGGGC CTGCACGACCTGGTCCGGGGCGACAACGAGTGGAGCGGACCACCGGCCGCTACC AGCCGGCCATCAGCGGACTTCAGGACCTCGCCGACCTGATGTCCTCCGGGTGGGAC CCGCTCGCCTCCGGCACGGCCGCGGACACGGTGGACACACACGATGA |

| **Amino acid sequence (SEQ ID NO:6)** |
|---|
| MGGAYRTVHRTPGGAGMPQDVVFDLPWPLRKHPGVAGAREHCLGWLAAQGLADRRGL TAETFVTWQLDELAGYFFPRATQEGLELATDLMVWYFAPFDDQFDGALGRDPRRTAGV CAGLAEVLYGVPEPGPVASSPVGRALGDLWRRSCTGMSPFWRTRARHNWTGYLAAHTAE SVPRYHGRTVDAAYCVRQRGYATSSHVIMDLIERTGGFEVPAMVWHHPVLVELRTLTSEM IGISNDLCSAEKEEADGDLSNNLLLVLENHEGLDRPEAIERARALTAERVARFLDVERAVTD VDCLLDGAGREAVRRFVEGLHDLVRGDNEWERTTGRYQPAISGLQDLADLMSSGWDPLA SGTAAGHGGHTR |

### Produced terpenoid compound

### (1)1,3,6-trimethyl-2-methylene-tricyclo[5.4.0.03,9]undecane

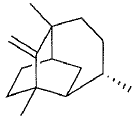

### (2) lactan-1-ene

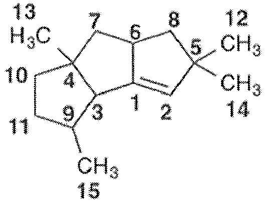

### 1-2-3. sgr_6065 (derived from Streptomyces griseus IFO 13350)

**[Table 8]**

| **Table 8** |
|---|
| **Base sequence (SEQ ID NO:7)** |
| GTGGCGCACACCCGTGGGCATCTGGACAGCTGGACCAGACGGACGGGTCTGGTCCA CCGCGAGTCCGCGAGGAACCGATTCGAACAAGCCGATTTCGGGGCGTTCGTCGGCA TGGTCTACCCGACGGCCGACGAGGAACACCTCGATCTGGTGGCCGACTGGTTCGTC TGGCTGTTCCTCGTCGACGACCAGCTCGACGACGGCCACCTCGGGCGTTCACCCGA CCGGGTGAGAGATGTCGTCGACCGGATGCGCGCGGTGGTCGACGGCTCCGCCCCC GAGGTGCTGCCGGACGAGGACGCCCCGGCCGCCGTGACGGCCCTGGTGGACCTGT GGAAACGCACGATGCCGGAAGCGGCCCCGCACTGGCGGACCCGGTTCGCCTGGCA CCTGGTCACCTACCTCACGACCGCCACGACCTGGGAGGCGGGGAACCGCGCCGAG GACGTGGTGCCCTCGGAGGAGACGTACATCGCCAAGCGCCGTCACACCGGGGCCAT CCACGTCTGCATGGACCTCATAGAGATCGTCGCGGGCATCGACGCCCCGGAGTCGG TCCACAACGACCCCCGCTTCATCACCGCGCTGGAAGCCGCCTGCAACCACGTGTGC TGGGCCAACGACGTGTACTCCTTCGAGAAGGAGCAGGTGCTCGGCGAGATCCACAA CCTCGTCCACCTGGTCCGCCACCACCGGGGGCTCGGGGAACAGCAGGCGCTGGAC CACGTCGCGGAGCGCCTCGCGATGGAGACCGAGCGGTTCCTGACCGCCGAGGACG AGCTGCTGGAGCTGTATCCGGAGCTGTCCGGGATGCTGGTTCCCTACCTTGACGGG ATGCGGAGCTGGATGCGCGGCAACCTGGACTGGTCCCGGCAGACGCCGCGCTACAA CCCCGCCGACGTGGGCCAGTACGAGGAACCCGAGGAGTATCTGGAGGAGACGGTGC TGGGCGTGCCGCCCGCGCGCAGCGAGACCGCCGCCCCTGCCCCGTGCGGGGCCGA GGCTCCCCGGGCCCGCTGA |

| **Amino acid sequence (SEQ ID NO:8)** |
|---|
| MAHTRGHLDSWTRRTGLVHRESARNRFEQADFGAFVGMVYPTADEEHLDLVADWFVWL FLVDDQLDDGHLGRSPDRVRDVVDRMRAVVDGSAPEVLPDEDAPAAVTALVDLWKRTMP EAAPHWRTRFAWHLVTYLTTATTWEAGNRAEDVVPSEETYIAKRRHTGAIHVCMDLIEIV AGIDAPESVHNDPRFITALEAACNHVCWANDVYSFEKEQVLGEIHNLVHLVRHHRGLGEQ QALDHVAERLAMETERFLTAEDELLELYPELSGMLVPYLDGMRSWMRGNLDWSRQTPRY NPADVGQYEEPEEYLEETVLGVPPARSETAAPAPCGAEAPRAR |

### Produced terpenoid compound

### (1) α-copaene

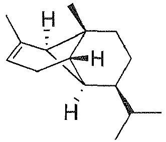

### (2) calarene

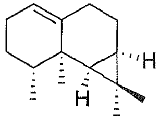

### (3) α-murolene

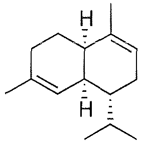

### (4) cadina-1(10),4-diene

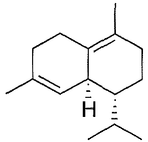

### (5) cadina-1,4-diene

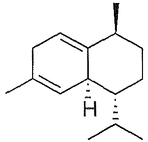

### (6) epicubenol

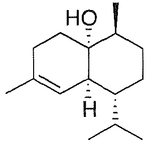

### 1-2-4. sven_0552 (derived from Streptomyces venezuelae ATCC 10712)

**[Table 9]**

| **Table 9** |
|---|
| **Base sequence (SEQ ID NO:9)** |
| ATGACAGTGCGTGCCGTCGAGCTCCCCCAGCTGTGGATGCCCTATCCGCTGCGGGT GAACCCCTACCTGTCGGCCCTGCGCGAGGAGAGCGAGACCTGGGCCCGCGAGATG GGCATGCTGGGCGGCGAAGGACCGTCCGCCCGCGGCGCGATATGGACCCGCTCCC AGTTCCACGCCATGACCGTGGAGCTGCTGACCGCCTGGACCCTGCCGGACGCCTCG CTCGCCGGTCTGCGGCTCAACCACCGCTTCAACATCTGGGCGCTGGCCTGGGACGA CTACTTCGCGTCCACCTTCAAGCAGACCGGCGACCTGCCCGGGGCGCTGGACTTCA CCGCCCGGCTGCACGCCTTCCTGCGACCAGAGGCCGACGCCCGGTCGCCAGAACC GGTGAACGCCGTCGAGCGCGGTATCGCCGATCTCCAGAAGCACCTGTTCCCGCCCC GACTGGCCCACTGGCGCCAGGAGTTCAACCTCGCGCTCGCCCACTACATCGACGCC GGCGTGCAGGAACTCACCAACAGCCGCGGCGGCCGCGTCCCCCATCTCATCGAGTA CGCGCCGTTCCGACGCGAGAGCTTCGCCGCCCACACCGCCCCCTACTCCGTCGAAC TGGCCACCGGCGCGCGGATACCCGAGCAGCTCCGGCACACCAGGACCGTCCACGC CCTGCTCGACGCGTTCATGGACGTCATGGCCTGGCAAACGATGTCGCCTCCTACG AACGGGAGGTCCACGAGGAACGCGACGTCAACAACCTCGTGGTGGTCATCGGAACG TCCCTCGGCATCACCCTCCACCAGGCCGTACCCGCCGCGCTGCACATGGTGAACGC CCGCATGCGGGACTTCGAGCACCTGCGGCACCACGAACTGCCGCCGCTGGTCGACC GGTTCGGGCTGCGCCCCGACGAGCGGGAGGAGCTGGAGACCTGGCTCGGGGGAGC CGCCAGCTTCCTCTCGGGCCTCCACGCCTGGTACACCGGGGCGCCCCGCTACGCG CCCACGGACCACTCCGTACCGGAGCAGCGTCAGCGCGGCGGTTCCGAGGGGGCTG CCGACAGACACGCGCCGGCAGCCTCCTCCCTGGCCCGGGGCGCGGAGGCCCAGAC GTGCGGGCCTACCGCCTCCGCAGGAAGCGCATGA |

| **Amino acid sequence (SEQ ID NO:10)** |
|---|
| MTVRAVELPQLWMPYPLRVNPYLSALREESETWAREMGMLGGEGPSARGAIWTRSQFHA MTVELLTAWTLPDASLAGLRLNHRFNIWALAWDDYFASTFKQTGDLPGALDFTARLHAF LRPEADARSPEPVNAVERGIADLQKHLFPPRLAHWRQEFNLALAHYIDAGVQELTNSRGG RVPHLIEYAPFRRESFAAHTAPYSVELATGARIPEQLRHTRTVHALLDAFMDVMGLANDVA SYEREVHEERDVNNLVVVIGTSLGITLHQAVPAALHMVNARMRDFEHLRHHELPPLVDRF GLRPDEREELETWLGGAASFLSGLHAWYTGAPRYAPTDHSVPEQRQRGGSEGAADRHAP AASSLARGAEAQTCGPTASAGSA |

### Produced terpenoid compound

### (1) α-chamigrene

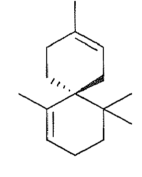

### (2) dauca-8,11-diene

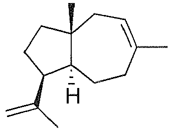

### 1-2-5. sk_3051 (derived from Streptomyces sp. SK 1894)

**[Table 10]**

| **Table 10** |
|---|
| **Base sequence (SEQ ID NO:11)** |
| GTGGGACCCCATTTGACCGTTCCGCCGATCTACGCGCCGATACCTCCGGCGATCCA TCCAAGACACGCCGTCATCGACGCGCGTACGGCGGCCTGGGCGGAGTCGTTCGAGA TCGGCTCGCCCGAGCTGCGCAGCAGACTCGTCCGGCACGACATCGGCACCTTCTGC GCCCGGATCCTGCCGGAGGGCAGAGAGGAGGTGGTCAGCCTTCTCGCCGACTTCGT GCTCTGGCTGTTCGGCGTGGACGACGGCTACTGCGAGGAGAGCGACCTGGGCCACC GGCCCGGCGATCTCGTCGGGGTCCTCCACCGGCTGCTGCGGGTCGCCCAGGACCC CGAGGGGCCCTTCCTGCTGGCCGACCCGCTCGCCAACGGGCTGCGCGACCTGCGG GCCCGGATCGACAGATTCGGTACGTACGCCCAGGCCACCCGGTGGGTGGACGCCCT GCGGGAGTACTTCTTCTCGGTGGTCTGGGAAGCCGACCACCGCCGGGCCGGTACGG TGCCGAACCTCGACGACTACACCCTGATGCGGCTCTACGACGGCGCCGCCACGGTC GTGTTCCCCCTGCTGGAGATGGGGCACGACTACGAGCTGACGCCCCAGGAACGGGA CCATCCGGCGACCCGGGCGCTCTGCGAGATGACGTCCTTCATCATCAGCTGGGACA ACGACATCTTCTCGCACCACAAGGAGGTGTCCGGCAAGGGCTTCTACCTCAACGCC CTGCGCGTACTCCAGCACCAGGACGGGCTCTCCCCGGCCGCCGCGCTCGCCACGG CCATCGCCCAGCGCGACCGGGTGATGTGCCTCTACCTCCGGCTCCACGACCACCGG GCAGGGACGGCGAGCCCCCGGCTGCGCCGCTATCTGTCGAGCCTGCGGCACTTCGT CCGGGCCACCCAGGACTGGAGCGTCAGCTCCCGCCGCTACACCACCCCCGACAAC CCGGCCGACCTGCCGAGCAGCTTCCGCAGCACCCCGACGGACGCGGGCACGGAGC CGCTGGACATTCCGGTGATCCGGTGGTGGTGGTCGCTGCTGCCCGAACCGGAAGGA GCCCGGGATGACGAGACGCCGTTACGCAGGCCCGCCGTCGCGGGCCGCTGA |

| **Amino acid sequence (SEQ ID NO:12)** |
|---|
| VGPHLTVPPIYAPIPPAIHPRHAVIDARTAAWAESFEIGSPELRSRLVRHDIGTFCARILPEGR EEVVSLLADFVLWLFGVDDGYCEESDLGHRPGDLVGVLHRLLRVAQDPEGPFLLADPLAN GLRDLRARIDRFGTYAQATRWVDALREYFFSVVWEADHRRAGTVPNLDDYTLMRLYDGA ATVVFPLLEMGHDYELTPQERDHPATRALCEMTSFIISWDNDIFSHHKEVSGKGFYLNALR VLQHQDGLSPAAALATAIAQRDRVMCLYLRLHDHRAGTASPRLRRYLSSLRHFVRATQDW SVSSRRYTTPDNPADLPSSFRSTPTDAGTEPLDIPVIRWWWSLLPEPEGARDDETPLRRPAV AGR |

### Produced terpenoid compound

### (1) β-maaliene

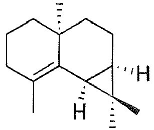

### (2) selina-3,7(11)-diene

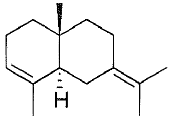

### (3) selina-7(11)-ene-4-ol

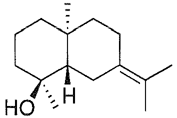

### (4) germacrene A

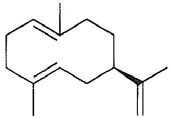

### 1-2-6. slt_1718 (derived from Streptomyces lactacystinaeus)

**[Table 11]**

| **Table 11** |
|---|
| **Base sequence (SEQ ID NO:13)** |
| ATGACCGATCTCCCCGACGGGCAGCCGCACAGCGCTCCGGACGACTTGTGGTGCCC CTTTCCCTCCCTGACGAACCCCCACGCCGAAGAGGCCGACCGGCAGAGCCTGGCGT GGCTGGCGCACCACCGGCTGCTGGACGGCGCCTCCGGCAGTCCGACCGGATCGG GCGCCAGCGCAGCGGCCACCTGGCGGCACGGACCAACCCCGAGGTCTCCGCCGGG ATGCTGCGCCTGCTGTCCGACTGGTACGTCTGGCTCTTCGCCTTCGACGACGGGTA CTGCGAGGACCGCGAACACGGCCATCGCCCCGGGCCGTTGGCCCGCACGACCGCT CTCCTCGCCCGGGTGATCGACCCCGATCCGCCCGGCGCCAGGATGTCCGACGACG GGCTGTCCGGACCGGCCGGCGCCTACGCCAGGGCCCTGCGGGAGATCCGGACCG GGTCGCCGCGCAGGCCTCGCCATCCCAGCTGTTCCGCTGGGAGTGCACGGTCCGCG AGTACCTCTCCGCCCAGGTGTGGGAGGCCGCCAACCGCGAGGCGGGCACGGTGCC GGGCCCGTCCGAGTACGTGGCCGTGCGCAGGCACGCCGGGGCGACGTACACCTGC CTCGCCCTGGTCGACATCGCGGCCGGCCACGAAGTCCCGGCCGCGGAGGCGGACG GGCCCTCGCTGCGCAGGCTCAGGGACCTCACGGCGAACCTGGTGAGCTGGGACAAC GACCTGTACTCCCACGCCAAGGAGACCGCGGGCGGCCAGGGCCGCCACAACCTGG TCGAGGTCCTCGCCCGCCACCACCGCTGTTCTCCGGCCGAGGCCCGACGGTCGGC CGTCGCCATGCGGGACGCCGAGATGCGCGCGTTCATCGAGCTGAGCCGAACCGTCC GCGCGGACGGCGGCGAGGCGGCCTCCGCGTACGCCCGGAGCCTGGGGCTGTGGCT GCGCGGACACATCGACTGGTCCCGCGGCAGCTCCCGGTTCGACGTGCCGGCCCCC ACGGCGAGGCCCTGA |

| **Amino acid sequence (SEQ ID NO:14)** |
|---|
| MTDLPDGQPHSAPDDLWCPFPSLTNPHAEEADRQSLAWLAHHRLLDGRLRQSDRIGRQRS GHLAARTNPEVSAGMLRLLSDWYVWLFAFDDGYCEDREHGHRPGPLARTTALLARVIDPD PPGARMSDDGLSGPAGAYARALREIRDRVAAQASPSQLFRWECTVREYLSAQVWEAANRE AGTVPGPSEYVAVRRHAGATYTCLALVDIAAGHEVPAAEADGPSLRRLRDLTANLVSWDN DLYSHAKETAGGQGRHNLVEVLARHHRCSPAEARRSAVAMRDAEMRAFIELSRTVRADGG EAASAYARSLGLWLRGHIDWSRGSSRFDVPAPTARP |

### Produced terpenoid compound

### (1) β-elemene

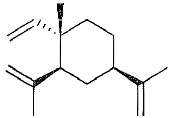

### (2) α-cadinene

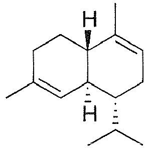

### (3) β-cadinene

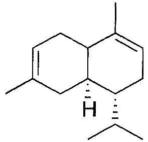

### (4) γ-cadinene

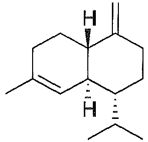

### (5) germacrene D 4-ol

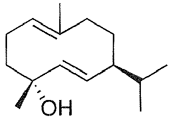

### (6) α-copaene

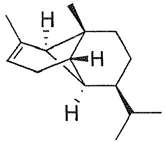

### (7) α-cubebene

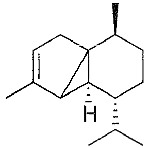

### (8) β-cubebene

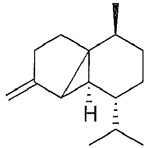

### (9) β-aromadendrene

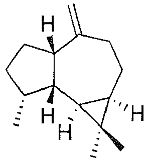

### (10) T-cadinol

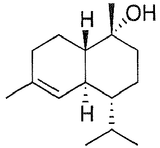

### (11) viridiflorene

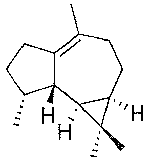

### 1-2-7. mmar_3220 (derived from Mycobacterium marinum M)

**[Table 12-1]**

| **Table 12** |
|---|
| **Base sequence (SEQ ID NO:15)** |
| GTGAACGTTATTGACTTCCCCCAGGTCGACTTGGGTACCGAGGCGACAATCAGCCC CGATGTCGACAAAGCAGGGGAACACCTGAAGACATGGTCTCGTGCCACAGGCATCG TCCTCGACGGCCCCAACGGTGACGCCTTCGATGCACTCGCGCAGCATCGCCTGGCG GCATGGACCTATCCGGCGGCAACCGGTGCGGAGCTTGATCTGATGGCGGACTGGAT CGGTTGGCTCTTTGCCTTCGACGACGTTTTCGATGAATCGATAGACGGCTGCGATCA GGACTTTGCCATTGCGACGGCGGCGGCCACCAACACCGTCTACACCGGGCTGCCCT CAGCACCCCCGAGCCCAGTAGTCAGGCCCTATGTGGTGGCGTTGGAGGATCTTTGG GAGCGGACCACCCAGGGCATGCCGGCGTACTGGTGCAGACGACTGGCCAACGACAT GGTCGATTACGTCAACTCCTACCGAAGCCATGCCCTGATCAACGCATCACGCATTG CGCTCGACGAACAGTCATATCGAGCCCACCGGCTGATCTCCAGCGCAGTATTCATC ACGCTCGACCTCGGCGAAGCCGCCGCTAGACGTGCCCTATCGGAGTCGCTGTTGGT GCACCCATACATACGGGCCGCTCGCGAAGCCGCCAACAACATCGTGTCCTGGAGCA ACGACCTCTACTCCGCGCCCAAAGAGCTCAGCCTGGGCGACCTGTGCAACTACATC GCCGTCCTGCAGTCTCAGGATGATCTCACGACTGCGCAAGCAGCCGACCGGGTCAG TCAGTACCTGCACGAGGAGATCGCGCGCTTCGATCAAACCAAACAGCTCATCCACA CCCACCTCATCCCACGCCTGCCAACCGAAGATCAACAAGGCGTGATCTCCATGCTG GATACCTGCAGCAACTGGATAACCGGCAACGTGGCGTGGTCATTGGAGACCGCACG CTACGCATCGCGCGCCATCGACATGGAGCGCGAAAAGGAAGTGTTCGGGGACTTGC GAACCTAG |

| **Amino acid sequence (SEQ ID NO:16)** |
|---|
| MNVIDFPQVDLGTEATISPDVDKAGEHLKTWSRATGIVLDGPNGDAFDALAQHRLAAWT YPAATGAELDLMADWIGWLFAFDDVFDESIDGCDQDFAIATAAATNTVYTGLPSAPPSPV VRPYVVALEDLWERTTQGMPAYWCRRLANDMVDYVNSYRSHALINASRIALDEQSYRAHR LISSAVFITLDLGEAAARRALSESLLVHPYIRAAREAANNIVSWSNDLYSAPKELSLGDLCNYI AVLQSQDDLTTAQAADRVSQYLHEEIARFDQTKQLIHTHLIPRLPTEDQQGVISMLDTCSN WITGNVAWSLETARYASRAIDMEREKEVFGDLRT |

**[Table 12-2]**

| **mmar_3220A: base sequence obtained by optimizing codons of mmar_3220 for expression in Streptomyces (SEQ ID NO:58)** |
|---|
| ATGAACGTCATTGACTTCCCCCAGGTCGACTTGGGCACCGAGGCGACAATCAGCCC CGACGTCGACAAAGCAGGGGAGCACCTGAAGACATGGTCCCGTGCCACAGGCATCG TCCTCGACGGCCCCAACGGCGACGCCTTCGACGCACTCGCGCAGCACCGCCTGGC GGCATGGACCTACCCGGCGGCAACCGGCGCGGAGCTTGACCTGATGGCGGACTGGA TCGGCTGGCTCTTCGCCTTCGACGACGTCTTCGACGAGTCGATCGACGGCTGCGAC CAGGACTTCGCCATTGCGACGGCGGCGGCCACCAACACCGTCTACACCGGGCTGCC CTCAGCACCCCCGAGCCCAGTAGTCAGGCCCTACGTGGTGGCGTTGGAGGACCTTT GGGAGCGGACCACCCAGGGCATGCCGGCGTACTGGTGCAGGCGACTGGCCAACGA CATGGTCGACTACGTCAACTCCTACCGAAGCCACGCCCTGATCAACGCATCACGCA TTGCGCTCGACGAGCAGTCATACCGAGCCCACCGGCTGATCTCCAGCGCAGTATTC ATCACGCTCGACCTCGGCGAGGCCGCCGCCAGGCGTGCCCTGTCGGAGTCGCTGTT GGTGCACCCATACATCCGGGCCGCCCGCGAGGCCGCCAACAACATCGTGTCCTGGA GCAACGACCTCTACTCCGCGCCCAAAGAGCTCAGCCTGGGCGACCTGTGCAACTAC ATCGCCGTCCTGCAGTCCCAGGACGACCTCACGACCGCGCAGGCAGCCGACCGGGT CAGTCAGTACCTGCACGAGGAGATCGCGCGCTTCGACCAGACCAAACAGCTCATCC ACACCCACCTCATCCCACGCCTGCCAACCGAGGACCAGCAGGGCGTGATCTCCATG CTGGACACCTGCAGCAACTGGATCACCGGCAACGTGGCGTGGTCATTGGAGACCGC ACGCTACGCATCGCGCGCCATCGACATGGAGCGCGAGAAGGAGGTGTTCGGGGACT TGCGAACCTAG |

### Produced terpenoid compound

### (1) β-eudesmol

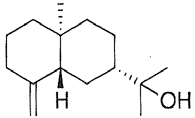

### (2) β-elemene

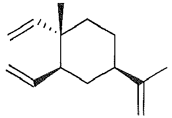

### (3) γ-elemene

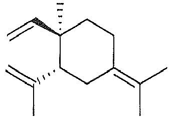

### 1-3. Diterpenoid synthetase gene

### 1-3-1. sclav_p0765 (derived from Streptomyces clavuligerus ATCC 27064)

**[Table 13]**

| **Table 13** |
|---|
| **Base sequence (SEQ ID NO:17)** |
| ATGACCATCTCCGTTCCCCAGCTCGACTGCCCGCTGAGCCGTCCGGTCCACCCGGAAGGAGAACGGGCCGACGCGTAC GCCGTGGAGTGGTTGCGCGGCGTGGGCCTCATGGCGGACGAGGCCGACGCCGCCCCGGTCCTGGCCGTCGGGCTCGGC AGGCTCGCCGCGTGCTATGTCGACGAGAACGCCTCCTGGACACGTTGGCCTTCATGACCATCCTGATGGCCTGGTAC GCCGAGTACGACGACCGCGCGATCGACTCCACCGGCGCGATCGACGGTCTGACGGACGCGGAGGTGGCGGAACTCCAC CGCGCCCTCGGGGAGATCCTGCGCGACCGGCCCGCACCCGACCCCTCCGACCCCGTACAACGCGGACTGGCCGACGTC TGGCGGACGCTGAACGGCCTCGCGTCCGACTGGGACCGCGCTGCCTTCGTCGACACCACCCTCCGCTACTTCGAAGCC AACCGGTACGAGCGGGTCAACATCCGCCGGGGAATCCCTCCGACTCCTTCCGCCCATATCGGCATGCGCCGCCACGGC GGCCATGTGTACGGCATGTACATCCTGGGTGCGGCGGTCAACGGCTACCGGCCCGAGCGCCGGGTGCTCGACCACGCC GCCGTCCGCGAACTGGAGACCCTCGCCGCGAACTACACCTCGTGGGCGAACGATCTGCACTCCTTCGCCCGCGAACAC CGCATGGGCCAGGTCAACAATCTGGTCTGGTCGGTCCACCACCACGAGGGGCTGACCTTCCAGCAGGCGGCGGACCGG GTCGCCGATCTGTGCGACAAGGAACTGGCCGCCTATCTGGAGCTGCGGCAGACGCTGCCCGAGTTGGGCATCCCCCTC ACCGGGGCCACCGGACGGCATGTCAGGTTCCTGGAGGACATGATGTGGTCCATGGTCGACTGGTCTGCGCGGAGCGCG CGCTACGACGTCGTGCCCGAGGCCGCCTGA |

| **Amino acid sequence (SEQ ID NO:18)** |
|---|
| MTISVPQLDCPLSRPVHPEGERADAYAVEWLRGVGLMADEADAAPVLAVGLGRLAACYVDENASWDTLAFMTILMAWY AEYDDRAIDSTGAIDGLTDAEVAELHRALGEILRDRPAPDPSDPVQRGLADVWRTLNGLASDWDRAAFVDTTLRYFEA NRYERVNIRRGIPPTPSAHIGMRRHGGHVYGMYILGAAVNGYRPERRVLDHAAVRELETLAANYTSWANDLHSFAREH RMGQVNNLVWSVHHHEGLTFQQAADRVADLCDKELAAYLELRQTLPELGIPLTGATGRHVRFLEDMMWSMVDWSARSA RYDVVPEAA |

### Produced terpenoid compound

### (1) trimethyl-methylenehexadecahydropyrene

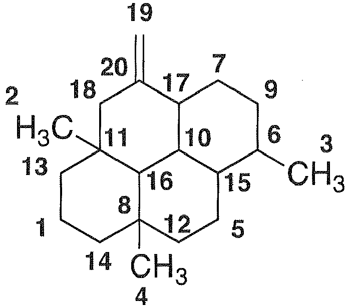

### (2) tetramethylhexadecahydropyrenol

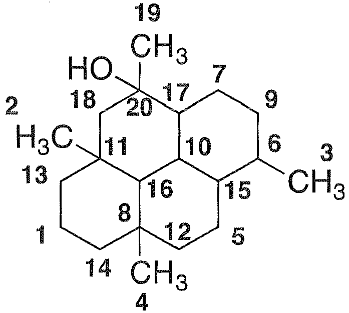

### (3) isoelisabethatriene

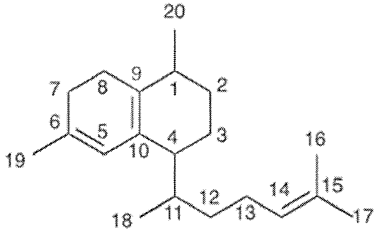

### 1-3-2. sclav_p1169 (derived from Streptomyces clavuligerus ATCC 27064)

**[Table 14]**

| **Table 14** |
|---|
| **Base sequence (SEQ ID NO:19)** |
| ATGCGCGGAGCCGGTCCCGGGAGCGTCCGATTTTCGTCGCTCTTCAGCGGTCTTCAGC GTTCTTCGGCGTTCTTCAGTGCTCTTCGGGAATCTTCGGCTTTTGACGTTCGACACAGG AGGCAGAGAGTGAGTCATGAACAGCGGGTGCCGTCGGTGGCGGCGCGGTACGGGGGA CCGCCGTGCACAGCGAGGACAGTGGACCGGAGGACGTCATGGTCCTCCGGGTCCCGGA ACTCGGACGGCGGTTACGGCCGCCCCGGCTGCACCCCGCCACACCCGCCGTGGTCGGG CAGCGGGTTCCGTGGCTGCGGACGCGCTCGCGGACATCGAGTTCCCCGGGGGGACGC CGGAGGACTTCCTCCGGCAGGACGTCCACCACTGGAGCGTCTACTGTCTGCCCACCGC GCGGGCCGACCGCATCGCCGATCTGAGCAATATCCATGAACTGGCCTTCGCCATGGAC GACATGCTCGAATCCGTGCACGACACCGACGCCGACGCCGACGTCCACACCGCTTCCG ATCTCGATCCCGCTTCCGGTTCCGCTTCCAATCCCGGTTCCGGTTCCGGTTCCGGTTTC GGTTCCGGTTTCGGTCGCGCGGCCCGCCGGGAGCGGGTCGAGCCGCTGGCCCGCGCG CTGGAGAGGGCGCTCGCTGGGCTGCCGCCCACCGGCCCGGTGCCGTCCTATCTGCGGG CGGCCGACGGCTGCTTCCGGACGCTGCGCGAGACGGGGCCCGCCCCCTGGTACCGGC GGTTCGGCGAGGCCGTCCTGTCCTGGTTCCACGGGGCCGTCAAGGAGAGTTCGATGAT GGCGACGGGGCGGCTGACCGGCTTCGAGGAGACGCTGGACAGCCGTATCGACACCGCG GGCGGGTTCTTCATCGCCACCAGCATCGAGTACGGCCTCGGCATCGACCTCACGGACG CCATCGCCGCCGGTCCTGAGCTGGGCGAGGTGGAGCGGGCGGCCTGGGTGCACGGCGT GCTGGTCAACGATCTGTTCTCGTACCGTAAGGAGCACTTCGGGGAGGCGGGCCGTGCT CCGGACGGCCGCGCTCCGGACGGCCGTGCGAACACCCTTCGTGTCCTCGCCGACGAGT ACGCGTGTTCCCTCCAGGAGGCGGTGGACCTGCTGGTCGAGCACGTCGACGCGGCGGA GGCCCGGTTCCTGGAACTGCGCGCGGACGTCCTCGGCGCTCCCCTCGGCGCCCGTCCC GGCGTCCGGGAGTATCTCGACGCCCTCGAACTGGTACTGCCCGGGAACATCGTCTGGT CCCGGACCTCACGGCGCTACCACGGACCGGCTGCCCCTGGACCGGGACCGCCCGGA CGGCGATGGCCCTCCACCCCGACAGGACCGTGTTCACCCCCTGGTAG |

| **Amino acid sequence (SEQ ID NO:20)** |
|---|
| MRGAGPGSVRFSSLFSGLQRSSAFFSALRESSAFDVRHRRQRVSHEQRVPSVAARYGGTAVHSE DSGPEDVMVLRVPELGRRLRPPRLHPATPAVVGQRVPWLRDALADIEFPGGTPEDFLRQDVH HWSVYCLPTARADRIADLSNIHELAFAMDDMLESVHDTDADADVHTASDLDPASGSASNPGSG SGSGFGSGFGRAARRERVEPLARALERALAGLPPTGPVPSYLRAADGCFRTLRETGPAPWYRR FGEAVLSWFHGAVKESSMMATGRLTGFEETLDSRIDTAGGFFIATSIEYGLGIDLTDAIAAGPE LGEVERAAWVHGVLVNDLFSYRKEHFGEAGRAPDGRAPDGRANTLRVLADEYACSLQEAVDL LVEHVDAAEARFLELRADVLGAPLGARPGVREYLDALELVLPGNIVWSRTSRRYHGTGCPWT GTARTAMALHPDRTVFTPW |

### Produced terpenoid compound

### (1) clavulatriene A

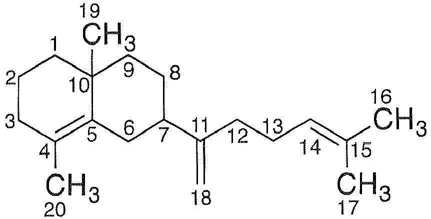

### (2) prenyl-β-elemene

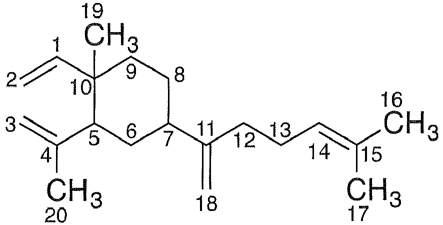

### (3) prenylgermacrene

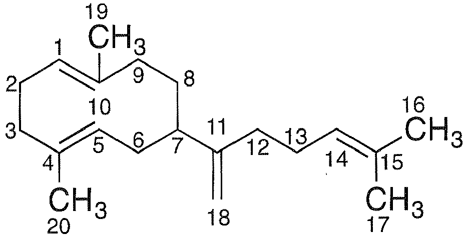

### (4) lobophytumin C

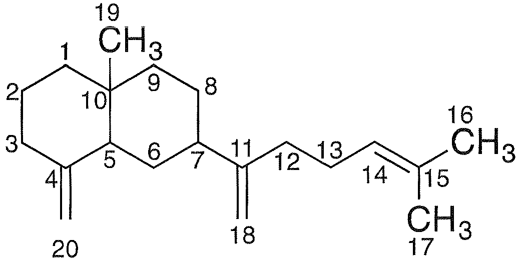

### (5) clavulatriene B

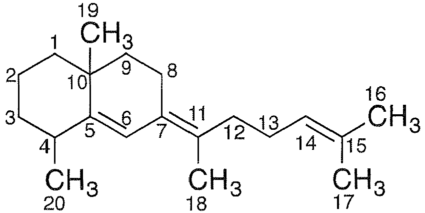

### (6) prenylgermacrene B

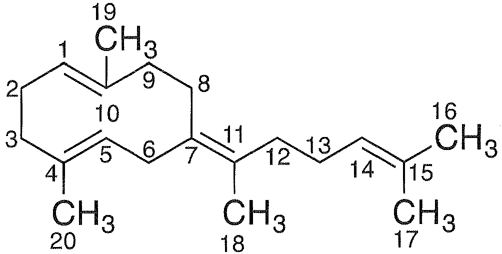

### 1-3-3. salt_1078 (derived from Streptomyces lactacystinaeus)

**[Table 15]**

| **Table 15** |
|---|
| **Base sequence (SEQ ID NO:21)** |
| ATGACAACCGTGCGCCGCACACTTGGCGTTCCGCCATTTTTCTGCCCTTTGCCCGACGC CGTGTACGACAAGCCGGAGGAAATAGATGCCGGCGCCGTCGCCTGGATGTTCCGGCAA GGGTTCCGCAAGGAGGGTGACTGGCTGCCGCGCATGAATGCGGGTTCGTACACCGCAC ACTGCATGCCGTACGTCCTCGACCGGTGCGCCGAGATCATCACCTATGTCATGTACTGG GGCGGCGCCTGGGACGACGACCTCGACGTGATCCCGGGGCTGGACCAGCCCGCACCGC TGACGGTCCACGTCAGCCGTCTCAAGCACATCCTCGCCTCGCCCAAGTCCGTCGTGCC GCCCGGAGACGCGTACCTCTCCTCCTTCCAGGACTTCTGGAACCACATCTCCACCGTGT CGACCCCGGGCCAGCTCGAGCACCTGCGCCAGGGTTTCTGCAGCTACTTCGACGGCGT GCTGTGGAAATGTGCGAACCAGAGCCTCGGTGAACTCCCCGAACTGGGCGACTACCTG ATCATGCGTGGCAACGATGTGGCCGGCCCCTGGCTGACCGCACTCACTCCGATCGGCG CCGGCTACGAACTGACCGCAGCCGAACGCCTCGATCCCCGGGTGCGCGCCGCCTCCGA AGCCGTATCCGTCATCGTCGGTCTCGACAACGACCTGATCTCCATTCACCGGGAGAATC TCCAGGGCTTGTTCGACCAGAACGCCATCAGCATCATGGTGCGCGACCGGCAATGCAC GCCCGAGGAAGCGGTCGCCGAGGTCCGCGCGCTGCGCGACCGAATCATGTCCCTTTAT CTGAATCTGCGTGACCAGGTCTCCGCGACCGCCCGCCCGGAACTGCGACGATACGTCC ACGAGATCGGTTACTGGATTCGCGGGCACATCATCTGGGCGGACTTCACCACCCGTTAC TGCGCGCCTGTCGACCCGGAGACCGGCGAAGAACAGGACTTCGGTGGCTACGGCCTGC TCTGGGCCGACGAGCCGGCTGACGCCAACCCTGCGCCCCTGCCGATTCCACAGATCGC CTGGTGGTGGGACCAGTTGGAGAAGTGA |

| **Amino acid sequence (SEQ ID NO:22)** |
|---|
| MTTVRRTLGVPPFFCPLPDAVYDKPEEIDAGAVAWMFRQGFRKEGDWLPRMNAGSYTAHCM PYVLDRCAEIITYVMYWGGAWDDDLDVIPGLDQPAPLTVHVSRLKHILASPKSVVPPGDAYLSS FQDFWNHISTVSTPGQLEHLRQGFCSYFDGVLWKCANQSLGELPELGDYLIMRGNDVAGPWL TALTPIGAGYELTAAERLDPRVRAASEAVSVIVGLDNDLISIHRENLQGLFDQNAISIMVRDRQC TPEEAVAEVRALRDRIMSLYLNLRDQVSATARPELRRYVHEIGYWIRGHIIWADFTTRYCAPVD PETGEEQDFGGYGLLWADEPADANPAPLPIPQIAWWWDQLEK |

### Produced terpenoid compound

### (1) cycloocta-7(8),10(14)-diene

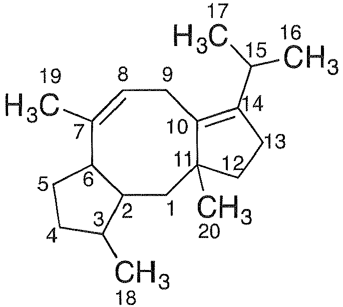

### 1-3-4. rxyl_0493 (derived from Rubrobacter xylanophilus DSM 9941)

**[Table 16-1]**

| **Table 16** |
|---|
| **Base sequence (SEQ ID NO:23)** |
| TTGTCTACTGAGAACGGGCATCGGACGGTCTGGGGCCCCTGCGCTGTCCCTTCCC CGCGGCGATAAACCCGCACGCCGACGAGGTGCACCGGGAGACCGTGGAGTGGGCC GAGGGTTTCGGGCTGCTCGGGCCCGGCGACGGCACCGGATGATGCGGACACCG GGATCGGGCGGCTCGCCGGGCGCTTCCACCCCGGGGCCGGGCGGAGGAGCTCCG GCTGATCTCCGACTGGTGCGCCTGGATGTTCCTGCGCGACGACCTCGCCGACGCGC CGGCGTACTTCCGGCACCCGGAGCGGCTCGCGGCCCTGGACGCGGCCTTTCTGGAC ATCCTCTCCGGCAGGGCCTCCGGGGAGGGCTGCGGGTCCTTCGGGCGGGCGCTGC GGGATCTCCGGGAGCGGCTGCTGCCCAAGGTTCCCGCGCCGCTCTGGCTGCGGCG CTTTCTCAGGTCCGTGGAGGAGCACTTCGAGTCCACGCTGTGGGAGGCCACCAACC GGGCCCGCGGGGTCGTCCCGGACCTCGAGACCTACCTCAGGATGCGCCCCATAACC GGCGGGATGCACGTGGACACGGACTTCATAGAGATATCCTCCGGCGTCTACCTGCC GCCCGAGGTGCGCCGGCACCCGGCCGTAAGCGCCCTCACCGGGGCCTCCAACAAC GTGGTCTGCTGGGCAAACGACATCATCTCGCTCGCCAAGGAGCGCTCGCGCGGCGA CGTGCACAACCTGGTCCTGGTCCTCCGCGCGAGCCGGCGTCTCACCACCCGGGAGG CGGTGGCGGAGGCCGCCCGGATGTACGAGGCGGAGGTGCGGAGGTTCGTCCGGCT GGAGCGGGAGCTGCCCCCCTTCGGGCCGGCCATCGACGCCAACCTCCGGCGCTAC GTCTCGGTGCTCAAGAGCAGGATGCGGGGCAATCTGGACTGGACCTACGAGTCGGC GCGCTACCGGGCCGGCGCGGCCTCCCGCTAG |

| **Amino acid sequence (SEQ ID NO:24)** |
|---|
| MSTENGHRDGLGPLRCPFPAAINPHADEVHRETVEWAEGFGLLGPGDGHRMMRDTGIGR LAGRFHPGAGREELRLISDWCAWMFLRDDLADAPAYFRHPERLAALDAAFLDILSGRASG EGCGSFGRALRDLRERLLPKVPAPLWLRRFLRSVEEHFESTLWEATNRARGVVPDLETYLR MRPITGGMHVDTDFIEISSGVYLPPEVRRHPAVSALTGASNNVVCWANDIISLAKERSRGDV HNLVLVLRASRRLTTREAVAEAARMYEAEVRRFVRLERELPPFGPAIDANLRRYVSVLKSR MRGNLDWTYESARYRAGAASR |

**[Table 16-2]**

| **rxyl_0493A: base sequence obtained by optimizing codons of rxyl_0493 for expression in Streptomyces (SEQ ID NO:59)** |
|---|
| ATGTCTACTGAGAACGGGCATCGGGACGGTCTGGGGCCCCTGCGCTGTCCCTTCCC CGCGGCGATAAACCCGCACGCCGACGAGGTGCACCGGGAGACCGTGGAGTGGGCC GAGGGTTTCGGGCTGCTCGGCCCGGCGACGGCACCGGATGATGCGGACACCG GGATCGGGCGGCTCGCCGGGCGCTTCCACCCCGGGGCCGGGCGGGAGGAGCTCCG GCTGATCTCCGACTGGTGCGCCTGGATGTTCCTGCGCGACGACCTCGCCGACGCGC CGGCGTACTTCCGGCACCCGGAGCGGCTCGCGGCCCTGGACGCGGCCTTTCTGGAC ATCCTCTCCGGCAGGGCCTCCGGGGAGGGCTGCGGGTCCTTCGGGCGGGCGCTGC GGGATCTCCGGGAGCGGCTGCTGCCCAAGGTTCCCGCGCCGCTCTGGCTGCGGCG CTTTCTCAGGTCCGTGGAGGAGCACTTCGAGTCCACGCTGTGGGAGGCCACCAACC GGGCCCGCGGGGTCGTCCCGGACCTCGAGACCTACCTCAGGATGCGCCCCATAACC GGCGGGATGCACGTGGACACGGACTTCATAGAGATATCCTCCGGCGTCTACCTGCC GCCCGAGGTGCGCCGGCACCCGGCCGTAAGCGCCCTCACCGGGGCCTCCAACAAC GTGGTCTGCTGGGCAAACGACATCATCTCGCTCGCCAAGGAGCGCTCGCGCGGCGA CGTGCACAACCTGGTCCTGGTCCTCCGCGCGAGCCGGCGTCTCACCACCCGGGAGG CGGTGGCGGAGGCCGCCCGGATGTACGAGGCGGAGGTGCGGAGGTTCGTCCGGCT GGAGCGGGAGCTGCCCCCCTTCGGGCCGGCCATCGACGCCAACCTCCGGCGCTAC GTCTCGGTGCTCAAGAGCAGGATGCGGGGCAATCTGGACTGGACCTACGAGTCGGC GCGCTACCGGGCCGGCGCGGCCTCCCGCTAG |

### Produced terpenoid compound

### (1) cembrene C (cembrene C)

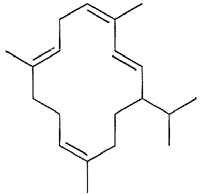

### 1-3-5. haur_2987 (derived from Herpetosiphon aurantiacus DSM 785)

**[Table 17-1]**

| **Table 17** |
|---|
| **Base sequence (SEQ ID NO:25)** |
| ATGCCAACCCGTACTACATTGCATCACTTAGCCACGCTTATGCCTCGTGATCTTCCG GTGCTCTATTGCCCATTTCCGGCGGCGATTCATCCCGATGCGGTTGCGATTCAAGC CTCAACCCAAGCATGGGCTGATAGCTTTGGCTTGCTCGATGATTTGGAGACGCGGC TCTCTTTTAATCAACGTGGCTATGGTACCTTGATGGCGCGAGCTTATCCTAATGCTT TAACTGAGCGCATGCCAACCATTGCCGATTGGAGCACCAGCCTCTTTCTCTTAGATG ATCAATTGGATCGCTTTGGCCGTGAGCATGATGCCGGGGCCATGGCAACCTTGTTG CAACGCTTGATGAAGGTACTCAAAGGTTCGCCTGCCAAGCGCCACGATTTGCCAATT GTGCAAGCAATGGGCGATGTAACCAGCCGACTTGTGCAAGGTTCGCCCGAAGCCTG GCAACACTACTTTTTTGATTCATTGCGCAAATCGTTTGCAGCAGTGCAATGGGAAGC TAACAACCGTGCTCAGGGCTATATTCCAACTCAAAAAACCTATGCAATGCAACGTTT ATATACCAGTGGTACTTATTTATGCTTGGCTTTGATTGAATTTGGCAATAACGATTTC CTGCATCCCCAAGTGCGTTTGCATCCCATGGTGCGGCGTTTGTGTGAATATGTTAAT CGGGTGGTGTTCTACTCAAATGATATTATTTCGTTGCGTAAAGAATTATTAGCTGGC GATTGGCAAAATTTGGTGTATCTCGTGCATACGCAACATCAATTACCAATCGGCGAA GCATTAAATTATGTCGCCCGTTTGCATAATGATACAATCAACCAATTCACTCGCGAA CGCATTATGCTCGATAAATTGGCTCCTGAGATTTGTTCACCAAGCTTGTATCGCTTT GTCGATGGTTTAGCTACCTGGATGCGAGCAAACCACGATTGGTCTACCATGACCTCT CGTTATCAGACATCTACCGAGGAATCCCATGGATCTCAGTATTGTGCGTGA |

| **Amino acid sequence (SEQ ID NO:26)** |
|---|
| MPTRTTLHHLATLMPRDLPVLYCPFPAAIHPDAVAIQASTQAWADSFGLLDDLETRLSFN QRGYGTLMARAYPNALTERMPTIADWSTSLFLLDDQLDRFGREHDAGAMATLLQRLMKV LKGSPAKRHDLPIVQAMGDVTSRLVQGSPEAWQHYFFDSLRKSFAAVQWEANNRAQGYIP TQKTYAMQRLYTSGTYLCLALIEFGNNDFLHPQVRLHPMVRRLCEYVNRVVFYSNDIISLR KELLAGDWQNLVYLVHTQHQLPIGEALNYVARLHNDTINQFTRERIMLDKLAPEICSPSLY RFVDGLATWMRANHDWSTMTSRYQTSTEESHGSQYCA |

**[Table 17-2]**

| **haur_2987A: base sequence obtained by optimizing codons of haur_2987 for expression in Streptomyces (SEQ ID NO:60)** |
|---|
| ATGCCAACCCGTACCACATTGCACCACTTGGCCACGCTTATGCCTCGTGACCTTCC GGTGCTCTACTGCCCATTCCCGGCGGCGATTCACCCCGACGCGGTCGCGATTCAGG CCTCAACCCAGGCATGGGCCGACAGCTTCGGCTTGCTCGACGACTTGGAGACGCGG CTCTCCTTCAACCAGCGTGGCTACGGCACCTTGATGGCGCGAGCCTACCCTAACGC CTTGACCGAGCGCATGCCAACCATTGCCGACTGGAGCACCAGCCTCTTCCTCTTGG ACGACCAGTTGGACCGCTTCGGCCGTGAGCACGACGCCGGGGCCATGGCAACCTTG TTGCAGCGCTTGATGAAGGTACTCAAAGGCTCGCCTGCCAAGCGCCACGACTTGCC AATTGTGCAGGCAATGGGCGACGTAACCAGCCGACTTGTGCAGGGCTCGCCCGAGG CCTGGCAGCACTACTTCTTCGACTCATTGCGCAAATCGTTCGCAGCAGTGCAGTGG GAGGCCAACAACCGTGCCCAGGGCTACATTCCAACCCAGAAAACCTACGCAATGCA GCGTTTGTACACCAGTGGCACCTACTTGTGCTTGGCCTTGATTGAGTTCGGCAACAA CGACTTCCTGCACCCCCAGGTGCGTTTGCACCCCATGGTGCGGCGTTTGTGCGAGT ACGTCAACCGGGTGGTGTTCTACTCAAACGACATTATTTCGTTGCGTAAAGAGTTGT TGGCCGGCGACTGGCAGAACTTGGTGTACCTCGTGCACACGCAGCACCAGTTGCCA ATCGGCGAGGCATTGAACTACGTCGCCCGTTTGCACAACGACACAATCAACCAGTT CACCCGCGAGCGCATTATGCTCGACAAATTGGCCCCTGAGATTTGCTCACCGAGCT TGTACCGCTTCGTCGACGGCTTGGCCACCTGGATGCGAGCAAACCACGACTGGTCC ACCATGACCTCCCGTTACCAGACATCCACCGAGGAGTCCCACGGATCCCAGTACTG CGCGTGA |

### Produced terpenoid compound

### (1) obscuronatin

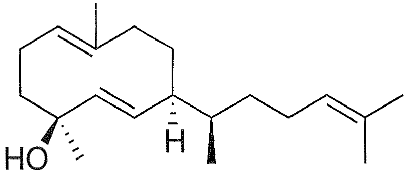

### 1-4. Sesquiterpenoid synthetase gene (II)

### 1-4-1. haur_2988 (derived from Herpetosiphon aurantiacus DSM 785)

**[Table 18-1]**

| **Table 18** |
|---|
| **Base sequence (SEQ ID NO:27)** |
| ATGCCAAATACAGCACACCTACAACAATTAGCGAGAACGGTTCCAGCAACCATTCC CAAGTTGGAATCACCGTTTCCTGCGCAAATCCATCCATTGGCAGCGGAGGTTCAACA GCAGACGACCAAATGGGTGCTTGAACAAGGTTTATTGAGCGAAGCGCGGCGTAAAG CTTTTCTTGATTACAACTATACCTTGTTGATTGCCCGTTGTTATCCGTATGCGAGTG TTGAGCGGCTGCAAATTATCAGCGATTGGAATGCTTGGCTGTTTGAACTTGATGATC AATGTGACGAAGCGCGAATTGGCCATGACCCTCAAGCGTTGGCTCGCTTGCATCAG CGGCTTGGGGCAGTTATTCGCGGAGCCACGGTTGCTGCCGACGAACCACTATTAGT TACAGCCCTCGCTGATATTATGGGTCGGATGCGGGCGCTAGCACCACTGGCATGGT TTGAGGTTTTACCCAACAGGTTGACGATTATTTTATTGGCAATCAATGGGAAGCCC ACAATCGGCTGTACTCGATTATTCCCAGCGAAATTGAATACTTGCACTATCGTAAAT TTACTAGTGCTGTTGCGATCTATATTGCGTTTATTGAATTTATGAATGCAAGTTATTT AACCGCTGATGAACGTCAATCAATCGACTTACGCAATCTACAAAATTGGACGAACCT TGTACTGAGTTTATGTAATGATCTTGTTTCAGTTAAAAAAGAATTACGTCAAGGCGAT TGGCATAATTATGGAGCAATTGTTGCATTAAAACGAGGTATGAGTATTCCTGAAGCC TTAGAAATAGTCGCCAATGAGCATAATCATTGTATTCAACAATTTATAACAACTACC ACTCAACTGAAAAATTGTAGCGTGGCACTTCAAAAAGTATTAAACGGTATGATGACA TTGATGCGCGGAAATTATGATTGGTCGGATATTACTAGCCGTTATGCTACTAAACTT ACCGAATTGTCGTATGCTGCATGA |

| **Amino acid sequence (SEQ ID NO:28)** |
|---|
| MPNTAHLQQLARTVPATIPKLESPFPAQIHPLAAEVQQQTTKWVLEQGLLSEARRKAFLD YNYTLLIARCYPYASVERLQIISDWNAWLFELDDQCDEARIGHDPQALARLHQRLGAVIRG ATVAADEPLLVTALADIMGRMRALAPLAWFEVFTQQVDDYFIGNQWEAHNRLYSIIPSEIE YLHYRKFTSAVAIYIAFIEFMNASYLTADERQSIDLRNLQNWTNLVLSLCNDLVSVKKELRQ GDWHNYGAIVALKRGMSIPEALEIVANEHNHCIQQFITTTTQLKNCSVALQKVLNGMMTL MRGNYDWSDITSRYATKLTELSYAA |

**[Table 18-2]**

| **haur_2988A: base sequence obtained by optimizing codons of haur_2988 for expression in Streptomyces (SEQ ID NO:61)** |
|---|
| ATGCCAAACACAGCACACCTGCAGCAGTTGGCGAGGACGGTCCCAGCAACCATTCC CAAGTTGGAGTCACCGTTCCCTGCGCAGATCCACCCATTGGCAGCGGAGGTCCAGC AGCAGACGACCAAATGGGTGCTTGAGCAGGGCTTGTTGAGCGAGGCGCGGCGTAAA GCCTTCCTTGACTACAACTACACCTTGTTGATTGCCCGTTGCTACCCGTACGCGAGT GTCGAGCGGCTGCAGATTATCAGCGACTGGAACGCCTGGCTGTTCGAGCTTGACGA CCAGTGCGACGAGGCGCGAATTGGCCACGACCCTCAGGCGTTGGCCCGCTTGCACC AGCGGCTTGGGGCAGTCATTCGCGGAGCCACGGTCGCCGCCGACGAGCCACTGTTG GTCACAGCCCTCGCCGACATTATGGGCCGGATGCGGGCGCTGGCACCACTGGCATG GTTCGAGGTCTTCACCCAGCAGGTCGACGACTACTTCATTGGCAACCAGTGGGAGG CCCACAACCGGCTGTACTCGATTATTCCCAGCGAGATTGAGTACTTGCACTACCGTA AATTCACCAGTGCCGTCGCGATCTACATTGCGTTCATTGAGTTCATGAACGCAAGTT ACTTGACCGCCGACGAGCGTCAGTCAATCGACTTGCGCAACCTGCAGAACTGGACG AACCTTGTACTGAGTTTGTGCAACGACCTTGTCTCAGTCAAAAAAGAGTTGCGTCAG GGCGACTGGCACAACTACGGAGCAATTGTCGCATTGAAACGAGGCATGAGTATTCC TGAGGCCTTGGAGATCGTCGCCAACGAGCACAACCACTGCATTCAGCAGTTCATCA CAACCACCACCCAGCTGAAAAACTGCAGCGTGGCACTTCAGAAAGTATTGAACGGC ATGATGACATTGATGCGCGGAAACTACGACTGGTCGGACATTACCAGCCGTTACGC CACCAAACTTACCGAGTTGTCGTACGCCGCATGA |

### Produced terpenoid compound

### (1) α-serinene

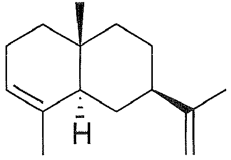

### 1-4-2. rcas_0622 (derived from Roseiflexus castenholzii DSM 13941)

**[Table 19-1]**

| **Table 19** |
|---|
| **Base sequence (SEQ ID NO:29)** |
| GTGGATCAGGACTATCGCGCACGACTTGTCTACCCATTTTCCGGAGCGATAAGCCC GCATGCCGATATAGTCGATCAGGCAACGCTCGCCTGGGCGGCGATGTTTGGCTTGT TGACGGACTCATTGCGCCACAAAAGTCGTCGCCTCCAGTATGGCTTGCTGGCTGCA CGCGCCTATCCACGAGCGGACCGTGAGATGTTGCAGATTGCCGCCGATTGGATTGC ATGGCTGTTCTTCATGGATGACCAATGCGACGAAACTGGCATTGGGCGTGATCTCCA ACGCATGATTGCGCTGCACGAACGATTTCTTGCAATTCTTGACGGCGCAACGCCGG AGGCGCATGATTGCGCGCTGACCTATGCGCTCGCCGATCTTCGTCGTCGACTGGCA CTGCGCGCTCCTGATAACTGGCTGCGCCGATTCAGCGAGCATGTGCGTCTCTACTT TACTGCCAATCGCTGGGAAACGGTCAACCGGCAGCGCGGTGCAACGCCGAATGTGG CGACATATTGCGCCGCGCGCCTGTTTTCGGGCGCCGTCTATGCATGCTTCGATCTG ATAGAACTGGCAGAGCAGATCGAACTGCCGTTCTATGCCCGTCACCACAGCATTGT GCAGCAACTGGAGCAGGCTGCGAACAATATCATTTGCTGGTGCAATGATGTGCTGT CGTATCCCAAGGAAATGCAGCACGGCGACCGGCACAATCTGGTGCTGGTCATTCAG GGCGAGCATCAATGCTCATTGCCCGAAGCCATTGACCGCGCGCTCGACCTTCATGC GCGTGAAGTCGCTACGTTTGTGCGGAAACGCACCTGTGTCCCATATTTCGATGCAG CGGTCAATACAGCGCTTGAAAAATATGTCACCGGATTGCAGTTCTGGATCTGCGCCA ATCGCGATTGGTCGCTGACGGCAACGCGCTACGCGCCGACGCACAAATCGCAGGAA ATGGTGATGGCAGTCGCGCAACAGTAA |

| **Amino acid sequence (SEQ ID NO:30)** |
|---|
| MDQDYRARLVYPFSGAISPHADIVDQATLAWAAMFGLLTDSLRHKSRRLQYGLLAARAYP RADREMLQIAADWIAWLFFMDDQCDETGIGRDLQRMIALHERFLAILDGATPEAHDCALT YALADLRRRLALRAPDNWLRRFSEHVRLYFTANRWETVNRQRGATPNVATYCAARLFSGA VYACFDLIELAEQIELPFYARHHSIVQQLEQAANNIICWCNDVLSYPKEMQHGDRHNLVLV IQGEHQCSLPEAIDRALDLHAREVATFVRKRTCVPYFDAAVNTALEKYVTGLQFWICANR DWSLTATRYAPTHKSQEMVMAVAQQ |

**[Table 19-2]**

| **rcas_0622A: base sequence obtained by optimizing codons of rcas_0622 for expression in Streptomyces (SEQ ID NO:62)** |
|---|
| ATGGACCAGGACTACCGCGCACGACTTGTCTACCCATTCTCCGGAGCGATCAGCCC GCACGCCGACATCGTCGACCAGGCAACGCTCGCCTGGGCGGCGATGTTCGGCTTGT TGACGGACTCATTGCGCCACAAAAGTCGTCGCCTCCAGTACGGCTTGCTGGCCGCA CGCGCCTACCCACGAGCGGACCGTGAGATGTTGCAGATTGCCGCCGACTGGATTGC ATGGCTGTTCTTCATGGACGACCAGTGCGACGAGACCGGCATTGGGCGTGACCTCC AGCGCATGATTGCGCTGCACGAGCGATTCCTTGCAATTCTTGACGGCGCAACGCCG GAGGCGCACGACTGCGCGCTGACCTACGCGCTCGCCGACCTTCGTCGTCGACTGGC ACTGCGCGCCCCTGACAACTGGCTGCGCCGATTCAGCGAGCACGTGCGTCTCTACT TCACCGCCAACCGCTGGGAGACGGTCAACCGGCAGCGCGGCGCAACGCCGAACGT GGCGACATACTGCGCCGCGCGCCTGTTCTCGGGCGCCGTCTACGCATGCTTCGACC TGATCGAGCTGGCAGAGCAGATCGAGCTGCCGTTCTACGCCCGTCACCACAGCATT GTGCAGCAGCTGGAGCAGGCCGCGAACAACATCATTTGCTGGTGCAACGACGTGCT GTCGTACCCCAAGGAGATGCAGCACGGCGACCGGCACAACCTGGTGCTGGTCATTC AGGGCGAGCACCAGTGCTCATTGCCCGAGGCCATTGACCGCGCGCTCGACCTTCAC GCGCGTGAGGTCGCCACGTTCGTGCGGAAACGCACCTGCGTCCCATACTTCGACGC AGCGGTCAACACAGCGCTTGAGAAATACGTCACCGGATTGCAGTTCTGGATCTGCG CCAACCGCGACTGGTCGCTGACGGCAACGCGCTACGCGCCGACGCACAAATCGCAG GAGATGGTGATGGCAGTCGCGCAGCAGTAG |

### Produced terpenoid compound

### (1) (+)-T-muurolol

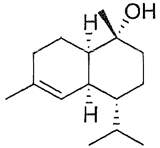

### 1-4-3. roseRS_3509 (derived from Roseiflexus sp. RS-1)

**[Table 20-1]**

| **Table 20** |
|---|
| **Base sequence (SEQ ID NO:31)** |
| GTGGATCGGGTCGATGGCGCACAGATCGTCTACCCGTTTACCGGTGCGATAAGCCC CTATGCTGGCGATGTCGATCAGGCAACACTTATCTGGGCAGAGGCGTCTGGACTTC TTACCGATGGGTTGCGCCAGAAAAGTCAGCGTCTCCAGTATGGAGTACTGGCTGCG CGCGCCTATCCACAGGCAGATCGTGATACGCTGCAAATTGCTGCCGATTGGATAGC CTGGCTGTTTTTCATGGACGATCAGTGTGATGAGGCTGGCATTGGTCGCGATCCGCA GCGCCTGGCTGCGCTGCACGAACGATTCCTCGCAGTGCTCGAAGGAGACCCACCTG ATAGTGGCGACTGGAATCTGACCCGCGCTCTCGCCGATATTCGCCGTCGGCTGGCC GCGCGCGCAACCGATGATTGGTTGCGCCGATTCGGAGAGCACGTGCGTCTCTACTT TACGGCGAATCGCTGGGAAGCGGCAAACCGATGCCAGAGCATCGTTCCAAACGTGG CGACCTATTGTGCTGCGCGCCTGTTTTCAGGCGCAGTCTATGCCTGCTTTGACCTGA TAGAACTGGCAGCAGGGATCGATCTTCCCTTTATGCACGTTATCATGCTGCCGTTC AACAACTCGAGCGGACGGCAAACAATATCATTTGCTGGTGCAATGATATGTTGTCGT ACCCCAAAGAGATGCAGCACGGTGATGTGCACAATCTGGTACTGGCTATCCGGCAG GAATATCAATGTTCGTTGAGTGAGGCGCTGCAGCAGGCGCTTCTGCTCCACGACCG GGAGACGGACACGTTTATGAAAACACAGAAGCAACTGCCGCGCTTCAATCCCGCCG TTGATATGGCGCTTGAGCGCTATATTGATGGTTTACAGTACTGGATATGCGCCAACC GTGACTGGTCATTGACAGCAATGCGCTATGCTTTGTCCGGAACCGAACCCGTACTTC GGACGAGGTTTGCACATTCGTGA |

| **Amino acid sequence (SEQ ID NO:32)** |
|---|
| MDRVDGAQIVYPFTGAISPYAGDVDQATLIWAEASGLLTDGLRQKSQRLQYGVLAARAYP QADRDTLQIAADWIAWLFFMDDQCDEAGIGRDPQRLAALHERFLAVLEGDPPDSGDWNL TRALADIRRRLAARATDDWLRRFGEHVRLYFTANRWEAANRCQSIVPNVATYCAARLFSG AVYACFDLIELAAGIDLPFYARYHAAVQQLERTANNIICWCNDMLSYPKEMQHGDVHNLV LAIRQEYQCSLSEALQQALLLHDRETDTFMKTQKQLPRFNPAVDMALERYIDGLQYWICA NRDWSLTAMRYALSGTEPVLRTRFAHS |

**[Table 20-2]**

| roseRS_3509A: base sequence obtained by optimizing codons of roseRS_3509 for expression in Streptomyces (SEQ ID NO:63) |
|---|
| ATGGACCGGGTCGACGGCGCACAGATCGTCTACCCGTTCACCGGCGCGATCAGCCC CTACGCCGGCGACGTCGACCAGGCAACACTTATCTGGGCAGAGGCGTCCGGACTTC TTACCGACGGGTTGCGCCAGAAAAGTCAGCGTCTCCAGTACGGAGTACTGGCCGCG CGCGCCTACCCACAGGCAGACCGTGACACGCTGCAGATTGCCGCCGACTGGATCGC CTGGCTGTTCTTCATGGACGACCAGTGCGACGAGGCCGGCATTGGCCGCGACCCGC AGCGCCTGGCCGCGCTGCACGAGCGATTCCTCGCAGTGCTCGAGGGAGACCCACCT GACAGTGGCGACTGGAACCTGACCCGCGCCCTCGCCGACATTCGCCGTCGGCTGGC CGCGCGCGCAACCGACGACTGGTTGCGCCGATTCGGAGAGCACGTGCGTCTCTACT TCACGGCGAACCGCTGGGAGGCGGCAAACCGATGCCAGAGCATCGTCCCAAACGTG GCGACCTACTGCGCCGCGCGCCTGTTCTCAGGCGCAGTCTACGCCTGCTTCGACCT GATCGAGCTGGCAGCAGGGATCGACCTTCCCTTCTACGCACGTTACCACGCCGCCG TCCAGCAGCTCGAGCGGACGGCAAACAACATCATTTGCTGGTGCAACGACATGTTG TCGTACCCCAAAGAGATGCAGCACGGCGACGTGCACAACCTGGTACTGGCCATCCG GCAGGAGTACCAGTGCTCGTTGAGTGAGGCGCTGCAGCAGGCGCTTCTGCTCCACG ACCGGGAGACGGACACGTTCATGAAAACACAGAAGCAGCTGCCGCGCTTCAACCCC GCCGTCGACATGGCGCTTGAGCGCTACATTGACGGCTTGCAGTACTGGATCTGCGC CAACCGTGACTGGTCATTGACAGCAATGCGCTACGCCTTGTCCGGAACCGAGCCCG TACTTCGGACGAGGTTCGCACACTCGTGA |

### Produced terpenoid compound

### (1) (+)-T-muurolol

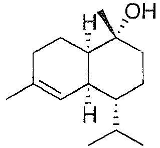

### 1-4-4. sce6369 (derived from Sorangium cellulosum 'So ce 56')

**[Table 21]**

| Table 21 |
|---|
| Base sequence (SEQ ID NO:33) |
| GTGCACAGAGCTCTACTTTGTCCTTTTCCCGCCACGACGCCCCACCCGCAGGCGGC ACAGCTGGCGAACGATTGCCTCGAATGGACTCGCAAGTGCGGCCTGCTCCCCGATG AATCGCCACGAACGCTCGACAAGGTGCGCAGCTACTCGGCGCTCGCCGCGCACTGT TACCCCGATGCGCATTTCGAGCGGCTTCGGGCGATCTGCGACTACTACAGCTGGCT CTTCTTCTTCGATGACGTGTGCGAGAATACGTCACTGAACGGCGCCGAACCGAAGG TTGTCTCGAGCCTGCTGTTCGATGTATACGGGGTGCTGCGAGGGCCCACGGCTGCC GTTGGGCATGCGCCATTTGCCCAAGCATTGGCGGACATCTGGCGACGCATCGGCGA TGGTTGTCCTGGGTTCTGGCGGCGGCGCCTCATCCGCCACGTCGAAAACTACATCG ATGGCTGTGTCTGGGAAGCGCAAAACCGCCAGCTCGACCGCGTACCCAGTCGCGCG GTGTTCGAGGGTATGCGCATGCACACGTCCACCATGTACGAGTTTTGGGACTTCATC GAGTATGCCGGCGACCTGTTCTTGCCGGATGAGGTCGTAGAACATCCCTTGGTGGC GGAGGTGCGTCGCGCCGGCAACGCCATCGCGTCTTTTGCCAATGACATTTACTCGC TGCGCAAAGAGACGAGCAACCGCGACGTGCACAATTTGGTTGTCGTGCTCATGCAC GAAGAGCGCATCGAGCTCGAAGCAGCGTACGCGCGCGCCGCTGGTATCCACGATGC TCAAGTCGAGCACTTCCTGGATCTGGTGAAGCACCTGCCTACGTTTTCTGCGACCAT CGATCGGAACCTGGCGCGTTACGTGGAGGGCATACGCATCTGGATCCGCGCCAACC ACGATTGGTCGATTGTCACGCCCCGTTACAATGAGCCCGATGCGCGATGA |

| Amino acid sequence (SEQ ID NO:34) |
|---|
| MHRALLCPFPATTPHPQAAQLANDCLEWTRKCGLLPDESPRTLDKVRSYSALAAHCYPDA HFERLRAICDYYSWLFFFDDVCENTSLNGAEPKVVSSLLFDVYGVLRGPTAAVGHAPFAQ ALADIWRRIGDGCPGFWRRRLIRHVENYIDGCVWEAQNRQLDRVPSRAVFEGMRMHTSTM YEFWDFIEYAGDLFLPDEVVEHPLVAEVRRAGNAIASFANDIYSLRKETSNRDVHNLVVVL MHEERIELEAAYARAAGIHDAQVEHFLDLVKHLPTFSATIDRNLARYVEGIRIWIRANHDWS IVTPRYNEPDAR |

| sce6369A: base sequence obtained by optimizing codons of sce6369 for expression in Streptomyces (SEQ ID NO:64) |
|---|
| ATGCACAGAGCCCTCCTGTGTCCTTTCCCCGCCACGACGCCCCACCCGCAGGCGGC ACAGCTGGCGAACGATTGCCTCGAATGGACTCGCAAGTGCGGCCTGCTCCCCGATG AATCGCCACGAACGCTCGACAAGGTGCGCAGCTACTCGGCGCTCGCCGCGCACTGT TACCCCGATGCGCATTTCGAGCGGCTTCGGGCGATCTGCGACTACTACAGCTGGCT CTTCTTCTTCGATGACGTGTGCGAGAATACGTCACTGAACGGCGCCGAACCGAAGG TTGTCTCGAGCCTGCTGTTCGATGTATACGGGGTGCTGCGAGGGCCCACGGCTGCC GTTGGGCATGCGCCATTCGCCCAGGCGTTGGCGGACATCTGGCGTCGCATCGGCGA TGGTTGTCCTGGGTTCTGGCGGCGGCGCCTCATCCGCCACGTCGAAAACTACATCG ATGGCTGTGTCTGGGAAGCGCAAAACCGCCAGCTCGACCGCGTACCCAGTCGCGCG GTGTTCGAGGGTATGCGCATGCACACGTCCACCATGTACGAGTTCTGGGACTTCAT CGAGTATGCCGGCGACCTGTTCTTGCCGGATGAGGTCGTAGAACATCCCTTGGTGG CGGAGGTGCGTCGCGCCGGCAACGCCATCGCGTCTTTCGCCAATGACATTTACTCG CTGCGCAAAGAGACGAGCAACCGCGACGTGCACAATTTGGTTGTCGTGCTCATGCA CGAAGAGCGCATCGAGCTCGAAGCAGCGTACGCGCGCGCCGCTGGTATCCACGATG CTCAAGTCGAGCACTTCCTGGATCTGGTGAAGCACCTGCCCACGTTCTCTGCGACC ATCGACCGGAACCTGGCGCGTTACGTGGAGGGCATACGCATCTGGATCCGCGCCAA CCACGATTGGTCGATTGTCACGCCCCGTTACAATGAGCCCGATGCGCGATGA |

### Produced terpenoid compound

### (1) α-cubebene

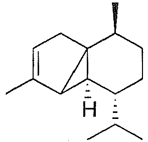

### (2) β-cubebene

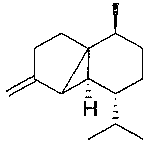

### (3) cadina-3,5-diene

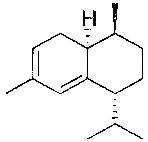

### (4) bicyclosesquiphellandrene

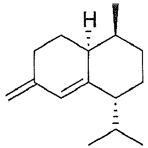

### 1-4-5. sce8552 (derived from Sorangium cellulosum 'So ce 56')

**[Table 22-1]**

| Table 22 |
|---|
| Base sequence (SEQ ID NO:35) |
| ATGTCATCTGATCGAACTAGCGTTGTCGTCAGCAAGCGCGACGCTGGTGGATTCGA ATATCCGTTTGCTGCGTCGTGTCACCCCGGGAGAGAGGTCACGGAGCAAAGGACCC TGGCGTGGGTTCGCCGGTTGCGTCTGGTGCCAGACGGTCGGTCGCTTTCACGTCTC AAGGCGACGAACTTCTCCCACCTGGCGGCATGGCTCTTACCGTCCGCGAGCACGCA GACGCTCCAGCTGGCATCGGACTTCACGGCCGTGCTGTTCCTCCTGGATGATGCGT ACGACGAGGGGCAGCTCAGCACGGACCCGGAATCCGTCGAGTGGCTCAACGAGAAG TACCTGGGTGAGCTCTTCGGGTACACCGAAGCGGACATGAGCGACCCCCTGACGCG CGGGATGCTCGATGTACGTGAGCGCATCCGACGCAGCCATCCGCACTTCTTCCTGA ATCGCTGGCTCTCGCACTTTCAGTACTACTACGAGGCCAACCTGTGGGAGGCGAAT AACCGAAAGCAGATGAGGGTGCCGCATCTTGAAGAATATTTGATGATGCGGCGCTA CAGCGGCGCTGTCTATACCTACTGTGACCTGCTCGAGCTGCTGCTCGAACGGCCGC TGCCGCTCGAGGTCGTCCAGCACCCGTTGATTCAGACGGTTCGAGACATCTGTAAT GACATCCTCTGCTGGACGAACGACTACTTCTCGCTCGGGAAGGAGCTGACCAACGG CGAGACGCACAACCTGATCGTCGTTCTCCGGAACGAATGCGTGAGCACGCTGGAAG AGGCGATCGACCGGCTCAAGGACATGCACGACCGCCGGGTCGCGGAGTACCAAGGC GTGAAGGAGAAGGTGCTCGCGCTCTGGGCGGACGACGAGATCCGCCTCTACCTGGA CGCGGTCGAGGCCATGATCGCCGGGAATCAGCGCTGGGCGCTCGAGGCCGGTCGC TATTCTGGACTAGAAAGTTTGATCGTGCGCGCTGGATGA |

| Amino acid sequence (SEQ ID NO:36) |
|---|
| MSSDRTSVVVSKRDAGGFEYPFAASCHPGREVTEQRTLAWVRRLRLVPDGRSLSRLKATN FSHLAAWLLPSASTQTLQLASDFTAVLFLLDDAYDEGQLSTDPESVEWLNEKYLGELFGY TEADMSDPLTRGMLDVRERIRRSHPHFFLNRWLSHFQYYYEANLWEANNRKQMRVPHLEE YLMMRRYSGAVYTYCDLLELLLERPLPLEVVQHPLIQTVRDICNDILCWTNDYFSLGKELT NGETHNLIVVLRNECVSTLEEAIDRLKDMHDRRVAEYQGVKEKVLALWADDEIRLYLDAV EAMIAGNQRWALEAGRYSGLESLIVRAG |

**[Table 22-2]**

| sce8552A: base sequence obtained by optimizing codons of sce8552 for expression in Streptomyces (SEQ ID NO:65) |
|---|
| ATGTCATCCGACCGAACCAGCGTCGTCAGCAAGCGCGACGCCGGCGGATTCGA GTACCCGTTCGCCGCGTCGTGCCACCCCGGAGGAGGTCACGGAGCAGAGGACC CTGGCGTGGGTCCGCCGGTTGCGTCTGGTGCCAGACGGCCGGTCGCTTTCACGTCT CAAGGCGACGAACTTCTCCCACCTGGCGGCATGGCTCTTGCCGTCCGCGAGCACGC AGACGCTCCAGCTGGCATCGGACTTCACGGCCGTGCTGTTCCTCCTGGACGACGCG TACGACGAGGGGCAGCTCAGCACGGACCCGGAGTCCGTCGAGTGGCTCAACGAGAA GTACCTGGGCGAGCTCTTCGGGTACACCGAGGCGGACATGAGCGACCCCCTGACGC GCGGGATGCTCGACGTACGTGAGCGCATCCGACGCAGCCACCCGCACTTCTTCCTG AACCGCTGGCTCTCGCACTTCCAGTACTACTACGAGGCCAACCTGTGGGAGGCGAA CAACCGAAAGCAGATGAGGGTGCCGCACCTTGAGGAGTACTTGATGATGCGGCGCT ACAGCGGCGCCGTCTACACCTACTGCGACCTGCTCGAGCTGCTGCTCGAGCGGCCG CTGCCGCTCGAGGTCGTCCAGCACCCGTTGATTCAGACGGTCCGAGACATCTGCAA CGACATCCTCTGCTGGACGAACGACTACTTCTCGCTCGGGAAGGAGCTGACCAACG GCGAGACGCACAACCTGATCGTCGTCCTCCGGAACGAGTGCGTGAGCACGCTGGAG GAGGCGATCGACCGGCTCAAGGACATGCACGACCGCCGGGTCGCGGAGTACCAGG GCGTGAAGGAGAAGGTGCTCGCGCTCTGGGCGGACGACGAGATCCGCCTCTACCTG GACGCGGTCGAGGCCATGATCGCCGGGAACCAGCGCTGGGCGCTCGAGGCCGGCC GCTACTCCGGACTGGAGAGTTTGATCGTGCGCGCCGGATGA |

### Produced terpenoid compound

### (1) eremophilene

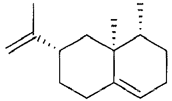

### 1-4-6. slt_1880 (derived from Streptomyces lactacystinaeus)

**[Table A]**

| : Table A Base sequence (SEQ ID NO:92) |
|---|
| ATGACAACCACGGCAGAGATCCTCTCCCCGGACCCGGAGGCCCCGGCCCTCGACGAGCT CCCCTTCCCCCGCCGGATCAGCCCGGACTTCCGGCCGCCCACGACCGCCACCTCGCCT GGCCGCGCTCCTTCGGCTTCCTGTCCACGGAAGCGGAGCGGGCCCACCACCTCAAGGGC CAGTTCCCGCTGATCGCGGCCATGTTCTATCCGAACGCGACCGGGAGCGAACTGGACAT CGGCGTCGACCAGCAGAGCTGGTACTTCCTGTTCGACGACGCGCTCGACGAACAGTGGG GCGGCTCGCCCGAGCGGGTCCGCCACCTCGTCGGACTCGTCCAGGAGGGTGTCACCGGC CAGGCCTCGCCGCTGCCGCCGGCCGCCGCCTTCGCCGACATGCGACGACGCAGCTGCCA CGGCATGCCCGAGGACTGGATCCGGCGCTCGGCGGCCCACTGGTCCTCCTACCTCGACC ACCACGTCCACGAGGCGCGCAGCCGGCAGTCCGGTGCGCCCATGCCGCTGAGCACCTAC CTGCGGGTCCGGCGCCACACCATCGGGGTGGCACCGGTCATCGACCTCGCCGAGCGGCT GTCCTCCTGCGTCCTGCCCGACCACCTGTACGCGCTGCCGCACCTGTCCGTCATGCGCG AGATGACGAAGACCTTCATCATCTGCGACAACGACATCGTCTCCCTGGAGAAGGACGCGG CCCTGGGGGAGCAGAACAACCTGGTGCTCTGCCTGGAACGGGACCACGGGCTCTCCCGG CCGCAGGCGGTCGACCGGGCGCTGCGCCGCCGCGCCGAAGCCCTGGAGCTGTTCACCGG CGCCCACCGGGCCCTGCTCGGCAGCACGGCGACGGGCCACCTCGACCCGGCCGAACGC GACCTGCTGCAGCGCTACTGCACCGAGGCGCTGCAGACCACCATCCGCGGCGCGTACGA CTGGCACCACGCCTCCACCCGGTACCACGGGTAG |

| Amino acid sequence (SEQ ID NO:93) |
|---|
| MTTTAEILSPDPEAPALDELPFPRRISPDFRAAHDRHLAWPRSFGFLSTEAERAHHLKGQFPLIAA MFYPNATGSELDIGVDQQSWYFLFDDALDEQWGGSPERVRHLVGLVQEGVTGQASPLPPAAAF ADMRRRSCHGMPEDWIRRSAAHWSSYLDHHVHEARSRQSGAPMPLSTYLRVRRETIGVAPVIDL AERLSSCVLPDHLYALPHLSVMREMTKTFIICDNDIVSLEKDAALGEQNNLVLCLERDHGLSRPQ AVDRALRRRAEALELFTGAHRALLGSTATGHLDPAERDLLQRYCTEALQTTIRGAYDWHHAST RYHG |

### Produced terpenoid compound

### (1) Lactan-4(5)-ene

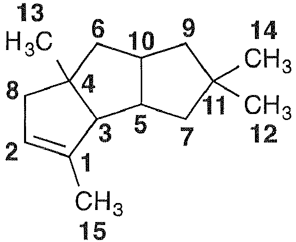

A protein consisting of an amino acid sequence having 80% or more, 90% or more, 95% or more, or 97% or more identity to the amino acid sequence selected from the group consisting of SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, and SEQ ID NO:93, wherein the protein is capable acting as a terpenoid synthetase is included within the scope of the present invention.

A terpenoid synthetase gene of the present invention include the gene encoding a protein consisting of an amino acid sequence having 80% or more, 90% or more, 95% or more, or 97% or more identity to the amino acid sequence selected from the group consisting of SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, and SEQ ID NO:93. Examples of terpenoid synthetase gene include the gene consisting of a base sequence having 80% or more, 90% or more, 95% or more, or 97% or more identity to gene selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:58, SEQ ID NO:17, SEQ ID NO:19, SEQ ID NO:21, SEQ ID NO:23, SEQ ID NO:59, SEQ ID NO:25, SEQ ID NO:60, SEQ ID NO:27, SEQ ID NO:61, SEQ ID NO:29, SEQ ID NO:62, SEQ ID NO:31, SEQ ID NO:63, SEQ ID NO:33, SEQ ID NO:64, SEQ ID NO:35, SEQ ID NO:65, and SEQ ID NO:92.

### [Example]

The following Production examples serve to illustrate the present invention more specifically, which does not intend to limit the present invention.

### Synthesis of monoterpenoid

### Production example 1: sclav_p0982 gene (SEQ ID NO:1)

### 1. Construction of Chromosomal integration vector used for Synthesis

(1) PCR was performed to amplify sclav_p0982 gene (SEQ ID NO:1) by using the DNA derived from *S. clavuligerus* ATCC 27064 as a template and the primer pair in the following table.

| | |
|---|---|
| Forward primer | 5' -T T T T C T A G A A T G C C C G C C G G C C A C G A A G A -3' (SEQ ID No: 4 4) |
| | (The underlined TCTAGA is Xbal site. The ATG indicated by boldface is the initiation codon of sclav_p0982.) |
| Reverse primer | 5'-TTTAAGCTTTCACCAAGGGGTGGTGGCCC -3' (SEQ ID No:45) |
| | (The underlined AAGCTT is HindIII site. The TCA indicated by boldface is the termination codon of sclav_p0982.) |

The initial denaturation step (98°C, 2 min) was followed by 30 cycles of amplification (95°C, 15 sec.; 55°C, 15 sec.; 72°C, 60 sec.) and then a final incubation (72° C, 10 min) (Roche; Expand High Fidelity PCR System, Biometra GmbH (Germany); Tgradient Thermocycler).
(2) The resulting segment was digested with XbaI and HindIII (New England Biolabs) to give a monoterpenoid synthetase gene sclav_p0982.

pKU460-rpsJp was prepared by the method according to Non-patent document 12. "rpsJ" and "rpsJp" used in the description, the claims, and the figures of this application is the same as "rpsJp" described in Non-patent document 12.

Specifically, PCR was performed by using pBSL141 (Non-patent document 14) as a template and the forward primer (5'-CTCGAGcaattgGACATAAGCCTGTTCGGTTCGTAA-3' (SEQ ID NO:66)) and the reverse primer (5'-CTCGAGgctagcGATCTCGGCTTGAACGAATTGTTA-3' (SEQ ID NO:67)). The initial denaturation step (98° C, 2 min) was followed by 30 cycles of amplification (95°C, 15 sec.; 55°C, 15 sec.; 72°C, 50 sec.) and then a final incubation (72° C, 10 min). The resulting 794-bp fragment comprising a gentamicin-resistant gene aac(3)I was digested with NheI and MfeI (Invitrogen).

PCR was performed by using a cosmid clone CL_218_C01 (http://avermitilis.ls.kitasato-u.ac.jp/cosmid/index.cgi), which comprises rpsJ gene from *S. avermitilis* as a template and the forward primer (5'-CTCGAGcaattgGACATAAGCCTGTTCGGTTCGTAA-3' (SEQ ID NO:68)) and the reverse primer (5'-CTCGAGgctagcGATCTCGGCTTGAACGAATTGTTA-3' (SEQ ID NO:69)). The initial denaturation step (98° C, 2 min) was followed by 30 cycles of amplification (95°C, 15 sec.; 55°C, 15 sec.; 72°C, 50 sec.) and then a final incubation (72°C, 10 min). The resulting 573-bp fragment comprising a rpsJ promoter sequence was digested with BamHI and EcoRI (Invitrogen).

The NheI/MfeI fragment comprising a gentamicin-resistant gene aac(3)I, the EcoRI/BamHI fragment comprising a rpsJ promoter, and pKU460 which had been digested with NheI and BamHI were mixed, and ligated using T4 DNA ligase (Invitrogen) in the presence of ATP. *E.coli* was transformed with the resulting fragment, and then spread on LA agar medium containing kanamycin and gentamicin. The plasmid DNA was extracted from the resulting colony, and digested with restriction enzymes (NdeI and HindIII: Invitrogen) to give pKU460-rpsJp.

PCR was performed to amplify a gps gene by using a chromosomal DNA of *Streptomyces* sp. KO-3988, which has a gps gene, as a template and the forward primer: 5'-CTCGAGCTCATATGACCACCGACACCGGCCAGGAC-3' (SEQ ID NO:70) (CATATG=NdeI); and the reverse primer: 5'-AGTCTAGATCAGGCGGTGCGGTAGGCGACCCG-3' (SEQ ID NO:71) (TCTAGA=XbaI). The initial denaturation step (98° C, 2 min) was followed by 30 cycles of amplification (95° C, 15 sec.; 55° C, 15 sec.; 72° C, 70 sec.) and then a final incubation (72° C, 10 min). The resulting PCR-amplified fragment was digested with NdeI/XbaI, and pKU460-rpsJp was digested with NdeI/XbaI (New England Biolabs). The digested PCR-amplified fragment, the digested pKU460-rpsJp, an aqueous solution (15 µL) containing 20 mM Tris-HCl (pH 7.5), 10 mM MgCl₂, 10 mM DTT, and 0.1 mM ATP, and T4 DNA ligase (0.25 µL) are mixed. The resulting mixture was allowed to stand at 14°C overnight for the ligation reaction, to give pKU460-rpsJ-gps.

pKU460-rpsJp-gps was digested with XbaI/HindIII. The digested pKU460-rpsJp-gps (0.1 µg) and the above monoterpenoid synthetase gene sclav_p0982 (0.2 µg) digested with XbaI/HindIII were mixed.

To the mixture were added an aqueous solution (15 µL) containing 20 mM Tris-HCl (pH 7.5), 10 mM MgCl₂, 10 mM DTT, and 0.1 mM ATP, and T4 DNA ligase (0.25 µl). The resulting mixture was allowed to stand at 14°C overnight for the ligation reaction, to give pKU460-rpsJ-gps-sclav_p0982.

(3) Ethanol precipitation was performed to remove salts from the ligation product (50 to 100 ng). The resultant was suspended into sterilized water (5 µL). The mixture was added to *E. coli* DH5α 50 µL (concentration: 1 x 10⁹/ mL), and then transferred to a cuvette with a 1 mm gap. Electroporation wherein electrical pulse was applied at 18 KV/cm (200 Ω, 25 µF) by using Gene Pulser (BioRad) was performed.

The resulting transformant was selected by using a kanamycin (50 µg/mL)-containing LA medium (1% triptone (Difco), 0.5% yeast extract (Difco), 0.5% NaCl, 1.5% agar, pH 7.2). The plasmid was extracted from the resulting colony by using QIAquick Plasmid DNA Extraction Kit (QIAGEN), and then was digested with restriction enzymes XbaI and HindIII to confirm that the fragment of interest was inserted.

The sclav_p0982 sequence in the recombinant plasmid was confirmed by DNA sequencer (Life Technologies, ABI3100).

### 2. Introduction of Chromosomal integration vector into S. avermitilis SUKA 17

To prepare an unmethylated DNA, the recombinant plasmid: pKU460-rpsJ-gps-sclav_p0982 was introduced into *E. coli* GM2929 hsdS::Tn10 (Non-patent document 13, Non-patent document 5) by electroporation in a similar way to the above.

Specifically, ethanol precipitation was performed to remove salts from the recombinant plasmid. The resultant was suspended into sterilized water. The mixture was added to *E. coli* GM2929 hsdS::Tn10, and then transferred to a cuvette with a 1 mm gap. Electroporation wherein electrical pulse was applied at 18KV/cm (200 Ω, 25 µF) by using Gene Pulser (BioRad) was performed.

The resulting transformant was selected by using LA medium (1% triptone (Difco), 0.5% yeast extract (Difco), 0.5% NaCl, 1.5% agar, pH 7.2) which contains kanamycin (50 µg/mL) and chloramphenicol (15µg/ml). The plasmid was extracted from the resulting colony by using QIAquick Plasmid DNA Extraction Kit (QIAGEN).

A suspension of spores of SUKA 17 was inoculated into 10 mL of TSB medium (Tryptone soya bouillon; NISSUI PHARMACEUTICAL CO., LTD) and incubated at 30°C for 2 days to give a preculture. The preculture (0.5 mL) (1%) was inoculated into TSB medium (50 mL) which contains 0.4% (final concentration) glycine and 5 mM magnesium chloride and incubated at 30°C for 16 to 20 hours. After the incubation, the bacterial cells were harvested by centrifugation at 3,000 rpm (10 min) and washed twice with 20 mL of P10 medium.

The P10 medium was prepared as follows.

Sucrose 103 g, potassium sulfate 0.25 g, magnesium chloride 2.03 g, and a microelement solution 2 mL were dissolved in 800 mL of water. The resulting solution was sterilized by high-pressure steam. When used, to the resulting solution were added 0.5% monopotassium phosphate 10 mL, 3.68% calcium chloride dihydrate 100 mL, and 0.25 M MES (2-morpholinoethanesulfonic acid, pH 6.5) 100 mL, each of which had been sterilized separately, to give the P10 medium.

The microelement solution was prepared as follows:

Zinc chloride hexahydrate 40 mg, ferric chloride hexahydrate 200 mg, copper(II) chloride dihydrate 10 mg, manganese chloride tetrahydrate 10 mg, sodium borate decahydrate 10 mg, and ammonium molybdate tetrahydrate 10 mg were dissolved in 1000 mL of water and then the solution was sterilized by filtration.

To the resulting bacterial cell was added 32 mL of P10 medium which contains 32 mg of lysozyme. The mixture was warmed at 30°C for 30 min. During warming, the mixture was occasionally shaken softly so that the protoplast was formed. To the mixture was further added 40 mL of P20 medium to give a suspension. Then the suspension was filtered through a cotton plug to remove the mycelia. The filtrate was centrifuged at 3,000 rpm for 10 min to harvest the protoplast. The protoplast was softly mixed with 10 mL of P20 medium. The resulting suspension was again centrifuged to precipitate the protoplast. This was repeated twice. The resulting protoplast was softly mixed with 1 mL of P20 medium which contains 7% (final concentration) DMSO. The resulting suspension was divided into each 50 µL, and cryopreserved at -80°C.

The above P20 medium was prepared as follows.

Sucrose 200 g, potassium sulfate 0.25 g, magnesium chloride 2.03 g, and the microelement solution 2 mL were dissolved in 800 mL of water. The resulting solution was sterilized by high-pressure steam. When used, to resulting solution were added 0.5% dipotassium phosphate 10 mL, 3.68% calcium chloride dihydrate 100 mL, 0.25 M MES 100 mL, each of which had been sterilized separately, to give the P20 medium.

The DNA 5 µL prepared by using *E.coli* GM2929 hsdS::Tn10 and the protoplast 50 µL were mixed. To the mixture was added 500 µL of 25% PEG solution. After still standing (1 min), 450 µL of P20 medium was added and the resulting mixture was shaken.

The above 25% PEG solution was prepared as follows.

Sucrose 2.5 g and polyethyleneglycol (#6000) 25 g were dissolved in water 100 mL. The solution was adjusted to pH 9.0 by adding 2 N potassium hydroxide. The resulting solution was sterilized by high-pressure steam (121°C, 15 min). When used, to the solution were added 0.5% dipotassium phosphate 10 µL/ mL, 5 M calcium chloride 20 µL/ mL, 1 M Tris-maleic acid (pH 8.0) 50 µL/ mL, each of which had been sterilized separately, to give the 25% PEG solution.

A portion of the above resulting suspension containing the DNA and the protoplast was spread together with 2.5 mL of RM14 soft agar medium on RM14 medium, and then the resulting medium was incubated at 30°C for 16 to 20 hours. After incubation, 2.5 mL of RM14 soft agar medium containing 100 µg/mL neomycin was spread on the medium, and the resulting medium was incubated at 30°C for 5 days.

The above RM14 soft agar medium was prepared as follows.

Sucrose 200 g, dipotassium sulfate 0.25 g, magnesium chloride hexahydrate 10.12 g, glucose 10 g, casamino acid (manufactured by Difco) 0.1 g, L-proline 3 g, yeast extract (manufactured by Difco) 2 g, and the microelement solution 2 mL were dissolved in water 870 mL. To the solution were added oatmeal agar medium (manufactured by Difco) 3 g and agar 5 g, and the mixture was sterilized by high-pressure steam. When used, to the resulting mixture were added 0.5% monopotassium phosphate 10 mL, 3.68% calcium chloride dihydrate 80 mL, and 0.25 M MES 40 mL, each of which had been sterilized separately, to give the RM14 soft agar medium.

The above RM14 medium was prepared as follows.

Sucrose 200 g, dipotassium sulfate 0.25 g, magnesium chloride hexahydrate 10.12 g, glucose 10 g, casamino acid (manufactured by Difco) 0.1 g, L-proline 3 g, yeast extract (manufactured by Difco) 2 g, and the microelement solution 2 mL were dissolved in water 870 mL. To the solution were added oatmeal agar medium (manufactured by Difco) 3 g and agar 20 g, and the mixture was sterilized by high-pressure steam. When used, 0.5% monopotassium phosphate 10 mL, 3.68% calcium chloride dihydrate 80 mL, 0.25 M MES 40 mL, each of which had been sterilized separately, to give the RM14 medium.

The resulting colony was transferred on YMS⁺⁺ medium containing 1 µg/mL neomycin in a patchy fashion and incubated. Spores of the resistant colony were suspended in 200 µL of sterilized water, spread on YMS⁺⁺ medium, and then incubated at 30°C for 5 days. The resulting spores were suspended in 20% glycerol, and then stored at -30°C.

The above YMS⁺⁺ medium was prepared as follows.

Yeast extract (manufactured by Difco) 4g, malt extract (manufactured by Difco) 10 g, and soluble starch 4g were dissolved in 1000 mL of water. The solution was adjusted to pH 7.4 by adding 2 N potassium hydroxide. Then agar was added to the solution so that the concentration is 2%. The mixture was sterilized by high-pressure steam (121°C, 15 min). When used, to the mixture were added 1 M magnesium chloride (final concentration 10 mM) and 1 M potassium nitrate(final concentration 8 mM), each of which had been sterilized separately.

### 3. Cultivation for Synthesizing Monoterpenoid compound and Analysis of Product

10 mL of a seed medium was added into a large-size test tube containing a spring coil. The suspension of spores of the SUKA 17-transformant was inoculate into the tube, and cultured with shaking at 30°C for 2 days to give a seed culture.

The above seed medium was prepared as follows.

Glucose 5 g, defatted soy flour 15 g, and yeast extract (manufactured by Difco) 5 g were dissolved in 1000 mL of water. The solution was adjusted to pH 7.4 by adding 2 N potassium hydroxide, and sterilized by high-pressure steam (121°C, 15 min)

10 mL of a medium for synthesis was added into a 125 mL Erlenmeyer flask made of polycarbonate. The seed culture 0.1 mL (1%) was inoculated into the flask, and cultured with shaking (200 rpm) at 28°C for 5 days.

The above medium for synthesis was prepared as follows.

Glucose 60 g, calcium carbonate 5 g, sodium chloride 2 g, dipotassium hydrogen phosphate 0.5 g, 0.5% iron sulfate heptahydrate 10 mL, 0.5% zinc sulfate heptahydrate 10 mL, 0.5% manganese sulfate tetrahydrate 10 mL, 1% magnesium sulfate heptahydrate 10 mL, ammonium sulfate 2 g, and yeast extract 2 g were dissolved in 1000 mL of water. The solution was adjusted to pH 7.0 by adding 2 N potassium hydroxide, and sterilized by high-pressure steam (110°C, 10 min).

Since the monoterpenoid compound was accumulated in the mycelia, the culture was centrifuged at 5,000 rpm for 15 min. The harvested mycelia were extracted with methanol (500 mL) with stirring. The resulting suspension of mycelia was filtered to give a clear filtrate. The resulting filtrate was further extracted twice with *n-*hexane (250 mL). The combined hexane extract was washed twice with 0.1 N NaOH (250 mL) to remove free fatty acids, and dried over Na₂SO₄. A portion of the organic layer was directly analyzed by GC-MS. The observed spectrum was compared with charts of compound libraries of GC-MS for identifying products.

### Analysis condition:

GC-MS SHIMADZU CORPORATION GCMS-QP5050A;
Column: Neutral Bond-5 capillary column (30m X 0.25mm) (GL sciences);
Temperature: Initiation temperature 50°C, Temperature gradient 50-250°C (25°C/min);
Measuring time: 12 min.

Three compounds were determined by GC-MS analysis, which were identified as following compounds: β-pinene (M⁺ m/z136, rt 6.02 min):

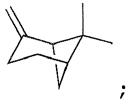

### camphene (M⁺ m/z136, rt 5.73 min):

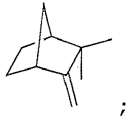

### 1,8-cineol (M⁺ m/z154, rt 6.57 min):

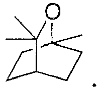

### Synthesis of sesquiterpenoid (I)

Production example 2: sclav_p0985 (SEQ ID NO:3), sclav_p1407 (SEQ ID NO:5), sgr_6065 (SEQ ID NO:7), sven_0552 (SEQ ID NO:9), sk_3051 (SEQ ID NO:11), slt_1718 (SEQ ID NO:13), and mmar_3220A (SEQ ID NO:58)

### 1. Construction of Chromosomal integration vector used for Synthesis

A chromosomal integration vector for synthesis of a sesquiterpenoid was constructed by the method according to Production example 1. The following is an explanation of the method of constructing chromosomal integration vector, which is focused on the difference in procedure of Production example 1.

(1) The PCR basal condition described in Table 23 is the same as Production example 1.

In Tables 24 to 29, each primer sequence of the genes and the elongation period of PCR at 72°C are indicated. The upper sequence is the forward primer. The XbaI site is underlined. The initiation codon is indicated by boldface. The lower sequence is the reverse primer. The HindIII site is underlined. The termination codon is indicated by boldface. The DNA derived from *S. clavuligerus* ATCC 27064 was used as a template for sclav_p0985 and sclav_p1407. The DNA derived from *S.griseus* IFO 13350 was used as a template for sgr_6065. The DNA derived from *S.venezuerae* ATCC 10712 was used as a template for sven_0552. The DNA derived from *Streptomyces* sp. SK1894 was used as a template for sk_3051. The DNA derived from *S. lactacyctinaeus* was used as a template for slt_1718.

**[Table 23]**

| Table 23 | | |
|---|---|---|
| PCR basal condition | | |
| 98°C- | 2 min | 1 cycle |
| 98°C- | 15 sec. | 30 cycles |
| 55°C- | 15 sec. | |
| 72°C- | indicated in Tables 24-29 | |

**[Table 24]**

| **Table 24** |
|---|
| sclav_p0985 |
| 5'-TTTTCTAGAATGCCGTTCCCCGCCCGCCC-3' (T CTAGA=XbaI) (SEQ ID No:46) |
| 5'-TTTAAGCTTTCAGTCGGCACCGGTGCGTG-3'  (A AGCTT=HindIII)  (SEQ ID No:47) |
| 72 °C - 60sec |

**[Table 25]**

| Table 25 |
|---|
| sclav_p1407 |
| 5'-TTTTCTAGAATGGGTGGGCGTACAGGAC-3'　(T CTAGA=XbaI)　(SEQ ID No:48) |
| 5'-TTTAAGCTTTCATCGTGTGTGTCCACCGT-3' (A AGCTT=HindIII) (SEQ ID No:49) |
| 72 °C - 70 sec |

**[Table 26]**

| Table 26 |
|---|
| sgr_6005 |
| 5'-TTTTCTAGAGTGGCGCACACCCGTGGGCA-3'  (T CTAGA=XbaI)  (SEQ ID No:50) |
| 5'-TTTAAGCTTTCAGCGGCCCGGGGAGCCT-3' (A AGCTT=HindIII) (SEQ ID No:5 1) |
| 72 °C *-* 70 sec |

**[Table 27]**

| Table 27 |
|---|
| sven_0552 |
| 5'-TTTTCTAGAATGACAGTGCGTGCCGTCGA-3' (T CTAGA=XbaI) (SEQ ID No52) |
| 5' -TTTAAGCTTTCATGCGCTTCCTGCGGAGG 3' (A AGCTT=HindIII) (SEQ ID No:5 3) |
| 72 °C - 80 sec |

**[Table 28]**

| Table 28 |
|---|
| sk_3051. |
| 5'-TTTTCTAGAGTGGACCCCATTTGACCGT-3'　(T CTAGA=XbaI)　(SEQ ID No: 54) |
| 5'-TTTAAGCTTTCAGCGGCCCGCGACGGCGG-3' (A AGCTT=HindIII) (SEQ ID No:55) |
| 72 °C 80 sec |

**[Table 29]**

| Table 29 |
|---|
| slt_1718 |
| 5'-TTTTCTAGAATGACCGATCTCCCCGACGG-3'  (T CTAGA=XbaI)  (SEQ ID No: 56) |
| 5'-TTTAAGCTTTCAGGGCCTCGCCGTGGGGG-3'  (A AGCTT=HindIII)  (SEQ ID No:57) |
| 72 °C - 60 sec |

The mmar_3220A was obtained by optimizing codons of sesquiterpenoid synthetase gene mmar_3220 for expression in Streptomyces. The DNA segments wherein the XbaI site was added to the initiation codon side of mmar_3220A and the HindIII site was added to the termination codon side of mmar_3220A was totally synthesized by Operon Biotechnology Kabushikigaisya.

The DNA segment prepared by Operon Biotechnology Kabushikigaisya has the following sequence.

**[Table 30]**

| Gene obtained by optimizing codons of mmar 3220 for expression in Streptomyces. Xbal site was added to initiation codon side and Hindlll site was added to termination codon side (obtained from Operon Biotechnology Kabushikigaisya) (SEQ ID NO:90) |
|---|
| tctagAGGAGGTtgcatATGAACGTCATTGACTTCCCCCAGGTCGACTTGGGCACCGAGGC GACAATCAGCCCCGACGTCGACAAAGCAGGGGAGCACCTGAAGACATGGTCCCGTGC CACAGGCATCGTCCTCGACGGCCCCAACGGCGACGCCTTCGACGCACTCGCGCAGCA CCGCCTGGCGGCATGGACCTACCCGGCGGCAACCGGCGCGGAGCTTGACCTGATGGC GGACTGGATCGGCTGGCTCTTCGCCTTCGACGACGTCTTCGACGAGTCGATCGACGG CTGCGACCAGGACTTCGCCATTGCGACGGCGGCGGCCACCAACACCGTCTACACCGG GCTGCCCTCAGCACCCCCGAGCCCAGTAGTCAGGCCCTACGTGGTGGCGTTGGAGGA CCTTTGGGAGCGGACCACCCAGGGCATGCCGGCGTACTGGTGCAGGCGACTGGCCAA CGACATGGTCGACTACGTCAACTCCTACCGAAGCCACGCCCTGATCAACGCATCACGC ATTGCGCTCGACGAGCAGTCATACCGAGCCCACCGGCTGATCTCCAGCGCAGTATTCA TCACGCTCGACCTCGGCGAGGCCGCCGCCAGGCGTGCCCTGTCGGAGTCGCTGTTGG TGCACCCATACATCCGGGCCGCCCGCGAGGCCGCCAACAACATCGTGTCCTGGAGCA ACGACCTCTACTCCGCGCCCAAAGAGCTCAGCCTGGGCGACCTGTGCAACTACATCG CCGTCCTGCAGTCCCAGGACGACCTCACGACCGCGCAGGCAGCCGACCGGGTCAGTC AGTACCTGCACGAGGAGATCGCGCGCTTCGACCAGACCAAACAGCTCATCCACACCC ACCTCATCCCACGCCTGCCAACCGAGGACCAGCAGGGCGTGATCTCCATGCTGGACA CCTGCAGCAACTGGATCACCGGCAACGTGGCGTGGTCATTGGAGACCGCACGCTACG CATCGCGCGCCATCGACATGGAGCGCGAGAAGGAGGTGTTCGGGGACTTGCGAACCT AGgaagctt |

(2) The segments obtained under the above PCR condition and the segment prepared in Operon Biotechnology Kabushikigaisya were digested with XbaI and HindIII to give sesquiterpenoid synthetase genes sclav_p0985, sclav_p1407, sgr_6065, sven_0552, sk_3051, slt_1718, and mmar_3220A, respectively.

PCR was performed to amplify the fps gene by using the chromosomal DNA derived from *S. avermitilis* MA-4680 (ATCC 31267, NRRL 8165, NCBIM 12804, JCM 5070), which has the fps gene, as a template and by using the forward primer 5'-CTCGAGCTCATATGACCGTGACCCCGGAAAGT-3' (SEQ ID NO:72) (CATATG=NdeI); and the reverse primer 5'-AGTCTAGATCACACCTCCCGGTCGACGACGAA-3' (SEQ ID NO:73) (TCTAGA=XbaI). The initial denaturation step (98° C, 2 min) was followed by 30 cycles of amplification (95° C, 15 sec.; 55° C, 15 sec.; 72° C, 70 sec.) and then a final incubation (72° C, 10 min).

The resulting PCR-amplified fragment was digested with NdeI/XbaI, and pKU460-rpsJp was digested with NdeI/XbaI. The digested PCR-amplified fragment, the digested pKU460-rpsJp, an aqueous solution (15 µL) containing 20 mM Tris-HCl (pH 7.5), 10 mM MgCl₂, 10 mM DTT, and 0.1 mM ATP, and T4 DNA ligase (0.25 µL) are mixed. The resulting mixture was allowed to stand at 14°C overnight for the ligation reaction, to give pKU460-rpsJ-fps.

In a similar manner to Production example 1, pKU460-rpsJ-fps was digested with the restriction enzymes XbaI and HindIII, and then to the digested pKU460-rpsJ-fps was added each of the sesquiterpenoid synthetase genes sclav_p0985, sclav_p140 sgr_6065, sven_0552, sk_3051, and slt_1718 which were amplified by PCR and mmar_3220A which was totally synthesized to give pKU460-rpsJ-fps-sclav_p0985, pKU460-rpsJ-fps-sclav_p1407, pKU460-rpsJ-fps-sgr_6065, pKU460-rpsJ-fps-sven_0552, pKU460-rpsJ-fps-sk_3051, pKU460-rpsJ-fps-slt_1718, and pKU460-rpsJ-fps-mmar_3220A, respectively.

(3) In a similar manner to Production example 1, the above ligation products were introduced into *E. coli* DH5α. Each sequence of sclav_p0985, sclav_p140 sgr_6065, sven_0552, sk_3051, slt_1718, and mmar_3220A in the resulting recombinant plasmid was confirmed.

### 2. Introduction of Chromosomal integration vector into S. avermitilis SUKA 17

In a similar manner to Production example 1, after the introduction into *E. coli* GM2929 hsdS::Tn10, each of the resulting recombinant plasmids: pKU460-rpsJ-fps-sclav_p0985, pKU460-rpsJ-fps-sclav_p1407, pKU460-rpsJ-fps-sgr_6065, pKU460-rpsJ-fps-sven_0552, pKU460-rpsJ-fps-sk_3051, pKU460-rpsJ-fps-slt_1718, and pKU460-rpsJ-fps-mmar_3220A was introduced into SUKA 17. Each of the transformants was cultivated, and the resulting spores were suspended in 20% glycerol, and then stored at -30°C.

### 3. Cultivation for Synthesizing Sesquiterpenoid compound and Analysis of Product

The transformants were cultivated in a similar manner to Production example 1 in order to give sesquiterpenoid compounds. The resulting products were analyzed by GC-MS in a similar manner to Production example 1.

As for sclav_p140 the resulting hexane extract was further filtered to remove Na₂SO₄, and the filtrate was concentrated to dryness with an evaporator to give 250 mg of a crude extract as an oil. The crude extract was dissolved in a small amount of *n*-pentane, and the mixture was loaded onto a silica gel (10 g) column with *n*-pentane. The silica gel column was washed with n-pentane, and then treated with a mixed solvent *n*-pentane:dichloromethane (solvent composition, 50:1 to 0:100).

A compound (2.51 mg) which was eluted with *n-*pentane:dichloromethane = 20:1 and whose retention time of GC-MS was 5.90 min compound, was obtained. The compound was dissolved in chloroform labeled with deuterium, and then determined by NMR, JEOL JNM-ECP500FT NMR SYSTEM (¹H-NMR:500 MHz, ¹³C-NMR:125 MHz).

EI-MS analysis of the compound whose retention time of GC-MS was 5.90 min showed a molecular ion peak at m/z 204[M⁺], which suggested that the molecular formula of the compound was C₁₅H₂₄, and confirmed that the index of hydrogen deficiency was 4. Twenty four hydrogen signals were observed from ¹H-NMR. Fifteen carbon signals were observed from ¹³C-NMR. The DEPT spectrum analysis thereof showed the presences of four methyl carbons, four methylene carbons, four methine carbons and three quaternary carbons. Among them, the two signals (δ 154.1 and 127.4 ppm) which shifted to low magnetic field suggested the presence of one double bond. These suggested that the compound was a tricyclic sesquiterpenoid compound. The detailed analysis of the correlation of ¹H-¹³C on the basis of ¹H-¹H-COSY and HMBC spectrum did not show the correlation of the two methylene groups (41.2 ppm, 25.1 ppm), but it was determined that the compound was a sesquiterpenoid compound having a 5-5-5-membered ring.

NMR analysis result of Compound whose retention time of GC-MS was 5.90 min

| No. | ¹³C (M) | ¹H (Int, M, *J*=Hz) |
|---|---|---|
| 1 | 154.1 (s) | - |
| 2 | 127.4 (d) | 5.01 (1H, s) |
| 3 | 57.7 (d) | 1.89 (1H, m) |
| 4 | 55.3 (s) | - |
| 5 | 50.8 (s) | - |
| 6 | 47.9 (d) | 3.16 (1H, quin, J=8.3) |
| 7 | 47.8 (t) | 1.22 (1H, m), 1.82 (1H, dd, *J*=7.0, 11.5) |
| 8 | 47.7 (t) | 0.84 (1H, m), 1.78 (1H, dd, *J*=7.1,11.5) |
| 9 | 43.7 (d) | ? |
| 10 | 41.2 (t) | 1.50 (2H, m) |
| 11 | 35.1 (t) | 1.67 (1H, m), 120 (1H, m) |
| 12 | 30.1 (q) | 1.08 (3H, s) |
| 13 | 30.1 (q) | 1.06 (3H, s) |
| 14 | 28.1 (q) | 1.02 (3H, s) |
| 15 | 20.1 (q) | 1.03 (3H, d, *J*=6.8) |

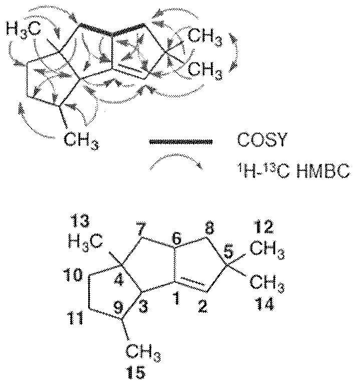

molecular formula : C₁₅H₂₄
molecular weight : 204
Index of hydrogen deficiency = 4

**[Table 31]**

| sclav_p0985 |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-250°C (25°C/min) |
| Measuring time: 12 min |

### Produced terpenoid compound

### african-2-ene (M⁺ m/z204, rt 6.68 min):

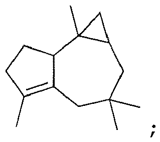

### african-1-ene (M⁺ m/z204, rt 6.75 min):

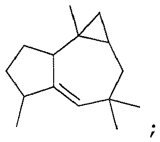

### α-gurjunene (M⁺ m/z204, rt 7.02 min):

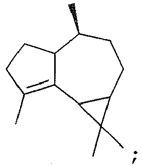

### α-humulene (M⁺ m/z204, rt 7.20 min):

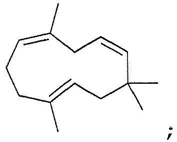

### β-caryophyllene (M⁺ m/z204, rt 7.30 min):

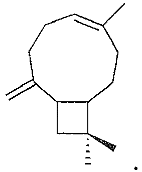

**[Table 32]**

| sclav_p1407 |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-250°C (25°C/min) |
| Measuring time: 12 min. |

### Produced terpenoid compound

### 1,3,6-trimethyl-2-methylene-tricyclo[5.4.0.03,9]undecane (M⁺ m/z204, rt 6.56 min):

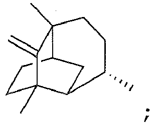

### Lactan-1-ene (M⁺ m/z204, rt 5.90 min):

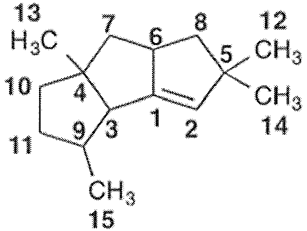

**[Table 33]**

| sgr_6065 |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-250°C (25°C/min) |
| Measuring time: 12 min |

### Produced terpenoid compound

### α-copaene (M⁺ m/z204, rt 6.70 min):

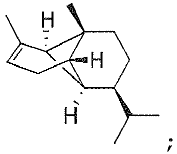

### calarene (M⁺ m/z204, rt 7.10 min):

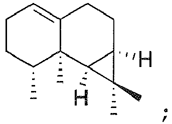

### α-murolene (M⁺ m/z204, rt 7.42 min):

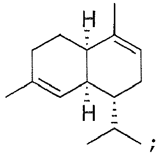

### cadina-1(10),4-diene (M⁺ m/z204, rt 7.55 min):

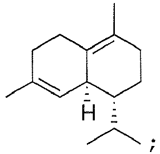

### cadina-1,4-diene (M⁺ m/z204, rt 7.61 min):

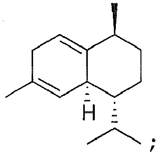

### epicubenol (M⁺ m/z222, rt 8.17 min):

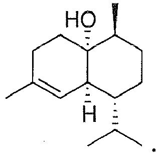

**[Table 34]**

| sven_0552 |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-280°C (20°C/min) |
| Measuring time: 12 min |

### Produced terpenoid compound

### α-chamigrene (M⁺ m/z204, rt 7.02 min):

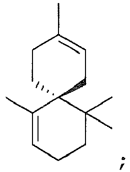

### dauca-8,11-diene (M⁺ m/z204, rt 7.48 min):

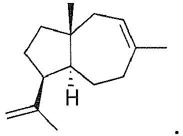

**[Table 35]**

| sk_3051 |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-280°C (20°C/min) |
| Measuring time: 12 min |

### Produced terpenoid compound

### β-maaliene (M⁺ m/z204, rt 10.67 min):

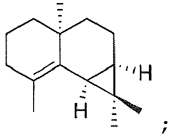

### selina-3,7(11)-diene (M⁺ m/z204, rt 10.75 min):

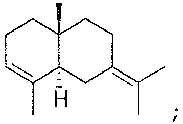

### selina-7(11)-ene-4-ol (M⁺ m/z222, rt 11.80 min):

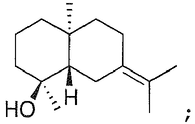

### germacrene A (M⁺ m/z204, rt 9.55 min, 9.91 min):

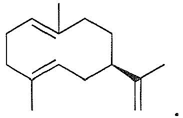

**[Table 36]**

| slt_1718 |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-250°C (25°C/min) |
| Measuring time: 12 min. |

### Produced terpenoid compound

### β-elemene (M⁺ m/z204, rt 9.62 min):

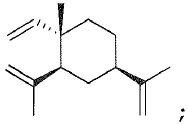

### α-cadinene (M⁺ m/z204, rt 10.68 min):

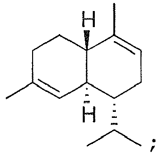

### β-cadinene (M⁺ m/z204, rt 10.55 min):

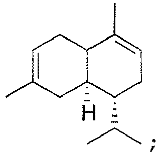

### γ-cadinene (M⁺ m/z204, rt 10.52 min):

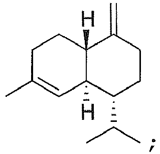

### germacrene D 4-ol (M⁺ m/z222, rt 10.98 min):

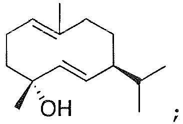

### α-copaene (M⁺ m/z204, rt 9.53 min):

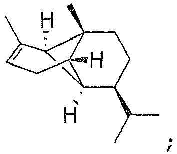

### α-cubebene (M⁺ m/z204, rt 10.05 min):

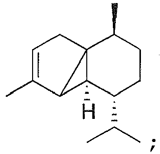

### β-cubebene (M⁺ m/z204, rt 10.21 min):

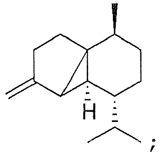

### β-aromadendrene (M⁺ m/z204, rt 10.25 min) :

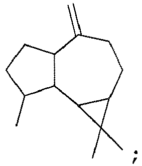

### T-cadinol (M⁺ m/z222, rt 11.39 min):

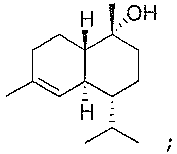

### viridiflorene (M⁺ m/z204, rt 10.41 min):

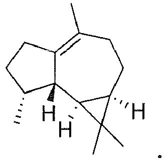

**[Table 37]**

| mmar_3220A |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-250°C (25°C/min) |
| Measuring time: 12 min |

### Produced terpenoid compound

### β-eudesmol (M⁺ m/z204, rt 11.08 min):

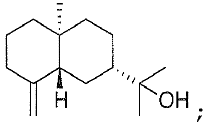

### β-element (M⁺ m/z204, rt 10.00 min):

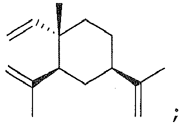

### γ-elemene (M⁺ m/z204, rt 9.60 min):

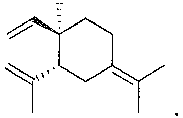

### Synthesis of diterpenoid

### Production example 3: sclav_p0765 (SEQ ID NO:17), sclav_1169 (SEQ ID NO:19), slt_1078 (SEQ ID NO:21), rxyl_0493A (SEQ ID NO:59), and haur_2987A (SEQ ID NO:60)

### (1) The PCR basal condition described in Table 38 is the same as Production example 1.

In Table 39 to 41, each primer sequence of the genes and the elongation period of PCR at 72°C are indicated. The upper sequence is the forward primer. The XbaI site is underlined. The initiation codon is indicated by boldface. The lower sequence is the reverse primer. The HindIII, site is underlined. The termination codon is indicated by boldface. The DNA derived from *S. clavuligerus* ATCC 27064 was used as a template for sclav_p0765 and sclav_p1169. The DNA derived from *S. lactacyctinaeus* was used as a template for slt_1078.

**[Table 38]**

| Table 38 | | |
|---|---|---|
| PCR basal condition | | |
| 98°C- | 2 min | 1 cycle |
| 98°C- | 15 sec. | 30 cycles |
| 55°C- | 15 sec. | |
| 72°C- | indicated in Tables 39-41. | |

**[Table 39]**

| Table 39 |
|---|
| sclav_p0765 |
| 5'-TTTTCTAGAATGACCATCTCCGTTCCCCA-3'  (T CTAGA=XbaI)  (SEQ ID No:78) |
| 5'-TTTAAGCTTTCAGGCGGCCTCGGGCACGA-3' (A AGCTT=HindIII) (SEQ ID No:79) |
| 72 °C - 60 sec |

**[Table 40]**

| Table 40 |
|---|
| sclav_p1169 |
| 5'-TTTTCTAGAATGCGCGGAGCCGGTCCCGG-3' (T CTAGA=XbaI) (SEQ ID No:80) |
| 5'-TTTAAGCTTCTACCAGGGGGTGAACACGG-3' (A AGCTT=HindIII) (SEQ ID No:81) |
| 72 °C-- 70 sec |

**[Table 41]**

| Table 41 |
|---|
| slt_10788 |
| 5'-TTTTCTAGAATGACAACCGTGCGCCGCAC-3'  (T CTAGA=XbaI)  (SEQ ID No: 82) |
| 5'-TTTAAGCTTTCACTTCTCCAACTGGTCCC-3' (A AGCTT=HindIII) (SEQ ID No:83) |
| 72 °C - 60 sec |

The rxyl_0493A and haur_2987A were respectively obtained by optimizing codons of sesquiterpenoid synthetase genes: rxyl_0493 and haur_2987 for expression in Streptomyces. The DNA segments wherein the XbaI site was added to the initiation codon side and the HindIII site was added to the termination codon side were totally synthesized by Operon Biotechnology Kabushikigaisya.

The DNA segments prepared by Operon Biotechnology Kabushikigaisya have the following sequences.

**[Table 42]**

| Gene obtained by optimizing codons of rxyl_0493 for expression in Streptomyces. Xbal site was added to initiation codon side, and Hindlll site was added to termination codon side (obtained from Operon Biotechnology Kabushikigaisya) (SEQ ID NO:84) |
|---|
| tctagAGGAGGTtgcatATGTCTACTGAGAACGGGCATCGGACGGTCTG GGGCCCCTGCGCTGTCCCTTCCCCGCGGCGATAAACCCGCACGCCGACGA GGTGCACCGGGAGACCGTGGAGTGGCCGAGGGTTTCGGGCTGCTCGGGC CCGGCGACGGGCACCGGATGATGCGGGACACCGGGATCGGGCGGCTCGCC GGGCGCTTCCACCCCGGGGCCGGGCGGGAGGAGCTCCGGCTGATCTCCGA CTGGTGCGCCTGGATGTTCCTGCGCGACGACCTCGCCGACGCGCCGGCGT ACTTCCGGCACCCGGAGCGGCTCGCGGCCCTGGACGCGGCCTTTCTGGAC ATCCTCTCCGGCAGGGCCTCCGGGGAGGGCTGCGGGTCCTTCGGGCGGGC GCTGCGGGATCTCCGGGAGCGGCTGCTGCCCAAGGTTCCCGCGCCGCTCT GGCTGCGGCGCTTTCTCAGGTCCGTGGAGGAGCACTTCGAGTCCACGCTG TGGGAGGCCACCAACCGGGCCCGCGGGGTCGTCCCGGACCTCGAGACCTA CCTCAGGATGCGCCCCATAACCGGCGGGATGCACGTGGACACGGACTTCA TAGAGATATCCTCCGGCGTCTACCTGCCGCCCGAGGTGCGCCGGCACCCG GCCGTAAGCGCCCTCACCGGGGCCTCCAACAACGTGGTCTGCTGGGCAAA CGACATCATCTCGCTCGCCAAGGAGCGCTCGCGCGGCGACGTGCACAACC TGGTCCTGGTCCTCCGCGCGAGCCGGCGTCTCACCACCCGGAGGCGGTG GCGGAGGCCGCCCGGATGTACGAGGCGGAGGTGCGGAGGTTCGTCCGGCT GGAGCGGGAGCTGCCCCCCTTCGGGCCGGCCATCGACGCCAACCTCCGGC GCTACGTCTCGGTGCTCAAGAGCAGGATGCGGGGCAATCTGGACTGGACC TACGAGTCGGCGCGCTACCGGGCCGGCGCGGCCTCCCGCTAGgaagctt |

**[Table 43]**

| Gene obtained by optimizing codons of haur_2987 for expression in Streptomyces. Xbal site was added to initiation codon side, and Hindlll site was added to termination codon side. (obtained from Operon Biotechnology Kabushikigaisya) (SEQ ID NO:91) |
|---|
| tctagAGGAGGTtgcatATGCCAACCCGTACCACATTGCACCACTTGGCCACGCTTA TGCCTCGTGACCTTCCGGTGCTCTACTGCCCATTCCCGGCGGCGATTCACCCCG ACGCGGTCGCGATTCAGGCCTCAACCCAGGCATGGGCCGACAGCTTCGGCTTG CTCGACGACTTGGAGACGCGGCTCTCCTTCAACCAGCGTGGCTACGGCACCTTG ATGGCGCGAGCCTACCCTAACGCCTTGACCGAGCGCATGCCAACCATTGCCGA CTGGAGCACCAGCCTCTTCCTCTTGGACGACCAGTTGGACCGCTTCGGCCGTGA GCACGACGCCGGGGCCATGGCAACCTTGTTGCAGCGCTTGATGAAGGTACTCAA AGGCTCGCCTGCCAAGCGCCACGACTTGCCAATTGTGCAGGCAATGGGCGACG TAACCAGCCGACTTGTGCAGGGCTCGCCCGAGGCCTGGCAGCACTACTTCTTCG ACTCATTGCGCAAATCGTTCGCAGCAGTGCAGTGGGAGGCCAACAACCGTGCCC AGGGCTACATTCCAACCCAGAAAACCTACGCAATGCAGCGTTTGTACACCAGTG GCACCTACTTGTGCTTGGCCTTGATTGAGTTCGGCAACAACGACTTCCTGCACC CCCAGGTGCGTTTGCACCCCATGGTGCGGCGTTTGTGCGAGTACGTCAACCGGG TGGTGTTCTACTCAAACGACATTATTTCGTTGCGTAAAGAGTTGTTGGCCGGCG ACTGGCAGAACTTGGTGTACCTCGTGCACACGCAGCACCAGTTGCCAATCGGCG AGGCATTGAACTACGTCGCCCGTTTGCACAACGACACAATCAACCAGTTCACCC GCGAGCGCATTATGCTCGACAAATTGGCCCCTGAGATTTGCTCACCGAGCTTGT ACCGCTTCGTCGACGGCTTGGCCACCTGGATGCGAGCAAACCACGACTGGTCC ACCATGACCTCCCGTTACCAGACATCCACCGAGGAGTCCCACGGATCCCAGTAC TGCGCGTGAgaagctt |

### 1. Construction of Chromosomal integration vector used for Synthesis

A chromosomal integration vector for synthesis of a diterpenoid was constructed by the method according to Production example 1. The following is an explanation of the method of constructing chromosomal integration vector, which is focused on the difference in procedure of Production example 1.

(1) The DNA segment, wherein the XbaI site was added to the initiation codon side of the diterpenoid synthetase gene, the HindIII site was added to the termination codon side of the diterpenoid synthetase gene, and the codons of the diterpenoid synthetase gene were optimized for expression in Streptomyces, were totally synthesized by Operon Biotechnology Kabushikigaisya.
(2) The segments prepared by the total synthesis were digested with XbaI and HindIII to give diterpenoid synthetase genes sclav_p0765, sclav_p1169, slt_1078, rxyl_0493A, and haur_2987A, respectively.

PCR was performed to amplify the ggs gene by using the chromosomal DNA derived from *S. avermitilis* MA-4680, which has the ggs gene, as a template and by using the forward primer: 5'-CTCGAGCTCATATGATGGCCGGTACCGCATGGGAC-3' (SEQ ID NO:74) (CATATG=NdeI); and the reverse primer: 5'-AGTCTAGATCATGACTGCCTTTCCGTGGCCAT-3' (SEQ ID NO:75) (TCTAGA=XbaI). The initial denaturation step (98° C, 2 min) was followed by 30 cycles of amplification (95° C, 15 sec.; 55° C, 15 sec.; 72° C, 70 sec.) and then a final incubation (72° C, 10 min).

The resulting PCR-amplified fragment was digested with NdeI/XbaI, and pKU460-rpsJp was digested with NdeI/XbaI. The digested PCR-amplified fragment, the digested pKU460-rpsJp, an aqueous solution (15 µL) containing 20 mM Tris-HCl (pH 7.5), 10 mM MgCl₂, 10 mM DTT, and 0.1 mM ATP and T4 DNA ligase (0.25 µL) are mixed. The resulting mixture was allowed to stand at 14°C overnight for the ligation reaction, to give pKU460-rpsJ-ggs.

In a similar manner to Production example 1, pKU460-rpsJ-ggs was digested with the restriction enzymes XbaI and HindIII, and then into the digested pKU460-rpsJ-ggs was inserted each of the diterpenoid synthetase genes sclav_p0765, sclav_p1169, slt_1078, rxyl_0493A, and haur_2987A to give pKU460-rpsJ-ggs-sclav_p0765, pKU460-rpsJ-ggs-sclav_p1169, pKU460-rpsJ-ggs-slt_1078, pKU460-rpsJ-ggs-rxyl_0493A, and pKU460-rpsJ-ggs-haur_2987A.

(3) In a similar manner to Production example 1, the above ligation products were introduced into *E. coli* DH5α. Each sequence of sclav_p0765, sclav_p1169, slt_1078, rxyl_0493A, and haur_2987A in the resulting recombinant plasmid was confirmed.

### 2. Introduction of Chromosomal integration vector into S. avermitilis SUKA 17

In a similar manner to Production example 1, each of the recombinant plasmids: pKU460-rpsJ-ggs-sclav_p0765, pKU460-rpsJ-ggs-sclav_p1169, pKU460-rpsJ-ggs-slt_1078, pKU460-rpsJ-ggs-rxyl_0493A, and pKU460-rpsJ-ggs-haur_2987A was introduced into SUKA 17. Each of the transformants is cultivated. The resulting spores were suspended in 20% glycerol (v/v), and then stored at -30°C.

### 3. Cultivation for Synthesizing Diterpenoid compound and Analysis of Product

### (1) sclav_p0765

100 mL of a seed medium added into a 500 mL Erlenmeyer flask. The suspension of spore of the SUKA 17-transformant: pKU460-rpsJp-ggs-sclav_p0765 was inoculated into the flask, and cultured with shaking (200 rpm) at 30°C for 2 days to give a seed culture.

The above seed medium was prepared as follows.

Glucose 5 g, defatted soy flour 15 g, and yeast extract (manufactured by Difco) 5 g were dissolved in 1000 mL of water. The solution was adjusted to pH 7.4 by adding 2 N potassium hydroxide, and sterilized by high-pressure steam (121°C, 15 min).

Each 100 mL of a medium for synthesis is added into seventy five 500 mL Erlenmeyer flasks. The seed culture 1 mL (1%) was inoculated into each of the flasks, and cultured with shaking (200 rpm) at 28°C for 5 days.

The above medium for synthesis was prepared as follows.

Glucose 60 g, calcium carbonate 5 g, sodium chloride 2 g, dipotassium hydrogen phosphate 0.5 g, 0.5% iron sulfate heptahydrate 10 mL, 0.5% zinc sulfate heptahydrate 10 mL, 0.5% manganese sulfate tetrahydrate 10 mL, 1% magnesium sulfate heptahydrate 10 mL, ammonium sulfate 2 g, and yeast extract 2 g were dissolved in 1000 mL of water. The solution was adjusted to pH 7.0 by adding 2 N potassium hydroxide, and sterilized by high-pressure steam (110°C, 10 min).

The culture (7L) was centrifuged at 5,000 rpm for 15 min. The harvested mycelia were extracted with methanol (3 L) with stirring. The suspension of mycelia was filtered to give a clear filtrate. The filtrate was further extracted twice with *n*-hexane (1 L). The combined hexane extract was washed twice with 0.1 N NaOH (1 L) to remove free fatty acids, and dried over Na₂SO₄ (A portion of the organic layer was directly analyzed by GC-MS for identifying products). After filtration of the hexane extract, the filtrate was concentrated to dryness with an evaporator to give a crude extract (324mg) as an oil. The crude extract was dissolved in a small amount of *n*-pentane. The mixture was loaded onto a silica gel (20 g) column with *n*-pentane. The silica gel column was washed with *n*-pentane, and then treated with a mixed solvent *n-*pentane:dichloromethane (solvent composition, 50:1 to 0:100).

A compound (trimethyl-methylenehexadecahydropyrene) (3.87 mg), which was eluted with *n*-pentane:dichloromethane = 20:1, was obtained. The retention time of GC-MS was 13.88 min.

Another compound (tetramethylhexadecahydropyrenol) (4.40 mg), which was eluted with 100% dichloromethane, was obtained. The retention time of GC-MS was 15.11 min.

Each of the compounds was dissolved in chloroform labeled with deuterium, and then determined by NMR, JEOL JNM-ECP500FT NMR SYSTEM (¹H-NMR:500 MHz, ¹³C-NMR:125 MHz).

EI-MS analysis of the compound whose retention time of GC-MS was 13.88 min showed a molecular ion peak at m/z272[M⁺], which suggested that the molecular formula of the compound was C20H32, and confirmed that the index of hydrogen deficiency was 5. Thirty two hydrogen signals were observed from ¹H-NMR. Twenty carbon signals were observed from ¹³C-NMR. The DEPT spectrum analysis thereof showed the presences of three methyl carbons, nine methylene carbons, five methine carbons and three quaternary carbons. Among them, the two signals (δ 106.7 and 151.8 ppm) which shifted to low magnetic field suggested the presence of one double bond. These suggested that the compound is a tetracyclic diterpene compound. The detailed analysis of the correlation of ¹H-¹³C on the basis of ¹H-¹H-COSY and HMBC spectrum did not show the correlation of one methylene group (1.44 ppm, 18.4 ppm), but it was determined that the compound was a diterpene compound having a 6-6-6-6-membered ring, trimethyl-methylenehexadecahydropyrene.

NMR analysis result of Compound whose retention time of GC-MS was 13.88 min

**[Table 44]**

| No. | ¹³C (M) | ¹H (Int, M, *J*=Hz) |
|---|---|---|
| 1 | 18.2(t) | 1.44(2H.m) |
| 2 | 20.4(q) | 0.77(3H, s) |
| 3 | 23.7(q) | 0.83(3H, d, *J=*7.6) |
| 4 | 21.0(q) | 0.90)(3H, s) |
| 5 | 22.7(t) | 1.51(1H, m), 1.63(1H, m) |
| 6 | 28.2(d) | 1.66(1H, m) |
| 7 | 29.6(t) | 1.26(1H, m), 1.74(1H, m) |
| 8 | 34.6(s) | - |
| 9 | 35.9(t) | 1.02(1H, m), 1.66(1H, m) |
| 10 | 36.1(d) | 1.89(1H, m) |
| 11 | 36.2(s) | - |
| 12 | 39.8(t) | 0.91(1H, m), 1.24(1H, m) |
| 13 | 42.3(t) | 1.15(1H, m), 1.33(1H, m) |
| 14 | 42.4(t) | 1.17(1H, m), 1.33(1H, m) |
| 15 | 44.0(d) | 1.23(1H, m) |
| 16 | 45.8(d) | 1.37(1H, m) |
| 17 | 47.6(d) | 2.27(1H, m) |
| 18 | 49.8(t) | 1.50(1H, m), 2.06(1H, m) |
| 19 | 106.7(t) | 4.42(1H, d, *J*=2.0), 4.65(1H, t, *J*=2.0) |
| 20 | 151.8(s) | - |

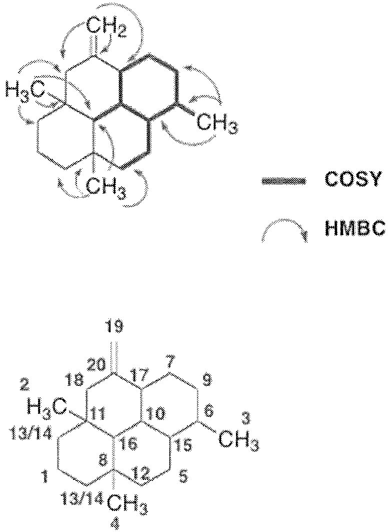

molecular formula: C₂₀H₃₂
molecular weight : 272
Index of hydrogen deficiency = 5

EI-MS analysis of the compound whose retention time of GC-MS was 15.11 min showed a molecular ion peak at m/z 290[M⁺], which suggested that the molecular formula of the compound was C₂₀H₃₄O and confirmed that the index of hydrogen deficiency was 4. The similarity to the mass fragmentation pattern of compound 5 suggested that the compound is a hydroxide of compound 5. Thirty two hydrogen signals were observed from ¹H-NMR. Twenty carbon signals were observed from ¹³C-NMR. The DEPT spectrum analysis thereof showed the presences of four methyl carbons, eight methylene carbons, five methine carbons, and three quaternary carbons. The signal shifted to low magnetic field, which was observed in the compound whose retention time of GC-MS was 13.88 min, was not observed, and a quaternary carbon which is directly linked to a methyl carbon and an oxygen atom was newly observed. The detailed analysis of the correlation of ¹H⁻¹³C on the basis of ¹H-¹H-COSY and HMBC spectrum did not show the correlation of one methylene group (1.41 ppm, 18.2 ppm: ¹H NMR, ¹³C NMR) as with compound 5, and the upper formula to the right of Table 45, which has a 6-6-membered ring, was determined as a partial structure of the compound. From the partial structure, it was determined that the compound whose retention time of GC-MS was 15.11 min is tetramethylhexadecahydropyrenol which is a hydroxide of the compound whose retention time of GC-MS was 13.88 min.

NMR analysis result of Compound whose retention time of GC-MS was 15.11 min

**[Table 45]**

| No. | ¹³C (M) | ¹H (Int, M, *J*=Hz) |
|---|---|---|
| 1 | 18.2(t) | 1.41(2H, m) |
| 2 | 20.6(q) | 0.82(3H, d, *J*=7.6) |
| 3 | 22.0(q) | 0.99(3H, s) |
| 4 | 22.8(t) | 1.55(2H, m) |
| 5 | 24.1(q) | 1.13(3H, s) |
| 6 | 26.1(t) | 1.24(1H, m). 1.49(1H, m) |
| 7 | 28.3(d) | 1.67(1H, m) |
| 8 | 31.1(q) | 1.08(3H, s) |
| 9 | 31.6(d) | 1.17(1H, m) |
| 10 | 34.6(s) | - |
| 11 | 34.9(s) | - |
| 12 | 36.1(t) | 0.97(1H, m), 1.68(1H, m) |
| 13 | 39.8(t) | 0.92(1H, m), 1.29(1H, m) |
| 14 | 42.6(t) | 1.11(1H, m), 1.36(1H, m) |
| 15 | 43.2(t) | 1.02(2H, m) |
| 16 | 44.(d) | 1.25(1H, m) |
| 17 | 45.2(d) | 1.26(1H, m) |
| 18 | 49.8(d) | 1.47(1H, m) |
| 19 | 52.3(t) | 1.14(1H, m), 1.33(1H, m) |
| 20 | 73.8(s) | - |

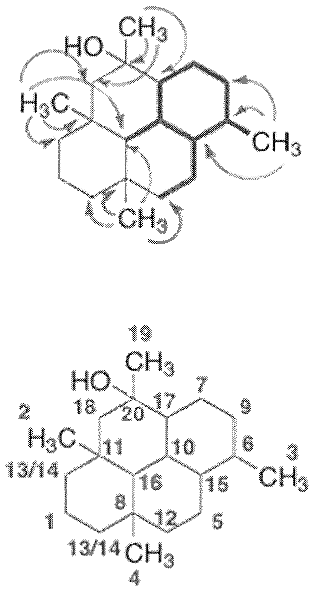

molecular formula: C₂₀H₃₂O
molecular weight : 290
Index of hydrogen deficiency = 4

**[Table 46]**

| sclav_p0765 |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-280°C (20°C/min) |
| Measuring time: 18 min |

### Produced terpenoid compound trimethyl-methylenehexadecahydropyrene (M⁺ m/z272, rt 13.88 min) :

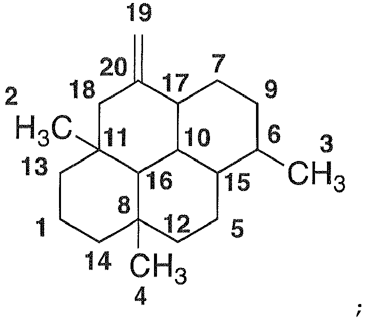

### tetramethylhexadecahydropyrenol (M⁺ m/z290, rt 15.11 min):

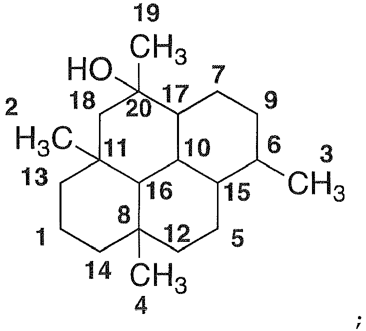

### isoelisabethatriene (M⁺ m/z272, rt 13.1 min)

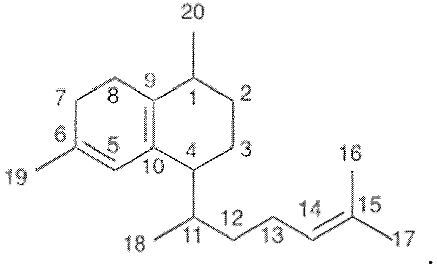

### (2) sclav_p1169

100 mL of a seed medium added into a 500 mL Erlenmeyer flask. The suspension of spore of the SUKA 17-transformant: pKU460-rpsJp-ggs-sclav_p1169 was inoculated into the flask, and cultured with shaking (200 rpm) at 30°C for 2 days to give a seed culture.

The above seed medium was prepared as follows.

Glucose 5 g, defatted soy flour 15 g, and yeast extract (manufactured by Difco) 5 g were dissolved in 1000 mL of water. The solution was adjusted to pH 7.4 by adding 2 N potassium hydroxide, and sterilized by high-pressure steam (121°C, 15 min).

Each 100 mL of a medium for synthesis is added into seventy five 500 mL Erlenmeyer flasks. The seed culture 1 mL (1%) was inoculated into each of the flasks, and cultured with shaking (200 rpm) at 28°C for 5 days.

The above medium for synthesis was prepared as follows.

Glucose 60 g, calcium carbonate 5 g, sodium chloride 2 g, dipotassium hydrogen phosphate 0.5 g, 0.5% iron sulfate heptahydrate 10 mL, 0.5% zinc sulfate heptahydrate 10 mL, 0.5% manganese sulfate tetrahydrate 10 mL, 1% magnesium sulfate heptahydrate 10 mL, ammonium sulfate 2 g, and yeast extract 2 g were dissolved in 1000 mL of water. The solution was adjusted to pH 7.0 by adding 2 N potassium hydroxide, and sterilized by high-pressure steam (110°C, 10 min).

The culture (7L) was centrifuged at 5,000 rpm for 15 min. The harvested mycelia were extracted with methanol (2 L) with stirring.

The suspension of mycelia was filtered to give a clear filtrate. The filtrate was further extracted twice with *n-*hexane (1 L). The combined hexane extract was washed twice with 0.1 N NaOH (1 L) to remove free fatty acids, and dried over Na₂SO₄ (A portion of the organic layer was directly analyzed by GC-MS for identifying products). After filtration of the hexane extract, the filtrate was concentrated to dryness with an evaporator to give a crude extract (323 mg) as an oil. The crude extract was dissolved in a small amount of *n*-pentane, and the mixture was loaded onto a silica gel (20 g) column with *n*-pentane. A fraction (37 mg) which was eluted with *n*-pentane and another fraction (2.8 mg) which was eluted with a mixed solvent of *n*-pentane:dichloromethane (solvent composition, 20:1) were obtained.

The fraction eluted with *n*-pentane was loaded into a silica gel (2 g) column which had been treated with 10% silver nitrate.

A fraction (a compound whose retention time of GC-MS was 13.57 min: clavulatriene B, 4.2 mg) was eluted with n-pentane:dichloromethane (1:1).

A fraction (a compound whose retention time of GC-MS was 13.11 min: prenylgermacrene, 1.15 mg) was eluted with *n*-pentane:dichloromethane (1:20).

A fraction (30 mg) was eluted with dichloromethane: methanol (10:1). The fraction eluted with dichloromethane: methanol (10:1) was again loaded into a silica gel (1.5 g) column which had been treated with 10% silver nitrate, and eluted with each 3 mL of dichloromethane: methanol (50:1). Fractions 10 to 13 contained a compound whose retention time of GC-MS was 13.22 min: clavulatriene A (22.3 mg). Fraction 15 to 20 contained a compound whose retention time of GC-MS was 13.38 min: lobophytumin C (4.8 mg).

Next, the above fraction (2.8 mg) eluted with n-pentane:dichloromethane (20:1) was loaded into a silica gel column which had been treated with 10% silver nitrate.

A fraction (a compound whose retention time of GC-MS was 12.63 min: prenyl-β-elemene, 0.9 mg) eluted with dichloromethane: methanol (20:1) was obtained.

A fraction (a compound whose retention time of GC-MS was 12.73 min: prenylgermacrene B, 1.3 mg) eluted with dichloromethane: methanol (1:1) was obtained.

Each of the compounds was dissolved in chloroform labeled with deuterium, and then determined by NMR, JEOL JNM-ECP500FT NMR SYSTEM (¹H-NMR:500 MHz, ¹³C-NMR:125 MHz).

The analysis of the extract of sclav_p1169 transformant culture showed six specific peaks. These peaks showed a molecular ion peak at m/z272[M⁺], which suggested that the products were diterpene hydrocarbons. Based on GC-MS databases, the compound whose retention time of GC-MS was 13.11 min and the compound whose retention time was 13.38 min were identified as prenylgermacrene and lobophytumin C, respectively.

Structures of compounds which had no correspondence in the GC-MS databases were analyzed by NMR (COSY, HMQC and HMBC).

NMR analysis result of Compound (clavulatriene B) whose retention time of GC-MS was 13.57 min:

**[Table 47]**

| No. | ¹³C (M) | ¹H (Int, M, *J*=Hz) |
|---|---|---|
| 1 | 41.7 | 1.15 (1H, m), 1.53(1H, m) |
| 2 | 17.5 | 1.47 (1H, m), 1.54(1H, m) |
| 3 | 33.2 | 1.55 (2H, m) |
| 4 | 38.2 | 2.50 (1H, m) |
| 5 | 148.5 | - |
| 6 | 120.8 | 6.12 (1H, s) |
| 7 | 128.5 | - |
| 8 | 22.9 | 2.20 (1H, m), 2.48(1H, m) |
| 9 | 41.5 | 1.35 (1H, m), 1.45(1H, m) |
| 10 | 34.6 | - |
| 11 | 128.4 | - |
| 12 | 34.9 | 2.01 (1H, m), 2.15(1H, m) |
| 13 | 26.7 | 2.02 (1H, m), 1.66(1H, m)2 |
| 14 | 124.5 | 5.14 (1H, m) |
| 15 | 131.7 | - |
| 16 | 25.7 | 1.67 (3H, s) |
| 17 | 17.8 | 1.60 (3H, s) |
| 18 | 17.7 | 1.78 (3H, d, *J*=2.1) |
| 19 | 26.2 | 1.13 (3H, s) |
| 20 | 23.3 | 1.15 (3H, d, *J=*12.2) |

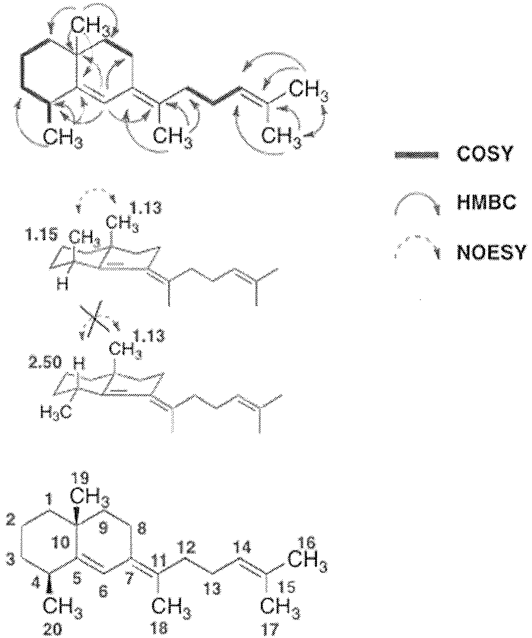

NMR analysis result of Compound (clavulatriene A) whose retention time of GC-MS was 13.22 min:

**[Table 48]**

| No, | ¹³C (M) | ¹H (int. M. *J*=Hz) |
|---|---|---|
| 1 | 42.4(t) | 1.26(1H, m),1.55(1H, m) |
| 2 | 19.1(t) | 1.55(2H, m) |
| 3 | 333.2(t) | 33.2(2H, m) |
| 4 | 124.5(s) | - |
| 5 | 135.1(s) | - |
| 6 | 31.3(t) | 2.55(2H, d J=7.4) |
| 7 | 45.6(t) | 1.80(1H, m) |
| 8 | 28.2(t) | 1.54(1H m), 1.63(1H, m) |
| 9 | 40.3(t) | 1.28(1H, m), 1.50(1H, m) |
| 10 | 34.5(s) | - |
| 11 | 154.8(s) | - |
| 12 | 35.0(t) | 2.07(2H, m) |
| 13 | 26.9(t) | 2.11(2H, m) |
| 14 | 24.3(d) | 5.14(1H, m) |
| 15 | 131.5(s) | - |
| 16 | 25.7(q) | 1.69(3H, s) |
| 17 | 17.7(q) | 1.62(3H, s) |
| 18 | 106.8(t) | 4.74(1H, d, J=1.6), 4.80(1H, brs) |
| 19 | 24.7(q) | 1.64(3H, s) |
| 20 | 19.3(q) | 1.60(3H, s) |

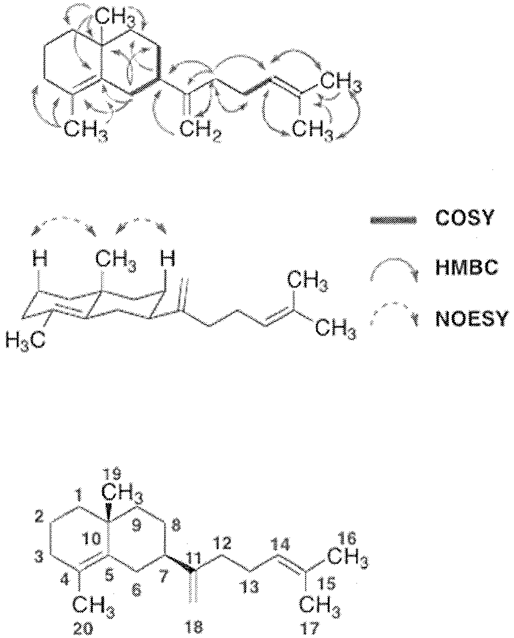

molecular formula: C₂₀H₃₂
molecular weight : 272
Index of hydrogen deficiency = 5

NMR analysis result of Compound (prenyl-β-elemene) whose retention time of GC-MS was 12.63 min:

**[Table 49]**

| No. | ¹³C (M) | ¹H (Int, M, *J*=Hz) |
|---|---|---|
| 1 | 150.1 | 5.81 (1H, dd, *J*=10.7, 17.7) |
| 2 | 109.7 | 4.91 (1H, d, *J*=17.5), 4.89 (1H, s) |
| 3 | 1120 | 4.81 (1H, brs), 4.59 (1H, d *J*=1.2) |
| 4 | 147.8 | - |
| 5 | 52.8 | 1.99 (1H, m) |
| 6 | 33.3 | 1.57 (1H, m), 1.53 (1H, M) |
| 7 | 44.4 | 1.95 (1H, m) |
| 8 | 26.8 | 1.62 (1H, m), 1.41 (1H, m) |
| 9 | 40.1 | 1.44 (1H, m), 1.41 (1H, m) |
| 10 | 40.0 | - |
| 11 | 154.5 | - |
| 12 | 34.9 | 2.06(1H, m), 2.03 (1H, m) |
| 13 | 27.3 | 2.11 (2H, m) |
| 14 | 124.2 | 5.13 (1H, m) |
| 15 | 131.6 | - |
| 16 | 25.7 | 1.68 (3H, s) |
| 17 | 17.7 | 1.62 (3H, s) |
| 18 | 107.0 | 4.78 (1H, brs), 4.72 (1H, brs) |
| 19 | 16.6 | 0.99 (3H, s) |
| 20 | 24.7 | 1.69 (3H, s) |

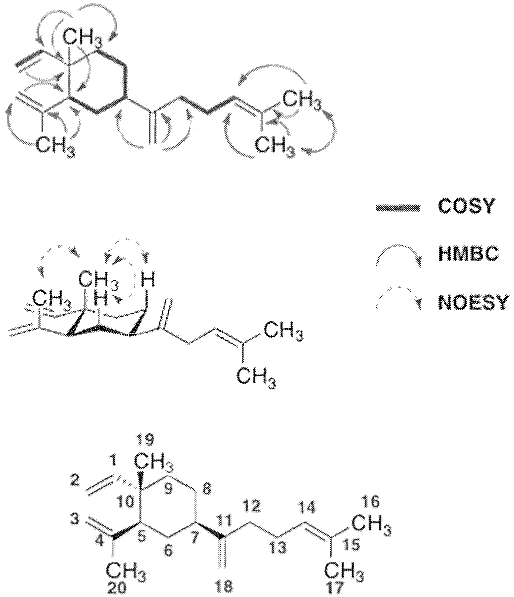

molecular formula : C₂₀H₃₂
molecular weight: 272
Index of hydrogen deficiency = 5

NMR analysis result of compound (prenylgermacrene B) whose retention time of GC-MS was 12.73 min:

**[Table 50]**

| No. | ¹³C(M) | ¹H (Int, M, *J*=Hz) |
|---|---|---|
| 1 | 135.8 (s) | - |
| 2 | 131.7 (s) | - |
| 3 | 131.6 (s) | - |
| 4 | 131.4 (s) | - |
| | 128.4 (s) | 4.71 (1H, m) |
| 6 | 124.7 (d) | - |
| 7 | 124.6 (d) | 4.51 (1H. m) |
| 8 | 124.6 (d) | 5.04 (1H, m) |
| 9 | 40.0 (t) | 1.90 - 2.00 (2H, m) |
| 10 | 39.8 (t) | 1.90 - 200 (2H, m) |
| 11 | 39.0 (t) | 2.18 (1H, m), 2.13 (1H, m) |
| 12 | 34.7 (t) | 2.08 (1H, m), 2.02 (1H, m) |
| 13 | 28.4 (t) | 2.00 (2H, m) |
| 14 | 26.9 (t) | 2.30 (1H, m), 2.08 (1H, m) |
| 15 | 26.0 (t) | 2.02 (2H, m) |
| 16 | 25.8 (q) | 1.69 (3H, s) |
| 17 | 18.0 (q) | 1.65 (3H, s) |
| 18 | 17.7 (q) | 1.62 (3H), s) |
| | 16.4 (q) | 1.58 (3H, s) |
| 20 | 16.1 (q) | 1.53 (3H, s) |

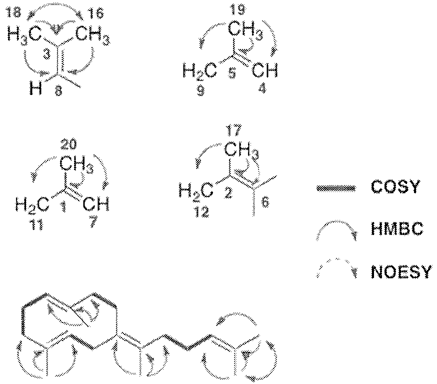

molecular formula : C₂₀H₃₂
molecular weight : 272
Index of hydrogen deficiency = 5

**[Table 51]**

| sclav_p1169 |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-280°C (20°C/min) |
| Measuring time: 18 min |

### Produced terpenoid compound

### clavulatriene A (M⁺ m/z272, rt 13.22 min):

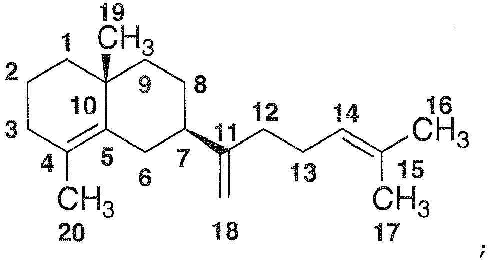

### prenyl-β-elemene (M⁺ m/z272, rt 12.63 min):

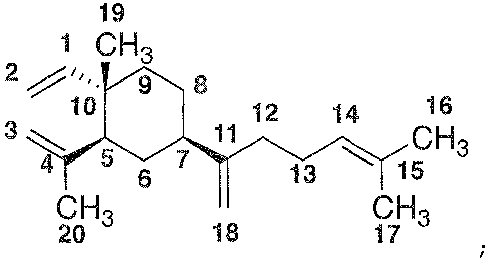

### prenylgermacrene (M⁺ m/z272, rt 13.11 min):

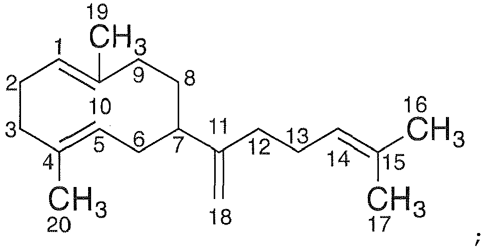

### lobophytumin C (M⁺ m/z272, rt 13.38 min):

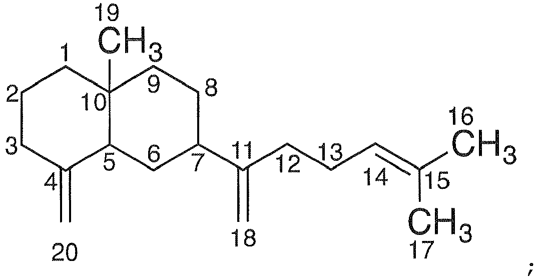

### clavulatriene B (M⁺ m/z272, rt 13.57 min):

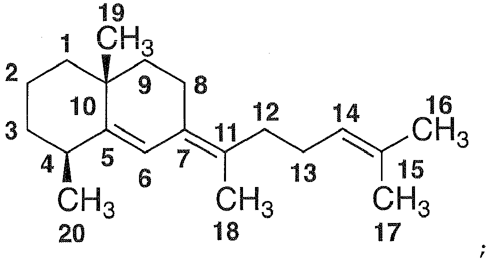

### prenylgermacrene B (M⁺ m/z272, rt 12.73 min):

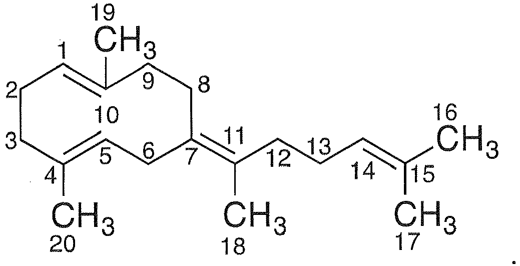

### (3) slt_1078

100 mL of a seed medium added into a 500 mL Erlenmeyer flask. The suspension of spore of the SUKA 17-transformant: pKU460-rpsJp-ggs-slt_1078 was inoculated into the flask, and cultured with shaking (200 rpm) at 30°C for 2 days to give a seed culture.

The above seed medium was prepared as follows.

Glucose 5 g, defatted soy flour 15 g, and yeast extract (manufactured by Difco) 5 g were dissolved in 1000 mL of water. The solution was adjusted to pH 7.4 by adding 2 N potassium hydroxide, and sterilized by high-pressure steam (121°C, 15 min).

Each 100 mL of a medium for synthesis is added into seventy five 500 mL Erlenmeyer flasks. The seed culture 1 mL (1%) was inoculated into each of the flasks, and cultured with shaking (200 rpm) at 28°C for 5 days.

The above medium for synthesis was prepared as follows.

Glucose 60 g, calcium carbonate 5 g, sodium chloride 2 g, dipotassium hydrogen phosphate 0.5 g, 0.5% iron sulfate heptahydrate 10 mL, 0.5% zinc sulfate heptahydrate 10 mL, 0.5% manganese sulfate tetrahydrate 10 mL, 1% magnesium sulfate heptahydrate 10 mL, ammonium sulfate 2 g, and yeast extract 2 g were dissolved in 1000 mL of water. The solution was adjusted to pH 7.0 by adding 2 N potassium hydroxide, and sterilized by high-pressure steam (110°C, 10 min).

The culture (7 L) was centrifuged at 5,000 rpm for 15 min. The harvested mycelia were extracted with methanol (2 L) with stirring. The resulting suspension of mycelia was filtered to give a clear filtrate. The filtrate was further extracted twice with *n*-hexane (1 L). The combined hexane extract was washed twice with 0.1 N NaOH (1 L) to remove free fatty acids, and dried over Na₂ SO₄ (A portion of the organic layer was directly analyzed by GC-MS for identifying products). After filtration of the hexane extract, the filtrate was concentrated to dryness with an evaporator to give a crude extract (300 mg) as an oil. The crude extract was dissolved in a small amount of *n*-pentane, and the mixture was loaded onto a silica gel (20 g) column with *n*-pentane, and the silica gel column was washed with *n*-pentane. A fraction (23.9 mg) eluted with n-pentane:ethyl acetate (9:1) was obtained. The fraction contained only a compound whose retention time of GC-MS was 9.30 min. Th compound had no correspondence in the GC-MS databases. The structure of the compound was further analyzed by NMR.

EI-MS analysis of the compound whose retention time of GC-MS was 9.30 min showed a molecular ion peak at m/z272[M⁺], which suggested that the compound was a diterpene hydrocarbon compound having C₂₀H₃₂. Thirty two hydrogen signals were observed from ¹H-NMR. Twenty carbon signals were observed from ¹³C-NMR. The DEPT spectrum analysis thereof showed the presences of five methyl carbons, six methylene carbons, five methine carbons, and four quaternary carbons. Among them, the four signals (δ 141.9, 140.1, 139.8 and 124.0 ppm) which shifted to low magnetic field suggested the presence of two double bonds. Each carbon and protons attaching to the carbon were determined by HMQC. Based on the detailed analysis of the correlation of ¹H⁻¹³C on the basis of ¹H-¹H-COSY and HMBC spectrum, it was determined that the compound was a 5-8-5-membered ring compound having double bonds at the 7-8 position and 10-14 position, cycloocta-7(8),10(14)-diene.

NMR analysis result of compound (cycloocta-7(8),10(14)-diene) whose retention time of GC-MS was 9.30 min

**[Table 52]**

| No. | ¹³C (M) | ¹H (Int. M, *J*=Hz) |
|---|---|---|
| 1 | 48.2(t) | 1.60(1H, m), 1.73(1H, m) |
| 2 | 53.5(d) | 0.91(1H, m) |
| 3 | 43.0(d) | 1.36(1H, m) |
| 4 | 35.9(t) | 1.05(1H, m), 1.69(1H, m) |
| 5 | 29.7(t) | 1.24(1H, m), 1.62(1H, m) |
| 6 | 49.1(d) | 2.73(1H, dd, *J*=5.5, 8.3) |
| 7 | 139.6(t) | - |
| 8 | 123.8(d) | 5.44(1H, dd. *J*=12.8, 7.7) |
| 9 | 23.4(t) | 2.45(2H, m) |
| 10 | 141.9(t) | - |
| 11 | 51.3(s) | - |
| 12 | 37.3(t) | 1.40(1H, m), 1.51(1H, m) |
| 13 | 26.3(t) | 1.99(1H, m) 2.11(1H, m) |
| 14 | 140.0(s) | - |
| 15 | 27.8(d) | 2.63(1H, m) |
| 16 | 20.9(q) | 0.90(3H, d, *J*=7.6) |
| 17 | 21.5(q) | 0.97(3H, d, *J*=7.6) |
| 18 | 18.0(q) | 0.93(3H, d, *J*=7.6) |
| 19 | 20.6(q) | 1.65(3H, s) |
| 20 | 26.0(q) | 0.92(3H, s) |

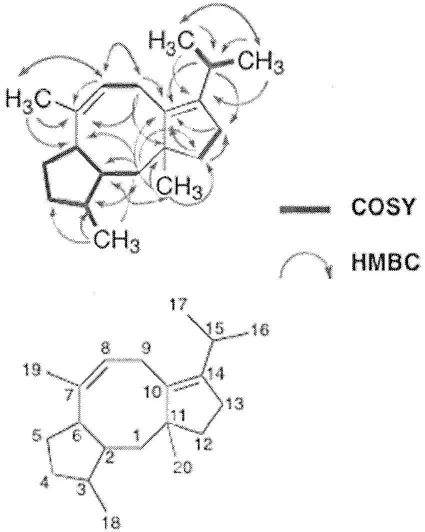

molecular formula: C₂₀H₃₂
molecular weight : 272
Index of hydrogen deficiency = 5

**[Table 53]**

| slt_1078 |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-280°C (20°C/min) |
| Measuring time: 18 min |

### Produced terpenoid compound

### cycloocta-7(8),10(14)-diene (M⁺ m/z272, rt 9.30 min):

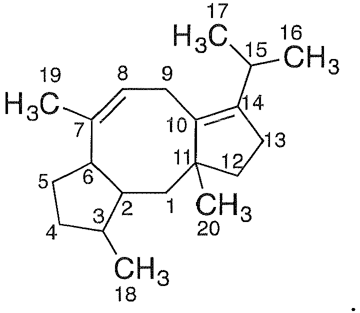

### (4) rxyl_0493A

In order to produce a diterpenoid compound, the transformant was cultured in a similar manner to Production example 1. In a similar manner to Production example 1, the produced compound was identified by GC-MS analysis.

**[Table 54]**

| rxyl_0493A |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-280°C (20°C/min) |
| Measuring time: 18 min |

### Produced terpenoid compound

### cembrene C (M⁺ m/z272, rt 13.62 min):

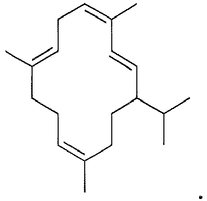

### (5) haur_2987A

In order to produce a diterpenoid compound, the transformant was cultured in a similar manner to Production example 1. In a similar manner to Production example 1, the produced compound was identified by GC-MS analysis.

**[Table 55]**

| haur_2987A |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-280°C (20°C/min) |
| Measuring time: 18 min |

### Produced terpenoid compound

### obscuronatin (M⁺ m/z290, rt 13.84 min):

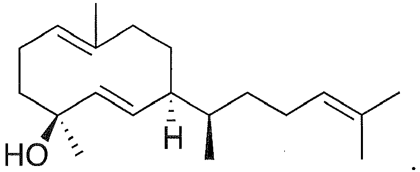

### Synthesis of sesquiterpenoid (II)

### Production example 4: haur_2988A (SEQ ID NO:61), rcas_0622A (SEQ ID NO:62), roseRS_3509A (SEQ ID NO:63), sce6369A (SEQ ID NO:64), and sce8552A (SEQ ID NO:65)

### 1. Construction of Chromosomal integration vector used for Synthesis

A chromosomal integration vector for synthesis of a sesquiterpenoid was constructed by the method according to Production example 1. The following is an explanation of the method of constructing chromosomal integration vector, which is focused on the difference in procedure of Production example 1.

(1) The DNA segment, wherein the XbaI site was added to the initiation codon side of the sesquiterpenoid synthetase gene, the HindIII site was added to the termination codon side of the sesquiterpenoid synthetase gene, and the codons of the sesquiterpenoid synthetase gene were optimized for expression in Streptomyces, were totally synthesized by Operon Biotechnology Kabushikigaisya.

**[Table 56]**

| Gene obtained by optimizing codons of haur_2988 for expression in Streptomyces. XbaI site was added to initiation codon side, and HindIII site was added to termination codon side. (obtained from Operon Biotechnology Kabushikigaisya) (SEQ ID NO:85) |
|---|
| tctagAGGAGGTtgcatATGCCAAACACAGCACACCTGCAGCAGTTGGCG AGGACGGTCCCAGCAACCATTCCCAAGTTGGAGTCACCGTTCCCTGCGCA GATCCACCCATTGGCAGCGGAGGTCCAGCAGACGACCAAATGGGTGC TTGAGCAGGGCTTGTTGAGCGAGGCGCGGCGTAAAGCCTTCCTTGACTAC AACTACACCTTGTTGATTGCCCGTTGCTACCCGTACGCGAGTGTCGAGCG GCTGCAGATTATCAGCGACTGGAACGCCTGGCTGTTCGAGCTTGACGACC AGTGCGACGAGGCGCGAATTGGCCACGACCCTCAGGCGTTGGCCCGCTTG CACCAGCGGCTTGGGGCAGTCATTCGCGGAGCCACGGTCGCCGCCGACGA GCCACTGTTGGTCACAGCCCTCGCCGACATTATGGGCCGGATGCGGGCGC TGGCACCACTGGCATGGTTCGAGGTCTTCACCCAGCAGGTCGACGACTAC TTCATTGGCAACCAGTGGGAGGCCCACAACCGGCTGTACTCGATTATTCC CAGCGAGATTGAGTACTTGCACTACCGTAAATTCACCAGTGCCGTCGCGA TCTACATTGCGTTCATTGAGTTCATGAACGCAAGTTACTTGACCGCCGAC GAGCGTCAGTCAATCGACTTGCGCAACCTGCAGAACTGGACGAACCTTGT ACTGAGTTTGTGCAACGACCTTGTCTCAGTCAAAAAAGAGTTGCGTCAGG GCGACTGGCACAACTACGGAGCAATTGTCGCATTGAAACGAGGCATGAGT ATTCCTGAGGCCTTGGAGATCGTCGCCAACGAGCACAACCACTGCATTCA GCAGTTCATCACAACCACCACCCAGCTGAAAAACTGCAGCGTGGCACTTC AGAAAGTATTGAACGGCATGATGACATTGATGCGCGGAAACTACGACTGG TCGGACATTACCAGCCGTTACGCCACCAAACTTACCGAGTTGTCGTACGC CGCATGAgaagctt |

**[Table 57]**

| Gene obtained by optimizing codons of rcas_0622 for expression in Streptomyces. XbaI site was added to initiation codon side, and HindIII site was added to termination codon side. (obtained from Operon Biotechnology Kabushikigaisya) (SEQ ID NO:86) |
|---|
| tctagAGGAGGTtgcatATGGACCAGGACTACCGCGCACGACTTGTCTAC CCATTCTCCGGAGCGATCAGCCCGCACGCCGACATCGTCGACCAGGCAAC GCTCGCCTGGGCGGCGATGTTCGGCTTGTTGACGGACTCATTGCGCCACA AAAGTCGTCGCCTCCAGTACGGCTTGCTGGCCGCACGCGCCTACCCACGA GCGGACCGTGAGATGTTGCAGATTGCCGCCGACTGGATTGCATGGCTGTT CTTCATGGACGACCAGTGCGACGAGACCGGCATTGGGCGTGACCTCCAGC GCATGATTGCGCTGCACGAGCGATTCCTTGCAATTCTTGACGGCGCAACG CCGGAGGCGCACGACTGCGCGCTGACCTACGCGCTCGCCGACCTTCGTCG TCGACTGGCACTGCGCGCCCCTGACAACTGGCTGCGCCGATTCAGCGAGC ACGTGCGTCTCTACTTCACCGCCAACCGCTGGGAGACGGTCAACCGGCAG CGCGGCGCAACGCCGAACGTGGCGACATACTGCGCCGCGCGCCTGTTCTC GGGCGCCGTCTACGCATGCTTCGACCTGATCGAGCTGGCAGAGCAGATCG AGCTGCCGTTCTACGCCCGTCACCACAGCATTGTGCAGCAGCTGGAGCAG GCCGCGAACAACATCATTTGCTGGTGCAACGACGTGCTGTCGTACCCCAA GGAGATGCAGCACGGCGACCGGCACAACCTGGTGCTGGTCATTCAGGGCG AGCACCAGTGCTCATTGCCCGAGGCCATTGACCGCGCGCTCGACCTTCAC GCGCGTGAGGTCGCCACGTTCGTGCGGAAACGCACCTGCGTCCCATACTT CGACGCAGCGGTCAACACAGCGCTTGAGAAATACGTCACCGGATTGCAGT TCTGGATCTGCGCCAACCGCGACTGGTCGCTGACGGCAACGCGCTACGCG CCGACGCACAAATCGCAGGAGATGGTGATGGCAGTCGCGCAGCAGTAGga agctt |

**[Table 58]**

| Gene obtained by optimizing codons of roseRS_3509 for expression in Streptomyces. XbaI site was added to initiation codon side, and HindIII site was added to termination codon side. (obtained from Operon Biotechnology Kabushikigaisya) (SEQ ID NO:87) |
|---|
| tctagAGGAGGTtgcatATGGACCGGGTCGACGGCGCACAGATCGTCTAC CCGTTCACCGGCGCGATCAGCCCCTACGCCGGCGACGTCGACCAGGCAAC ACTTATCTGGGCAGAGGCGTCCGGACTTCTTACCGACGGGTTGCGCCAGA AAAGTCAGCGTCTCCAGTACGGAGTACTGGCCGCGCGCGCCTACCCACAG GCAGACCGTGACACGCTGCAGATTGCCGCCGACTGGATCGCCTGGCTGTT CTTCATGGACGACCAGTGCGACGAGGCCGGCATTGGCCGCGACCCGCAGC GCCTGGCCGCGCTGCACGAGCGATTCCTCGCAGTGCTCGAGGGAGACCCA CCTGACAGTGGCGACTGGAACCTGACCCGCGCCCTCGCCGACATTCGCCG TCGGCTGGCCGCGCGCGCAACCGACGACTGGTTGCGCCGATTCGGAGAGC ACGTGCGTCTCTACTTCACGGCGAACCGCTGGGAGGCGGCAAACCGATGC CAGAGCATCGTCCCAAACGTGGCGACCTACTGCGCCGCGCGCCTGTTCTC AGGCGCAGTCTACGCCTGCTTCGACCTGATCGAGCTGGCAGCAGGGATCG ACCTTCCCTTCTACGCACGTTACCACGCCGCCGTCCAGCAGCTCGAGCGG ACGGCAAACAACATCATTTGCTGGTGCAACGACATGTTGTCGTACCCCAA AGAGATGCAGCACGGCGACGTGCACAACCTGGTACTGGCCATCCGGCAGG AGTACCAGTGCTCGTTGAGTGAGGCGCTGCAGCAGGCGCTTCTGCTCCAC GACCGGGAGACGGACACGTTCATGAAAACACAGAAGCAGCTGCCGCGCTT CAACCCCGCCGTCGACATGGCGCTTGAGCGCTACATTGACGGCTTGCAGT ACTGGATCTGCGCCAACCGTGACTGGTCATTGACAGCAATGCGCTACGCC TTGTCCGGAACCGAGCCCGTACTTCGGACGAGGTTCGCACACTCGTGAga agctt |

**[Table 59]**

| Gene obtained by optimizing codons of sce6369 for expression in Streptomyces. XbaI site was added to initiation codon side, and HindIII site was added to termination codon side. (obtained from Operon Biotechnology Kabushikigaisya) (SEQ ID NO:88) |
|---|
| tctagAGGAGGTtgcatATGCACAGAGCCCTCCTGTGTCCTTTCCCCGCC ACGACGCCCCACCCGCAGGCGGCACAGCTGGCGAACGATTGCCTCGAATG GACTCGCAAGTGCGGCCTGCTCCCCGATGAATCGCCACGAACGCTCGACA AGGTGCGCAGCTACTCGGCGCTCGCCGCACTGTTACCCCGATGCGCAT TTCGAGCGGCTTCGGGCGATCTGCGACTACTACAGCTGGCTCTTCTTCTT CGATGACGTGTGCGAGAATACGTCACTGAACGGCGCCGAACCGAAGGTTG TCTCGAGCCTGCTGTTCGATGTATACGGGGTGCTGCGAGGGCCCACGGCT GCCGTTGGGCATGCGCCATTCGCCCAGGCGTTGGCGGACATCTGGCGTCG CATCGGCGATGGTTGTCCTGGGTTCTGGCGGCGGCGCCTCATCCGCCACG TCGAAAACTACATCGATGGCTGTGTCTGGGAAGCGCAAAACCGCCAGCTC GACCGCGTACCCAGTCGCGCGGTGTTCGAGGGTATGCGCATGCACACGTC CACCATGTACGAGTTCTGGGACTTCATCGAGTATGCCGGCGACCTGTTCT TGCCGGATGAGGTCGTAGAACATCCCTTGGTGGCGGAGGTGCGTCGCGCC GGCAACGCCATCGCGTCTTTCGCCAATGACATTTACTCGCTGCGCAAAGA GACGAGCAACCGCGACGTGCACAATTTGGTTGTCGTGCTCATGCACGAAG AGCGCATCGAGCTCGAAGCAGCGTACGCGCGCGCCGCTGGTATCCACGAT GCTCAAGTCGAGCACTTCCTGGATCTGGTGAAGCACCTGCCCACGTTCTC TGCGACCATCGACCGGAACCTGGCGCGTTACGTGGAGGGCATACGCATCT GGATCCGCGCCAACCACGATTGGTCGATTGTCACGCCCCGTTACAATGAG CCCGATGCGCGATGAgaagctt |

**[Table 60]**

| Gene obtained by optimizing codons of sce8552 for expression in Streptomyces. XbaI site was added to initiation codon side, and HindIII site was added to termination codon side. (obtained from Operon Biotechnology Kabushikigaisya) (SEQ ID NO:89) |
|---|
| tctagAGGAGGTtgcatATGTCATCCGACCGAACCAGCGTCGTCGTCAGC AAGCGCGACGCCGGCGGATTCGAGTACCCGTTCGCCGCGTCGTGCCACCC CGGGAGGGAGGTCACGGAGCAGAGGACCCTGGCGTGGGTCCGCCGGTTGC GTCTGGTGCCAGACGGCCGGTCGCTTTCACGTCTCAAGGCGACGAACTTC TCCCACCTGGCGGCATGGCTCTTGCCGTCCGCGAGCACGCAGACGCTCCA GCTGGCATCGGACTTCACGGCCGTGCTGTTCCTCCTGGACGACGCGTACG ACGAGGGGCAGCTCAGCACGGACCCGGAGTCCGTCGAGTGGCTCAACGAG AAGTACCTGGGCGAGCTCTTCGGGTACACCGAGGCGGACATGAGCGACCC CCTGACGCGCGGGATGCTCGACGTACGTGAGCGCATCCGACGCAGCCACC CGCACTTCTTCCTGAACCGCTGGCTCTCGCACTTCCAGTACTACTACGAG GCCAACCTGTGGGAGGCGAACAACCGAAAGCAGATGAGGGTGCCGCACCT TGAGGAGTACTTGATGATGCGGCGCTACAGCGGCGCCGTCTACACCTACT GCGACCTGCTCGAGCTGCTGCTCGAGCGGCCGCTGCCGCTCGAGGTCGTC CAGCACCCGTTGATTCAGACGGTCCGAGACATCTGCAACGACATCCTCTG CTGGACGAACGACTACTTCTCGCTCGGGAAGGAGCTGACCAACGGCGAGA CGCACAACCTGATCGTCGTCCTCCGGAACGAGTGCGTGAGCACGCTGGAG GAGGCGATCGACCGGCTCAAGGACATGCACGACCGCCGGGTCGCGGAGTA CCAGGGCGTGAAGGAGAAGGTGCTCGCGCTCTGGGCGGACGACGAGATCC GCCTCTACCTGGACGCGGTCGAGGCCATGATCGCCGGGAACCAGCGCTGG GCGCTCGAGGCCGGCCGCTACTCCGGACTGGAGAGTTTGATCGTGCGCGC CGGATGAgaagctt |

(2) The segments prepared by the total synthesis were digested with XbaI and HindIII to give sesquiterpenoid synthetase genes haur_2988A, rcas_0622A, roseRS_3509A, sce6369A, and sce8552A, respectively.

PCR was performed to amplify the fps gene by using a chromosomal DNA derived from *S. avermitilis* MA-4680, which has the fps gene, as a template and by using the forward primer 5'-CTCGAGCTCATATGACCGTGACCCCGGAAAGT-3' (SEQ ID NO:76) (CATATG=NdeI); and the reverse primer 5'-AGTCTAGATCACACCTCCCGGTCGACGACGAA-3' (SEQ ID NO:77) (TCTAGA=XbaI). The initial denaturation step (98° C, 2 min) was followed by 30 cycles of amplification (95° C, 15 sec.; 55° C, 15 sec.; 72° C, 70 sec.) and then a final incubation (72° C, 10 min).

The resulting PCR-amplified fragment was digested with NdeI/XbaI. pKU460-rpsJp was digested with NdeI/XbaI. The digested PCR-amplified fragment, the digested pKU460-rpsJp, an aqueous solution (15 µL) containing 20 mM Tris-HCl (pH 7.5), 10 mM MgCl₂, 10 mM DTT, and 0.1 mM ATP, and T4 DNA ligase (0.25 µL) were mixed. The resulting mixture was allowed to stand at 14°C overnight for the ligation reaction, to give pKU460-rpsJ-fps.

In a similar manner to Production example 1, pKU460-rpsJ-fps was digested with the restriction enzymes XbaI and HindIII, and then into the digested pKU460-rpsJ-fps was inserted each of the sesquiterpenoid synthetase genes prepared by the total synthesis, haur_2988A, rcas_0622A, roseRS_3509A, sce6369A, and sce8552A, to give pKU460-rpsJ-fps-haur_2988A, pKU460-rpsJ-fps-rcas_0622A, pKU460-rpsJ-fps-roseRS_3509A, pKU460-rpsJ-fps-sce6369A, and pKU460-rpsJ-fps-sce8552A, respectively.

(3) In a similar manner to Production example 1, the above ligation products were introduced into *E. coli* DH5α. Each sequence of haur_2988A, rcas_0622A, roseRS_3509A, sce6369A, and sce8552A in the resulting recombinant plasmid was confirmed.

### 2. Introduction of Chromosomal integration vector into S. avermitilis SUKA 17

In a similar manner to Production example 1, each of the recombinant plasmids: pKU460-rpsJ-fps-haur_2988A, pKU460-rpsJ-fps-rcas_0622A, pKU460-rpsJ-fps-roseRS_3509A, pKU460-rpsJ-fps-sce6369A, and pKU460-rpsJ-fps-sce8552A was introduced into SUKA 17, and each of the transformants is cultivated. The resulting spores were suspended in 20% glycerol (v/v), and then stored at -30°C.

### 3. Cultivation for Synthesizing Sesquiterpenoid compound and Analysis of Product

In order to produce sesquiterpenoid compounds, the transformants were cultured in a similar manner to Production example 1. In a similar manner to Production example 1, GC-MS analysis was performed and the observed spectrum was compared with charts of compound libraries of GC-MS for identifying the sesquiterpenoid compounds.

**[Table 61]**

| haur_2988A |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-280°C (20°C/min) |
| Measuring time: 18 min |

### Produced terpenoid compound

### α-serinene (M⁺ m/z204, rt 7.43 min):

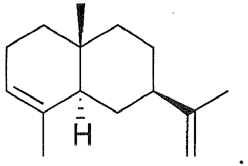

**[Table 62]**

| rcas_0622A |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-280°C (20°C/min) |
| Measuring time: 18 min |

### Produced terpenoid compound

### (+)-T-muurolol (M⁺ m/z222, rt 11.39 min):

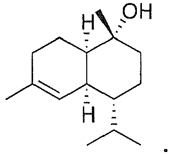

**[Table 63]**

| roseRS_3509A |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-280°C (20°C/min) |
| Measuring time: 18 min |

### Produced terpenoid compound

### (+)-T-muurolol (M⁺ m/z222, rt 11.39 min):

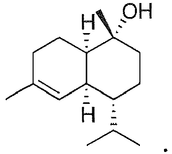

**[Table 64]**

| sce6369A |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-280°C (20°C/min) |
| Measuring time: 18 min |

### Produced terpenoid compound

### α-cubebene (M⁺ m/z204, rt 9.64 min):

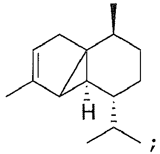

### β-cubebene (M⁺ m/z204, rt 9.93 min):

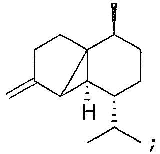

### cadina-3,5-diene (M⁺ m/z204, rt 10.04 min):

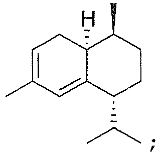

### bicyclosesquiphellandrene (M⁺ m/z204, rt 10.18 min):

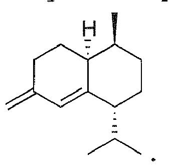

**[Table 65]**

| sce8552A |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-280°C (20°C/min) |
| Measuring time: 18 min |

### Produced terpenoid compound

### eremophilene (M⁺ m/z204, rt 7.37 min):

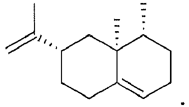

### Synthesis of sesquiterpenoid (III)

### Production example 5: slt_1880 (SEQ ID NO:92)

### 1. Construction of Chromosomal integration vector used for Synthesis

A chromosomal integration vector for synthesis of a sesquiterpenoid was constructed by the method according to Production example 1. The following is an explanation of the method of constructing chromosomal integration vector, which is focused on the difference in procedure of Production example 1.

(1) The PCR basal condition described in Table 66 is the same as Production example 1.
In Table 67, each primer sequence of the genes and the elongation period of PCR at 72°C are indicated. The upper sequence is the forward primer. The XbaI site is underlined. The initiation codon is indicated by boldface. The lower sequence is the reverse primer. The HindIII site is underlined. The termination codon is indicated by boldface. The DNA derived from *S.lactacyctinaeus* was used as a template.

**[Table 66]**

| Table 66 | | |
|---|---|---|
| PCR basal condition | | |
| 98°C- | 2 min | 1 cycle |
| 98°C- | 15 sec. | 30 cycles |
| 55°C- | 15 sec. | |
| 72°C- | indicated in Table 67. | |

**[Table 67]**

| Table 67 |
|---|
| slt_1880 |
| 5'-TTTTCTAGAATGACAACCACGGCAGAGAT-3' (TCT AGA=XbaI) (SEQ ID NO:94) |
| 5'-TTTAAGCTTCTACCCGTGGTACCGGGTGG-3' (AAG CTT=HindIII) (SEQ ID NO:95) |
| 72°C-60sec |

(2) The segment obtained under the above PCR condition was digested with XbaI and HindIII to give the sesquiterpenoid synthetase gene slt_1880.

PCR was performed to amplify the fps gene by using a chromosomal DNA derived from *S. avermitilis* MA-4680 (ATCC 31267, NRRL 8165, NCBIM 12804 , JCM 5070), which has the fps gene, as a template and by using the forward primer 5'-CTCGAGCTCATATGACCGTGACCCCGGAAAGT-3' (SEQ ID NO:72) (CATATG=NdeI); and the reverse primer 5'-AGTCTAGATCACACCTCCCGGTCGACGACGAA-3' (SEQ ID NO:73) (TCTAGA=XbaI). The initial denaturation step (98° C, 2 min) was followed by 30 cycles of amplification (95° C, 15 sec.; 55° C, 15 sec.; 72° C, 70 sec.) and then a final incubation (72° C, 10 min).

The resulting PCR-amplified fragment was digested with NdeI/XbaI, and pKU460-rpsJp was digested with NdeI/XbaI. The digested PCR-amplified fragment, the digested pKU460-rpsJp, an aqueous solution (15 µL) containing 20 mM Tris-HCl (pH 7.5), 10 mM MgCl₂, 10 mM DTT, and 0.1 mM ATP, and T4 DNA ligase (0.25 µL) are mixed. The resulting mixture was allowed to stand at 14°C overnight for the ligation reaction, to give pKU460-rpsJ-fps.

In a similar manner to Production example 1, pKU460-rpsJ-fps was digested with the restriction enzymes XbaI and HindIII, and then into the digested pKU460-rpsJ-fps was inserted the sesquiterpenoid synthetase gene slt_1880 which were amplified by PCR to give pKU460-rpsJ-fps-slt_1880.

(3) In a similar manner to Production example 1, the above ligation product was introduced into *E. coli* DH5α. The sequence of s slt_1880 in the resulting recombinant plasmid was confirmed.

### 2. Introduction of Chromosomal integration vector into S. avermitilis SUKA 17

In a similar manner to Production example 1, after the introduction into *E. coli* GM2929 hsdS::Tn10, the resulting recombinant plasmid: pKU460-rpsJ-fps-slt_1880 was introduced into SUKA 17. The transformant was cultivated, and the resulting spores were suspended in 20% glycerol, and then stored at -30°C.

### 3. Cultivation for Synthesizing Sesquiterpenoid compound and Analysis of Product

### (1) slt_1880

Each 100 mL of a medium for synthesis is added into seventy five 500 mL Erlenmeyer flasks. The seed culture 1 mL (1%) was inoculated into each of the flasks, and cultured with shaking (200 rpm) at 28°C for 5 days.

The above medium for synthesis was prepared as follows.

Glucose 60 g, calcium carbonate 5 g, sodium chloride 2 g, dipotassium hydrogen phosphate 0.5 g, 0.5% iron sulfate heptahydrate 10 mL, 0.5% zinc sulfate heptahydrate 10 mL, 0.5% manganese sulfate tetrahydrate 10 mL, 1% magnesium sulfate heptahydrate 10 mL, ammonium sulfate 2 g, and yeast extract 2 g were dissolved in 1000 mL of water. The solution was adjusted to pH 7.0 by adding 2 N potassium hydroxide, and sterilized by high-pressure steam (110°C, 10 min).

The culture (7 L) was centrifuged at 5,000 rpm for 15 min. The harvested mycelia were extracted with methanol (3 L) with stirring. The resulting suspension of mycelia was filtered to give a clear filtrate. The filtrate was further extracted twice with n-hexane (1 L). The combined hexane extract was washed twice with 0.1 N NaOH (1 L) to remove free fatty acids, and dried over Na₂SO₄ (A portion of the organic layer was directly analyzed by GC-MS for identifying products). After filtration of the hexane extract, the filtrate was concentrated to dryness with an evaporator to give a crude extract (1240 mg) as an oil. The crude extract was dissolved in a small amount of n-pentane, and the mixture was loaded onto a silica gel (20 g) column with *n*-pentane. The silica gel column was washed with *n*-pentane, and then treated with a mixed solvent, n-pentane:dichloromethane (solvent composition, 50:1 to 0:100).

A compound which was eluted with *n-*pentane:dichloromethane = 20:1 (a compound whose retention time of GC-MS was 6.30 min.; lactan-4(5)-ene) (4.27 mg) was obtained. The compounds was dissolved in chloroform labeled with deuterium, and then determined by NMR, JEOL JNM-ECP500FT NMR SYSTEM (¹H-NMR:500 MHz, ¹³C-NMR:125 MHz).

EI-MS analysis of the compound whose retention time of GC-MS was 6.30 min showed a molecular ion peak at m/z204[M⁺], which suggested that the molecular formula of the compound was C₁₅H₂₉, and confirmed that the index of hydrogen deficiency was 4. Twenty four hydrogen signals were observed from ¹H-NMR. Fifteen carbon signals were observed from ¹³C-NMR. The DEPT spectrum analysis thereof showed the presences of four methyl carbons, four methylene carbons, four methine carbons and three quaternary carbons. Among them, the two signals (δ 142.8 and 121.8 ppm) which shifted to low magnetic field suggested the presence of one double bond. These suggested that the compound is a tricyclic sesquiterpenoid compound. From these results, it was determined that the compound was a sesquiterpenoid compound having a 5-5-5-membered ring, lactan-4(5)-ene.

NMR analysis result of Compound whose retention time of GC-MS was 6.30 min

**[Table 68]**

| No. | ¹³C (M) | ¹H(Int. M. *J*=Hz) |
|---|---|---|
| 1 | 142.8 (s) | - |
| 2 | 121.8 (d) | 5.06 (1H, brs) |
| 3 | 66.7 (d) | 2.11 (1 H, brs) |
| 4 | 52.9 (s) | - |
| 5 | 50.1 (d) | 2.34(1H, q, *J*=10.0) |
| 6 | 48.9 (t) | 1.81(1H, dd, *J*=13.0, 7.8), 1.32 (1 H, dd, *J*=13.0, 7.7) |
| 7 | 48.8 (t) | 158 (1 H, dd, *J*=13.0, 8.6), 1.30 (1H, m) |
| 8 | 47.5 (t) | 2.19 (1H, dq. *J*=15.7, 2.2), 2.01 (1H, dt, *J*=15.7, 2.1) |
| 9 | 47.4 (t) | 1.64 (1 H, ddd, J=11.6, 8.7, 1,1), 1.27 (1 H, m) |
| 10 | 44.3 (d) | 2.52 (1 H, m) |
| 11 | 41.6 (s) | - |
| 12 | 30.5 (q) | 1,06 (3H, s) |
| 13 | 29.4 (q) | 1.17 (3H, s) |
| 14 | 29.2 (q) | 0.92 (3H, s) |
| 15 | 15.9 (q) | 1.68 (3H, s) |

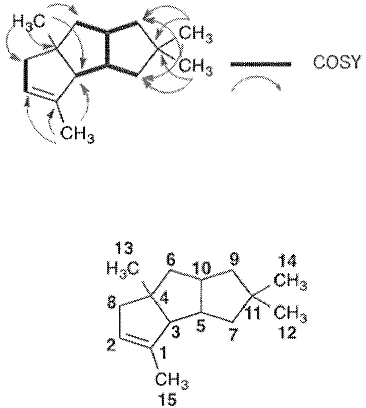

molecular formula : C₁₅H₂₄
molecular weigh : 204
Index of hydrogen deficiency = 4

**[Table 69]**

| slt_1880 |
|---|
| Analysis condition: |
| GC-MS SHIMADZU CORPORATION GCMS-QP5050A; |
| Column: Neutral Bond-5 capillary column (30m X 0.25mm) (GL sciences); |
| Temperature: Initiation temperature 50°C; |
| Temperature gradient: 50-250°C (25°C/min) |
| Measuring time: 7.75 min |

### Produced terpenoid compound

### lactan-4(5)-ene (M⁺ m/z204, rt 6.30 min):

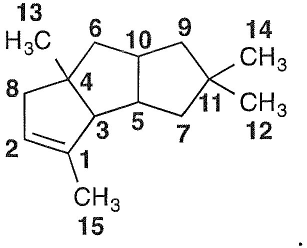

### Assay of Terpenoid compound

### Cytotoxicity of Novel diterpene compound to Organism cell

An in vitro assay was performed by using the following organism cell lines: Jurkat (T-cell lymphoblastic leukemia), SKOV (ovarian cancer), and *Mycobacterium smegmatis* which belongs to the same genus as tuberculosis.

Jurkat and SKOV were grown in a CO₂ incubator at 37°C using RPMI-1640 medium (manufactured by Life Technologies Japan). The mycobacterium was grown at 37°C using Mueller-Hinton medium (manufactured by DIFCO).

In this assay, the novel diterpene compound was diluted with 1% DMSO (dimethylsulfoxide) to give the following final concentrations: 5 µg/mL, 10 µg/mL, 25 µg/mL, 50 µg/mL, and 100 µg/mL. The cytotoxicity was measured by a colorimetric method using the MTT assay. The organism cells (5 x 10⁴ cells/mL) were added into a Saarsted flat bottom 96 well-plate, and then the solution of the novel diterpene compound in 1% DMSO (20 µL) was added into each well so as to give the above final concentrations of the diterpene compound. The plate was incubated for 72 hours under the standard temperature and pressure. For each of the diterpene concentrations, three wells were used. 1% DMSO (20 µL) without the novel diterpene was added into three wells, which were used as a control.

After the incubation, 20 µL of the MTT solution (manufactured by SIGMA) was added into each well and then the plate was incubated for 10 min. at 37°C. Optical density (OD) at 540 nm was measured using a plate reader.

MTT is metabolized to a water-insoluble formazan by a living cell, thereby the optical density of a sample solution is changed from yellow to dark blue in direct proportion to an amount of the living cells.

For each sample, three wells were used.

Viability (%) was calculated by an equation: (Average value of OD of Sample / Average value of OD of Control) x 100.

The results are shown below.

**[Table 70]**

| 72 h treatment | | | |
|---|---|---|---|
| | SKOV | Jurkat | Mycobacterium smegmatis |
| 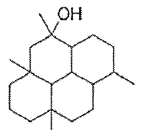 | IC50 = 35.38 *µ* g/ml | IC50 = 29. 35 *µ* g/ml | IC50 = 49.78 *µ*g/ml |
| tetramethylhexadecahydropyrenol | | | |
| 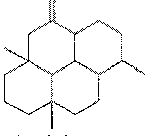 | not active | not active | IC50 = 50.08 *µ*g/ml |
| trimethyl-methylenehexadecahydropyrene | | | |
| 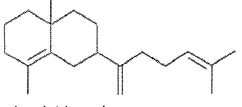 | not active | IC50 = 5.63 *µ*g/ml. | not active |
| clavulatriene A | | | |

## Claims

1. A method of producing a terpenoid compound, comprising:
introducing a gene encoding a protein consisting of an amino acid sequence having 90% or more identity to an amino acid sequence selected from the group consisting of SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, and SEQ ID NO:93 into a host cell, and
culturing the host cell.

2. The method of producing a terpenoid compound according to Claim 1, comprising
introducing a gene consisting of a base sequence having 90% or more identity to a gene selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:58, SEQ ID NO:17, SEQ ID NO:19, SEQ ID NO:21, SEQ ID NO:23, SEQ ID NO:59, SEQ ID NO:25, SEQ ID NO:60, SEQ ID NO:27, SEQ ID NO:61, SEQ ID NO:29, SEQ ID NO:62, SEQ ID NO:31, SEQ ID NO:63, SEQ ID NO:33, SEQ ID NO:64, SEQ ID NO:35, SEQ ID NO:65, and SEQ ID NO:92 into a host cell, and
culturing the host cell.

3. The method of producing a terpenoid compound according to Claim 1 or 2, comprising
introducing:
(1) a gene encoding a protein consisting of an amino acid sequence having 90% or more identity to an amino acid sequence of SEQ ID NO:39; and
(2) a gene encoding a protein consisting of an amino acid sequence having 90% or more identity to an amino acid sequence of SEQ ID NO:2,
into a host cell.

4. The method of producing a terpenoid compound according to any one of Claims 1 to 3, comprising
introducing:
(1) a gene consisting of a base sequence having 90% or more identity to a gene of SEQ ID NO:38; and
(2) a gene consisting of a base sequence having 90% or more identity to a gene of SEQ ID NO:1,
into a host cell.

5. The method of producing a terpenoid compound according to Claim 1 or 2, comprising
introducing:
(1) a gene encoding a protein consisting of an amino acid sequence having 90% or more identity to an amino acid sequence of SEQ ID NO:41; and
(2) a gene encoding a protein consisting of an amino acid sequence having 90% or more identity to an amino acid sequence selected from the group consisting of SEQ ID NO:4, SEQ ID NO:6, SEQ ID NO:8, SEQ ID NO:10, SEQ ID NO:12, SEQ ID NO:14, SEQ ID NO:16, SEQ ID NO:28, SEQ ID NO:30, SEQ ID NO:32, SEQ ID NO:34, SEQ ID NO:36, and SEQ ID NO:93,
into a host cell.

6. The method of producing a terpenoid compound according to any one of Claims 1, 2 and 5, comprising
introducing:
(1) a gene consisting of a base sequence having 90% or more identity to a gene of SEQ ID NO:40; and
(2) a gene consisting of a base sequence having 90% or more identity to a gene selected from the group consisting of SEQ ID NO:3, SEQ ID NO:5, SEQ ID NO:7, SEQ ID NO:9, SEQ ID NO:11, SEQ ID NO:13, SEQ ID NO:15, SEQ ID NO:58, SEQ ID NO:27, SEQ ID NO:61, SEQ ID NO:29, SEQ ID NO:62, SEQ ID NO:31, SEQ ID NO:63, SEQ ID NO:33, SEQ ID NO:64, SEQ ID NO:35, SEQ ID NO:65, and SEQ ID NO:92,
into a host cell.

7. The method of producing a terpenoid compound according to Claim 1 or 2, comprising
introducing
(1) a gene encoding a protein consisting of an amino acid sequence having 90% or more identity to an amino acid sequence of SEQ ID NO:43; and
(2) a gene encoding a protein consisting of an amino acid sequence having 90% or more identity to an amino acid sequence selected from the group consisting of SEQ ID NO:18, SEQ ID NO:20, SEQ ID NO:22, SEQ ID NO:24, and SEQ ID NO:26,
into a host cell.

8. The method of producing a terpenoid compound according to any one of Claims 1, 2 and 7, comprising
introducing
(1) a gene consisting of a base sequence having 90% or more identity to a gene of SEQ ID NO:42; and
(2) a gene consisting of a base sequence having 90% or more identity to a gene selected from the group consisting of SEQ ID NO:17, SEQ ID NO:19, SEQ ID NO:21, SEQ ID NO:23, SEQ ID NO:59, SEQ ID NO:25, and SEQ ID NO:60,
into a host cell.

9. The method of producing a terpenoid compound according to any one of Claims 1 to 8, further comprising
introducing rpsJ (SEQ ID NO:37) into the host cell.

10. The method of producing a terpenoid compound according to any one of Claims 1 to 9, wherein the host cell is a bacterium belonging to the genus Streptomyces, *E.coli,* or a mutant thereof.

11. A protein consisting of an amino acid sequence having 90% or more identity to an amino acid sequence selected from the group consisting of SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, and SEQ ID NO:93.

12. A gene encoding a protein consisting of an amino acid sequence having 90% or more identity to an amino acid sequence selected from the group consisting of SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, and SEQ ID NO:93.

13. A gene consisting of a base sequence having 90% or more identity to a gene selected from the group consisting of SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:58, SEQ ID NO:17, SEQ ID NO:19, SEQ ID NO:21, SEQ ID NO:23, SEQ ID NO:59, SEQ ID NO:25, SEQ ID NO:60, SEQ ID NO:27, SEQ ID NO:61, SEQ ID NO:29, SEQ ID NO:62, SEQ ID NO:31, SEQ ID NO:63, SEQ ID NO:33, SEQ ID NO:64, SEQ ID NO:35, SEQ ID NO:65, and SEQ ID NO:92.

14. A compound selected from the group consisting of:


SEQUENCE LISTING

<110>  THE KITASATO INSTITUTE
       NAGASE & CO., LTD.

<120>  NOVEL TERPENOID COMPOUNDS AND METHOD FOR PRODUCING THE SAME

<130>  672013

<150>  JP 2013-168230
<151>  2013-08-13

<160>  95

<170>  PatentIn version 3.5

<210>  1
<211>  993
<212>  DNA
<213>  Streptomyces clavuligerus ATCC 27064

<400>  1
atgcccgccg gccacgaaga gttcgacata cccttcccgt cccgagtgaa ccccttccac      60

gcccgggccg aggaccgcca tgtggcctgg atgcgcgcca tgggtctgat caccggcgac     120

gccgccgaag cgacgtaccg ccgctggagt cccgccaagg tcggagcccg ctggttctat     180

ctcgcacagg gtgaggacct ggacctgggc tgcgacatct tcggctggtt cttcgcctat     240

gacgaccact tcgacggacc caccggcacc gatccccggc agaccgccgc gttcgtgaac     300

cgcaccgtgg ccatgctcga cccccgcgcc gacccgaccg gcgagcaccc cctcaacatc     360

gcctttcacg acttatggca acgtgagagc gcccccatgt cccccctctg gcagcggcgt     420

gccgtcgacc actggacgca gtacctcacc gcccacatca ccgaggcgac caaccgcacc     480

cgccacacca gtcccaccat cgccgactac ctggagctgc gccaccgcac cggcttcatg     540

cccccgctgc tcgacctgat cgaacgggtc tggcgcgccg aaatccccgc ccccgtctac     600

accacccccg aagtgcagac ccttctccat acgaccaacc agaacatcaa catcgtcaat     660

gacgtcctgt ccctggagaa ggaagaagcc cacggcgacc cgcacaacct cgtcctggtc     720

atccagcacg agcggcagag tacccgacag caggccctcg ccaccgcccg gcgcatgatc     780

gacgagtgga ccgatacctt catccgcacc gaaccccggc tccccgccct ctgcggccgc     840

ctcggcattc ctctcgccga ccgcacatcc ctctacaccg ccgtcgaagg catgcgcgcc     900

gccatccgcg gcaactacga ctggtgcgcc gagaccaacc gctacgccgt tcaccgcccc     960

accggcacgg ggcgggccac caccccttgg tga                                 993

<210>  2
<211>  330
<212>  PRT
<213>  Streptomyces clavuligerus ATCC 27064

<400>  2

Met Pro Ala Gly His Glu Glu Phe Asp Ile Pro Phe Pro Ser Arg Val
1               5                   10                  15

Asn Pro Phe His Ala Arg Ala Glu Asp Arg His Val Ala Trp Met Arg
            20                  25                  30

Ala Met Gly Leu Ile Thr Gly Asp Ala Ala Glu Ala Thr Tyr Arg Arg
        35                  40                  45

Trp Ser Pro Ala Lys Val Gly Ala Arg Trp Phe Tyr Leu Ala Gln Gly
    50                  55                  60

Glu Asp Leu Asp Leu Gly Cys Asp Ile Phe Gly Trp Phe Phe Ala Tyr
65                  70                  75                  80

Asp Asp His Phe Asp Gly Pro Thr Gly Thr Asp Pro Arg Gln Thr Ala
                85                  90                  95

Ala Phe Val Asn Arg Thr Val Ala Met Leu Asp Pro Arg Ala Asp Pro
            100                 105                 110

Thr Gly Glu His Pro Leu Asn Ile Ala Phe His Asp Leu Trp Gln Arg
        115                 120                 125

Glu Ser Ala Pro Met Ser Pro Leu Trp Gln Arg Arg Ala Val Asp His
    130                 135                 140

Trp Thr Gln Tyr Leu Thr Ala His Ile Thr Glu Ala Thr Asn Arg Thr
145                 150                 155                 160

Arg His Thr Ser Pro Thr Ile Ala Asp Tyr Leu Glu Leu Arg His Arg
                165                 170                 175

Thr Gly Phe Met Pro Pro Leu Leu Asp Leu Ile Glu Arg Val Trp Arg
            180                 185                 190

Ala Glu Ile Pro Ala Pro Val Tyr Thr Thr Pro Glu Val Gln Thr Leu
        195                 200                 205

Leu His Thr Thr Asn Gln Asn Ile Asn Ile Val Asn Asp Val Leu Ser
    210                 215                 220

Leu Glu Lys Glu Glu Ala His Gly Asp Pro His Asn Leu Val Leu Val
225                 230                 235                 240

Ile Gln His Glu Arg Gln Ser Thr Arg Gln Gln Ala Leu Ala Thr Ala
                245                 250                 255

Arg Arg Met Ile Asp Glu Trp Thr Asp Thr Phe Ile Arg Thr Glu Pro
            260                 265                 270

Arg Leu Pro Ala Leu Cys Gly Arg Leu Gly Ile Pro Leu Ala Asp Arg
        275                 280                 285

Thr Ser Leu Tyr Thr Ala Val Glu Gly Met Arg Ala Ala Ile Arg Gly
    290                 295                 300

Asn Tyr Asp Trp Cys Ala Glu Thr Asn Arg Tyr Ala Val His Arg Pro
305                 310                 315                 320

Thr Gly Thr Gly Arg Ala Thr Thr Pro Trp
                325                 330

<210>  3
<211>  1005
<212>  DNA
<213>  Streptomyces clavuligerus ATCC 27064

<400>  3
atgccgttcc ccgcccgccc gctgaacgcg ggaatggagc aggccaggca gcggatgtgg      60

gcatggatcg accacttcgg cctcggccct tccgaggcgt cccaccagcg cctgtcacag     120

ctgcggttag aggtgatcac ggcccgctac taccccttcg tggagccgtc cgccctgccc     180

ctgctcgcgc tgcacatggc atgggcctgg tgtgtcgacg agcagttcga cgacgggccc     240

gccggtcgtg atccgcgctg gtgtctgacg gccctccgcg gtctgcggga cgccgtactg     300

ggcgggagcc acataccggg caaccccctc gaagccgccg ccgccgactt ccaccggaga     360

ctgggagagg tccattcgcc ccggtgggtc cgggactaca cggacgtcac cacccgctgg     420

ctgtggagtt actacgccga gagcctggac cgggccaccg accggcacca accgctgccg     480

gactaccggc gccatcggca ggtcaccagc ggcgagcaca tgttcttcat gctcgccgag     540

cacggagcgg gcagggagct tcccgacagt gtgagcaggc tcccggccta catcgcgttg     600

caggacgcgg cggcggagca catgggactg ctcaacgaca tcgtctccct gcccaaggag     660

gaacccatcg gcgacgtgca caacgcggtc actcttgtgt cgcgccactc ccgtgtgccc     720

gccgcggagg cggccgaggc cgtcaacgac atgctgaccg actgcgtcca ccggatgctg     780

gccgcggagg acgaactccc gggacagctc gacgccgtgg gcgtcacgga gcggacccgg     840

gcggacgcgc tggccatggt cgaggcgatc aaggcccata cccggggaaa cttcgactgg     900

cacttcgagg tgagccgcta cgcggggcca ggacagctcg acgaagccgg aagccccgcg     960

tatgtggccg atgtgctggg cggcgcacgc accggtgccg actga                    1005

<210>  4
<211>  334
<212>  PRT
<213>  Streptomyces clavuligerus ATCC 27064

<400>  4

Met Pro Phe Pro Ala Arg Pro Leu Asn Ala Gly Met Glu Gln Ala Arg
1               5                   10                  15

Gln Arg Met Trp Ala Trp Ile Asp His Phe Gly Leu Gly Pro Ser Glu
            20                  25                  30

Ala Ser His Gln Arg Leu Ser Gln Leu Arg Leu Glu Val Ile Thr Ala
        35                  40                  45

Arg Tyr Tyr Pro Phe Val Glu Pro Ser Ala Leu Pro Leu Leu Ala Leu
    50                  55                  60

His Met Ala Trp Ala Trp Cys Val Asp Glu Gln Phe Asp Asp Gly Pro
65                  70                  75                  80

Ala Gly Arg Asp Pro Arg Trp Cys Leu Thr Ala Leu Arg Gly Leu Arg
                85                  90                  95

Asp Ala Val Leu Gly Gly Ser His Ile Pro Gly Asn Pro Leu Glu Ala
            100                 105                 110

Ala Ala Ala Asp Phe His Arg Arg Leu Gly Glu Val His Ser Pro Arg
        115                 120                 125

Trp Val Arg Asp Tyr Thr Asp Val Thr Thr Arg Trp Leu Trp Ser Tyr
    130                 135                 140

Tyr Ala Glu Ser Leu Asp Arg Ala Thr Asp Arg His Gln Pro Leu Pro
145                 150                 155                 160

Asp Tyr Arg Arg His Arg Gln Val Thr Ser Gly Glu His Met Phe Phe
                165                 170                 175

Met Leu Ala Glu His Gly Ala Gly Arg Glu Leu Pro Asp Ser Val Ser
            180                 185                 190

Arg Leu Pro Ala Tyr Ile Ala Leu Gln Asp Ala Ala Ala Glu His Met
        195                 200                 205

Gly Leu Leu Asn Asp Ile Val Ser Leu Pro Lys Glu Glu Pro Ile Gly
    210                 215                 220

Asp Val His Asn Ala Val Thr Leu Val Ser Arg His Ser Arg Val Pro
225                 230                 235                 240

Ala Ala Glu Ala Ala Glu Ala Val Asn Asp Met Leu Thr Asp Cys Val
                245                 250                 255

His Arg Met Leu Ala Ala Glu Asp Glu Leu Pro Gly Gln Leu Asp Ala
            260                 265                 270

Val Gly Val Thr Glu Arg Thr Arg Ala Asp Ala Leu Ala Met Val Glu
        275                 280                 285

Ala Ile Lys Ala His Thr Arg Gly Asn Phe Asp Trp His Phe Glu Val
    290                 295                 300

Ser Arg Tyr Ala Gly Pro Gly Gln Leu Asp Glu Ala Gly Ser Pro Ala
305                 310                 315                 320

Tyr Val Ala Asp Val Leu Gly Gly Ala Arg Thr Gly Ala Asp
                325                 330

<210>  5
<211>  1104
<212>  DNA
<213>  Streptomyces clavuligerus ATCC 27064

<400>  5
atgggtgggg cgtacaggac cgtccaccgt acgccgggag gggctgggat gccgcaggat      60

gttgtgttcg atcttccgtg gccgctgcgg aagcaccccg gtgtggccgg ggcccgggag     120

cactgcctcg gctggctggc cgcccagggg ctggcggacc ggcgggggct gaccgccgag     180

acctttgtga cctggcagct cgacgagttg gcgggctact tcttcccgcg tgccacccag     240

gagggccttg aactcgccac ggacctgatg gtctggtact tcgccccgtt cgacgaccag     300

ttcgacggcg cgctcggccg cgacccccgc cgcacggccg gtgtctgcgc cgggctggcc     360

gaggtcctgt acggggtgcc ggagcccggc ccggtcgcct cgtccccggt gggccgggcg     420

ctgggggacc tgtggcggcg gagttgcacg ggcatgagcc cgttctggcg cacccgtgcc     480

cgtcacaact ggacgggcta tctcgccgcg cacaccgcgg aatcggttcc ccggtaccac     540

gggcggaccg tggacgccgc ttactgcgtg cgccagcggg gatacgcgac ctcgtcacac     600

gtgatcatgg atctgatcga gcggaccggg ggcttcgagg ttccggccat ggtctggcac     660

caccccgtcc tcgtcgagct gcggacgctg acctccgaga tgatcggaat ctccaacgac     720

ctctgctccg cggagaagga ggaggcggac ggcgatctga gcaacaatct gctgctggtc     780

ctggagaacc acgaggggct ggaccgcccg gaggccatcg agcgggcgcg cgccctgacg     840

gcggaacggg tcgcccgctt cctcgacgtc gaacgcgccg tcacggacgt ggactgcctg     900

ctcgacggcg cgggccgcga ggcggtgcgc cggttcgtgg agggcctgca cgacctggtc 960

cggggcgaca acgagtggga gcggaccacc ggccgctacc agccggccat cagcggactt 1020

caggacctcg ccgacctgat gtcctccggg tgggacccgc tcgcctccgg cacggccgcg 1080

ggacacggtg gacacacacg atga 1104

<210> 6
<211> 367
<212> PRT
<213> Streptomyces clavuligerus ATCC 27064

<400> 6

Met Gly Gly Ala Tyr Arg Thr Val His Arg Thr Pro Gly Gly Ala Gly
1               5                   10                  15

Met Pro Gln Asp Val Val Phe Asp Leu Pro Trp Pro Leu Arg Lys His
            20                  25                  30

Pro Gly Val Ala Gly Ala Arg Glu His Cys Leu Gly Trp Leu Ala Ala
        35                  40                  45

Gln Gly Leu Ala Asp Arg Arg Gly Leu Thr Ala Glu Thr Phe Val Thr
    50                  55                  60

Trp Gln Leu Asp Glu Leu Ala Gly Tyr Phe Phe Pro Arg Ala Thr Gln
65                  70                  75                  80

Glu Gly Leu Glu Leu Ala Thr Asp Leu Met Val Trp Tyr Phe Ala Pro
                85                  90                  95

Phe Asp Asp Gln Phe Asp Gly Ala Leu Gly Arg Asp Pro Arg Arg Thr
            100                 105                 110

Ala Gly Val Cys Ala Gly Leu Ala Glu Val Leu Tyr Gly Val Pro Glu
        115                 120                 125

Pro Gly Pro Val Ala Ser Ser Pro Val Gly Arg Ala Leu Gly Asp Leu
    130                 135                 140

Trp Arg Arg Ser Cys Thr Gly Met Ser Pro Phe Trp Arg Thr Arg Ala
145                 150                 155                 160

Arg His Asn Trp Thr Gly Tyr Leu Ala Ala His Thr Ala Glu Ser Val
                165                 170                 175

Pro Arg Tyr His Gly Arg Thr Val Asp Ala Ala Tyr Cys Val Arg Gln
            180                 185                 190

Arg Gly Tyr Ala Thr Ser Ser His Val Ile Met Asp Leu Ile Glu Arg
        195                 200                 205

Thr Gly Gly Phe Glu Val Pro Ala Met Val Trp His His Pro Val Leu
    210                 215                 220

Val Glu Leu Arg Thr Leu Thr Ser Glu Met Ile Gly Ile Ser Asn Asp
225                 230                 235                 240

Leu Cys Ser Ala Glu Lys Glu Glu Ala Asp Gly Asp Leu Ser Asn Asn
                245                 250                 255

Leu Leu Leu Val Leu Glu Asn His Glu Gly Leu Asp Arg Pro Glu Ala
            260                 265                 270

Ile Glu Arg Ala Arg Ala Leu Thr Ala Glu Arg Val Ala Arg Phe Leu
        275                 280                 285

Asp Val Glu Arg Ala Val Thr Asp Val Asp Cys Leu Leu Asp Gly Ala
    290                 295                 300

Gly Arg Glu Ala Val Arg Arg Phe Val Glu Gly Leu His Asp Leu Val
305                 310                 315                 320

Arg Gly Asp Asn Glu Trp Glu Arg Thr Thr Gly Arg Tyr Gln Pro Ala
                325                 330                 335

Ile Ser Gly Leu Gln Asp Leu Ala Asp Leu Met Ser Ser Gly Trp Asp
            340                 345                 350

Pro Leu Ala Ser Gly Thr Ala Ala Gly His Gly Gly His Thr Arg
        355                 360                 365

<210>  7
<211>  1020
<212>  DNA
<213>  Streptomyces griseus IFO 13350

<400>  7
gtggcgcaca cccgtgggca tctggacagc tggaccagac ggacgggtct ggtccaccgc      60

gagtccgcga ggaaccgatt cgaacaagcc gatttcgggg cgttcgtcgg catggtctac     120

ccgacggccg acgaggaaca cctcgatctg gtggccgact ggttcgtctg gctgttcctc     180

gtcgacgacc agctcgacga cggccacctc gggcgttcac ccgaccgggt gagagatgtc     240

gtcgaccgga tgcgcgcggt ggtcgacggc tccgcccccg aggtgctgcc ggacgaggac     300

gccccggccg ccgtgacggc cctggtggac ctgtggaaac gcacgatgcc ggaagcggcc     360

ccgcactggc ggacccggtt cgcctggcac ctggtcacct acctcacgac cgccacgacc 420

tgggaggcgg ggaaccgcgc cgaggacgtg gtgccctcgg aggagacgta catcgccaag 480

cgccgtcaca ccggggccat ccacgtctgc atggacctca tagagatcgt cgcgggcatc 540

gacgccccgg agtcggtcca caacgacccc cgcttcatca ccgcgctgga agccgcctgc 600

aaccacgtgt gctgggccaa cgacgtgtac tccttcgaga aggagcaggt gctcggcgag 660

atccacaacc tcgtccacct ggtccgccac caccgggggc tcggggaaca gcaggcgctg 720

gaccacgtcg cggagcgcct cgcgatggag accgagcggt tcctgaccgc cgaggacgag 780

ctgctggagc tgtatccgga gctgtccggg atgctggttc cctaccttga cgggatgcgg 840

agctggatgc gcggcaacct ggactggtcc cggcagacgc cgcgctacaa ccccgccgac 900

gtgggccagt acgaggaacc cgaggagtat ctggaggaga cggtgctggg cgtgccgccc 960

gcgcgcagcg agaccgccgc ccctgccccg tgcggggccg aggctccccg ggcccgctga 1020

<210> 8
<211> 339
<212> PRT
<213> Streptomyces griseus IFO 13350

<400> 8

Met Ala His Thr Arg Gly His Leu Asp Ser Trp Thr Arg Arg Thr Gly
1               5                   10                  15

Leu Val His Arg Glu Ser Ala Arg Asn Arg Phe Glu Gln Ala Asp Phe
            20                  25                  30

Gly Ala Phe Val Gly Met Val Tyr Pro Thr Ala Asp Glu Glu His Leu
        35                  40                  45

Asp Leu Val Ala Asp Trp Phe Val Trp Leu Phe Leu Val Asp Asp Gln
    50                  55                  60

Leu Asp Asp Gly His Leu Gly Arg Ser Pro Asp Arg Val Arg Asp Val
65                  70                  75                  80

Val Asp Arg Met Arg Ala Val Val Asp Gly Ser Ala Pro Glu Val Leu
                85                  90                  95

Pro Asp Glu Asp Ala Pro Ala Ala Val Thr Ala Leu Val Asp Leu Trp
            100                 105                 110

Lys Arg Thr Met Pro Glu Ala Ala Pro His Trp Arg Thr Arg Phe Ala
        115                 120                 125

Trp His Leu Val Thr Tyr Leu Thr Thr Ala Thr Thr Trp Glu Ala Gly
    130                 135                 140

Asn Arg Ala Glu Asp Val Val Pro Ser Glu Glu Thr Tyr Ile Ala Lys
145                 150                 155                 160

Arg Arg His Thr Gly Ala Ile His Val Cys Met Asp Leu Ile Glu Ile
                165                 170                 175

Val Ala Gly Ile Asp Ala Pro Glu Ser Val His Asn Asp Pro Arg Phe
            180                 185                 190

Ile Thr Ala Leu Glu Ala Ala Cys Asn His Val Cys Trp Ala Asn Asp
        195                 200                 205

Val Tyr Ser Phe Glu Lys Glu Gln Val Leu Gly Glu Ile His Asn Leu
    210                 215                 220

Val His Leu Val Arg His His Arg Gly Leu Gly Glu Gln Gln Ala Leu
225                 230                 235                 240

Asp His Val Ala Glu Arg Leu Ala Met Glu Thr Glu Arg Phe Leu Thr
                245                 250                 255

Ala Glu Asp Glu Leu Leu Glu Leu Tyr Pro Glu Leu Ser Gly Met Leu
            260                 265                 270

Val Pro Tyr Leu Asp Gly Met Arg Ser Trp Met Arg Gly Asn Leu Asp
        275                 280                 285

Trp Ser Arg Gln Thr Pro Arg Tyr Asn Pro Ala Asp Val Gly Gln Tyr
    290                 295                 300

Glu Glu Pro Glu Glu Tyr Leu Glu Glu Thr Val Leu Gly Val Pro Pro
305                 310                 315                 320

Ala Arg Ser Glu Thr Ala Ala Pro Ala Pro Cys Gly Ala Glu Ala Pro
                325                 330                 335

Arg Ala Arg

<210>  9
<211>  1146
<212>  DNA
<213>  Streptomyces venezuelae ATCC 10712

<400>  9
atgacagtgc gtgccgtcga gctcccccag ctgtggatgc cctatccgct gcgggtgaac        60

ccctacctgt cggccctgcg cgaggagagc gagacctggg cccgcgagat gggcatgctg 120

ggcggcgaag gaccgtccgc ccgcggcgcg atatggaccc gctcccagtt ccacgccatg 180

accgtggagc tgctgaccgc ctggaccctg ccggacgcct cgctcgccgg tctgcggctc 240

aaccaccgct tcaacatctg ggcgctggcc tgggacgact acttcgcgtc caccttcaag 300

cagaccggcg acctgcccgg ggcgctggac ttcaccgccc ggctgcacgc cttcctgcga 360

ccagaggccg acgcccggtc gccagaaccg gtgaacgccg tcgagcgcgg tatcgccgat 420

ctccagaagc acctgttccc gccccgactg gcccactggc gccaggagtt caacctcgcg 480

ctcgcccact acatcgacgc cggcgtgcag gaactcacca acagccgcgg cggccgcgtc 540

ccccatctca tcgagtacgc gccgttccga cgcgagagct tcgccgccca caccgccccc 600

tactccgtcg aactggccac cggcgcgcgg atacccgagc agctccggca caccaggacc 660

gtccacgccc tgctcgacgc gttcatggac gtcatgggcc tggcaaacga tgtcgcctcc 720

tacgaacggg aggtccacga ggaacgcgac gtcaacaacc tcgtggtggt catcggaacg 780

tccctcggca tcaccctcca ccaggccgta cccgccgcgc tgcacatggt gaacgcccgc 840

atgcgggact tcgagcacct gcggcaccac gaactgccgc cgctggtcga ccggttcggg 900

ctgcgccccg acgagcggga ggagctggag acctggctcg ggggagccgc cagcttcctc 960

tcgggcctcc acgcctggta caccggggcg ccccgctacg cgcccacgga ccactccgta 1020

ccggagcagc gtcagcgcgg cggttccgag ggggctgccg acagacacgc gccggcagcc 1080

tcctccctgg cccggggcgc ggaggcccag acgtgcgggc ctaccgcctc cgcaggaagc 1140

gcatga 1146

<210> 10
<211> 381
<212> PRT
<213> Streptomyces venezuelae ATCC 10712

<400> 10

Met Thr Val Arg Ala Val Glu Leu Pro Gln Leu Trp Met Pro Tyr Pro
1               5                   10                  15

Leu Arg Val Asn Pro Tyr Leu Ser Ala Leu Arg Glu Glu Ser Glu Thr
            20                  25                  30

Trp Ala Arg Glu Met Gly Met Leu Gly Gly Glu Gly Pro Ser Ala Arg
        35                  40                  45

Gly Ala Ile Trp Thr Arg Ser Gln Phe His Ala Met Thr Val Glu Leu
    50                  55                  60

Leu Thr Ala Trp Thr Leu Pro Asp Ala Ser Leu Ala Gly Leu Arg Leu
65                  70                  75                  80

Asn His Arg Phe Asn Ile Trp Ala Leu Ala Trp Asp Asp Tyr Phe Ala
                85                  90                  95

Ser Thr Phe Lys Gln Thr Gly Asp Leu Pro Gly Ala Leu Asp Phe Thr
            100                 105                 110

Ala Arg Leu His Ala Phe Leu Arg Pro Glu Ala Asp Ala Arg Ser Pro
        115                 120                 125

Glu Pro Val Asn Ala Val Glu Arg Gly Ile Ala Asp Leu Gln Lys His
    130                 135                 140

Leu Phe Pro Pro Arg Leu Ala His Trp Arg Gln Glu Phe Asn Leu Ala
145                 150                 155                 160

Leu Ala His Tyr Ile Asp Ala Gly Val Gln Glu Leu Thr Asn Ser Arg
                165                 170                 175

Gly Gly Arg Val Pro His Leu Ile Glu Tyr Ala Pro Phe Arg Arg Glu
            180                 185                 190

Ser Phe Ala Ala His Thr Ala Pro Tyr Ser Val Glu Leu Ala Thr Gly
        195                 200                 205

Ala Arg Ile Pro Glu Gln Leu Arg His Thr Arg Thr Val His Ala Leu
    210                 215                 220

Leu Asp Ala Phe Met Asp Val Met Gly Leu Ala Asn Asp Val Ala Ser
225                 230                 235                 240

Tyr Glu Arg Glu Val His Glu Glu Arg Asp Val Asn Asn Leu Val Val
                245                 250                 255

Val Ile Gly Thr Ser Leu Gly Ile Thr Leu His Gln Ala Val Pro Ala
            260                 265                 270

Ala Leu His Met Val Asn Ala Arg Met Arg Asp Phe Glu His Leu Arg
        275                 280                 285

His His Glu Leu Pro Pro Leu Val Asp Arg Phe Gly Leu Arg Pro Asp
    290                 295                 300

Glu Arg Glu Glu Leu Glu Thr Trp Leu Gly Gly Ala Ala Ser Phe Leu
305                 310                 315                 320

Ser Gly Leu His Ala Trp Tyr Thr Gly Ala Pro Arg Tyr Ala Pro Thr
                325                 330                 335

Asp His Ser Val Pro Glu Gln Arg Gln Arg Gly Gly Ser Glu Gly Ala
            340                 345                 350

Ala Asp Arg His Ala Pro Ala Ala Ser Ser Leu Ala Arg Gly Ala Glu
        355                 360                 365

Ala Gln Thr Cys Gly Pro Thr Ala Ser Ala Gly Ser Ala
    370                 375                 380

<210>  11
<211>  1110
<212>  DNA
<213>  Streptomyces sp. SK 1894

<400>  11
gtgggacccc atttgaccgt tccgccgatc tacgcgccga tacctccggc gatccatcca      60

agacacgccg tcatcgacgc gcgtacggcg gcctgggcgg agtcgttcga gatcggctcg     120

cccgagctgc gcagcagact cgtccggcac gacatcggca ccttctgcgc ccggatcctg     180

ccggagggca gagaggaggt ggtcagcctt ctcgccgact tcgtgctctg gctgttcggc     240

gtggacgacg gctactgcga ggagagcgac ctgggccacc ggcccggcga tctcgtcggg     300

gtcctccacc ggctgctgcg ggtcgcccag gaccccgagg ggcccttcct gctggccgac     360

ccgctcgcca acgggctgcg cgacctgcgg gcccggatcg acagattcgg tacgtacgcc     420

caggccaccc ggtgggtgga cgccctgcgg gagtacttct tctcggtggt ctgggaagcc     480

gaccaccgcc gggccggtac ggtgccgaac ctcgacgact acaccctgat gcggctctac     540

gacggcgccg ccacggtcgt gttccccctg ctggagatgg ggcacgacta cgagctgacg     600

ccccaggaac gggaccatcc ggcgacccgg gcgctctgcg agatgacgtc cttcatcatc     660

agctgggaca acgacatctt ctcgcaccac aaggaggtgt ccggcaaggg cttctacctc     720

aacgccctgc gcgtactcca gcaccaggac gggctctccc cggccgccgc gctcgccacg     780

gccatcgccc agcgcgaccg ggtgatgtgc ctctacctcc ggctccacga ccaccgggca     840

gggacggcga gcccccggct gcgccgctat ctgtcgagcc tgcggcactt cgtccgggcc     900

acccaggact ggagcgtcag ctcccgccgc tacaccaccc ccgacaaccc ggccgacctg     960

ccgagcagct tccgcagcac cccgacggac gcgggcacgg agccgctgga cattccggtg    1020

atccggtggt ggtggtcgct gctgcccgaa ccggaaggag cccgggatga cgagacgccg    1080

ttacgcaggc ccgccgtcgc gggccgctga                                     1110

<210>  12

<211>  369
<212>  PRT
<213>  Streptomyces sp. SK 1894

<400>  12

Val Gly Pro His Leu Thr Val Pro Pro Ile Tyr Ala Pro Ile Pro Pro
1               5                   10                  15

Ala Ile His Pro Arg His Ala Val Ile Asp Ala Arg Thr Ala Ala Trp
            20                  25                  30

Ala Glu Ser Phe Glu Ile Gly Ser Pro Glu Leu Arg Ser Arg Leu Val
        35                  40                  45

Arg His Asp Ile Gly Thr Phe Cys Ala Arg Ile Leu Pro Glu Gly Arg
    50                  55                  60

Glu Glu Val Val Ser Leu Leu Ala Asp Phe Val Leu Trp Leu Phe Gly
65                  70                  75                  80

Val Asp Asp Gly Tyr Cys Glu Glu Ser Asp Leu Gly His Arg Pro Gly
                85                  90                  95

Asp Leu Val Gly Val Leu His Arg Leu Leu Arg Val Ala Gln Asp Pro
            100                 105                 110

Glu Gly Pro Phe Leu Leu Ala Asp Pro Leu Ala Asn Gly Leu Arg Asp
        115                 120                 125

Leu Arg Ala Arg Ile Asp Arg Phe Gly Thr Tyr Ala Gln Ala Thr Arg
    130                 135                 140

Trp Val Asp Ala Leu Arg Glu Tyr Phe Phe Ser Val Val Trp Glu Ala
145                 150                 155                 160

Asp His Arg Arg Ala Gly Thr Val Pro Asn Leu Asp Asp Tyr Thr Leu
                165                 170                 175

Met Arg Leu Tyr Asp Gly Ala Ala Thr Val Val Phe Pro Leu Leu Glu
            180                 185                 190

Met Gly His Asp Tyr Glu Leu Thr Pro Gln Glu Arg Asp His Pro Ala
        195                 200                 205

Thr Arg Ala Leu Cys Glu Met Thr Ser Phe Ile Ile Ser Trp Asp Asn
    210                 215                 220

Asp Ile Phe Ser His His Lys Glu Val Ser Gly Lys Gly Phe Tyr Leu

225                 230                 235                 240

Asn Ala Leu Arg Val Leu Gln His Gln Asp Gly Leu Ser Pro Ala Ala
                245                 250                 255

Ala Leu Ala Thr Ala Ile Ala Gln Arg Asp Arg Val Met Cys Leu Tyr
            260                 265                 270

Leu Arg Leu His Asp His Arg Ala Gly Thr Ala Ser Pro Arg Leu Arg
        275                 280                 285

Arg Tyr Leu Ser Ser Leu Arg His Phe Val Arg Ala Thr Gln Asp Trp
    290                 295                 300

Ser Val Ser Ser Arg Arg Tyr Thr Thr Pro Asp Asn Pro Ala Asp Leu
305                 310                 315                 320

Pro Ser Ser Phe Arg Ser Thr Pro Thr Asp Ala Gly Thr Glu Pro Leu
                325                 330                 335

Asp Ile Pro Val Ile Arg Trp Trp Trp Ser Leu Leu Pro Glu Pro Glu
            340                 345                 350

Gly Ala Arg Asp Asp Glu Thr Pro Leu Arg Arg Pro Ala Val Ala Gly
        355                 360                 365

Arg

<210>  13
<211>  1011
<212>  DNA
<213>  Streptomyces lactacystinaeus

<400>  13
atgaccgatc tccccgacgg gcagccgcac agcgctccgg acgacttgtg gtgccccttt      60

ccctccctga cgaaccccca cgccgaagag gccgaccggc agagcctggc gtggctggcg     120

caccaccggc tgctggacgg gcgcctccgg cagtccgacc ggatcgggcg ccagcgcagc     180

ggccacctgg cggcacggac caaccccgag gtctccgccg ggatgctgcg cctgctgtcc     240

gactggtacg tctggctctt cgccttcgac gacgggtact gcgaggaccg cgaacacggc     300

catcgccccg ggccgttggc ccgcacgacc gctctcctcg cccgggtgat cgaccccgat     360

ccgcccggcg ccaggatgtc cgacgacggg ctgtccggac cggccggcgc ctacgccagg     420

gccctgcggg agatccggga ccgggtcgcc gcgcaggcct cgccatccca gctgttccgc     480

tgggagtgca cggtccgcga gtacctctcc gcccaggtgt gggaggccgc caaccgcgag     540

gcgggcacgg tgccgggccc gtccgagtac gtggccgtgc gcaggcacgc cggggcgacg 600

tacacctgcc tcgccctggt cgacatcgcg gccggccacg aagtcccggc cgcggaggcg 660

gacgggccct cgctgcgcag gctcagggac ctcacggcga acctggtgag ctgggacaac 720

gacctgtact cccacgccaa ggagaccgcg ggcggccagg gccgccacaa cctggtcgag 780

gtcctcgccc gccaccaccg ctgttctccg gccgaggccc gacggtcggc cgtcgccatg 840

cgggacgccg agatgcgcgc gttcatcgag ctgagccgaa ccgtccgcgc ggacggcggc 900

gaggcggcct ccgcgtacgc ccggagcctg gggctgtggc tgcgcggaca catcgactgg 960

tcccgcggca gctcccggtt cgacgtgccg gcccccacgg cgaggccctg a 1011

<210> 14
<211> 336
<212> PRT
<213> Streptomyces lactacystinaeus

<400> 14

Met Thr Asp Leu Pro Asp Gly Gln Pro His Ser Ala Pro Asp Asp Leu
1               5                   10                  15

Trp Cys Pro Phe Pro Ser Leu Thr Asn Pro His Ala Glu Glu Ala Asp
            20                  25                  30

Arg Gln Ser Leu Ala Trp Leu Ala His His Arg Leu Leu Asp Gly Arg
        35                  40                  45

Leu Arg Gln Ser Asp Arg Ile Gly Arg Gln Arg Ser Gly His Leu Ala
    50                  55                  60

Ala Arg Thr Asn Pro Glu Val Ser Ala Gly Met Leu Arg Leu Leu Ser
65                  70                  75                  80

Asp Trp Tyr Val Trp Leu Phe Ala Phe Asp Asp Gly Tyr Cys Glu Asp
                85                  90                  95

Arg Glu His Gly His Arg Pro Gly Pro Leu Ala Arg Thr Thr Ala Leu
            100                 105                 110

Leu Ala Arg Val Ile Asp Pro Asp Pro Pro Gly Ala Arg Met Ser Asp
        115                 120                 125

Asp Gly Leu Ser Gly Pro Ala Gly Ala Tyr Ala Arg Ala Leu Arg Glu
    130                 135                 140

Ile Arg Asp Arg Val Ala Ala Gln Ala Ser Pro Ser Gln Leu Phe Arg
145                 150                 155                 160

Trp Glu Cys Thr Val Arg Glu Tyr Leu Ser Ala Gln Val Trp Glu Ala
                165                 170                 175

Ala Asn Arg Glu Ala Gly Thr Val Pro Gly Pro Ser Glu Tyr Val Ala
            180                 185                 190

Val Arg Arg His Ala Gly Ala Thr Tyr Thr Cys Leu Ala Leu Val Asp
        195                 200                 205

Ile Ala Ala Gly His Glu Val Pro Ala Ala Glu Ala Asp Gly Pro Ser
    210                 215                 220

Leu Arg Arg Leu Arg Asp Leu Thr Ala Asn Leu Val Ser Trp Asp Asn
225                 230                 235                 240

Asp Leu Tyr Ser His Ala Lys Glu Thr Ala Gly Gly Gln Gly Arg His
                245                 250                 255

Asn Leu Val Glu Val Leu Ala Arg His His Arg Cys Ser Pro Ala Glu
            260                 265                 270

Ala Arg Arg Ser Ala Val Ala Met Arg Asp Ala Glu Met Arg Ala Phe
        275                 280                 285

Ile Glu Leu Ser Arg Thr Val Arg Ala Asp Gly Gly Glu Ala Ala Ser
    290                 295                 300

Ala Tyr Ala Arg Ser Leu Gly Leu Trp Leu Arg Gly His Ile Asp Trp
305                 310                 315                 320

Ser Arg Gly Ser Ser Arg Phe Asp Val Pro Ala Pro Thr Ala Arg Pro
                325                 330                 335

<210>  15
<211>  1017
<212>  DNA
<213>  Mycobacterium marinum M

<400>  15
gtgaacgtta ttgacttccc ccaggtcgac ttgggtaccg aggcgacaat cagccccgat      60

gtcgacaaag caggggaaca cctgaagaca tggtctcgtg ccacaggcat cgtcctcgac     120

ggccccaacg gtgacgcctt cgatgcactc gcgcagcatc gcctggcggc atggacctat     180

ccggcggcaa ccggtgcgga gcttgatctg atggcggact ggatcggttg gctctttgcc     240

ttcgacgacg ttttcgatga atcgatagac ggctgcgatc aggactttgc cattgcgacg     300

gcggcggcca ccaacaccgt ctacaccggg ctgccctcag cacccccgag cccagtagtc     360

aggccctatg tggtggcgtt ggaggatctt tgggagcgga ccacccaggg catgccggcg 420

tactggtgca gacgactggc caacgacatg gtcgattacg tcaactccta ccgaagccat 480

gccctgatca acgcatcacg cattgcgctc gacgaacagt catatcgagc ccaccggctg 540

atctccagcg cagtattcat cacgctcgac ctcggcgaag ccgccgctag acgtgcccta 600

tcggagtcgc tgttggtgca cccatacata cgggccgctc gcgaagccgc caacaacatc 660

gtgtcctgga gcaacgacct ctactccgcg cccaaagagc tcagcctggg cgacctgtgc 720

aactacatcg ccgtcctgca gtctcaggat gatctcacga ctgcgcaagc agccgaccgg 780

gtcagtcagt acctgcacga ggagatcgcg cgcttcgatc aaaccaaaca gctcatccac 840

acccacctca tcccacgcct gccaaccgaa gatcaacaag gcgtgatctc catgctggat 900

acctgcagca actggataac cggcaacgtg gcgtggtcat tggagaccgc acgctacgca 960

tcgcgcgcca tcgacatgga gcgcgaaaag gaagtgttcg gggacttgcg aacctag 1017

<210> 16
<211> 338
<212> PRT
<213> Mycobacterium marinum M

<400> 16

Met Asn Val Ile Asp Phe Pro Gln Val Asp Leu Gly Thr Glu Ala Thr
1               5                   10                  15

Ile Ser Pro Asp Val Asp Lys Ala Gly Glu His Leu Lys Thr Trp Ser
            20                  25                  30

Arg Ala Thr Gly Ile Val Leu Asp Gly Pro Asn Gly Asp Ala Phe Asp
        35                  40                  45

Ala Leu Ala Gln His Arg Leu Ala Ala Trp Thr Tyr Pro Ala Ala Thr
    50                  55                  60

Gly Ala Glu Leu Asp Leu Met Ala Asp Trp Ile Gly Trp Leu Phe Ala
65                  70                  75                  80

Phe Asp Asp Val Phe Asp Glu Ser Ile Asp Gly Cys Asp Gln Asp Phe
                85                  90                  95

Ala Ile Ala Thr Ala Ala Ala Thr Asn Thr Val Tyr Thr Gly Leu Pro
            100                 105                 110

Ser Ala Pro Pro Ser Pro Val Val Arg Pro Tyr Val Val Ala Leu Glu
        115                 120                 125

Asp Leu Trp Glu Arg Thr Thr Gln Gly Met Pro Ala Tyr Trp Cys Arg

130                 135                 140

Arg Leu Ala Asn Asp Met Val Asp Tyr Val Asn Ser Tyr Arg Ser His
145                 150                 155                 160

Ala Leu Ile Asn Ala Ser Arg Ile Ala Leu Asp Glu Gln Ser Tyr Arg
                165                 170                 175

Ala His Arg Leu Ile Ser Ser Ala Val Phe Ile Thr Leu Asp Leu Gly
            180                 185                 190

Glu Ala Ala Ala Arg Arg Ala Leu Ser Glu Ser Leu Leu Val His Pro
        195                 200                 205

Tyr Ile Arg Ala Ala Arg Glu Ala Ala Asn Asn Ile Val Ser Trp Ser
    210                 215                 220

Asn Asp Leu Tyr Ser Ala Pro Lys Glu Leu Ser Leu Gly Asp Leu Cys
225                 230                 235                 240

Asn Tyr Ile Ala Val Leu Gln Ser Gln Asp Asp Leu Thr Thr Ala Gln
                245                 250                 255

Ala Ala Asp Arg Val Ser Gln Tyr Leu His Glu Glu Ile Ala Arg Phe
            260                 265                 270

Asp Gln Thr Lys Gln Leu Ile His Thr His Leu Ile Pro Arg Leu Pro
        275                 280                 285

Thr Glu Asp Gln Gln Gly Val Ile Ser Met Leu Asp Thr Cys Ser Asn
    290                 295                 300

Trp Ile Thr Gly Asn Val Ala Trp Ser Leu Glu Thr Ala Arg Tyr Ala
305                 310                 315                 320

Ser Arg Ala Ile Asp Met Glu Arg Glu Lys Glu Val Phe Gly Asp Leu
                325                 330                 335

Arg Thr

<210>  17
<211>  966
<212>  DNA
<213>  Streptomyces clavuligerus ATCC 27064

<400>  17
atgaccatct ccgttcccca gctcgactgc ccgctgagcc gtccggtcca cccggaagga        60

gaacgggccg acgcgtacgc cgtggagtgg ttgcgcggcg tgggcctcat ggcggacgag     120

gccgacgccg ccccggtcct ggccgtcggg ctcggcaggc tcgccgcgtg ctatgtcgac     180

gagaacgcct cctgggacac gttggccttc atgaccatcc tgatggcctg gtacgccgag     240

tacgacgacc gcgcgatcga ctccaccggc gcgatcgacg gtctgacgga cgcggaggtg     300

gcggaactcc accgcgccct cggggagatc ctgcgcgacc ggcccgcacc cgacccctcc     360

gaccccgtac aacgcggact ggccgacgtc tggcggacgc tgaacggcct cgcgtccgac     420

tgggaccgcg ctgccttcgt cgacaccacc ctccgctact tcgaagccaa ccggtacgag     480

cgggtcaaca tccgccgggg aatccctccg actccttccg cccatatcgg catgcgccgc     540

cacggcggcc atgtgtacgg catgtacatc ctgggtgcgg cggtcaacgg ctaccggccc     600

gagcgccggg tgctcgacca cgccgccgtc cgcgaactgg agaccctcgc cgcgaactac     660

acctcgtggg cgaacgatct gcactccttc gcccgcgaac accgcatggg ccaggtcaac     720

aatctggtct ggtcggtcca ccaccacgag gggctgacct tccagcaggc ggcggaccgg     780

gtcgccgatc tgtgcgacaa ggaactggcc gcctatctgg agctgcggca gacgctgccc     840

gagttgggca tccccctcac cggggccacc ggacggcatg tcaggttcct ggaggacatg     900

atgtggtcca tggtcgactg gtctgcgcgg agcgcgcgct acgacgtcgt gcccgaggcc     960

gcctga                                                                966

<210>  18
<211>  321
<212>  PRT
<213>  Streptomyces clavuligerus ATCC 27064

<400>  18

Met Thr Ile Ser Val Pro Gln Leu Asp Cys Pro Leu Ser Arg Pro Val
1               5                   10                  15

His Pro Glu Gly Glu Arg Ala Asp Ala Tyr Ala Val Glu Trp Leu Arg
            20                  25                  30

Gly Val Gly Leu Met Ala Asp Glu Ala Asp Ala Ala Pro Val Leu Ala
        35                  40                  45

Val Gly Leu Gly Arg Leu Ala Ala Cys Tyr Val Asp Glu Asn Ala Ser
    50                  55                  60

Trp Asp Thr Leu Ala Phe Met Thr Ile Leu Met Ala Trp Tyr Ala Glu
65                  70                  75                  80

Tyr Asp Asp Arg Ala Ile Asp Ser Thr Gly Ala Ile Asp Gly Leu Thr
                85                  90                  95

Asp Ala Glu Val Ala Glu Leu His Arg Ala Leu Gly Glu Ile Leu Arg
        100                 105                 110

Asp Arg Pro Ala Pro Asp Pro Ser Asp Pro Val Gln Arg Gly Leu Ala
    115                 120                 125

Asp Val Trp Arg Thr Leu Asn Gly Leu Ala Ser Asp Trp Asp Arg Ala
    130                 135                 140

Ala Phe Val Asp Thr Thr Leu Arg Tyr Phe Glu Ala Asn Arg Tyr Glu
145                 150                 155                 160

Arg Val Asn Ile Arg Arg Gly Ile Pro Pro Thr Pro Ser Ala His Ile
                165                 170                 175

Gly Met Arg Arg His Gly Gly His Val Tyr Gly Met Tyr Ile Leu Gly
        180                 185                 190

Ala Ala Val Asn Gly Tyr Arg Pro Glu Arg Arg Val Leu Asp His Ala
    195                 200                 205

Ala Val Arg Glu Leu Glu Thr Leu Ala Ala Asn Tyr Thr Ser Trp Ala
    210                 215                 220

Asn Asp Leu His Ser Phe Ala Arg Glu His Arg Met Gly Gln Val Asn
225                 230                 235                 240

Asn Leu Val Trp Ser Val His His His Glu Gly Leu Thr Phe Gln Gln
                245                 250                 255

Ala Ala Asp Arg Val Ala Asp Leu Cys Asp Lys Glu Leu Ala Ala Tyr
        260                 265                 270

Leu Glu Leu Arg Gln Thr Leu Pro Glu Leu Gly Ile Pro Leu Thr Gly
    275                 280                 285

Ala Thr Gly Arg His Val Arg Phe Leu Glu Asp Met Met Trp Ser Met
    290                 295                 300

Val Asp Trp Ser Ala Arg Ser Ala Arg Tyr Asp Val Val Pro Glu Ala
305                 310                 315                 320

Ala

<210>  19
<211>  1380

<212>  DNA
<213>  Streptomyces clavuligerus ATCC 27064

<400>  19
atgcgcggag ccggtcccgg gagcgtccga ttttcgtcgc tcttcagcgg tcttcagcgt      60

tcttcggcgt tcttcagtgc tcttcgggaa tcttcggctt ttgacgttcg acacaggagg     120

cagagagtga gtcatgaaca gcgggtgccg tcggtggcgg cgcggtacgg ggggaccgcc     180

gtgcacagcg aggacagtgg accggaggac gtcatggtcc tccgggtccc ggaactcgga     240

cggcggttac ggccgccccg gctgcacccc gccacacccg ccgtggtcgg gcagcgggtt     300

ccgtggctgc gggacgcgct cgcggacatc gagttccccg gggggacgcc ggaggacttc     360

ctccggcagg acgtccacca ctggagcgtc tactgtctgc ccaccgcgcg ggccgaccgc     420

atcgccgatc tgagcaatat ccatgaactg gccttcgcca tggacgacat gctcgaatcc     480

gtgcacgaca ccgacgccga cgccgacgtc cacaccgctt ccgatctcga tcccgcttcc     540

ggttccgctt ccaatcccgg ttccggttcc ggttccggtt tcggttccgg tttcggtcgc     600

gcggcccgcc gggagcgggt cgagccgctg gcccgcgcgc tggagagggc gctcgctggg     660

ctgccgccca ccggcccggt gccgtcctat ctgcgggcgg ccgacggctg cttccggacg     720

ctgcgcgaga cggggcccgc cccctggtac cggcggttcg gcgaggccgt cctgtcctgg     780

ttccacgggg ccgtcaagga gagttcgatg atggcgacgg ggcggctgac cggcttcgag     840

gagacgctgg acagccgtat cgacaccgcg ggcgggttct tcatcgccac cagcatcgag     900

tacggcctcg gcatcgacct cacggacgcc atcgccgccg gtcctgagct gggcgaggtg     960

gagcgggcgg cctgggtgca cggcgtgctg gtcaacgatc tgttctcgta ccgtaaggag    1020

cacttcgggg aggcgggccg tgctccggac ggccgcgctc cggacggccg tgcgaacacc    1080

cttcgtgtcc tcgccgacga gtacgcgtgt tccctccagg aggcggtgga cctgctggtc    1140

gagcacgtcg acgcggcgga ggcccggttc ctggaactgc gcgcggacgt cctcggcgct    1200

cccctcggcg cccgtcccgg cgtccgggag tatctcgacg ccctcgaact ggtactgccc    1260

gggaacatcg tctggtcccg gacctcacgg cgctaccacg ggaccggctg cccctggacc    1320

gggaccgccc ggacggcgat ggccctccac cccgacagga ccgtgttcac cccctggtag    1380

<210>  20
<211>  459
<212>  PRT
<213>  Streptomyces clavuligerus ATCC 27064

<400>  20

Met Arg Gly Ala Gly Pro Gly Ser Val Arg Phe Ser Ser Leu Phe Ser
1               5                   10                  15

Gly Leu Gln Arg Ser Ser Ala Phe Phe Ser Ala Leu Arg Glu Ser Ser

20                  25                  30

Ala Phe Asp Val Arg His Arg Arg Gln Arg Val Ser His Glu Gln Arg
        35                  40                  45

Val Pro Ser Val Ala Ala Arg Tyr Gly Gly Thr Ala Val His Ser Glu
    50                  55                  60

Asp Ser Gly Pro Glu Asp Val Met Val Leu Arg Val Pro Glu Leu Gly
65                  70                  75                  80

Arg Arg Leu Arg Pro Pro Arg Leu His Pro Ala Thr Pro Ala Val Val
                85                  90                  95

Gly Gln Arg Val Pro Trp Leu Arg Asp Ala Leu Ala Asp Ile Glu Phe
            100                 105                 110

Pro Gly Gly Thr Pro Glu Asp Phe Leu Arg Gln Asp Val His His Trp
        115                 120                 125

Ser Val Tyr Cys Leu Pro Thr Ala Arg Ala Asp Arg Ile Ala Asp Leu
    130                 135                 140

Ser Asn Ile His Glu Leu Ala Phe Ala Met Asp Asp Met Leu Glu Ser
145                 150                 155                 160

Val His Asp Thr Asp Ala Asp Ala Asp Val His Thr Ala Ser Asp Leu
                165                 170                 175

Asp Pro Ala Ser Gly Ser Ala Ser Asn Pro Gly Ser Gly Ser Gly Ser
            180                 185                 190

Gly Phe Gly Ser Gly Phe Gly Arg Ala Ala Arg Arg Glu Arg Val Glu
        195                 200                 205

Pro Leu Ala Arg Ala Leu Glu Arg Ala Leu Ala Gly Leu Pro Pro Thr
    210                 215                 220

Gly Pro Val Pro Ser Tyr Leu Arg Ala Ala Asp Gly Cys Phe Arg Thr
225                 230                 235                 240

Leu Arg Glu Thr Gly Pro Ala Pro Trp Tyr Arg Arg Phe Gly Glu Ala
                245                 250                 255

Val Leu Ser Trp Phe His Gly Ala Val Lys Glu Ser Ser Met Met Ala
            260                 265                 270

Thr Gly Arg Leu Thr Gly Phe Glu Glu Thr Leu Asp Ser Arg Ile Asp
        275                 280                 285

Thr Ala Gly Gly Phe Phe Ile Ala Thr Ser Ile Glu Tyr Gly Leu Gly
    290                 295                 300

Ile Asp Leu Thr Asp Ala Ile Ala Ala Gly Pro Glu Leu Gly Glu Val
305                 310                 315                 320

Glu Arg Ala Ala Trp Val His Gly Val Leu Val Asn Asp Leu Phe Ser
                325                 330                 335

Tyr Arg Lys Glu His Phe Gly Glu Ala Gly Arg Ala Pro Asp Gly Arg
            340                 345                 350

Ala Pro Asp Gly Arg Ala Asn Thr Leu Arg Val Leu Ala Asp Glu Tyr
        355                 360                 365

Ala Cys Ser Leu Gln Glu Ala Val Asp Leu Leu Val Glu His Val Asp
    370                 375                 380

Ala Ala Glu Ala Arg Phe Leu Glu Leu Arg Ala Asp Val Leu Gly Ala
385                 390                 395                 400

Pro Leu Gly Ala Arg Pro Gly Val Arg Glu Tyr Leu Asp Ala Leu Glu
                405                 410                 415

Leu Val Leu Pro Gly Asn Ile Val Trp Ser Arg Thr Ser Arg Arg Tyr
            420                 425                 430

His Gly Thr Gly Cys Pro Trp Thr Gly Thr Ala Arg Thr Ala Met Ala
        435                 440                 445

Leu His Pro Asp Arg Thr Val Phe Thr Pro Trp
    450                 455

<210>  21
<211>  1077
<212>  DNA
<213>  Streptomyces lactacystinaeus

<400>  21
   atgacaaccg tgcgccgcac acttggcgtt ccgccatttt tctgcccttt gcccgacgcc      60
   gtgtacgaca agccggagga aatagatgcc ggcgccgtcg cctggatgtt ccggcaaggg     120
   ttccgcaagg agggtgactg gctgccgcgc atgaatgcgg gttcgtacac cgcacactgc     180
   atgccgtacg tcctcgaccg gtgcgccgag atcatcacct atgtcatgta ctggggcggc     240
   gcctgggacg acgacctcga cgtgatcccg gggctggacc agcccgcacc gctgacggtc     300
   cacgtcagcc gtctcaagca catcctcgcc tcgcccaagt ccgtcgtgcc gcccggagac 360
   gcgtacctct cctccttcca ggacttctgg aaccacatct ccaccgtgtc gaccccgggc 420
   cagctcgagc acctgcgcca gggtttctgc agctacttcg acggcgtgct gtggaaatgt 480
   gcgaaccaga gcctcggtga actccccgaa ctgggcgact acctgatcat gcgtggcaac 540
   gatgtggccg gcccctggct gaccgcactc actccgatcg gcgccggcta cgaactgacc 600
   gcagccgaac gcctcgatcc ccgggtgcgc gccgcctccg aagccgtatc cgtcatcgtc 660
   ggtctcgaca acgacctgat ctccattcac cgggagaatc tccagggctt gttcgaccag 720
   aacgccatca gcatcatggt gcgcgaccgg caatgcacgc ccgaggaagc ggtcgccgag 780
   gtccgcgcgc tgcgcgaccg aatcatgtcc ctttatctga atctgcgtga ccaggtctcc 840
   gcgaccgccc gcccggaact gcgacgatac gtccacgaga tcggttactg gattcgcggg 900
   cacatcatct gggcggactt caccacccgt tactgcgcgc ctgtcgaccc ggagaccggc 960
   gaagaacagg acttcggtgg ctacggcctg ctctgggccg acgagccggc tgacgccaac 1020
   cctgcgcccc tgccgattcc acagatcgcc tggtggtggg accagttgga gaagtga 1077

<210> 22
<211> 358
<212> PRT
<213> Streptomyces lactacystinaeus

<400> 22

Met Thr Thr Val Arg Arg Thr Leu Gly Val Pro Pro Phe Phe Cys Pro
1               5                   10                  15

Leu Pro Asp Ala Val Tyr Asp Lys Pro Glu Glu Ile Asp Ala Gly Ala
            20                  25                  30

Val Ala Trp Met Phe Arg Gln Gly Phe Arg Lys Glu Gly Asp Trp Leu
        35                  40                  45

Pro Arg Met Asn Ala Gly Ser Tyr Thr Ala His Cys Met Pro Tyr Val
    50                  55                  60

Leu Asp Arg Cys Ala Glu Ile Ile Thr Tyr Val Met Tyr Trp Gly Gly
65                  70                  75                  80

Ala Trp Asp Asp Asp Leu Asp Val Ile Pro Gly Leu Asp Gln Pro Ala
                85                  90                  95

Pro Leu Thr Val His Val Ser Arg Leu Lys His Ile Leu Ala Ser Pro
            100                 105                 110

Lys Ser Val Val Pro Pro Gly Asp Ala Tyr Leu Ser Ser Phe Gln Asp
        115                 120                 125

Phe Trp Asn His Ile Ser Thr Val Ser Thr Pro Gly Gln Leu Glu His
    130                 135                 140

Leu Arg Gln Gly Phe Cys Ser Tyr Phe Asp Gly Val Leu Trp Lys Cys
145                 150                 155                 160

Ala Asn Gln Ser Leu Gly Glu Leu Pro Glu Leu Gly Asp Tyr Leu Ile
                165                 170                 175

Met Arg Gly Asn Asp Val Ala Gly Pro Trp Leu Thr Ala Leu Thr Pro
            180                 185                 190

Ile Gly Ala Gly Tyr Glu Leu Thr Ala Ala Glu Arg Leu Asp Pro Arg
        195                 200                 205

Val Arg Ala Ala Ser Glu Ala Val Ser Val Ile Val Gly Leu Asp Asn
    210                 215                 220

Asp Leu Ile Ser Ile His Arg Glu Asn Leu Gln Gly Leu Phe Asp Gln
225                 230                 235                 240

Asn Ala Ile Ser Ile Met Val Arg Asp Arg Gln Cys Thr Pro Glu Glu
                245                 250                 255

Ala Val Ala Glu Val Arg Ala Leu Arg Asp Arg Ile Met Ser Leu Tyr
            260                 265                 270

Leu Asn Leu Arg Asp Gln Val Ser Ala Thr Ala Arg Pro Glu Leu Arg
        275                 280                 285

Arg Tyr Val His Glu Ile Gly Tyr Trp Ile Arg Gly His Ile Ile Trp
    290                 295                 300

Ala Asp Phe Thr Thr Arg Tyr Cys Ala Pro Val Asp Pro Glu Thr Gly
305                 310                 315                 320

Glu Glu Gln Asp Phe Gly Gly Tyr Gly Leu Leu Trp Ala Asp Glu Pro
                325                 330                 335

Ala Asp Ala Asn Pro Ala Pro Leu Pro Ile Pro Gln Ile Ala Trp Trp
            340                 345                 350

Trp Asp Gln Leu Glu Lys
        355

<210> 23
<211> 975
<212> DNA
<213> Rubrobacter xylanophilus DSM 9941

<400> 23
ttgtctactg agaacgggca tcgggacggt ctggggcccc tgcgctgtcc cttccccgcg 60

gcgataaacc cgcacgccga cgaggtgcac cgggagaccg tggagtgggc cgagggtttc 120

gggctgctcg ggcccggcga cgggcaccgg atgatgcggg acaccgggat cgggcggctc 180

gccgggcgct tccaccccgg ggccgggcgg gaggagctcc ggctgatctc cgactggtgc 240

gcctggatgt tcctgcgcga cgacctcgcc gacgcgccgg cgtacttccg gcacccggag 300

cggctcgcgg ccctggacgc ggcctttctg gacatcctct ccggcagggc ctccggggag 360

ggctgcgggt ccttcgggcg ggcgctgcgg gatctccggg agcggctgct gcccaaggtt 420

cccgcgccgc tctggctgcg gcgctttctc aggtccgtgg aggagcactt cgagtccacg 480

ctgtgggagg ccaccaaccg ggcccgcggg gtcgtcccgg acctcgagac ctacctcagg 540

atgcgcccca taaccggcgg gatgcacgtg gacacggact tcatagagat atcctccggc 600

gtctacctgc cgcccgaggt gcgccggcac ccggccgtaa gcgccctcac cggggcctcc 660

aacaacgtgg tctgctgggc aaacgacatc atctcgctcg ccaaggagcg ctcgcgcggc 720

gacgtgcaca acctggtcct ggtcctccgc gcgagccggc gtctcaccac ccgggaggcg 780

gtggcggagg ccgcccggat gtacgaggcg gaggtgcgga ggttcgtccg gctggagcgg 840

gagctgcccc ccttcgggcc ggccatcgac gccaacctcc ggcgctacgt ctcggtgctc 900

aagagcagga tgcggggcaa tctggactgg acctacgagt cggcgcgcta ccgggccggc 960

gcggcctccc gctag 975

<210> 24
<211> 324
<212> PRT
<213> Rubrobacter xylanophilus DSM 9941

<400> 24

Met Ser Thr Glu Asn Gly His Arg Asp Gly Leu Gly Pro Leu Arg Cys
1               5                   10                  15

Pro Phe Pro Ala Ala Ile Asn Pro His Ala Asp Glu Val His Arg Glu
            20                  25                  30

Thr Val Glu Trp Ala Glu Gly Phe Gly Leu Leu Gly Pro Gly Asp Gly
        35                  40                  45

His Arg Met Met Arg Asp Thr Gly Ile Gly Arg Leu Ala Gly Arg Phe
    50                  55                  60

His Pro Gly Ala Gly Arg Glu Glu Leu Arg Leu Ile Ser Asp Trp Cys
65                  70                  75                  80

Ala Trp Met Phe Leu Arg Asp Asp Leu Ala Asp Ala Pro Ala Tyr Phe
                85                  90                  95

Arg His Pro Glu Arg Leu Ala Ala Leu Asp Ala Ala Phe Leu Asp Ile
            100                 105                 110

Leu Ser Gly Arg Ala Ser Gly Glu Gly Cys Gly Ser Phe Gly Arg Ala
        115                 120                 125

Leu Arg Asp Leu Arg Glu Arg Leu Leu Pro Lys Val Pro Ala Pro Leu
    130                 135                 140

Trp Leu Arg Arg Phe Leu Arg Ser Val Glu Glu His Phe Glu Ser Thr
145                 150                 155                 160

Leu Trp Glu Ala Thr Asn Arg Ala Arg Gly Val Val Pro Asp Leu Glu
                165                 170                 175

Thr Tyr Leu Arg Met Arg Pro Ile Thr Gly Gly Met His Val Asp Thr
            180                 185                 190

Asp Phe Ile Glu Ile Ser Ser Gly Val Tyr Leu Pro Pro Glu Val Arg
        195                 200                 205

Arg His Pro Ala Val Ser Ala Leu Thr Gly Ala Ser Asn Asn Val Val
    210                 215                 220

Cys Trp Ala Asn Asp Ile Ile Ser Leu Ala Lys Glu Arg Ser Arg Gly
225                 230                 235                 240

Asp Val His Asn Leu Val Leu Val Leu Arg Ala Ser Arg Arg Leu Thr
                245                 250                 255

Thr Arg Glu Ala Val Ala Glu Ala Ala Arg Met Tyr Glu Ala Glu Val
            260                 265                 270

Arg Arg Phe Val Arg Leu Glu Arg Glu Leu Pro Pro Phe Gly Pro Ala
        275                 280                 285

Ile Asp Ala Asn Leu Arg Arg Tyr Val Ser Val Leu Lys Ser Arg Met
    290                 295                 300

Arg Gly Asn Leu Asp Trp Thr Tyr Glu Ser Ala Arg Tyr Arg Ala Gly

305                 310                 315                 320

Ala Ala Ser Arg

<210>  25
<211>  1017
<212>  DNA
<213>  Herpetosiphon aurantiacus DSM 785

<400>  25
atgccaaccc gtactacatt gcatcactta gccacgctta tgcctcgtga tcttccggtg      60

ctctattgcc catttccggc ggcgattcat cccgatgcgg ttgcgattca agcctcaacc     120

caagcatggg ctgatagctt tggcttgctc gatgatttgg agacgcggct ctcttttaat     180

caacgtggct atggtacctt gatggcgcga gcttatccta atgctttaac tgagcgcatg     240

ccaaccattg ccgattggag caccagcctc tttctcttag atgatcaatt ggatcgcttt     300

ggccgtgagc atgatgccgg ggccatggca accttgttgc aacgcttgat gaaggtactc     360

aaaggttcgc ctgccaagcg ccacgatttg ccaattgtgc aagcaatggg cgatgtaacc     420

agccgacttg tgcaaggttc gcccgaagcc tggcaacact acttttttga ttcattgcgc     480

aaatcgtttg cagcagtgca atgggaagct aacaaccgtg ctcagggcta tattccaact     540

caaaaaacct atgcaatgca acgtttatat accagtggta cttatttatg cttggctttg     600

attgaatttg gcaataacga tttcctgcat ccccaagtgc gtttgcatcc catggtgcgg     660

cgtttgtgtg aatatgttaa tcgggtggtg ttctactcaa atgatattat ttcgttgcgt     720

aaagaattat tagctggcga ttggcaaaat ttggtgtatc tcgtgcatac gcaacatcaa     780

ttaccaatcg gcgaagcatt aaattatgtc gcccgtttgc ataatgatac aatcaaccaa     840

ttcactcgcg aacgcattat gctcgataaa ttggctcctg agatttgttc accaagcttg     900

tatcgctttg tcgatggttt agctacctgg atgcgagcaa accacgattg gtctaccatg     960

acctctcgtt atcagacatc taccgaggaa tcccatggat ctcagtattg tgcgtga       1017

<210>  26
<211>  338
<212>  PRT
<213>  Herpetosiphon aurantiacus DSM 785

<400>  26

Met Pro Thr Arg Thr Thr Leu His His Leu Ala Thr Leu Met Pro Arg
1               5                   10                  15

Asp Leu Pro Val Leu Tyr Cys Pro Phe Pro Ala Ala Ile His Pro Asp
            20                  25                  30

Ala Val Ala Ile Gln Ala Ser Thr Gln Ala Trp Ala Asp Ser Phe Gly
        35                  40                  45

Leu Leu Asp Asp Leu Glu Thr Arg Leu Ser Phe Asn Gln Arg Gly Tyr
    50                  55                  60

Gly Thr Leu Met Ala Arg Ala Tyr Pro Asn Ala Leu Thr Glu Arg Met
65                  70                  75                  80

Pro Thr Ile Ala Asp Trp Ser Thr Ser Leu Phe Leu Leu Asp Asp Gln
                85                  90                  95

Leu Asp Arg Phe Gly Arg Glu His Asp Ala Gly Ala Met Ala Thr Leu
            100                 105                 110

Leu Gln Arg Leu Met Lys Val Leu Lys Gly Ser Pro Ala Lys Arg His
        115                 120                 125

Asp Leu Pro Ile Val Gln Ala Met Gly Asp Val Thr Ser Arg Leu Val
    130                 135                 140

Gln Gly Ser Pro Glu Ala Trp Gln His Tyr Phe Phe Asp Ser Leu Arg
145                 150                 155                 160

Lys Ser Phe Ala Ala Val Gln Trp Glu Ala Asn Asn Arg Ala Gln Gly
                165                 170                 175

Tyr Ile Pro Thr Gln Lys Thr Tyr Ala Met Gln Arg Leu Tyr Thr Ser
            180                 185                 190

Gly Thr Tyr Leu Cys Leu Ala Leu Ile Glu Phe Gly Asn Asn Asp Phe
        195                 200                 205

Leu His Pro Gln Val Arg Leu His Pro Met Val Arg Arg Leu Cys Glu
    210                 215                 220

Tyr Val Asn Arg Val Val Phe Tyr Ser Asn Asp Ile Ile Ser Leu Arg
225                 230                 235                 240

Lys Glu Leu Leu Ala Gly Asp Trp Gln Asn Leu Val Tyr Leu Val His
                245                 250                 255

Thr Gln His Gln Leu Pro Ile Gly Glu Ala Leu Asn Tyr Val Ala Arg
            260                 265                 270

Leu His Asn Asp Thr Ile Asn Gln Phe Thr Arg Glu Arg Ile Met Leu
        275                 280                 285

Asp Lys Leu Ala Pro Glu Ile Cys Ser Pro Ser Leu Tyr Arg Phe Val
    290                 295                 300

Asp Gly Leu Ala Thr Trp Met Arg Ala Asn His Asp Trp Ser Thr Met
305                 310                 315                 320

Thr Ser Arg Tyr Gln Thr Ser Thr Glu Glu Ser His Gly Ser Gln Tyr
                325                 330                 335

Cys Ala

<210>  27
<211>  990
<212>  DNA
<213>  Herpetosiphon aurantiacus DSM 785

<400>  27
atgccaaata cagcacacct acaacaatta gcgagaacgg ttccagcaac cattcccaag      60

ttggaatcac cgtttcctgc gcaaatccat ccattggcag cggaggttca acagcagacg     120

accaaatggg tgcttgaaca aggtttattg agcgaagcgc ggcgtaaagc ttttcttgat     180

tacaactata ccttgttgat tgcccgttgt tatccgtatg cgagtgttga gcggctgcaa     240

attatcagcg attggaatgc ttggctgttt gaacttgatg atcaatgtga cgaagcgcga     300

attggccatg accctcaagc gttggctcgc ttgcatcagc ggcttggggc agttattcgc     360

ggagccacgg ttgctgccga cgaaccacta ttagttacag ccctcgctga tattatgggt     420

cggatgcggg cgctagcacc actggcatgg tttgaggttt ttacccaaca ggttgacgat     480

tattttattg gcaatcaatg ggaagcccac aatcggctgt actcgattat tcccagcgaa     540

attgaatact tgcactatcg taaatttact agtgctgttg cgatctatat tgcgtttatt     600

gaatttatga atgcaagtta tttaaccgct gatgaacgtc aatcaatcga cttacgcaat     660

ctacaaaatt ggacgaacct tgtactgagt ttatgtaatg atcttgtttc agttaaaaaa     720

gaattacgtc aaggcgattg gcataattat ggagcaattg ttgcattaaa acgaggtatg     780

agtattcctg aagccttaga aatagtcgcc aatgagcata atcattgtat tcaacaattt     840

ataacaacta ccactcaact gaaaaattgt agcgtggcac ttcaaaaagt attaaacggt     900

atgatgacat tgatgcgcgg aaattatgat tggtcggata ttactagccg ttatgctact     960

aaacttaccg aattgtcgta tgctgcatga                                      990

<210>  28
<211>  329
<212>  PRT
<213>  Herpetosiphon aurantiacus DSM 785

<400> 28

Met Pro Asn Thr Ala His Leu Gln Gln Leu Ala Arg Thr Val Pro Ala
1               5                   10                  15

Thr Ile Pro Lys Leu Glu Ser Pro Phe Pro Ala Gln Ile His Pro Leu
            20                  25                  30

Ala Ala Glu Val Gln Gln Gln Thr Thr Lys Trp Val Leu Glu Gln Gly
        35                  40                  45

Leu Leu Ser Glu Ala Arg Arg Lys Ala Phe Leu Asp Tyr Asn Tyr Thr
    50                  55                  60

Leu Leu Ile Ala Arg Cys Tyr Pro Tyr Ala Ser Val Glu Arg Leu Gln
65                  70                  75                  80

Ile Ile Ser Asp Trp Asn Ala Trp Leu Phe Glu Leu Asp Asp Gln Cys
                85                  90                  95

Asp Glu Ala Arg Ile Gly His Asp Pro Gln Ala Leu Ala Arg Leu His
            100                 105                 110

Gln Arg Leu Gly Ala Val Ile Arg Gly Ala Thr Val Ala Ala Asp Glu
        115                 120                 125

Pro Leu Leu Val Thr Ala Leu Ala Asp Ile Met Gly Arg Met Arg Ala
    130                 135                 140

Leu Ala Pro Leu Ala Trp Phe Glu Val Phe Thr Gln Gln Val Asp Asp
145                 150                 155                 160

Tyr Phe Ile Gly Asn Gln Trp Glu Ala His Asn Arg Leu Tyr Ser Ile
                165                 170                 175

Ile Pro Ser Glu Ile Glu Tyr Leu His Tyr Arg Lys Phe Thr Ser Ala
            180                 185                 190

Val Ala Ile Tyr Ile Ala Phe Ile Glu Phe Met Asn Ala Ser Tyr Leu
        195                 200                 205

Thr Ala Asp Glu Arg Gln Ser Ile Asp Leu Arg Asn Leu Gln Asn Trp
    210                 215                 220

Thr Asn Leu Val Leu Ser Leu Cys Asn Asp Leu Val Ser Val Lys Lys
225                 230                 235                 240

Glu Leu Arg Gln Gly Asp Trp His Asn Tyr Gly Ala Ile Val Ala Leu

245                 250                 255

Lys Arg Gly Met Ser Ile Pro Glu Ala Leu Glu Ile Val Ala Asn Glu
            260                 265                 270

His Asn His Cys Ile Gln Gln Phe Ile Thr Thr Thr Thr Gln Leu Lys
        275                 280                 285

Asn Cys Ser Val Ala Leu Gln Lys Val Leu Asn Gly Met Met Thr Leu
    290                 295                 300

Met Arg Gly Asn Tyr Asp Trp Ser Asp Ile Thr Ser Arg Tyr Ala Thr
305                 310                 315                 320

Lys Leu Thr Glu Leu Ser Tyr Ala Ala
                325

<210>  29
<211>  981
<212>  DNA
<213>  Roseiflexus castenholzii DSM 13941

<400>  29
gtggatcagg actatcgcgc acgacttgtc tacccatttt ccggagcgat aagcccgcat      60

gccgatatag tcgatcaggc aacgctcgcc tgggcggcga tgtttggctt gttgacggac     120

tcattgcgcc acaaaagtcg tcgcctccag tatggcttgc tggctgcacg cgcctatcca     180

cgagcggacc gtgagatgtt gcagattgcc gccgattgga ttgcatggct gttcttcatg     240

gatgaccaat gcgacgaaac tggcattggg cgtgatctcc aacgcatgat tgcgctgcac     300

gaacgatttc ttgcaattct tgacggcgca acgccggagg cgcatgattg cgcgctgacc     360

tatgcgctcg ccgatcttcg tcgtcgactg gcactgcgcg ctcctgataa ctggctgcgc     420

cgattcagcg agcatgtgcg tctctacttt actgccaatc gctgggaaac ggtcaaccgg     480

cagcgcggtg caacgccgaa tgtggcgaca tattgcgccg cgcgcctgtt ttcgggcgcc     540

gtctatgcat gcttcgatct gatagaactg gcagagcaga tcgaactgcc gttctatgcc     600

cgtcaccaca gcattgtgca gcaactggag caggctgcga acaatatcat ttgctggtgc     660

aatgatgtgc tgtcgtatcc caaggaaatg cagcacggcg accggcacaa tctggtgctg     720

gtcattcagg gcgagcatca atgctcattg cccgaagcca ttgaccgcgc gctcgacctt     780

catgcgcgtg aagtcgctac gtttgtgcgg aaacgcacct gtgtcccata tttcgatgca     840

gcggtcaata cagcgcttga aaaatatgtc accggattgc agttctggat ctgcgccaat     900

cgcgattggt cgctgacggc aacgcgctac gcgccgacgc acaaatcgca ggaaatggtg     960

atggcagtcg cgcaacagta a                                               981

<210>  30
<211>  326
<212>  PRT
<213>  Roseiflexus castenholzii DSM 13941

<400>  30

Met Asp Gln Asp Tyr Arg Ala Arg Leu Val Tyr Pro Phe Ser Gly Ala
1               5                   10                  15

Ile Ser Pro His Ala Asp Ile Val Asp Gln Ala Thr Leu Ala Trp Ala
            20                  25                  30

Ala Met Phe Gly Leu Leu Thr Asp Ser Leu Arg His Lys Ser Arg Arg
        35                  40                  45

Leu Gln Tyr Gly Leu Leu Ala Ala Arg Ala Tyr Pro Arg Ala Asp Arg
    50                  55                  60

Glu Met Leu Gln Ile Ala Ala Asp Trp Ile Ala Trp Leu Phe Phe Met
65                  70                  75                  80

Asp Asp Gln Cys Asp Glu Thr Gly Ile Gly Arg Asp Leu Gln Arg Met
                85                  90                  95

Ile Ala Leu His Glu Arg Phe Leu Ala Ile Leu Asp Gly Ala Thr Pro
            100                 105                 110

Glu Ala His Asp Cys Ala Leu Thr Tyr Ala Leu Ala Asp Leu Arg Arg
        115                 120                 125

Arg Leu Ala Leu Arg Ala Pro Asp Asn Trp Leu Arg Arg Phe Ser Glu
    130                 135                 140

His Val Arg Leu Tyr Phe Thr Ala Asn Arg Trp Glu Thr Val Asn Arg
145                 150                 155                 160

Gln Arg Gly Ala Thr Pro Asn Val Ala Thr Tyr Cys Ala Ala Arg Leu
                165                 170                 175

Phe Ser Gly Ala Val Tyr Ala Cys Phe Asp Leu Ile Glu Leu Ala Glu
            180                 185                 190

Gln Ile Glu Leu Pro Phe Tyr Ala Arg His His Ser Ile Val Gln Gln
        195                 200                 205

Leu Glu Gln Ala Ala Asn Asn Ile Ile Cys Trp Cys Asn Asp Val Leu
    210                 215                 220

Ser Tyr Pro Lys Glu Met Gln His Gly Asp Arg His Asn Leu Val Leu
225                 230                 235                 240

Val Ile Gln Gly Glu His Gln Cys Ser Leu Pro Glu Ala Ile Asp Arg
                245                 250                 255

Ala Leu Asp Leu His Ala Arg Glu Val Ala Thr Phe Val Arg Lys Arg
            260                 265                 270

Thr Cys Val Pro Tyr Phe Asp Ala Ala Val Asn Thr Ala Leu Glu Lys
        275                 280                 285

Tyr Val Thr Gly Leu Gln Phe Trp Ile Cys Ala Asn Arg Asp Trp Ser
    290                 295                 300

Leu Thr Ala Thr Arg Tyr Ala Pro Thr His Lys Ser Gln Glu Met Val
305                 310                 315                 320

Met Ala Val Ala Gln Gln
                325

<210>  31
<211>  981
<212>  DNA
<213>  Roseiflexus sp. RS-1

<400>  31
gtggatcggg tcgatggcgc acagatcgtc tacccgttta ccggtgcgat aagcccctat      60

gctggcgatg tcgatcaggc aacacttatc tgggcagagg cgtctggact tcttaccgat     120

gggttgcgcc agaaaagtca gcgtctccag tatggagtac tggctgcgcg cgcctatcca     180

caggcagatc gtgatacgct gcaaattgct gccgattgga tagcctggct gtttttcatg     240

gacgatcagt gtgatgaggc tggcattggt cgcgatccgc agcgcctggc tgcgctgcac     300

gaacgattcc tcgcagtgct cgaaggagac ccacctgata gtggcgactg gaatctgacc     360

cgcgctctcg ccgatattcg ccgtcggctg gccgcgcgcg caaccgatga ttggttgcgc     420

cgattcggag agcacgtgcg tctctacttt acggcgaatc gctgggaagc ggcaaaccga     480

tgccagagca tcgttccaaa cgtggcgacc tattgtgctg cgcgcctgtt ttcaggcgca     540

gtctatgcct gctttgacct gatagaactg gcagcaggga tcgatcttcc cttttatgca     600

cgttatcatg ctgccgttca acaactcgag cggacggcaa acaatatcat ttgctggtgc     660

aatgatatgt tgtcgtaccc caaagagatg cagcacggtg atgtgcacaa tctggtactg     720

gctatccggc aggaatatca atgttcgttg agtgaggcgc tgcagcaggc gcttctgctc     780

cacgaccggg agacggacac gtttatgaaa acacagaagc aactgccgcg cttcaatccc     840

gccgttgata tggcgcttga gcgctatatt gatggtttac agtactggat atgcgccaac     900

cgtgactggt cattgacagc aatgcgctat gctttgtccg gaaccgaacc cgtacttcgg     960

acgaggtttg cacattcgtg a                                               981

<210>  32
<211>  326
<212>  PRT
<213>  Roseiflexus sp. RS-1

<400>  32

Met Asp Arg Val Asp Gly Ala Gln Ile Val Tyr Pro Phe Thr Gly Ala
1               5                   10                  15

Ile Ser Pro Tyr Ala Gly Asp Val Asp Gln Ala Thr Leu Ile Trp Ala
            20                  25                  30

Glu Ala Ser Gly Leu Leu Thr Asp Gly Leu Arg Gln Lys Ser Gln Arg
        35                  40                  45

Leu Gln Tyr Gly Val Leu Ala Ala Arg Ala Tyr Pro Gln Ala Asp Arg
    50                  55                  60

Asp Thr Leu Gln Ile Ala Ala Asp Trp Ile Ala Trp Leu Phe Phe Met
65                  70                  75                  80

Asp Asp Gln Cys Asp Glu Ala Gly Ile Gly Arg Asp Pro Gln Arg Leu
                85                  90                  95

Ala Ala Leu His Glu Arg Phe Leu Ala Val Leu Glu Gly Asp Pro Pro
            100                 105                 110

Asp Ser Gly Asp Trp Asn Leu Thr Arg Ala Leu Ala Asp Ile Arg Arg
        115                 120                 125

Arg Leu Ala Ala Arg Ala Thr Asp Asp Trp Leu Arg Arg Phe Gly Glu
    130                 135                 140

His Val Arg Leu Tyr Phe Thr Ala Asn Arg Trp Glu Ala Ala Asn Arg
145                 150                 155                 160

Cys Gln Ser Ile Val Pro Asn Val Ala Thr Tyr Cys Ala Ala Arg Leu
                165                 170                 175

Phe Ser Gly Ala Val Tyr Ala Cys Phe Asp Leu Ile Glu Leu Ala Ala
            180                 185                 190

Gly Ile Asp Leu Pro Phe Tyr Ala Arg Tyr His Ala Ala Val Gln Gln

195                 200                 205

Leu Glu Arg Thr Ala Asn Asn Ile Ile Cys Trp Cys Asn Asp Met Leu
    210                 215                 220

Ser Tyr Pro Lys Glu Met Gln His Gly Asp Val His Asn Leu Val Leu
225                 230                 235                 240

Ala Ile Arg Gln Glu Tyr Gln Cys Ser Leu Ser Glu Ala Leu Gln Gln
                245                 250                 255

Ala Leu Leu Leu His Asp Arg Glu Thr Asp Thr Phe Met Lys Thr Gln
            260                 265                 270

Lys Gln Leu Pro Arg Phe Asn Pro Ala Val Asp Met Ala Leu Glu Arg
        275                 280                 285

Tyr Ile Asp Gly Leu Gln Tyr Trp Ile Cys Ala Asn Arg Asp Trp Ser
    290                 295                 300

Leu Thr Ala Met Arg Tyr Ala Leu Ser Gly Thr Glu Pro Val Leu Arg
305                 310                 315                 320

Thr Arg Phe Ala His Ser
                325

<210>  33
<211>  948
<212>  DNA
<213>  Sorangium cellulosum \201eSo ce 56\201f

<400>  33
gtgcacagag ctctactttg tccttttccc gccacgacgc cccacccgca ggcggcacag      60

ctggcgaacg attgcctcga atggactcgc aagtgcggcc tgctccccga tgaatcgcca     120

cgaacgctcg acaaggtgcg cagctactcg gcgctcgccg cgcactgtta ccccgatgcg     180

catttcgagc ggcttcgggc gatctgcgac tactacagct ggctcttctt cttcgatgac     240

gtgtgcgaga atacgtcact gaacggcgcc gaaccgaagg ttgtctcgag cctgctgttc     300

gatgtatacg gggtgctgcg agggcccacg gctgccgttg ggcatgcgcc atttgcccaa     360

gcattggcgg acatctggcg acgcatcggc gatggttgtc ctgggttctg gcggcggcgc     420

ctcatccgcc acgtcgaaaa ctacatcgat ggctgtgtct gggaagcgca aaaccgccag     480

ctcgaccgcg tacccagtcg cgcggtgttc gagggtatgc gcatgcacac gtccaccatg     540

tacgagtttt gggacttcat cgagtatgcc ggcgacctgt tcttgccgga tgaggtcgta     600

gaacatccct tggtggcgga ggtgcgtcgc gccggcaacg ccatcgcgtc ttttgccaat     660

gacatttact cgctgcgcaa agagacgagc aaccgcgacg tgcacaattt ggttgtcgtg 720

ctcatgcacg aagagcgcat cgagctcgaa gcagcgtacg cgcgcgccgc tggtatccac 780

gatgctcaag tcgagcactt cctggatctg gtgaagcacc tgcctacgtt ttctgcgacc 840

atcgatcgga acctggcgcg ttacgtggag ggcatacgca tctggatccg cgccaaccac 900

gattggtcga ttgtcacgcc ccgttacaat gagcccgatg cgcgatga 948

<210> 34
<211> 315
<212> PRT
<213> Sorangium cellulosum \201eSo ce 56\201f

<400> 34

Met His Arg Ala Leu Leu Cys Pro Phe Pro Ala Thr Thr Pro His Pro
1               5                   10                  15

Gln Ala Ala Gln Leu Ala Asn Asp Cys Leu Glu Trp Thr Arg Lys Cys
            20                  25                  30

Gly Leu Leu Pro Asp Glu Ser Pro Arg Thr Leu Asp Lys Val Arg Ser
        35                  40                  45

Tyr Ser Ala Leu Ala Ala His Cys Tyr Pro Asp Ala His Phe Glu Arg
    50                  55                  60

Leu Arg Ala Ile Cys Asp Tyr Tyr Ser Trp Leu Phe Phe Phe Asp Asp
65                  70                  75                  80

Val Cys Glu Asn Thr Ser Leu Asn Gly Ala Glu Pro Lys Val Val Ser
                85                  90                  95

Ser Leu Leu Phe Asp Val Tyr Gly Val Leu Arg Gly Pro Thr Ala Ala
            100                 105                 110

Val Gly His Ala Pro Phe Ala Gln Ala Leu Ala Asp Ile Trp Arg Arg
        115                 120                 125

Ile Gly Asp Gly Cys Pro Gly Phe Trp Arg Arg Arg Leu Ile Arg His
    130                 135                 140

Val Glu Asn Tyr Ile Asp Gly Cys Val Trp Glu Ala Gln Asn Arg Gln
145                 150                 155                 160

Leu Asp Arg Val Pro Ser Arg Ala Val Phe Glu Gly Met Arg Met His
                165                 170                 175

Thr Ser Thr Met Tyr Glu Phe Trp Asp Phe Ile Glu Tyr Ala Gly Asp

180                 185                 190

Leu Phe Leu Pro Asp Glu Val Val Glu His Pro Leu Val Ala Glu Val
        195                 200                 205

Arg Arg Ala Gly Asn Ala Ile Ala Ser Phe Ala Asn Asp Ile Tyr Ser
    210                 215                 220

Leu Arg Lys Glu Thr Ser Asn Arg Asp Val His Asn Leu Val Val Val
225                 230                 235                 240

Leu Met His Glu Glu Arg Ile Glu Leu Glu Ala Ala Tyr Ala Arg Ala
                245                 250                 255

Ala Gly Ile His Asp Ala Gln Val Glu His Phe Leu Asp Leu Val Lys
            260                 265                 270

His Leu Pro Thr Phe Ser Ala Thr Ile Asp Arg Asn Leu Ala Arg Tyr
        275                 280                 285

Val Glu Gly Ile Arg Ile Trp Ile Arg Ala Asn His Asp Trp Ser Ile
    290                 295                 300

Val Thr Pro Arg Tyr Asn Glu Pro Asp Ala Arg
305                 310                 315

<210>  35
<211>  990
<212>  DNA
<213>  Sorangium cellulosum \201eSo ce 56\201f

<400>  35
atgtcatctg atcgaactag cgttgtcgtc agcaagcgcg acgctggtgg attcgaatat      60

ccgtttgctg cgtcgtgtca ccccgggaga gaggtcacgg agcaaaggac cctggcgtgg     120

gttcgccggt tgcgtctggt gccagacggt cggtcgcttt cacgtctcaa ggcgacgaac     180

ttctcccacc tggcggcatg gctcttaccg tccgcgagca cgcagacgct ccagctggca     240

tcggacttca cggccgtgct gttcctcctg gatgatgcgt acgacgaggg gcagctcagc     300

acggacccgg aatccgtcga gtggctcaac gagaagtacc tgggtgagct cttcgggtac     360

accgaagcgg acatgagcga ccccctgacg cgcgggatgc tcgatgtacg tgagcgcatc     420

cgacgcagcc atccgcactt cttcctgaat cgctggctct cgcactttca gtactactac     480

gaggccaacc tgtgggaggc gaataaccga aagcagatga gggtgccgca tcttgaagaa     540

tatttgatga tgcggcgcta cagcggcgct gtctatacct actgtgacct gctcgagctg     600

ctgctcgaac ggccgctgcc gctcgaggtc gtccagcacc cgttgattca gacggttcga     660

gacatctgta atgacatcct ctgctggacg aacgactact tctcgctcgg gaaggagctg 720

accaacggcg agacgcacaa cctgatcgtc gttctccgga acgaatgcgt gagcacgctg 780

gaagaggcga tcgaccggct caaggacatg cacgaccgcc gggtcgcgga gtaccaaggc 840

gtgaaggaga aggtgctcgc gctctgggcg gacgacgaga tccgcctcta cctggacgcg 900

gtcgaggcca tgatcgccgg gaatcagcgc tgggcgctcg aggccggtcg ctattctgga 960

ctagaaagtt tgatcgtgcg cgctggatga 990

<210> 36
<211> 329
<212> PRT
<213> Sorangium cellulosum \201eSo ce 56\201f

<400> 36

Met Ser Ser Asp Arg Thr Ser Val Val Val Ser Lys Arg Asp Ala Gly
1               5                   10                  15

Gly Phe Glu Tyr Pro Phe Ala Ala Ser Cys His Pro Gly Arg Glu Val
            20                  25                  30

Thr Glu Gln Arg Thr Leu Ala Trp Val Arg Arg Leu Arg Leu Val Pro
        35                  40                  45

Asp Gly Arg Ser Leu Ser Arg Leu Lys Ala Thr Asn Phe Ser His Leu
    50                  55                  60

Ala Ala Trp Leu Leu Pro Ser Ala Ser Thr Gln Thr Leu Gln Leu Ala
65                  70                  75                  80

Ser Asp Phe Thr Ala Val Leu Phe Leu Leu Asp Asp Ala Tyr Asp Glu
                85                  90                  95

Gly Gln Leu Ser Thr Asp Pro Glu Ser Val Glu Trp Leu Asn Glu Lys
            100                 105                 110

Tyr Leu Gly Glu Leu Phe Gly Tyr Thr Glu Ala Asp Met Ser Asp Pro
        115                 120                 125

Leu Thr Arg Gly Met Leu Asp Val Arg Glu Arg Ile Arg Arg Ser His
    130                 135                 140

Pro His Phe Phe Leu Asn Arg Trp Leu Ser His Phe Gln Tyr Tyr Tyr
145                 150                 155                 160

Glu Ala Asn Leu Trp Glu Ala Asn Asn Arg Lys Gln Met Arg Val Pro
                165                 170                 175

His Leu Glu Glu Tyr Leu Met Met Arg Arg Tyr Ser Gly Ala Val Tyr
            180                 185                 190

Thr Tyr Cys Asp Leu Leu Glu Leu Leu Leu Glu Arg Pro Leu Pro Leu
        195                 200                 205

Glu Val Val Gln His Pro Leu Ile Gln Thr Val Arg Asp Ile Cys Asn
    210                 215                 220

Asp Ile Leu Cys Trp Thr Asn Asp Tyr Phe Ser Leu Gly Lys Glu Leu
225                 230                 235                 240

Thr Asn Gly Glu Thr His Asn Leu Ile Val Val Leu Arg Asn Glu Cys
                245                 250                 255

Val Ser Thr Leu Glu Glu Ala Ile Asp Arg Leu Lys Asp Met His Asp
            260                 265                 270

Arg Arg Val Ala Glu Tyr Gln Gly Val Lys Glu Lys Val Leu Ala Leu
        275                 280                 285

Trp Ala Asp Asp Glu Ile Arg Leu Tyr Leu Asp Ala Val Glu Ala Met
    290                 295                 300

Ile Ala Gly Asn Gln Arg Trp Ala Leu Glu Ala Gly Arg Tyr Ser Gly
305                 310                 315                 320

Leu Glu Ser Leu Ile Val Arg Ala Gly
                325

<210>  37
<211>  548
<212>  DNA
<213>  S. avermitilis

<400>  37
cacggacatg ttctgttcac ccggaagggg gctgccggga tgagcacatg tgctcacgtt      60

cccggccggc cggtggcacc ggggtcggac gcgtgtgccc cgggcggatc ggtgtgcgac     120

ccttgctgag tgcccggggt ttgacctgcg tcaccctcgg ggtacgatcc tcggcccgat     180

tggccaggcc cgtgccccgt atggcagact gtcggagttg ctcggtcgag tgccgatgct     240

gtgcgcctcc cgccgggagg actggaagcg agtcccacag tactcgtcgc ccgatctgtc     300

gaaaggcagc gctggggcgg acgtacggga atcttccggg aagtttcagc ggggtgccga     360

ccaggcgccc ggtgggtgtt gtgcccccgg tcctgcggtt tttttcgggc cgtgtccctc     420

ggtagggaaa tccatatcgg atatctctgt cagcaagagc gcgacacacc cgaccgcgtg     480

ggtcggaggt caggacgcga acccccgggc tccagagcgt tttatcgaga cgaaggacta 540

ctgagtac 548

<210> 38
<211> 1059
<212> DNA
<213> Streptomyces sp.

<400> 38
atgaccaccg acaccggcca ggacgccgtc ggcctcgccc gccgcaccgg cgccgatctg 60

ctgcaccgcg tggaggaccg gctgcgctcc ctcctcgcgg tcgagcgcga cgcctgggcc 120

gcggtgcacg aacaggcggt cgtccccgtc gacgcgctgt ccgagctgat cgcgtcgggc 180

ggcaagcgca tccggccggc cttctgcatc accggctatc tcgcggccgg cggcgatccc 240

gccgagcccg ggatcgtggc ggccggcgcg gctctggaga tgctccacct gtcggccctc 300

gtccacgacg acgtcctcga cgactccagc agccggcgcg gagtgccgac cgtgcacaca 360

caggccatgg cgctgcacga gagcagcgga tggcagggcg agccgcgcag gtacggcgag 420

ggcgtggcga tcctcgtcgg cgacctcgcg ctcgtctact ccgaggaact gatggccgag 480

gcgccgcggc gggtgctccc ggagtggaac aagctgcgct ccgaagtgat gatcggccag 540

tacatggacg tccacgcggc cgccgagttc tcggtcgacc cccgaagctc ccgactgatc 600

gccaggatca agtccggccg ctacaccatc caccggcccc tcgtggtggg cgcgaaacgc 660

gggcggggcc gaggcgacct cgcccccgcc ctggaggagt acggcgaggc ggtcggcgag 720

gcgttccagc tccgcgacga cctgctggac gcttcggcga ctcccgcgga gaccggcaag 780

cccaccggcc tcgacttcac ccagcacaag atgacgctgc tgctgggctg ggcgatgcag 840

cgcgacgagc acatccacac cctcgtcacg gagccgggcc acacccccga cgaggtgcgc 900

cgccgcctcc tggacacggg cgttcccgcc gacgtcgaac ggcacatcgc gggcctcgtc 960

gagcggggct gcaaggccat cgccgacgcg cccgtcgacc aggtgtggcg cggcgaactg 1020

gcggccatgg ccggacgggt cgcctaccgc accgcctga 1059

<210> 39
<211> 352
<212> PRT
<213> Streptomyces sp.

<400> 39

Met Thr Thr Asp Thr Gly Gln Asp Ala Val Gly Leu Ala Arg Arg Thr
1               5                   10                  15

Gly Ala Asp Leu Leu His Arg Val Glu Asp Arg Leu Arg Ser Leu Leu
            20                  25                  30

Ala Val Glu Arg Asp Ala Trp Ala Ala Val His Glu Gln Ala Val Val
        35                  40                  45

Pro Val Asp Ala Leu Ser Glu Leu Ile Ala Ser Gly Gly Lys Arg Ile
    50                  55                  60

Arg Pro Ala Phe Cys Ile Thr Gly Tyr Leu Ala Ala Gly Gly Asp Pro
65                  70                  75                  80

Ala Glu Pro Gly Ile Val Ala Ala Gly Ala Ala Leu Glu Met Leu His
                85                  90                  95

Leu Ser Ala Leu Val His Asp Asp Val Leu Asp Asp Ser Ser Ser Arg
            100                 105                 110

Arg Gly Val Pro Thr Val His Thr Gln Ala Met Ala Leu His Glu Ser
        115                 120                 125

Ser Gly Trp Gln Gly Glu Pro Arg Arg Tyr Gly Glu Gly Val Ala Ile
    130                 135                 140

Leu Val Gly Asp Leu Ala Leu Val Tyr Ser Glu Glu Leu Met Ala Glu
145                 150                 155                 160

Ala Pro Arg Arg Val Leu Pro Glu Trp Asn Lys Leu Arg Ser Glu Val
                165                 170                 175

Met Ile Gly Gln Tyr Met Asp Val His Ala Ala Ala Glu Phe Ser Val
            180                 185                 190

Asp Pro Arg Ser Ser Arg Leu Ile Ala Arg Ile Lys Ser Gly Arg Tyr
        195                 200                 205

Thr Ile His Arg Pro Leu Val Val Gly Ala Lys Arg Gly Arg Gly Arg
    210                 215                 220

Gly Asp Leu Ala Pro Ala Leu Glu Glu Tyr Gly Glu Ala Val Gly Glu
225                 230                 235                 240

Ala Phe Gln Leu Arg Asp Asp Leu Leu Asp Ala Ser Ala Thr Pro Ala
                245                 250                 255

Glu Thr Gly Lys Pro Thr Gly Leu Asp Phe Thr Gln His Lys Met Thr
            260                 265                 270

Leu Leu Leu Gly Trp Ala Met Gln Arg Asp Glu His Ile His Thr Leu
        275                 280                 285

Val Thr Glu Pro Gly His Thr Pro Asp Glu Val Arg Arg Arg Leu Leu
    290                 295                 300

Asp Thr Gly Val Pro Ala Asp Val Glu Arg His Ile Ala Gly Leu Val
305                 310                 315                 320

Glu Arg Gly Cys Lys Ala Ile Ala Asp Ala Pro Val Asp Gln Val Trp
                325                 330                 335

Arg Gly Glu Leu Ala Ala Met Ala Gly Arg Val Ala Tyr Arg Thr Ala
            340                 345                 350

<210>  40
<211>  1014
<212>  DNA
<213>  S. avermitilis MA-4680

<400>  40
atgaccgtga ccccggaaag tctgcaccgc agccgcgacg cgatcacacc ggcgttgcgc      60

accgcgctcc acctgctcga cgaggagacc cgccatcagg cggcgtacca cctcggctgg     120

atcgaggccg acggcacccc ggcggacggc ggcggaggca aggccgtacg gcccgcgctc     180

gcgctgctgt cggcgcaggc ggccggcgca ccggcggaaa ccggcctgcc cggtgcggtg     240

gccgtcgaac tggtgcacaa cttctcgttg ctgcacgacg acctgatgga cggcgacgag     300

gagcgtcggc atcgacgtac ggtctggacg ctgtggggcg cctcgggcgc gatcctcacc     360

ggtgacgcgc tgctggcgct cgcgcaggag gtgctgctcg actgcgggct gcccacgggg     420

gcggccgcgg cgcggctgct cgcccgcacc acccgcgacc tcatccgcgg gcaggtgcag     480

gacgtggcgt tcgagcggcg ggcccatgtg ccgatcgagg agtgcgtcga catggcggcc     540

ggcaagaccg gcgcgctgct gtcggccagc gccgcgatcg gtgccgtact ggccggcgcg     600

ccggtgacca ccgtcgacgc cctggcgctc tacgggcagc aggtgggcct cgccttccag     660

ctgaccgacg acgtcctcgg catctggggc gacccggctg tcaccggcaa gtcggtccac     720

tccgacctgc gggcccgcaa gaagtcgctg ccggtcacgt acgcgctcaa ccagggcggg     780

accgtgagtg tcgagctggc cacctggctc tccgcaccag gcgagcccga cgaggcagag     840

ctgcgccggg cggccgacct ggtggcggcg gccggcggcc gcgactgggc caccgccgag     900

gcggcccggc gcatggcgct cggggaggcc gcgctgcgcg gcgtcgacct ggacgccgcg     960

gcgcgcgacg agctcatcgc cctcggacac ttcgtcgtcg accgggaggt gtga          1014

<210>  41
<211>  337
<212>  PRT
<213>  S. avermitilis MA-4680

<400>  41

Met Thr Val Thr Pro Glu Ser Leu His Arg Ser Arg Asp Ala Ile Thr
1               5                   10                  15

Pro Ala Leu Arg Thr Ala Leu His Leu Leu Asp Glu Glu Thr Arg His
            20                  25                  30

Gln Ala Ala Tyr His Leu Gly Trp Ile Glu Ala Asp Gly Thr Pro Ala
        35                  40                  45

Asp Gly Gly Gly Gly Lys Ala Val Arg Pro Ala Leu Ala Leu Leu Ser
    50                  55                  60

Ala Gln Ala Ala Gly Ala Pro Ala Glu Thr Gly Leu Pro Gly Ala Val
65                  70                  75                  80

Ala Val Glu Leu Val His Asn Phe Ser Leu Leu His Asp Asp Leu Met
                85                  90                  95

Asp Gly Asp Glu Glu Arg Arg His Arg Arg Thr Val Trp Thr Leu Trp
            100                 105                 110

Gly Ala Ser Gly Ala Ile Leu Thr Gly Asp Ala Leu Leu Ala Leu Ala
        115                 120                 125

Gln Glu Val Leu Leu Asp Cys Gly Leu Pro Thr Gly Ala Ala Ala Ala
    130                 135                 140

Arg Leu Leu Ala Arg Thr Thr Arg Asp Leu Ile Arg Gly Gln Val Gln
145                 150                 155                 160

Asp Val Ala Phe Glu Arg Arg Ala His Val Pro Ile Glu Glu Cys Val
                165                 170                 175

Asp Met Ala Ala Gly Lys Thr Gly Ala Leu Leu Ser Ala Ser Ala Ala
            180                 185                 190

Ile Gly Ala Val Leu Ala Gly Ala Pro Val Thr Thr Val Asp Ala Leu
        195                 200                 205

Ala Leu Tyr Gly Gln Gln Val Gly Leu Ala Phe Gln Leu Thr Asp Asp
    210                 215                 220

Val Leu Gly Ile Trp Gly Asp Pro Ala Val Thr Gly Lys Ser Val His
225                 230                 235                 240

Ser Asp Leu Arg Ala Arg Lys Lys Ser Leu Pro Val Thr Tyr Ala Leu

245                 250                 255

Asn Gln Gly Gly Thr Val Ser Val Glu Leu Ala Thr Trp Leu Ser Ala
            260                 265                 270

Pro Gly Glu Pro Asp Glu Ala Glu Leu Arg Arg Ala Ala Asp Leu Val
        275                 280                 285

Ala Ala Ala Gly Gly Arg Asp Trp Ala Thr Ala Glu Ala Ala Arg Arg
    290                 295                 300

Met Ala Leu Gly Glu Ala Ala Leu Arg Gly Val Asp Leu Asp Ala Ala
305                 310                 315                 320

Ala Arg Asp Glu Leu Ile Ala Leu Gly His Phe Val Val Asp Arg Glu
                325                 330                 335

Val

<210>  42
<211>  1086
<212>  DNA
<213>  Streptomyces sp.

<400>  42
atggccggta ccgcatggga caccgatatc ccgaccctta tatccgttcg tgacgatgta      60

gacgcggtgc tcgaggactt cgtcgacgtg caggagcggg cagcgcacgg cccggaaatg     120

gcaccgctgt tcaccacggc ccgccggctc ctcttctcca acggcaagcg actgcgcccc     180

ttgctgtgcg tggcgggatg gcaggccgca ggcgcaccgg gagaccccca cgcggtcctt     240

cgcgtcgccg ccgccctgga gctgttccag accttcgccc tgatccacga cgacgtcatg     300

gacgacagtg acacccgccg cggacggccc tcggcccacc gcgcgctggc cgccgagtac     360

atgagcggcg gtgggcggct cagcaaggcg gaggcgcacg gccgaggtgc cgccatcctc     420

ctcggtgacc tcctgctcgt gtggtcggac gaaatgctgg cgggcgcggg gctcgaagcc     480

cgggcgcggg ccagggtcct gccactggtg gacagcatgc gcgccgagct ggtctacggg     540

cagtacctgg atctgctgtc gaccgaccgg atgtccgggg acgtcgaggc cgcgctgcgg     600

gtcgcccggt acaagacggg gaagtacacc gtggaacggc ccctgcacct cggcgtttcc     660

ctggccggcg gcgatccgcg gctgctgcag acctgcaccg atttcgccct cccgctggga     720

gaagcgtttc agctgcgcga tgacctgctc aacgtcttcg gtgacccggt gcggaccggc     780

aagcctgtcg gagacgacat ccgtgagggc aaagccaccg tgctcctggc tctcgcgatc     840

caggacgcct ccgaggccga cctgaagctg ctcggcaccc tggtcggcag cccggaactg     900

gggagcgagg acatcgaccg gttccgcacc gtcatggaga ccaccggtgc ccggcagcgg 960

gtcgaagaca tgatcatcgc caggcgcgac accgccctcg ccacgctgga agaagcagac 1020

ctgccccagg acgtcgccga gacgttgagg cagatcgtat ggatggccac ggaaaggcag 1080

tcatga 1086

<210> 43
<211> 361
<212> PRT
<213> Streptomyces sp.

<400> 43

Met Ala Gly Thr Ala Trp Asp Thr Asp Ile Pro Thr Leu Ile Ser Val
1               5                   10                  15

Arg Asp Asp Val Asp Ala Val Leu Glu Asp Phe Val Asp Val Gln Glu
            20                  25                  30

Arg Ala Ala His Gly Pro Glu Met Ala Pro Leu Phe Thr Thr Ala Arg
        35                  40                  45

Arg Leu Leu Phe Ser Asn Gly Lys Arg Leu Arg Pro Leu Leu Cys Val
    50                  55                  60

Ala Gly Trp Gln Ala Ala Gly Ala Pro Gly Asp Pro His Ala Val Leu
65                  70                  75                  80

Arg Val Ala Ala Ala Leu Glu Leu Phe Gln Thr Phe Ala Leu Ile His
                85                  90                  95

Asp Asp Val Met Asp Asp Ser Asp Thr Arg Arg Gly Arg Pro Ser Ala
            100                 105                 110

His Arg Ala Leu Ala Ala Glu Tyr Met Ser Gly Gly Gly Arg Leu Ser
        115                 120                 125

Lys Ala Glu Ala His Gly Arg Gly Ala Ala Ile Leu Leu Gly Asp Leu
    130                 135                 140

Leu Leu Val Trp Ser Asp Glu Met Leu Ala Gly Ala Gly Leu Glu Ala
145                 150                 155                 160

Arg Ala Arg Ala Arg Val Leu Pro Leu Val Asp Ser Met Arg Ala Glu
                165                 170                 175

Leu Val Tyr Gly Gln Tyr Leu Asp Leu Leu Ser Thr Asp Arg Met Ser
            180                 185                 190

Gly Asp Val Glu Ala Ala Leu Arg Val Ala Arg Tyr Lys Thr Gly Lys
        195                 200                 205

Tyr Thr Val Glu Arg Pro Leu His Leu Gly Val Ser Leu Ala Gly Gly
    210                 215                 220

Asp Pro Arg Leu Leu Gln Thr Cys Thr Asp Phe Ala Leu Pro Leu Gly
225                 230                 235                 240

Glu Ala Phe Gln Leu Arg Asp Asp Leu Leu Asn Val Phe Gly Asp Pro
                245                 250                 255

Val Arg Thr Gly Lys Pro Val Gly Asp Asp Ile Arg Glu Gly Lys Ala
            260                 265                 270

Thr Val Leu Leu Ala Leu Ala Ile Gln Asp Ala Ser Glu Ala Asp Leu
        275                 280                 285

Lys Leu Leu Gly Thr Leu Val Gly Ser Pro Glu Leu Gly Ser Glu Asp
    290                 295                 300

Ile Asp Arg Phe Arg Thr Val Met Glu Thr Thr Gly Ala Arg Gln Arg
305                 310                 315                 320

Val Glu Asp Met Ile Ile Ala Arg Arg Asp Thr Ala Leu Ala Thr Leu
                325                 330                 335

Glu Glu Ala Asp Leu Pro Gln Asp Val Ala Glu Thr Leu Arg Gln Ile
            340                 345                 350

Val Trp Met Ala Thr Glu Arg Gln Ser
        355                 360

<210>  44
<211>  29
<212>  DNA
<213>  Artificial sequence

<220>
<223>  primer

<400>  44
ttttctagaa tgcccgccgg ccacgaaga                                        29

<210>  45
<211>  29
<212>  DNA
<213>  Artificial sequence

<220>

<223>  primer

<400>  45
tttaagcttt caccaagggg tggtggccc                                          29


<210>  46
<211>  29
<212>  DNA
<213>  Artificial sequence

<220>
<223>  primer

<400>  46
ttttctagaa tgccgttccc cgcccgccc                                          29


<210>  47
<211>  29
<212>  DNA
<213>  Artificial sequence

<220>
<223>  primer

<400>  47
tttaagcttt cagtcggcac cggtgcgtg                                          29


<210>  48
<211>  29
<212>  DNA
<213>  Artificial sequence

<220>
<223>  primer

<400>  48
ttttctagaa tgggtggggc gtacaggac                                          29


<210>  49
<211>  29
<212>  DNA
<213>  Artificial sequence

<220>
<223>  primer

<400>  49
tttaagcttt catcgtgtgt gtccaccgt                                          29


<210>  50
<211>  29
<212>  DNA
<213>  Artificial sequence

<220>
<223>  primer

<400>  50

ttttctagag tggcgcacac ccgtgggca                                          29


<210>  51
<211>  29
<212>  DNA
<213>  Artificial sequence

<220>
<223>  primer

<400>  51
tttaagcttt cagcgggccc ggggagcct                                          29


<210>  52
<211>  29
<212>  DNA
<213>  Artificial sequence

<220>
<223>  primer

<400>  52
ttttctagaa tgacagtgcg tgccgtcga                                          29


<210>  53
<211>  29
<212>  DNA
<213>  Artificial sequence

<220>
<223>  primer

<400>  53
tttaagcttt catgcgcttc ctgcggagg                                          29


<210>  54
<211>  29
<212>  DNA
<213>  Artificial sequence

<220>
<223>  primer

<400>  54
ttttctagag tgggacccca tttgaccgt                                          29


<210>  55
<211>  29
<212>  DNA
<213>  Artificial sequence

<220>
<223>  primer

<400>  55
tttaagcttt cagcggcccg cgacggcgg                                          29

<210>  56
<211>  29
<212>  DNA
<213>  Artificial sequence

<220>
<223>  primer

<400>  56
ttttctagaa tgaccgatct ccccgacgg                                          29


<210>  57
<211>  29
<212>  DNA
<213>  Artificial sequence

<220>
<223>  primer

<400>  57
tttaagcttt cagggcctcg ccgtggggg                                          29


<210>  58
<211>  1017
<212>  DNA
<213>  Artificial sequence

<220>
<223>  Synthetic Construct

<400>  58
atgaacgtca ttgacttccc ccaggtcgac ttgggcaccg aggcgacaat cagccccgac        60

gtcgacaaag caggggagca cctgaagaca tggtcccgtg ccacaggcat cgtcctcgac       120

ggccccaacg gcgacgcctt cgacgcactc gcgcagcacc gcctggcggc atggacctac       180

ccggcggcaa ccggcgcgga gcttgacctg atggcggact ggatcggctg gctcttcgcc       240

ttcgacgacg tcttcgacga gtcgatcgac ggctgcgacc aggacttcgc cattgcgacg       300

gcggcggcca ccaacaccgt ctacaccggg ctgccctcag cacccccgag cccagtagtc       360

aggccctacg tggtggcgtt ggaggacctt tgggagcgga ccacccaggg catgccggcg       420

tactggtgca ggcgactggc caacgacatg gtcgactacg tcaactccta ccgaagccac       480

gccctgatca acgcatcacg cattgcgctc gacgagcagt cataccgagc ccaccggctg       540

atctccagcg cagtattcat cacgctcgac ctcggcgagg ccgccgccag gcgtgccctg       600

tcggagtcgc tgttggtgca cccatacatc cgggccgccc gcgaggccgc caacaacatc       660

gtgtcctgga gcaacgacct ctactccgcg cccaaagagc tcagcctggg cgacctgtgc       720

aactacatcg ccgtcctgca gtcccaggac gacctcacga ccgcgcaggc agccgaccgg       780

gtcagtcagt acctgcacga ggagatcgcg cgcttcgacc agaccaaaca gctcatccac       840

acccacctca tcccacgcct gccaaccgag gaccagcagg gcgtgatctc catgctggac       900

acctgcagca actggatcac cggcaacgtg gcgtggtcat tggagaccgc acgctacgca 960

tcgcgcgcca tcgacatgga gcgcgagaag gaggtgttcg gggacttgcg aacctag 1017

<210> 59
<211> 975
<212> DNA
<213> Artificial sequence

<220>
<223> Synthetic Construct

<400> 59
atgtctactg agaacgggca tcgggacggt ctggggcccc tgcgctgtcc cttccccgcg 60

gcgataaacc cgcacgccga cgaggtgcac cgggagaccg tggagtgggc cgagggtttc 120

gggctgctcg ggcccggcga cgggcaccgg atgatgcggg acaccgggat cgggcggctc 180

gccgggcgct tccaccccgg ggccgggcgg gaggagctcc ggctgatctc cgactggtgc 240

gcctggatgt tcctgcgcga cgacctcgcc gacgcgccgg cgtacttccg gcacccggag 300

cggctcgcgg ccctggacgc ggcctttctg gacatcctct ccggcagggc ctccggggag 360

ggctgcgggt ccttcgggcg ggcgctgcgg gatctccggg agcggctgct gcccaaggtt 420

cccgcgccgc tctggctgcg gcgctttctc aggtccgtgg aggagcactt cgagtccacg 480

ctgtgggagg ccaccaaccg ggcccgcggg gtcgtcccgg acctcgagac ctacctcagg 540

atgcgcccca taaccggcgg gatgcacgtg gacacggact tcatagagat atcctccggc 600

gtctacctgc cgcccgaggt gcgccggcac ccggccgtaa gcgccctcac cggggcctcc 660

aacaacgtgg tctgctgggc aaacgacatc atctcgctcg ccaaggagcg ctcgcgcggc 720

gacgtgcaca acctggtcct ggtcctccgc gcgagccggc gtctcaccac ccgggaggcg 780

gtggcggagg ccgcccggat gtacgaggcg gaggtgcgga ggttcgtccg gctggagcgg 840

gagctgcccc ccttcgggcc ggccatcgac gccaacctcc ggcgctacgt ctcggtgctc 900

aagagcagga tgcggggcaa tctggactgg acctacgagt cggcgcgcta ccgggccggc 960

gcggcctccc gctag 975

<210> 60
<211> 1017
<212> DNA
<213> Artificial sequence

<220>
<223> Synthetic Construct

<400> 60
atgccaaccc gtaccacatt gcaccacttg gccacgctta tgcctcgtga ccttccggtg 60

ctctactgcc cattcccggc ggcgattcac cccgacgcgg tcgcgattca ggcctcaacc 120

caggcatggg ccgacagctt cggcttgctc gacgacttgg agacgcggct ctccttcaac 180

cagcgtggct acggcacctt gatggcgcga gcctacccta acgccttgac cgagcgcatg 240

ccaaccattg ccgactggag caccagcctc ttcctcttgg acgaccagtt ggaccgcttc 300

ggccgtgagc acgacgccgg ggccatggca accttgttgc agcgcttgat gaaggtactc 360

aaaggctcgc ctgccaagcg ccacgacttg ccaattgtgc aggcaatggg cgacgtaacc 420

agccgacttg tgcagggctc gcccgaggcc tggcagcact acttcttcga ctcattgcgc 480

aaatcgttcg cagcagtgca gtgggaggcc aacaaccgtg cccagggcta cattccaacc 540

cagaaaacct acgcaatgca gcgtttgtac accagtggca cctacttgtg cttggccttg 600

attgagttcg gcaacaacga cttcctgcac ccccaggtgc gtttgcaccc catggtgcgg 660

cgtttgtgcg agtacgtcaa ccgggtggtg ttctactcaa acgacattat ttcgttgcgt 720

aaagagttgt tggccggcga ctggcagaac ttggtgtacc tcgtgcacac gcagcaccag 780

ttgccaatcg gcgaggcatt gaactacgtc gcccgtttgc acaacgacac aatcaaccag 840

ttcacccgcg agcgcattat gctcgacaaa ttggcccctg agatttgctc accgagcttg 900

taccgcttcg tcgacggctt ggccacctgg atgcgagcaa accacgactg gtccaccatg 960

acctcccgtt accagacatc caccgaggag tcccacggat cccagtactg cgcgtga 1017

<210> 61
<211> 990
<212> DNA
<213> Artificial sequence

<220>
<223> Synthetic Construct

<400> 61
atgccaaaca cagcacacct gcagcagttg gcgaggacgg tcccagcaac cattcccaag 60

ttggagtcac cgttccctgc gcagatccac ccattggcag cggaggtcca gcagcagacg 120

accaaatggg tgcttgagca gggcttgttg agcgaggcgc ggcgtaaagc cttccttgac 180

tacaactaca ccttgttgat tgcccgttgc tacccgtacg cgagtgtcga gcggctgcag 240

attatcagcg actggaacgc ctggctgttc gagcttgacg accagtgcga cgaggcgcga 300

attggccacg accctcaggc gttggcccgc ttgcaccagc ggcttggggc agtcattcgc 360

ggagccacgg tcgccgccga cgagccactg ttggtcacag ccctcgccga cattatgggc 420

cggatgcggg cgctggcacc actggcatgg ttcgaggtct tcacccagca ggtcgacgac 480

tacttcattg gcaaccagtg ggaggcccac aaccggctgt actcgattat tcccagcgag 540

attgagtact tgcactaccg taaattcacc agtgccgtcg cgatctacat tgcgttcatt 600

gagttcatga acgcaagtta cttgaccgcc gacgagcgtc agtcaatcga cttgcgcaac 660

ctgcagaact ggacgaacct tgtactgagt ttgtgcaacg accttgtctc agtcaaaaaa 720

gagttgcgtc agggcgactg gcacaactac ggagcaattg tcgcattgaa acgaggcatg 780

agtattcctg aggccttgga gatcgtcgcc aacgagcaca accactgcat tcagcagttc 840

atcacaacca ccacccagct gaaaaactgc agcgtggcac ttcagaaagt attgaacggc 900

atgatgacat tgatgcgcgg aaactacgac tggtcggaca ttaccagccg ttacgccacc 960

aaacttaccg agttgtcgta cgccgcatga 990

<210> 62
<211> 981
<212> DNA
<213> Artificial sequence

<220>
<223> Synthetic Construct

<400> 62
atggaccagg actaccgcgc acgacttgtc tacccattct ccggagcgat cagcccgcac 60

gccgacatcg tcgaccaggc aacgctcgcc tgggcggcga tgttcggctt gttgacggac 120

tcattgcgcc acaaaagtcg tcgcctccag tacggcttgc tggccgcacg cgcctaccca 180

cgagcggacc gtgagatgtt gcagattgcc gccgactgga ttgcatggct gttcttcatg 240

gacgaccagt gcgacgagac cggcattggg cgtgacctcc agcgcatgat tgcgctgcac 300

gagcgattcc ttgcaattct tgacggcgca acgccggagg cgcacgactg cgcgctgacc 360

tacgcgctcg ccgaccttcg tcgtcgactg gcactgcgcg cccctgacaa ctggctgcgc 420

cgattcagcg agcacgtgcg tctctacttc accgccaacc gctgggagac ggtcaaccgg 480

cagcgcggcg caacgccgaa cgtggcgaca tactgcgccg cgcgcctgtt ctcgggcgcc 540

gtctacgcat gcttcgacct gatcgagctg gcagagcaga tcgagctgcc gttctacgcc 600

cgtcaccaca gcattgtgca gcagctggag caggccgcga acaacatcat ttgctggtgc 660

aacgacgtgc tgtcgtaccc caaggagatg cagcacggcg accggcacaa cctggtgctg 720

gtcattcagg gcgagcacca gtgctcattg cccgaggcca ttgaccgcgc gctcgacctt 780

cacgcgcgtg aggtcgccac gttcgtgcgg aaacgcacct gcgtcccata cttcgacgca 840

gcggtcaaca cagcgcttga gaaatacgtc accggattgc agttctggat ctgcgccaac 900

cgcgactggt cgctgacggc aacgcgctac gcgccgacgc acaaatcgca ggagatggtg 960

atggcagtcg cgcagcagta g 981

<210> 63
<211> 981
<212> DNA
<213> Artificial sequence

<220>
<223> Synthetic Construct

<400>  63
atggaccggg tcgacggcgc acagatcgtc tacccgttca ccggcgcgat cagcccctac      60

gccggcgacg tcgaccaggc aacacttatc tgggcagagg cgtccggact tcttaccgac     120

gggttgcgcc agaaaagtca gcgtctccag tacggagtac tggccgcgcg cgcctaccca     180

caggcagacc gtgacacgct gcagattgcc gccgactgga tcgcctggct gttcttcatg     240

gacgaccagt gcgacgaggc cggcattggc cgcgacccgc agcgcctggc cgcgctgcac     300

gagcgattcc tcgcagtgct cgagggagac ccacctgaca gtggcgactg gaacctgacc     360

cgcgccctcg ccgacattcg ccgtcggctg gccgcgcgcg caaccgacga ctggttgcgc     420

cgattcggag agcacgtgcg tctctacttc acggcgaacc gctgggaggc ggcaaaccga     480

tgccagagca tcgtcccaaa cgtggcgacc tactgcgccg cgcgcctgtt ctcaggcgca     540

gtctacgcct gcttcgacct gatcgagctg gcagcaggga tcgaccttcc cttctacgca     600

cgttaccacg ccgccgtcca gcagctcgag cggacggcaa acaacatcat ttgctggtgc     660

aacgacatgt tgtcgtaccc caaagagatg cagcacggcg acgtgcacaa cctggtactg     720

gccatccggc aggagtacca gtgctcgttg agtgaggcgc tgcagcaggc gcttctgctc     780

cacgaccggg agacggacac gttcatgaaa acacagaagc agctgccgcg cttcaacccc     840

gccgtcgaca tggcgcttga gcgctacatt gacggcttgc agtactggat ctgcgccaac     900

cgtgactggt cattgacagc aatgcgctac gccttgtccg gaaccgagcc cgtacttcgg     960

acgaggttcg cacactcgtg a                                               981


<210>  64
<211>  948
<212>  DNA
<213>  Artificial sequence

<220>
<223>  Synthetic Construct

<400>  64
atgcacagag ccctcctgtg tcctttcccc gccacgacgc cccacccgca ggcggcacag      60

ctggcgaacg attgcctcga atggactcgc aagtgcggcc tgctccccga tgaatcgcca     120

cgaacgctcg acaaggtgcg cagctactcg gcgctcgccg cgcactgtta ccccgatgcg     180

catttcgagc ggcttcgggc gatctgcgac tactacagct ggctcttctt cttcgatgac     240

gtgtgcgaga atacgtcact gaacggcgcc gaaccgaagg ttgtctcgag cctgctgttc     300

gatgtatacg gggtgctgcg agggcccacg gctgccgttg ggcatgcgcc attcgcccag     360

gcgttggcgg acatctggcg tcgcatcggc gatggttgtc ctgggttctg gcggcggcgc     420

ctcatccgcc acgtcgaaaa ctacatcgat ggctgtgtct gggaagcgca aaaccgccag     480

ctcgaccgcg tacccagtcg cgcggtgttc gagggtatgc gcatgcacac gtccaccatg     540

tacgagttct gggacttcat cgagtatgcc ggcgacctgt tcttgccgga tgaggtcgta 600

gaacatccct tggtggcgga ggtgcgtcgc gccggcaacg ccatcgcgtc tttcgccaat 660

gacatttact cgctgcgcaa agagacgagc aaccgcgacg tgcacaattt ggttgtcgtg 720

ctcatgcacg aagagcgcat cgagctcgaa gcagcgtacg cgcgcgccgc tggtatccac 780

gatgctcaag tcgagcactt cctggatctg gtgaagcacc tgcccacgtt ctctgcgacc 840

atcgaccgga acctggcgcg ttacgtggag ggcatacgca tctggatccg cgccaaccac 900

gattggtcga ttgtcacgcc ccgttacaat gagcccgatg cgcgatga 948

<210> 65
<211> 990
<212> DNA
<213> Artificial sequence

<220>
<223> Synthetic Construct

<400> 65
atgtcatccg accgaaccag cgtcgtcgtc agcaagcgcg acgccggcgg attcgagtac 60

ccgttcgccg cgtcgtgcca ccccgggagg gaggtcacgg agcagaggac cctggcgtgg 120

gtccgccggt tgcgtctggt gccagacggc cggtcgcttt cacgtctcaa ggcgacgaac 180

ttctcccacc tggcggcatg gctcttgccg tccgcgagca cgcagacgct ccagctggca 240

tcggacttca cggccgtgct gttcctcctg gacgacgcgt acgacgaggg gcagctcagc 300

acggacccgg agtccgtcga gtggctcaac gagaagtacc tgggcgagct cttcgggtac 360

accgaggcgg acatgagcga ccccctgacg cgcgggatgc tcgacgtacg tgagcgcatc 420

cgacgcagcc acccgcactt cttcctgaac cgctggctct cgcacttcca gtactactac 480

gaggccaacc tgtgggaggc gaacaaccga aagcagatga gggtgccgca ccttgaggag 540

tacttgatga tgcggcgcta cagcggcgcc gtctacacct actgcgacct gctcgagctg 600

ctgctcgagc ggccgctgcc gctcgaggtc gtccagcacc cgttgattca gacggtccga 660

gacatctgca acgacatcct ctgctggacg aacgactact tctcgctcgg gaaggagctg 720

accaacggcg agacgcacaa cctgatcgtc gtcctccgga acgagtgcgt gagcacgctg 780

gaggaggcga tcgaccggct caaggacatg cacgaccgcc gggtcgcgga gtaccagggc 840

gtgaaggaga aggtgctcgc gctctgggcg gacgacgaga tccgcctcta cctggacgcg 900

gtcgaggcca tgatcgccgg gaaccagcgc tgggcgctcg aggccggccg ctactccgga 960

ctggagagtt tgatcgtgcg cgccggatga 990

<210> 66
<211> 36
<212> DNA
<213> Artificial sequence

<220>
<223>  primer

<400>  66
ctcgagcaat tggacataag cctgttcggt tcgtaa                                36


<210>  67
<211>  36
<212>  DNA
<213>  Artificial sequence

<220>
<223>  primer

<400>  67
ctcgaggcta gcgatctcgg cttgaacgaa ttgtta                                36


<210>  68
<211>  36
<212>  DNA
<213>  Artificial sequence

<220>
<223>  primer

<400>  68
ctcgagcaat tggacataag cctgttcggt tcgtaa                                36


<210>  69
<211>  36
<212>  DNA
<213>  Artificial sequence

<220>
<223>  primer

<400>  69
ctcgaggcta gcgatctcgg cttgaacgaa ttgtta                                36


<210>  70
<211>  35
<212>  DNA
<213>  Artificial sequence

<220>
<223>  primer

<400>  70
ctcgagctca tatgaccacc gacaccggcc aggac                                 35


<210>  71
<211>  32
<212>  DNA
<213>  Artificial sequence

<220>
<223>  primer

<400>  71
agtctagatc aggcggtgcg gtaggcgacc cg                                    32


<210>  72
<211>  32
<212>  DNA
<213>  Artificial sequence

<220>
<223>  primer

<400>  72
ctcgagctca tatgaccgtg accccggaaa gt                                    32


<210>  73
<211>  32
<212>  DNA
<213>  Artificial sequence

<220>
<223>  primer

<400>  73
agtctagatc acacctcccg gtcgacgacg aa                                    32


<210>  74
<211>  35
<212>  DNA
<213>  Artificial sequence

<220>
<223>  primer

<400>  74
ctcgagctca tatgatggcc ggtaccgcat gggac                                 35


<210>  75
<211>  32
<212>  DNA
<213>  Artificial sequence

<220>
<223>  primer

<400>  75
agtctagatc atgactgcct ttccgtggcc at                                    32


<210>  76
<211>  32
<212>  DNA
<213>  Artificial sequence

<220>
<223>  primer

<400>  76
ctcgagctca tatgaccgtg accccggaaa gt                                    32

<210>  77
<211>  32
<212>  DNA
<213>  Artificial sequence

<220>
<223>  primer

<400>  77
agtctagatc acacctcccg gtcgacgacg aa                                    32


<210>  78
<211>  29
<212>  DNA
<213>  Artificial sequence

<220>
<223>  primer

<400>  78
ttttctagaa tgaccatctc cgttcccca                                        29


<210>  79
<211>  29
<212>  DNA
<213>  Artificial sequence

<220>
<223>  primer

<400>  79
tttaagcttt caggcggcct cgggcacga                                        29


<210>  80
<211>  29
<212>  DNA
<213>  Artificial sequence

<220>
<223>  primer

<400>  80
ttttctagaa tgcgcggagc cggtcccgg                                        29


<210>  81
<211>  29
<212>  DNA
<213>  Artificial sequence

<220>
<223>  primer

<400>  81
tttaagcttc taccaggggg tgaacacgg                                        29


<210>  82

<211>  29
<212>  DNA
<213>  Artificial sequence

<220>
<223>  primer

<400>  82
ttttctagaa tgacaaccgt gcgccgcac                                          29


<210>  83
<211>  29
<212>  DNA
<213>  Artificial sequence

<220>
<223>  primer

<400>  83
tttaagcttt cacttctcca actggtccc                                          29


<210>  84
<211>  999
<212>  DNA
<213>  Artificial sequence

<220>
<223>  Synthetic Construct

<400>  84
tctagaggag gttgcatatg tctactgaga acgggcatcg ggacggtctg gggcccctgc        60

gctgtccctt ccccgcggcg ataaacccgc acgccgacga ggtgcaccgg gagaccgtgg       120

agtgggccga gggtttcggg ctgctcgggc ccggcgacgg gcaccggatg atgcgggaca       180

ccgggatcgg gcggctcgcc gggcgcttcc accccggggc cgggcgggag gagctccggc       240

tgatctccga ctggtgcgcc tggatgttcc tgcgcgacga cctcgccgac gcgccggcgt       300

acttccggca cccggagcgg ctcgcggccc tggacgcggc ctttctggac atcctctccg       360

gcagggcctc cggggagggc tgcgggtcct tcgggcgggc gctgcgggat ctccgggagc       420

ggctgctgcc caaggttccc gcgccgctct ggctgcggcg ctttctcagg tccgtggagg       480

agcacttcga gtccacgctg tgggaggcca ccaaccgggc ccgcggggtc gtcccggacc       540

tcgagaccta cctcaggatg cgccccataa ccggcgggat gcacgtggac acggacttca       600

tagagatatc ctccggcgtc tacctgccgc ccgaggtgcg ccggcacccg gccgtaagcg       660

ccctcaccgg ggcctccaac aacgtggtct gctgggcaaa cgacatcatc tcgctcgcca       720

aggagcgctc gcgcggcgac gtgcacaacc tggtcctggt cctccgcgcg agccggcgtc       780

tcaccacccg ggaggcggtg gcggaggccg cccggatgta cgaggcggag gtgcggaggt       840

tcgtccggct ggagcgggag ctgcccccct tcgggccggc catcgacgcc aacctccggc        900

gctacgtctc ggtgctcaag agcaggatgc ggggcaatct ggactggacc tacgagtcgg       960

cgcgctaccg ggccggcgcg gcctcccgct aggaagctt                              999

<210>  85
<211>  1014
<212>  DNA
<213>  Artificial sequence

<220>
<223>  Synthetic Construct

<400>  85
tctagaggag gttgcatatg ccaaacacag cacacctgca gcagttggcg aggacggtcc       60

cagcaaccat tcccaagttg gagtcaccgt tccctgcgca gatccaccca ttggcagcgg      120

aggtccagca gcagacgacc aaatgggtgc ttgagcaggg cttgttgagc gaggcgcggc      180

gtaaagcctt ccttgactac aactacacct tgttgattgc ccgttgctac ccgtacgcga      240

gtgtcgagcg gctgcagatt atcagcgact ggaacgcctg gctgttcgag cttgacgacc      300

agtgcgacga ggcgcgaatt ggccacgacc ctcaggcgtt ggcccgcttg caccagcggc      360

ttggggcagt cattcgcgga gccacggtcg ccgccgacga gccactgttg gtcacagccc      420

tcgccgacat tatgggccgg atgcgggcgc tggcaccact ggcatggttc gaggtcttca      480

cccagcaggt cgacgactac ttcattggca accagtggga ggcccacaac cggctgtact      540

cgattattcc cagcgagatt gagtacttgc actaccgtaa attcaccagt gccgtcgcga      600

tctacattgc gttcattgag ttcatgaacg caagttactt gaccgccgac gagcgtcagt      660

caatcgactt gcgcaacctg cagaactgga cgaaccttgt actgagtttg tgcaacgacc      720

ttgtctcagt caaaaaagag ttgcgtcagg gcgactggca caactacgga gcaattgtcg      780

cattgaaacg aggcatgagt attcctgagg ccttggagat cgtcgccaac gagcacaacc      840

actgcattca gcagttcatc acaaccacca cccagctgaa aaactgcagc gtggcacttc      900

agaaagtatt gaacggcatg atgacattga tgcgcggaaa ctacgactgg tcggacatta      960

ccagccgtta cgccaccaaa cttaccgagt tgtcgtacgc cgcatgagaa gctt           1014

<210>  86
<211>  1005
<212>  DNA
<213>  Artificial sequence

<220>
<223>  Synthetic Construct

<400>  86
tctagaggag gttgcatatg gaccaggact accgcgcacg acttgtctac ccattctccg       60

gagcgatcag cccgcacgcc gacatcgtcg accaggcaac gctcgcctgg gcggcgatgt      120

tcggcttgtt gacggactca ttgcgccaca aaagtcgtcg cctccagtac ggcttgctgg      180

ccgcacgcgc ctacccacga gcggaccgtg agatgttgca gattgccgcc gactggattg 240

catggctgtt cttcatggac gaccagtgcg acgagaccgg cattgggcgt gacctccagc 300

gcatgattgc gctgcacgag cgattccttg caattcttga cggcgcaacg ccggaggcgc 360

acgactgcgc gctgacctac gcgctcgccg accttcgtcg tcgactggca ctgcgcgccc 420

ctgacaactg gctgcgccga ttcagcgagc acgtgcgtct ctacttcacc gccaaccgct 480

gggagacggt caaccggcag cgcggcgcaa cgccgaacgt ggcgacatac tgcgccgcgc 540

gcctgttctc gggcgccgtc tacgcatgct tcgacctgat cgagctggca gagcagatcg 600

agctgccgtt ctacgcccgt caccacagca ttgtgcagca gctggagcag gccgcgaaca 660

acatcatttg ctggtgcaac gacgtgctgt cgtaccccaa ggagatgcag cacggcgacc 720

ggcacaacct ggtgctggtc attcagggcg agcaccagtg ctcattgccc gaggccattg 780

accgcgcgct cgaccttcac gcgcgtgagg tcgccacgtt cgtgcggaaa cgcacctgcg 840

tcccatactt cgacgcagcg gtcaacacag cgcttgagaa atacgtcacc ggattgcagt 900

tctggatctg cgccaaccgc gactggtcgc tgacggcaac gcgctacgcg ccgacgcaca 960

aatcgcagga gatggtgatg gcagtcgcgc agcagtagga agctt 1005

<210> 87
<211> 1005
<212> DNA
<213> Artificial sequence

<220>
<223> Synthetic Construct

<400> 87
tctagaggag gttgcatatg gaccgggtcg acggcgcaca gatcgtctac ccgttcaccg 60

gcgcgatcag cccctacgcc ggcgacgtcg accaggcaac acttatctgg gcagaggcgt 120

ccggacttct taccgacggg ttgcgccaga aaagtcagcg tctccagtac ggagtactgg 180

ccgcgcgcgc ctacccacag gcagaccgtg acacgctgca gattgccgcc gactggatcg 240

cctggctgtt cttcatggac gaccagtgcg acgaggccgg cattggccgc gacccgcagc 300

gcctggccgc gctgcacgag cgattcctcg cagtgctcga gggagaccca cctgacagtg 360

gcgactggaa cctgacccgc gccctcgccg acattcgccg tcggctggcc gcgcgcgcaa 420

ccgacgactg gttgcgccga ttcggagagc acgtgcgtct ctacttcacg gcgaaccgct 480

gggaggcggc aaaccgatgc cagagcatcg tcccaaacgt ggcgacctac tgcgccgcgc 540

gcctgttctc aggcgcagtc tacgcctgct tcgacctgat cgagctggca gcagggatcg 600

accttccctt ctacgcacgt taccacgccg ccgtccagca gctcgagcgg acggcaaaca 660

acatcatttg ctggtgcaac gacatgttgt cgtaccccaa agagatgcag cacggcgacg 720

tgcacaacct ggtactggcc atccggcagg agtaccagtg ctcgttgagt gaggcgctgc 780

agcaggcgct tctgctccac gaccgggaga cggacacgtt catgaaaaca cagaagcagc 840

tgccgcgctt caaccccgcc gtcgacatgg cgcttgagcg ctacattgac ggcttgcagt 900

actggatctg cgccaaccgt gactggtcat tgacagcaat gcgctacgcc ttgtccggaa 960

ccgagcccgt acttcggacg aggttcgcac actcgtgaga agctt 1005

<210> 88
<211> 972
<212> DNA
<213> Artificial sequence

<220>
<223> Synthetic Construct

<400> 88
tctagaggag gttgcatatg cacagagccc tcctgtgtcc tttccccgcc acgacgcccc 60

acccgcaggc ggcacagctg gcgaacgatt gcctcgaatg gactcgcaag tgcggcctgc 120

tccccgatga atcgccacga acgctcgaca aggtgcgcag ctactcggcg ctcgccgcgc 180

actgttaccc cgatgcgcat ttcgagcggc ttcgggcgat ctgcgactac tacagctggc 240

tcttcttctt cgatgacgtg tgcgagaata cgtcactgaa cggcgccgaa ccgaaggttg 300

tctcgagcct gctgttcgat gtatacgggg tgctgcgagg gcccacggct gccgttgggc 360

atgcgccatt cgcccaggcg ttggcggaca tctggcgtcg catcggcgat ggttgtcctg 420

ggttctggcg gcggcgcctc atccgccacg tcgaaaacta catcgatggc tgtgtctggg 480

aagcgcaaaa ccgccagctc gaccgcgtac ccagtcgcgc ggtgttcgag ggtatgcgca 540

tgcacacgtc caccatgtac gagttctggg acttcatcga gtatgccggc gacctgttct 600

tgccggatga ggtcgtagaa catcccttgg tggcggaggt gcgtcgcgcc ggcaacgcca 660

tcgcgtcttt cgccaatgac atttactcgc tgcgcaaaga gacgagcaac cgcgacgtgc 720

acaatttggt tgtcgtgctc atgcacgaag agcgcatcga gctcgaagca gcgtacgcgc 780

gcgccgctgg tatccacgat gctcaagtcg agcacttcct ggatctggtg aagcacctgc 840

ccacgttctc tgcgaccatc gaccggaacc tggcgcgtta cgtggagggc atacgcatct 900

ggatccgcgc caaccacgat tggtcgattg tcacgccccg ttacaatgag cccgatgcgc 960

gatgagaagc tt 972

<210> 89
<211> 1014
<212> DNA
<213> Artificial sequence

<220>
<223> Synthetic Construct

<400> 89

tctagaggag gttgcatatg tcatccgacc gaaccagcgt cgtcgtcagc aagcgcgacg 60

ccggcggatt cgagtacccg ttcgccgcgt cgtgccaccc cgggagggag gtcacggagc 120

agaggaccct ggcgtgggtc cgccggttgc gtctggtgcc agacggccgg tcgctttcac 180

gtctcaaggc gacgaacttc tcccacctgg cggcatggct cttgccgtcc gcgagcacgc 240

agacgctcca gctggcatcg gacttcacgg ccgtgctgtt cctcctggac gacgcgtacg 300

acgaggggca gctcagcacg gacccggagt ccgtcgagtg gctcaacgag aagtacctgg 360

gcgagctctt cgggtacacc gaggcggaca tgagcgaccc cctgacgcgc gggatgctcg 420

acgtacgtga gcgcatccga cgcagccacc cgcacttctt cctgaaccgc tggctctcgc 480

acttccagta ctactacgag gccaacctgt gggaggcgaa caaccgaaag cagatgaggg 540

tgccgcacct tgaggagtac ttgatgatgc ggcgctacag cggcgccgtc tacacctact 600

gcgacctgct cgagctgctg ctcgagcggc cgctgccgct cgaggtcgtc cagcacccgt 660

tgattcagac ggtccgagac atctgcaacg acatcctctg ctggacgaac gactacttct 720

cgctcgggaa ggagctgacc aacggcgaga cgcacaacct gatcgtcgtc ctccggaacg 780

agtgcgtgag cacgctggag gaggcgatcg accggctcaa ggacatgcac gaccgccggg 840

tcgcggagta ccagggcgtg aaggagaagg tgctcgcgct ctgggcggac gacgagatcc 900

gcctctacct ggacgcggtc gaggccatga tcgccgggaa ccagcgctgg gcgctcgagg 960

ccggccgcta ctccggactg gagagtttga tcgtgcgcgc cggatgagaa gctt 1014

<210> 90
<211> 1041
<212> DNA
<213> Artificial sequence

<220>
<223> Synthetic Construct

<400> 90
tctagaggag gttgcatatg aacgtcattg acttccccca ggtcgacttg ggcaccgagg 60

cgacaatcag ccccgacgtc gacaaagcag gggagcacct gaagacatgg tcccgtgcca 120

caggcatcgt cctcgacggc cccaacggcg acgccttcga cgcactcgcg cagcaccgcc 180

tggcggcatg gacctacccg gcggcaaccg gcgcggagct tgacctgatg gcggactgga 240

tcggctggct cttcgccttc gacgacgtct tcgacgagtc gatcgacggc tgcgaccagg 300

acttcgccat tgcgacggcg gcggccacca acaccgtcta caccgggctg ccctcagcac 360

ccccgagccc agtagtcagg ccctacgtgg tggcgttgga ggacctttgg gagcggacca 420

cccagggcat gccggcgtac tggtgcaggc gactggccaa cgacatggtc gactacgtca 480

actcctaccg aagccacgcc ctgatcaacg catcacgcat tgcgctcgac gagcagtcat 540

accgagccca ccggctgatc tccagcgcag tattcatcac gctcgacctc ggcgaggccg 600

ccgccaggcg tgccctgtcg gagtcgctgt tggtgcaccc atacatccgg gccgcccgcg 660

aggccgccaa caacatcgtg tcctggagca acgacctcta ctccgcgccc aaagagctca 720

gcctgggcga cctgtgcaac tacatcgccg tcctgcagtc ccaggacgac ctcacgaccg 780

cgcaggcagc cgaccgggtc agtcagtacc tgcacgagga gatcgcgcgc ttcgaccaga 840

ccaaacagct catccacacc cacctcatcc cacgcctgcc aaccgaggac cagcagggcg 900

tgatctccat gctggacacc tgcagcaact ggatcaccgg caacgtggcg tggtcattgg 960

agaccgcacg ctacgcatcg cgcgccatcg acatggagcg cgagaaggag gtgttcgggg 1020

acttgcgaac ctaggaagct t 1041

<210> 91
<211> 1041
<212> DNA
<213> Artificial sequence

<220>
<223> Synthetic Construct

<400> 91
tctagaggag gttgcatatg ccaacccgta ccacattgca ccacttggcc acgcttatgc 60

ctcgtgacct tccggtgctc tactgcccat tcccggcggc gattcacccc gacgcggtcg 120

cgattcaggc ctcaacccag gcatgggccg acagcttcgg cttgctcgac gacttggaga 180

cgcggctctc cttcaaccag cgtggctacg gcaccttgat ggcgcgagcc taccctaacg 240

ccttgaccga gcgcatgcca accattgccg actggagcac cagcctcttc ctcttggacg 300

accagttgga ccgcttcggc cgtgagcacg acgccggggc catggcaacc ttgttgcagc 360

gcttgatgaa ggtactcaaa ggctcgcctg ccaagcgcca cgacttgcca attgtgcagg 420

caatgggcga cgtaaccagc cgacttgtgc agggctcgcc cgaggcctgg cagcactact 480

tcttcgactc attgcgcaaa tcgttcgcag cagtgcagtg ggaggccaac aaccgtgccc 540

agggctacat tccaacccag aaaacctacg caatgcagcg tttgtacacc agtggcacct 600

acttgtgctt ggccttgatt gagttcggca acaacgactt cctgcacccc caggtgcgtt 660

tgcaccccat ggtgcggcgt ttgtgcgagt acgtcaaccg ggtggtgttc tactcaaacg 720

acattatttc gttgcgtaaa gagttgttgg ccggcgactg gcagaacttg gtgtacctcg 780

tgcacacgca gcaccagttg ccaatcggcg aggcattgaa ctacgtcgcc cgtttgcaca 840

acgacacaat caaccagttc acccgcgagc gcattatgct cgacaaattg gcccctgaga 900

tttgctcacc gagcttgtac cgcttcgtcg acggcttggc cacctggatg cgagcaaacc 960

acgactggtc caccatgacc tcccgttacc agacatccac cgaggagtcc cacggatccc 1020

agtactgcgc gtgagaagct t 1041

<210>  92
<211>  978
<212>  DNA
<213>  Streptomyces lactacystinaeus

<400>  92
atgacaacca cggcagagat cctctccccg gacccggagg ccccggccct cgacgagctc      60

cccttccccc gccggatcag ccccgacttc cgggccgccc acgaccgcca cctcgcctgg     120

ccgcgctcct tcggcttcct gtccacggaa gcggagcggg cccaccacct caagggccag     180

ttcccgctga tcgcggccat gttctatccg aacgcgaccg ggagcgaact ggacatcggc     240

gtcgaccagc agagctggta cttcctgttc gacgacgcgc tcgacgaaca gtggggcggc     300

tcgcccgagc gggtccgcca cctcgtcgga ctcgtccagg agggtgtcac cggccaggcc     360

tcgccgctgc cgccggccgc cgccttcgcc gacatgcgac gacgcagctg ccacggcatg     420

cccgaggact ggatccggcg ctcggcggcc cactggtcct cctacctcga ccaccacgtc     480

cacgaggcgc gcagccggca gtccggtgcg cccatgccgc tgagcaccta cctgcgggtc     540

cggcgccaca ccatcggggt ggcaccggtc atcgacctcg ccgagcggct gtcctcctgc     600

gtcctgcccg accacctgta cgcgctgccg cacctgtccg tcatgcgcga gatgacgaag     660

accttcatca tctgcgacaa cgacatcgtc tccctggaga aggacgcggc cctgggggag     720

cagaacaacc tggtgctctg cctggaacgg gaccacgggc tctcccggcc gcaggcggtc     780

gaccgggcgc tgcgccgccg cgccgaagcc ctggagctgt tcaccggcgc ccaccgggcc     840

ctgctcggca gcacggcgac gggccacctc gacccggccg aacgcgacct gctgcagcgc     900

tactgcaccg aggcgctgca gaccaccatc cgcggcgcgt acgactggca ccacgcctcc     960

acccggtacc acgggtag                                                   978

<210>  93
<211>  325
<212>  PRT
<213>  Streptomyces lactacystinaeus

<400>  93

Met Thr Thr Thr Ala Glu Ile Leu Ser Pro Asp Pro Glu Ala Pro Ala
1               5                   10                  15

Leu Asp Glu Leu Pro Phe Pro Arg Arg Ile Ser Pro Asp Phe Arg Ala
            20                  25                  30

Ala His Asp Arg His Leu Ala Trp Pro Arg Ser Phe Gly Phe Leu Ser
        35                  40                  45

Thr Glu Ala Glu Arg Ala His His Leu Lys Gly Gln Phe Pro Leu Ile
    50                  55                  60

Ala Ala Met Phe Tyr Pro Asn Ala Thr Gly Ser Glu Leu Asp Ile Gly
65                  70                  75                  80

Val Asp Gln Gln Ser Trp Tyr Phe Leu Phe Asp Asp Ala Leu Asp Glu
                85                  90                  95

Gln Trp Gly Gly Ser Pro Glu Arg Val Arg His Leu Val Gly Leu Val
            100                 105                 110

Gln Glu Gly Val Thr Gly Gln Ala Ser Pro Leu Pro Pro Ala Ala Ala
        115                 120                 125

Phe Ala Asp Met Arg Arg Arg Ser Cys His Gly Met Pro Glu Asp Trp
    130                 135                 140

Ile Arg Arg Ser Ala Ala His Trp Ser Ser Tyr Leu Asp His His Val
145                 150                 155                 160

His Glu Ala Arg Ser Arg Gln Ser Gly Ala Pro Met Pro Leu Ser Thr
                165                 170                 175

Tyr Leu Arg Val Arg Arg His Thr Ile Gly Val Ala Pro Val Ile Asp
            180                 185                 190

Leu Ala Glu Arg Leu Ser Ser Cys Val Leu Pro Asp His Leu Tyr Ala
        195                 200                 205

Leu Pro His Leu Ser Val Met Arg Glu Met Thr Lys Thr Phe Ile Ile
    210                 215                 220

Cys Asp Asn Asp Ile Val Ser Leu Glu Lys Asp Ala Ala Leu Gly Glu
225                 230                 235                 240

Gln Asn Asn Leu Val Leu Cys Leu Glu Arg Asp His Gly Leu Ser Arg
                245                 250                 255

Pro Gln Ala Val Asp Arg Ala Leu Arg Arg Arg Ala Glu Ala Leu Glu
            260                 265                 270

Leu Phe Thr Gly Ala His Arg Ala Leu Leu Gly Ser Thr Ala Thr Gly
        275                 280                 285

His Leu Asp Pro Ala Glu Arg Asp Leu Leu Gln Arg Tyr Cys Thr Glu
    290                 295                 300

Ala Leu Gln Thr Thr Ile Arg Gly Ala Tyr Asp Trp His His Ala Ser

305                 310                 315                 320

Thr Arg Tyr His Gly
                325

<210>  94
<211>  29
<212>  DNA
<213>  Artificial sequence

<220>
<223>  primer

<400>  94
ttttctagaa tgacaaccac ggcagagat                                         29

<210>  95
<211>  29
<212>  DNA
<213>  Artificial sequence

<220>
<223>  primer

<400>  95
tttaagcttc tacccgtggt accgggtgg                                         29